# EUROPEAN PATENT SPECIFICATION

(11) **EP 1 860 199 B1**
(45) Date of publication and mention of the grant of the patent: **05.03.2014**
(21) Application number: 07105501.6
(22) Date of filing: 11.01.2002
(51) Int. Cl.: C12Q 1/68, G01N 33/53

(54) **Modulating insulin receptor signaling**
Modulierte Signalisierung von Insulinrezeptoren
Modulation de signal de récépteur d'insuline

(30) Priority: 12.01.2001 US 261335 P; 12.01.2001 US 261532 P; 12.01.2001 US 261590 P; 12.01.2001 US 261361 P; 12.01.2001 US 261531 P; 12.01.2001 US 261457 P; 12.01.2001 US 261694 P; 12.01.2001 US 261226 P; 12.01.2001 US 261304 P; 12.01.2001 US 261459 P; 12.01.2001 US 261456 P; 12.01.2001 US 261589 P; 12.01.2001 US 261461 P; 12.01.2001 US 261697 P; 12.01.2001 US 261458 P; 12.01.2001 US 261695 P; 12.01.2001 US 261336 P; 12.01.2001 US 261518 P; 12.01.2001 US 261303 P
(43) Date of publication of application: 28.11.2007
(62) Divisional of application: 02713406.3
(73) Proprietor: Exelixis, Inc., South San Francisco, CA 94080 (US)
(72) Inventor: Seidel-Dugan, Cynthia, Benicia, CA 94510 (US); Ferguson, Kimberley, Carr, Pacifica, CA 94044 (US); Kidd, Thomas, San Francisco, CA 94118 (US)
(74) Representative: Main, Malcolm Charles

(56) References cited:
- US-A- 5 726 027
- US-A- 5 968 508
- US-A- 6 110 718
- US-B1- 6 406 875
- SHISHEVA A ET AL: "Cloning, characterization, and expression of a novel Zn2+-binding FYVE finger-containing phosphoinositide kinase in insulin-sensitive cells." MOLECULAR AND CELLULAR BIOLOGY JAN 1999, vol. 19, no. 1, January 1999 (1999-01), pages 623-634, XP002464528 ISSN: 0270-7306

### SEQUENCE LISTING

<110> EXELIXIS, INC.
<120> Modulating Insulin Receptor Signaling
<130> EX02-001C-PC
<150> US 60/261,335
   <151> 2001-01-12
<150> US 60/261,694
   <151> 2001-01-12
<150> US 60/261,532
   <151> 2001-01-12
<150> US 60/261,361
   <151> 2001-01-12
<150> US 60/261,531
   <151> 2001-01-12
<150> US 60/261,457
   <151> 2001-01-12
<150> US 60/261,226
   <151> 2001-01-12
<150> US 60/261,304
   <151> 2001-01-12
<150> US 60/261,459
   <151> 2001-01-12
<150> US 60/261,456
   <151> 2001-01-12
<150> US 60/261,589
   <151> 2001-01-12
<150> US 60/261,461
   <151> 2001-01-12
<150> US 60/261,697
   <151> 2001-01-12
<150> US 60/261,458
   <151> 2001-01-12
<150> US 60/261,695
   <151> 2001-01-12
<150> US 60/261,336
   <151> 2001-01-12
<150> US 60/261,518
   <151> 2001-01-12
<150> US 60/261,303
   <151> 2001-01-12
<150> US 60/261,590
   <151> 2001-01-12
<160> 76
<170> PatentIn version 3.1
<210> 1
   <211> 2244
   <212> DNA
   <213> Homo sapiens
<400> 1
   ctccccgccc cgaccatggt agtgttcaat ggccttctta agatcaaaat ctgcgaggcc     60
   gtgagcttga agcccacagc ctggtcgctg cgccatgcgg tgggaccccg gccgcagact    120
   ttccttctcg acccctacat tgccctcaat gtggacgact cgcgcatcgg ccaaacggcc    180
   accaagcaga agaccaacag cccggcctgg cacgacgagt tcgtcaccga tgtgtgcaac    240
   ggacgcaaga tcgagctggc tgtctttcac gatgccccca taggctacga cgacttcgtg    300
   gccaactgca ccatccagtt tgaggagctg ctgcagaacg ggagccgcca cttcgaggac    360
   tggattgatc tggagccaga aggaagagtg tatgtgatca tcgatctctc agggtcgtcg    420
   ggtgaagccc ctaaagacaa tgaagagcgt gtgttcaggg aacgcatgcg gccgaggaag    480
   cggcaggggg ccgtcaggcg cagggtccat caggtcaacg gccacaagtt catggccacc    540
   tatcttcggc agcccaccta ctgctcccat tgcagagact tcatctgggg tgtcatagga    600
   aagcagggat accagtgtca agtctgcacc tgcgtggtcc acaagcggtg ccacgagctc    660
   ataatcacaa agtgtgctgg gttaaagaag caggagaccc ccgaccaggt gggctcccag    720
   cggttcagcg tcaacatgcc ccacaagttc ggtatccaca actacaaggt ccctaccttc    780
   tgcgatcact gtgggtccct gctctgggga ctcttgcggc agggtttgca gtgtaaagtc    840
   tgcaaaatga atgttcaccg tcgatgtgag accaacgtgg ctcccaactg tggagtggat    900
   gccagaggaa tcgccaaagt actggccgac ctgggcgtta ccccagacaa aatcaccaac    960
   agcggccaga gaaggaaaaa gctcattgct ggtgccgagt ccccgcagcc tgcttctgga   1020
   agctcaccat ctgaggaaga tcgatccaag tcagcaccca cctccccttg tgaccaggaa   1080
   ataaaagaac ttgagaacaa cattcggaaa gccttgtcat ttgacaaccg aggagaggag   1140
   caccgggcag catcgtctcc tgatggccag ctgatgagcc ccggtgagaa tggcgaagtc   1200
   cggcaaggcc aggccaagcg cctgggcctg gatgagttca acttcatcaa ggtgttgggc   1260
   aaaggcagct ttggcaaggt catgttggca gaactcaagg gcaaagatga agtatatgct   1320
   gtgaaggtct taaagaagga cgtcatcctt caggatgatg acgtggactg cacaatgaca   1380
   gagaagagga ttttggctct ggcacggaaa cacccgtacc ttacccaact ctactgctgc   1440
   ttccagacca aggaccgcct ctttttcgtc atggaatatg taaatggtgg agacctcatg   1500
   tttcagattc agcgctcccg aaaattcgac gagcctcgtt cacggttcta tgctgcagag   1560
   gtcacatcgg ccctcatgtt cctccatcag catggagtca tctacaggga tttgaaactg   1620
   gacaacatcc ttctggatgc agaaggtcac tgcaagctgg ctgacttcgg gatgtgcaag   1680
   gaagggattc tgaatggtgt gacgaccacc acgttctgtg ggactcctga ctacatagct   1740
   cctgagatcc tgcaggagtt ggagtatggc ccctccgtgg actggtgggc cctgggggtg   1800
   ctgatgtacg agatgatggc tggacagcct ccctttgagg ccgacaatga ggacgaccta   1860
   tttgagtcca tcctccatga cgacgtgctg tacccagtct ggctcagcaa ggaggctgtc   1920
   agcatcttga aagctttcat gacgaagaat ccccacaagc gcctgggctg tgtggcatcg   1980
   cagaatggcg aggacgccat caagcagcac ccattcttca aagagattga ctgggtgctc   2040
   ctggagcaga agaagatcaa gccacccttc aaaccacgca ttaaaaccaa aagagacgtc   2100
   aataattttg accaagactt tacccgggaa gagccggtac tcacccttgt ggacgaagca   2160
   attgtaaagc agatcaacca ggaggaattc aaaggtttct cctactttgg tgaagacctg   2220
   atgccctgag agcccactgc agtt                                          2244
<210> 2
   <211> 737
   <212> PRT
   <213> Homo sapiens
<400> 2
<210> 3
   <211> 2389
   <212> DNA
   <213> Homo sapiens
<400> 3
   gaattcggac ggaggaggca gaatggccag tcgaggggcg cttaggcgtg gcctttcccc      60
   agggctgcct cgactcctgc acctgtcccg agggctggcc tgagacggga ctcccggttc     120
   tcccgctgcg aagcaggccc cccggggccg gggcagcggc gccggcatgt cgtctggcac     180
   catgaagttc aatggctatt tgagggtccg catcggtgag gcagtggggc tgcagcccac     240
   ccgctggtcc ctgcgccact cgctcttcaa gaagggccac cagctgctgg acccctatct     300
   gacggtgagc gtggaccagg tgcgcgtggg ccagaccagc accaagcaga agaccaacaa     360
   acccacgtac aacgaggagt tttgcgctaa cgtcaccgac ggcggccacc tcgagttggc     420
   cgtcttccac gagacccccc tgggctacga cttcgtggcc aactgcaccc tgcagttcca     480
   ggagctcgtc ggcacgaccg gcgcctcgga caccttcgag ggttgggtgg atctcgagcc     540
   agaggggaaa gtatttgtgg taataaccct taccgggagt ttcactgaag ctactctcca     600
   gagagaccgg atcttcaaac attttaccag gaagcgccaa agggctatgc gaaggcgagt     660
   ccaccagatc aatggacaca agttcatggc cacgtatctg aggcagccca cctactgctc     720
   tcactgcagg gagtttatct ggggagtgtt tgggaaacag ggttatcagt gccaagtgtg     780
   cacctgtgtc gtccataaac gctgccatca tctaattgtt acagcctgta cttgccaaaa     840
   caatattaac aaagtggatt caaagattgc agaacagagg ttcgggatca acatcccaca     900
   caagttcagc atccacaact acaaagtgcc aacattctgc gatcactgtg gctcactgct     960
   ctggggaata atgcgacaag gacttcagtg taaaatatgt aaaatgaatg tgcatattcg    1020
   atgtcaagcg aacgtggccc ctaactgtgg ggtaaatgcg gtggaacttg ccaagaccct    1080
   ggcagggatg ggtctccaac ccggaaatat ttctccaacc tcgaaactcg tttccagatc    1140
   gaccctaaga cgacagggaa aggagagcag caaagaagga aatgggattg gggttaattc    1200
   ttccaaccga cttggtatcg acaactttga gttcatccga gtgttgggga aggggagttt    1260
   tgggaaggtg atgcttgcaa gagtaaaaga aacaggagac ctctatgctg tgaaggtgct    1320
   gaagaaggac gtgattctgc tggatgatga tgtggaatgc accatgaccg agaaaaggat    1380
   cctgtctctg gcccgcaatc accccttcct cactcagttg ttctgctgct ttcagacccc    1440
   cgatcgtctg ttttttgtga tggagtttgt gaatgggggt gacttgatgt tccacattca    1500
   gaagtctcgt cgttttgatg aagcacgagc tcgcttctat gctgcagaaa tcatttcggc    1560
   tctcatgttc ctccatgata aaggaatcat ctatagagat ctgaaactgg acaatgtcct    1620
   gttggaccac gagggtcact gtaaactggc agacttcgga atgtgcaagg aggggatttg    1680
   caatggtgtc accacggcca cattctgtgg cacgccagac tatatcgctc cagagatcct    1740
   ccaggaaatg ctgtacgggc ctgcagtaga ctggtgggca atgggcgtgt tgctctatga    1800
   gatgctctgt ggtcacgcgc cttttgaggc agagaatgaa gatgacctct ttgaggccat    1860
   actgaatgat gaggtggtct accctacctg gctccatgaa gatgccacag ggatcctaaa    1920
   atctttcatg accaagaacc ccaccatgcg cttgggcagc ctgactcagg gaggcgagca    1980
   cgccatcttg agacatcctt tttttaagga aatcgactgg gcccagctga accatcgcca    2040
   aatagaaccg cctttcagac ccagaatcaa atcccgagaa gatgtcagta attttgaccc    2100
   tgacttcata aaggaagagc cagttttaac tccaattgat gagggacatc ttccaatgat    2160
   taaccaggat gagtttagaa acttttccta tgtgtctcca gaattgcaac catagcctta    2220
   tggggagtga gagagagggc acgagaaccc aaaggaatag agattctcca ggaatttcct    2280
   ctatcggacc ttcccagcat cagccttaga acaagaacct taccttcaag gagcaagtga    2340
   agaactctgt cgaaggatgg aactttcaga tatcaactat ttagagtcc                2389
<210> 4
   <211> 682
   <212> PRT
   <213> Homo sapiens
<400> 4
   Met Ser Ser Gly Thr Met Lys Phe Asn Gly Tyr Leu Arg Val Arg Ile
   1               5                   10                  15
   Gly Glu Ala Val Gly Leu Gln Pro Thr Arg Trp Ser Leu Arg His Ser
               20                  25                  30
   Leu Phe Lys Lys Gly His Gln Leu Leu Asp Pro Tyr Leu Thr Val Ser
           35                  40                  45
   Val Asp Gln Val Arg Val Gly Gln Thr Ser Thr Lys Gln Lys Thr Asn
       50                  55                  60
   Lys Pro Thr Tyr Asn Glu Glu Phe Cys Ala Asn Val Thr Asp Gly Gly
   65                  70                  75                  80
   His Leu Glu Leu Ala Val Phe His Glu Thr Pro Leu Gly Tyr Asp Phe
                   85                  90                  95
   Val Ala Asn Cys Thr Leu Gln Phe Gln Glu Leu Val Gly Thr Thr Gly
               100                 105                 110
   Ala Ser Asp Thr Phe Glu Gly Trp Val Asp Leu Glu Pro Glu Gly Lys
           115                 120                 125
   Val Phe Val Val Ile Thr Leu Thr Gly Ser Phe Thr Glu Ala Thr Leu
       130                 135                 140
   Gln Arg Asp Arg Ile Phe Lys His Phe Thr Arg Lys Arg Gln Arg Ala
   145                 150                 155                 160
   Met Arg Arg Arg Val His Gln Ile Asn Gly His Lys Phe Met Ala Thr
                   165                 170                 175
   Tyr Leu Arg Gln Pro Thr Tyr Cys Ser His Cys Arg Glu Phe Ile Trp
               180                 185                 190
   Gly Val Phe Gly Lys Gln Gly Tyr Gln Cys Gln Val Cys Thr Cys Val
           195                 200                 205
   Val His Lys Arg Cys His His Leu Ile Val Thr Ala Cys Thr Cys Gln
       210                 215                 220
   Asn Asn Ile Asn Lys Val Asp Ser Lys Ile Ala Glu Gln Arg Phe Gly
   225                 230                 235                 240
   Ile Asn Ile Pro His Lys Phe Ser Ile His Asn Tyr Lys Val Pro Thr
                   245                 250                 255
   Phe Cys Asp His Cys Gly Ser Leu Leu Trp Gly Ile Met Arg Gln Gly
               260                 265                 270
   Leu Gln Cys Lys Ile Cys Lys Met Asn Val His Ile Arg Cys Gln Ala
           275                 280                 285
   Asn Val Ala Pro Asn Cys Gly Val Asn Ala Val Glu Leu Ala Lys Thr
       290                 295                 300
   Leu Ala Gly Met Gly Leu Gln Pro Gly Asn Ile Ser Pro Thr Ser Lys
   305                 310                 315                 320
   Leu Val Ser Arg Ser Thr Leu Arg Arg Gln Gly Lys Glu Ser Ser Lys
                   325                 330                 335
   Glu Gly Asn Gly Ile Gly Val Asn Ser Ser Asn Arg Leu Gly Ile Asp
               340                 345                 350
   Asn Phe Glu Phe Ile Arg Val Leu Gly Lys Gly Ser Phe Gly Lys Val
           355                 360                 365
   Met Leu Ala Arg Val Lys Glu Thr Gly Asp Leu Tyr Ala Val Lys Val
       370                 375                 380
   Leu Lys Lys Asp Val Ile Leu Leu Asp Asp Asp Val Glu Cys Thr Met
   385                 390                 395                 400
   Thr Glu Lys Arg Ile Leu Ser Leu Ala Arg Asn His Pro Phe Leu Thr
                   405                 410                 415
   Gln Leu Phe Cys Cys Phe Gln Thr Pro Asp Arg Leu Phe Phe Val Met
               420                 425                 430
   Glu Phe Val Asn Gly Gly Asp Leu Met Phe His Ile Gln Lys Ser Arg
           435                 440                 445
   Arg Phe Asp Glu Ala Arg Ala Arg Phe Tyr Ala Ala Glu Ile Ile Ser
       450                 455                 460
   Ala Leu Met Phe Leu His Asp Lys Gly Ile Ile Tyr Arg Asp Leu Lys
   465 470 475 480
   Leu Asp Asn Val Leu Leu Asp His Glu Gly His Cys Lys Leu Ala Asp
   485 490 495
   Phe Gly Met Cys Lys Glu Gly Ile Cys Asn Gly Val Thr Thr Ala Thr
   500 505 510
   Phe Cys Gly Thr Pro Asp Tyr Ile Ala Pro Glu Ile Leu Gln Glu Met
   515 520 525
   Leu Tyr Gly Pro Ala Val Asp Trp Trp Ala Met Gly Val Leu Leu Tyr
   530 535 540
   Glu Met Leu Cys Gly His Ala Pro Phe Glu Ala Glu Asn Glu Asp Asp
   545 550 555 560
   Leu Phe Glu Ala Ile Leu Asn Asp Glu Val Val Tyr Pro Thr Trp Leu
   565 570 575
   His Glu Asp Ala Thr Gly Ile Leu Lys Ser Phe Met Thr Lys Asn Pro
   580 585 590
   Thr Met Arg Leu Gly Ser Leu Thr Gln Gly Gly Glu His Ala Ile Leu
   595 600 605
   Arg His Pro Phe Phe Lys Glu Ile Asp Trp Ala Gln Leu Asn His Arg
   610 615 620
   Gln Ile Glu Pro Pro Phe Arg Pro Arg Ile Lys Ser Arg Glu Asp Val
   625 630 635 640
   Ser Asn Phe Asp Pro Asp Phe Ile Lys Glu Glu Pro Val Leu Thr Pro
   645 650 655
   Ile Asp Glu Gly His Leu Pro Met Ile Asn Gln Asp Glu Phe Arg Asn
   660 665 670
   Phe Ser Tyr Val Ser Pro Glu Leu Gln Pro
   675 680
<210> 5
   <211> 2372
   <212> DNA
   <213> Homo sapiens
<400> 5
   gagaaatggc gtggcagggg acccagcgag cccagaggga ttttgccgct gcttcctcta 60
   cccctgtatt tcacgcagct ctctaaattg actcagctcc aggctagtgt gagaaacacc    120
   aacagcaggc ccatctcaga tcttcactat ggcaacttat gcaagaaact gttgaattag    180
   acccgtttcc tatagatgag aaaccataca agctgtggta tttatgagcc tccatttctt    240
   atactactgc agtgaaccaa cattggatgt gaaaattgcc ttttgtcagg gattcgataa    300
   acaagtggat gtgtcatata ttgccaaaca ttacaacatg agcaaaagca aagttgacaa    360
   ccagttctac agtgtggaag tgggagactc aaccttcaca gttctcaagc gctaccagaa    420
   tctaaagcct attggctctg gggctcaggg catagtttgt gccgcgtatg atgctgtcct    480
   tgacagaaat gtggccatta agaagctcag cagacccttt cagaaccaaa cacatgccaa    540
   gagagcgtac cgggagctgg tcctcatgaa gtgtgtgaac cataaaaaca ttattagttt    600
   attaaatgtc ttcacacccc agaaaacgct ggaggagttc caagatgttt acttagtaat    660
   ggaactgatg gatgccaact tatgtcaagt gattcagatg gaattagacc atgagcgaat    720
   gtcttacctg ctgtaccaaa tgttgtgtgg cattaagcac ctccattctg ctggaattat    780
   tcacagggat ttaaaaccaa gtaacattgt agtcaagtct gattgcacat tgaaaatcct    840
   ggactttgga ctggccagga cagcaggcac aagcttcatg atgactccat atgtggtgac    900
   acgttattac agagcccctg aggtcatcct ggggatgggc tacaaggaga acgtggatat    960
   atggtctgtg ggatgcatta tgggagaaat ggttcgccac aaaatcctct ttccaggaag   1020
   ggactatatt gaccagtgga ataaggtaat tgaacaacta ggaacaccat gtccagaatt   1080
   catgaagaaa ttgcaaccca cagtaagaaa ctatgtggag aatcggccca agtatgcggg   1140
   actcaccttc cccaaactct tcccagattc cctcttccca gcggactccg agcacaataa   1200
   actcaaagcc agccaagcca gggacttgtt gtcaaagatg ctagtgattg acccagcaaa   1260
   aagaatatca gtggacgacg ccttacagca tccctacatc aacgtctggt atgacccagc   1320
   cgaagtggag gcgcctccac ctcagatata tgacaagcag ttggatgaaa gagaacacac   1380
   aattgaagaa tggaaagaac ttatctacaa ggaagtaatg aattcagaag aaaagactaa   1440
   aaatggtgta gtaaaaggac agccttctcc ttcagcacag gtgcagcagt gaacagcagt   1500
   gagagtctcc ctccatcctc gtctgtcaat gacatctcct ccatgtccac cgaccagacc   1560
   ctggcatctg acactgacag cagcctggaa gcctcggcag gacccctggg ttgttgcagg   1620
   tgactagccg cctgcctgcg aaacccagcg ttcttcagga gatgatgtga tggaacacac   1680
   acacacgcag acacacacac acacacaaat gcagacacac aacatcaaga aaacagcaag   1740
   ggagagaatc caagcctaaa attaaataaa tctttcagcc tgcttcttcc ccagggttct   1800
   gtattgcagc taagctcaaa tgtatattta acttctagtt gctcttgctt tggtcttctt   1860
   ccaatgatgc ttactacaga aagcaaatca gacacaatta gagaagcctt ttccataaag   1920
   tgtaatttta atggctgcaa aaccggcaac ctgtaactgc ccttttaaat ggcatgacaa    1980
   ggtgtgcagt ggccccatcc agcatgtgtg tgtctctatc ttgcatctac ctgctccttg    2040
   gcctagtcag atggatgtag atacagatcc gcatgtgtct gtattcatac agcactactt    2100
   acttagagat gctactctca gtgtcctcag ggctctacca agacataatg cactggggta    2160
   ccacatggtc catttcatgt gatctattac tctgacataa acccatctgt aatatattgc    2220
   cagtatataa gctgtttagt ttgttaattg attaaactgt atgtcttata agaaaacatg    2280
   taaaggggga atatattggg ggagtgagct ctctcagacc cttgaagatg tagcttccaa    2340
   atttgaatgg attaaatggc acctgtatac ca                                  2372
<210> 6
   <211> 422
   <212> PRT
   <213> Homo sapiens
<400> 6
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65                  70                  75                  80
   Ala Tyr Asp Ala Val Leu Asp Arg Asn Val Ala Ile Lys Lys Leu Ser
                   85                  90                  95
   Arg Pro Phe Gln Asn Gln Thr His Ala Lys Arg Ala Tyr Arg Glu Leu
               100                 105                 110
   Val Leu Met Lys Cys Val Asn His Lys Asn Ile Ile Ser Leu Leu Asn
           115                 120                 125
   Val Phe Thr Pro Gln Lys Thr Leu Glu Glu Phe Gln Asp Val Tyr Leu
       130                 135                 140
   Val Met Glu Leu Met Asp Ala Asn Leu Cys Gln Val Ile Gln Met Glu
   145                 150                 155                 160
   Leu Asp His Glu Arg Met Ser Tyr Leu Leu Tyr Gln Met Leu Cys Gly
                   165                 170                 175
   Ile Lys His Leu His Ser Ala Gly Ile Ile His Arg Asp Leu Lys Pro
               180                 185                 190
   Ser Asn Ile Val Val Lys Ser Asp Cys Thr Leu Lys Ile Leu Asp Phe
           195                 200                 205
   Gly Leu Ala Arg Thr Ala Gly Thr Ser Phe Met Met Thr Pro Tyr Val
       210                 215                 220
   Val Thr Arg Tyr Tyr Arg Ala Pro Glu Val Ile Leu Gly Met Gly Tyr
   225                 230                 235                 240
   Lys Glu Asn Val Asp Ile Trp Ser Val Gly Cys Ile Met Gly Glu Met
                   245                 250                 255
   Val Arg His Lys Ile Leu Phe Pro Gly Arg Asp Tyr Ile Asp Gln Trp
               260                 265                 270
   Asn Lys Val Ile Glu Gln Leu Gly Thr Pro Cys Pro Glu Phe Met Lys
           275                 280                 285
   Lys Leu Gln Pro Thr Val Arg Asn Tyr Val Glu Asn Arg Pro Lys Tyr
       290                 295                 300
   Ala Gly Leu Thr Phe Pro Lys Leu Phe Pro Asp Ser Leu Phe Pro Ala
   305                 310                 315                 320
   Asp Ser Glu His Asn Lys Leu Lys Ala Ser Gln Ala Arg Asp Leu Leu
                   325                 330                 335
   Ser Lys Met Leu Val Ile Asp Pro Ala Lys Arg Ile Ser Val Asp Asp
               340                 345                 350
   Ala Leu Gln His Pro Tyr Ile Asn Val Trp Tyr Asp Pro Ala Glu Val
           355                 360                 365
   Glu Ala Pro Pro Pro Gln Ile Tyr Asp Lys Gln Leu Asp Glu Arg Glu
       370                 375                 380
   His Thr Ile Glu Glu Trp Lys Glu Leu Ile Tyr Lys Glu Val Met Asn
   385                 390                 395                 400
   Ser Glu Glu Lys Thr Lys Asn Gly Val Val Lys Gly Gln Pro Ser Pro
                   405                 410                 415
   Ser Ala Gln Val Gln Gln
               420
<210> 7
   <211> 1418
   <212> DNA
   <213> Homo sapiens
<400> 7
   cattaattgc ttgccatcat gagcagaagc aagcgtgaca acaattttta tagtgtagag      60
   attggagatt ctacattcac agtcctgaaa cgatatcaga atttaaaacc tataggctca     120
   ggagctcaag gaatagtatg cgcagcttat gatgccattc ttgaaagaaa tgttgcaatc     180
   aagaagctaa gccgaccatt tcagaatcag actcatgcca agcgggccta cagagagcta     240
   gttcttatga aatgtgttaa tcacaaaaat ataattggcc ttttgaatgt tttcacacca     300
   cagaaatccc tagaagaatt tcaagatgtt tacatagtca tggagctcat ggatgcaaat     360
   ctttgccaag tgattcagat ggagctagat catgaaagaa tgtcctacct tctctatcag     420
   atgctgtgtg gaatcaagca ccttcattct gctggaatta ttcatcggga cttaaagccc     480
   agtaatatag tagtaaaatc tgattgcact ttgaagattc ttgacttcgg tctggccagg     540
   actgcaggaa cgagttttat gatgacgcct tatgtagtga ctcgctacta cagagcaccc     600
   gaggtcatcc ttggcatggg ctacaaggaa aacgtggatt tatggtctgt ggggtgcatt     660
   atgggagaaa tggtttgcca caaaatcctc tttccaggaa gggactatat tgatcagtgg     720
   aataaagtta ttgaacagct tggaacacca tgtcctgaat tcatgaagaa actgcaacca     780
   acagtaagga cttacgttga aaacagacct aaatatgctg gatatagctt tgagaaactc     840
   ttccctgatg tccttttccc agctgactca gaacacaaca aacttaaagc cagtcaggca     900
   agggatttgt tatccaaaat gctggtaata gatgcatcta aaaggatctc tgtagatgaa     960
   gctctccaac acccgtacat caatgtctgg tatgatcctt ctgaagcaga agctccacca    1020
   ccaaagatcc ctgacaagca gttagatgaa agggaacaca caatagaaga gtggaaagaa    1080
   ttgatatata aggaagttat ggacttggag gagagaacca agaatggagt tatacggggg    1140
   cagccctctc ctttagcaca ggtgcagcag tgatcaatgg ctctcagcat ccatcatcat    1200
   cgtcgtctgt caatgatgtg tcttcaatgt caacagatcc gactttggcc tctgatacag    1260
   acagcagtct agaagcagca gctgggcctc tgggctgctg tagatgacta cttgggccat    1320
   cggggggtgg gagggatggg gagtcggtta gtcattgata gaactacttt gaaaacaatt    1380
   cagtggtctt atttttgggt gatttttcaa aaaatgta                            1418
<210> 8
   <211> 384
   <212> PRT
   <213> Homo sapiens
<400> 8
   Met Ser Arg Ser Lys Arg Asp Asn Asn Phe Tyr Ser Val Glu Ile Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Ile Leu
           35                  40                  45
   Glu Arg Asn Val Ala Ile Lys Lys Leu Ser Arg Pro Phe Gln Asn Gln
       50                  55                  60
   Thr His Ala Lys Arg Ala Tyr Arg Glu Leu Val Leu Met Lys Cys Val
   65                  70                  75                  80
   Asn His Lys Asn Ile Ile Gly Leu Leu Asn Val Phe Thr Pro Gln Lys
                   85                  90                  95
   Ser Leu Glu Glu Phe Gln Asp Val Tyr Ile Val Met Glu Leu Met Asp
               100                 105                 110
   Ala Asn Leu Cys Gln Val Ile Gln Met Glu Leu Asp His Glu Arg Met
           115                 120                 125
   Ser Tyr Leu Leu Tyr Gln Met Leu Cys Gly Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Gly Thr Ser Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Leu
           195                 200                 205
   Trp Ser Val Gly Cys Ile Met Gly Glu Met Val Cys His Lys Ile Leu
   210 215 220
   Phe Pro Gly Arg Asp Tyr Ile Asp Gln Trp Asn Lys Val Ile Glu Gln
   225 230 235 240
   Leu Gly Thr Pro Cys Pro Glu Phe Met Lys Lys Leu Gln Pro Thr Val
   245 250 255
   Arg Thr Tyr Val Glu Asn Arg Pro Lys Tyr Ala Gly Tyr Ser Phe Glu
   260 265 270
   Lys Leu Phe Pro Asp Val Leu Phe Pro Ala Asp Ser Glu His Asn Lys
   275 280 285
   Leu Lys Ala Ser Gln Ala Arg Asp Leu Leu Ser Lys Met Leu Val Ile
   290 295 300
   Asp Ala Ser Lys Arg Ile Ser Val Asp Glu Ala Leu Gln His Pro Tyr
   305 310 315 320
   Ile Asn Val Trp Tyr Asp Pro Ser Glu Ala Glu Ala Pro Pro Pro Lys
   325 330 335
   Ile Pro Asp Lys Gln Leu Asp Glu Arg Glu His Thr Ile Glu Glu Trp
   340 345 350
   Lys Glu Leu Ile Tyr Lys Glu Val Met Asp Leu Glu Glu Arg Thr Lys
   355 360 365
   Asn Gly Val Ile Arg Gly Gln Pro Ser Pro Leu Ala Gln Val Gln Gln
   370 375 380
<210> 9
   <211> 1392
   <212> DNA
   <213> Homo sapiens
<400> 9
   tctgaaactt gcccaccctt cgggatattg caggacgctg catcatgagc gacagtaaat 60
   gtgacagtca gttttatagt gtgcaagtgg cagactcaac cttcactgtc ctaaaacgtt 120
   accagcagct gaaaccaatt ggctctgggg cccaagggat tgtttgtgct gcatttgata 180
   cagttcttgg gataagtgtt gcagtcaaga aactaagccg tccttttcag aaccaaactc 240
   atgcaaagag agcttatcgt gaacttgtcc tcttaaaatg tgtcaatcat aaaaatataa 300
   ttagtttgtt aaatgtgttt acaccacaaa aaactctaga agaatttcaa gatgtgtatt 360
   tggttatgga attaatggat gctaacttat gtcaggttat tcacatggag ctggatcatg 420
   aaagaatgtc ctaccttctt taccagatgc tttgtggtat taaacatctg cattcagctg 480
   gtataattca tagagatttg aagcctagca acattgttgt gaaatcagac tgcaccctga 540
   agatccttga ctttggcctg gcccggacag cgtgcactaa cttcatgatg accccttacg 600
   tggtgacacg gtactaccgg gcgcccgaag tcatcctggg tatgggctac aaagagaacg 660
   ttgatatctg gtcagtgggt tgcatcatgg gagagctggt gaaaggttgt gtgatattcc 720
   aaggcactga ccatattgat cagtggaata aagttattga gcagctggga acaccatcag 780
   cagagttcat gaagaaactt cagccaactg tgaggaatta tgtcgaaaac agaccaaagt 840
   atcctggaat caaatttgaa gaactctttc cagattggat attcccatca gaatctgagc 900
   gagacaaaat aaaaacaagt caagccagag atctgttatc aaaaatgtta gtgattgatc 960
   ctgacaagcg gatctctgta gacgaagctc tgcgtcaccc atacatcact gtttggtatg 1020
   accccgccga agcagaagcc ccaccacctc aaatttatga tgcccagttg gaagaaagag 1080
   aacatgcaat tgaagaatgg aaagagctaa tttacaaaga agtcatggat tgggaagaaa 1140
   gaagcaagaa tggtgttgta aaagatcagc cttcagcaca gatgcagcag taagtagcaa 1200
   cgccactcct tctcagtctt catcgatcaa tgacatttca tccatgtcca ctgagcagac 1260
   gctggcctca gacacagaca gcagtcttga tgcctcgacg ggaccccttg aaggctgtcg 1320
   atgataggtt agaaatagca aacctgtcag cattgaagga actctcacct ccgtgggcct 1380
   gaaatgcttg gg 1392
<210> 10
   <211> 382
   <212> PRT
   <213> Homo sapiens
<400> 10
   Met Ser Asp Ser Lys Cys Asp Ser Gln Phe Tyr Ser Val Gln Val Ala
   1               5                   10                  15
   Asp Ser Thr Phe Thr Val Leu Lys Arg Tyr Gln Gln Leu Lys Pro Ile
               20                  25                  30
   Gly Ser Gly Ala Gln Gly Ile Val Cys Ala Ala Phe Asp Thr Val Leu
           35                  40                  45
   Gly Ile Ser Val Ala Val Lys Lys Leu Ser Arg Pro Phe Gln Asn Gln
       50                  55                  60
   Thr His Ala Lys Arg Ala Tyr Arg Glu Leu Val Leu Leu Lys Cys Val
   65                  70                  75                  80
   Asn His Lys Asn Ile Ile Ser Leu Leu Asn Val Phe Thr Pro Gln Lys
                   85                  90                  95
   Thr Leu Glu Glu Phe Gln Asp Val Tyr Leu Val Met Glu Leu Met Asp
               100                 105                 110
   Ala Asn Leu Cys Gln Val Ile His Met Glu Leu Asp His Glu Arg Met
           115                 120                 125
   Ser Tyr Leu Leu Tyr Gln Met Leu Cys Gly Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Cys Thr Asn Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Ile
           195                 200                 205
   Trp Ser Val Gly Cys Ile Met Gly Glu Leu Val Lys Gly Cys Val Ile
       210                 215                 220
   Phe Gln Gly Thr Asp His Ile Asp Gln Trp Asn Lys Val Ile Glu Gln
   225                 230                 235                 240
   Leu Gly Thr Pro Ser Ala Glu Phe Met Lys Lys Leu Gln Pro Thr Val
                   245                 250                 255
   Arg Asn Tyr Val Glu Asn Arg Pro Lys Tyr Pro Gly Ile Lys Phe Glu
               260                 265                 270
   Glu Leu Phe Pro Asp Trp Ile Phe Pro Ser Glu Ser Glu Arg Asp Lys
           275                 280                 285
   Ile Lys Thr Ser Gln Ala Arg Asp Leu Leu Ser Lys Met Leu Val Ile
       290                 295                 300
   Asp Pro Asp Lys Arg Ile Ser Val Asp Glu Ala Leu Arg His Pro Tyr
   305                 310                 315                 320
   Ile Thr Val Trp Tyr Asp Pro Ala Glu Ala Glu Ala Pro Pro Pro Gln
   325 330 335
   Ile Tyr Asp Ala Gln Leu Glu Glu Arg Glu His Ala Ile Glu Glu Trp
   340 345 350
   Lys Glu Leu Ile Tyr Lys Glu Val Met Asp Trp Glu Glu Arg Ser Lys
   355 360 365
   Asn Gly Val Val Lys Asp Gln Pro Ser Ala Gln Met Gln Gln
   370 375 380
<210> 11
   <211> 1167
   <212> DNA
   <213> Homo sapiens
<400> 11
   atgctgatcg ctgtcctcct cctccccctc aggcggcgct ggcggcgagc cctgggaccc      60
   gcggaagccg gcatgctgga gaagctggag ttcgaggacg aagcagtaga agactcagaa     120
   agtggtgttt acatgcgatt catgaggtca cacaagtgtt atgacatcgt tccaaccagt     180
   tcaaagcttg ttgtctttga tactacatta caagttaaaa aggccttctt tgctttggta     240
   gccaacggtg tccgagcagc gccactgtgg gagagtaaaa aacaaagttt tgtaggaatg     300
   ctaacaatta cagatttcat aaatatacta catagatact ataaatcacc tatggtacag     360
   atttatgaat tagaggaaca taaaattgaa acatggaggg agctttattt acaagaaaca     420
   tttaagcctt tagtgaatat atctccagat gcaagcctct tcgatgctgt atactccttg     480
   atcaaaaata aaatccacag attgcccgtt attgacccta tcagtgggaa tgcactttat     540
   atacttaccc acaaaagaat cctcaagttc ctccagcttt ttatgtctga tatgccaaag     600
   cctgccttca tgaagcagaa cctggatgag cttggaatag gaacgtacca caacattgcc     660
   ttcatacatc cagacactcc catcatcaaa gccttgaaca tatttgtgga aagacgaata     720
   tcagctctgc ctgttgtgga tgagtcagga aaagttgtag atatttattc caaatttgat     780
   gtaattaatc ttgctgctga gaaaacatac aataacctag atatcacggt gacccaggcc     840
   cttcagcacc gttcacagta ttttgaaggt gttgtgaagt gcaataagct ggaaatactg     900
   gagaccatcg tggacagaat agtaagagct gaggtccatc ggctggtggt ggtaaatgaa     960
   gcagatagta ttgtgggtat tatttccctg tcggacattc tgcaagccct gatcctcaca    1020
   ccagcaggtg ccaaacaaaa ggagacagaa acggagtgac cgccgtgaat gtagacgccc    1080
   taggaggaga acttgaacaa agtctctggg tcacgttttg cctcatgaac actggctgca    1140
   agtggttaag aatgtatatc agggttt                                        1167
<210> 12
   <211> 352
   <212> PRT
   <213> Homo sapiens
<400> 12
   Met Leu Ile Ala Val Leu Leu Leu Pro Leu Arg Arg Arg Trp Arg Arg
   1               5                   10                  15
   Ala Leu Gly Pro Ala Glu Ala Gly Met Leu Glu Lys Leu Glu Phe Glu
               20                  25                  30
   Asp Glu Ala Val Glu Asp Ser Glu Ser Gly Val Tyr Met Arg Phe Met
           35                  40                  45
   Arg Ser His Lys Cys Tyr Asp Ile Val Pro Thr Ser Ser Lys Leu Val
       50                  55                  60
   Val Phe Asp Thr Thr Leu Gln Val Lys Lys Ala Phe Phe Ala Leu Val
   65                  70                  75                  80
   Ala Asn Gly Val Arg Ala Ala Pro Leu Trp Glu Ser Lys Lys Gln Ser
                   85                  90                  95
   Phe Val Gly Met Leu Thr Ile Thr Asp Phe Ile Asn Ile Leu His Arg
               100                 105                 110
   Tyr Tyr Lys Ser Pro Met Val Gln Ile Tyr Glu Leu Glu Glu His Lys
           115                 120                 125
   Ile Glu Thr Trp Arg Glu Leu Tyr Leu Gln Glu Thr Phe Lys Pro Leu
       130                 135                 140
   Val Asn Ile Ser Pro Asp Ala Ser Leu Phe Asp Ala Val Tyr Ser Leu
   145                 150                 155                 160
   Ile Lys Asn Lys Ile His Arg Leu Pro Val Ile Asp Pro Ile Ser Gly
                   165                 170                 175
   Asn Ala Leu Tyr Ile Leu Thr His Lys Arg Ile Leu Lys Phe Leu Gln
               180                 185                 190
   Leu Phe Met Ser Asp Met Pro Lys Pro Ala Phe Met Lys Gln Asn Leu
           195                 200                 205
   Asp Glu Leu Gly Ile Gly Thr Tyr His Asn Ile Ala Phe Ile His Pro
       210                 215                 220
   Asp Thr Pro Ile Ile Lys Ala Leu Asn Ile Phe Val Glu Arg Arg Ile
   225 230 235 240
   Ser Ala Leu Pro Val Val Asp Glu Ser Gly Lys Val Val Asp Ile Tyr
   245 250 255
   Ser Lys Phe Asp Val Ile Asn Leu Ala Ala Glu Lys Thr Tyr Asn Asn
   260 265 270
   Leu Asp Ile Thr Val Thr Gln Ala Leu Gln His Arg Ser Gln Tyr Phe
   275 280 285
   Glu Gly Val Val Lys Cys Asn Lys Leu Glu Ile Leu Glu Thr Ile Val
   290 295 300
   Asp Arg Ile Val Arg Ala Glu Val His Arg Leu Val Val Val Asn Glu
   305 310 315 320
   Ala Asp Ser Ile Val Gly Ile Ile Ser Leu Ser Asp Ile Leu Gln Ala
   325 330 335
   Leu Ile Leu Thr Pro Ala Gly Ala Lys Gln Lys Glu Thr Glu Thr Glu
   340 345 350
<210> 13
   <211> 2223
   <212> DNA
   <213> Homo sapiens
<400> 13
   aggcccctcc tcccggcggc gcggcagagc caggccccag cgctcggccg gccgcgagcc 60
   cgccggccgg ggacgagcgt cgcagctcat gctgatcgct gtcctcctcc tccccctcag 120
   gcggcgctgg cggcggccct gggacccgcg gaagccggca tgctggagaa gctggagttc 180
   gaggacgaag cagtagaaga ctcagaaagt ggtgtttaca tgcgattcat gaggtcacac 240
   aagtgttatg acatcgttcc aaccagttca aagcttgttg tctttgatac tacattacaa 300
   gttaaaaagg ccttctttgc tttggtagcc aacggtgtcc gagcagcgcc actgtgggag 360
   agtaaaaaac aaagttttgt aggaatgcta acaattacag atttcataaa tatactacat 420
   agatactata aatcacctat ggtacagatt tatgaattag aggaacataa aattgaaaca 480
   tggagggagc tttatttaca agaaacattt aagcctttag tgaatatatc tccagatgca 540
   agcctcttcg atgctgtata ctccttgatc aaaaataaaa tccacagatt gcccgttatt 600
   gaccctatca gtgggaatgc actttatata cttacccaca aaagaatcct caagttcctc 660
   cagcttttta tgtctgatat gccaaagcct gccttcatga agcagaacct ggatgagctt 720
   ggaataggaa cgtaccacaa cattgccttc atacatccag acactcccat catcaaagcc     780
   ttgaacatat ttgtggaaag acgaatatca gctctgcctg ttgtggatga gtcaggaaaa     840
   gttgtagata tttattccaa atttgatgta attaatcttg ctgctgagaa aacatacaat     900
   aacctagata tcacggtgac ccaggccctt cagcaccgtt cacagtattt tgaaggtgtt     960
   gtgaagtgca ataagctgga aatactggag accatcgtgg acagaatagt aagagctgag    1020
   gtccatcggc tggtggtggt aaatgaagca gatagtattg tgggtattat ttccctgtcg    1080
   gacattctgc aagccctgat cctcacgcca gcaggtgcca aacaaaagga gacagaaacg    1140
   gagtgaccgc cgtgaatgta gacgccctag gaggagaact tgaacaaagt ctctgggtca    1200
   cgttttgcct catgaacact ggctgcaagt ggttaagaat gtatatcagg gtttaacgat    1260
   aggtatttct tccagtgatg ttgaaattaa gcttaaaaaa gaaagatttt atgtgcttga    1320
   agattcaggc ttgcattaaa agactgtttt cagacctttg tctgaaggat tttaaatgct    1380
   gtatgtcatt aaagtgcact gtgtcctgaa gttttcatta tttttcattt caaagaattc    1440
   actggtatgg aacaggtgat gtggcataag gtgagtgcac ggtatgttca gatcacagtg    1500
   ccttatgtcc gaatacagca atatgtcacc gccgcagccg gggcgcacgc gtgtgaaaca     1560
   acaccgagct tgaatgtgga agtctttgaa cctcttacca aatcagtttg ttttctttag    1620
   atttgtcaaa aagttgtaat ttgaatataa ataattactt taaaatttta atgacacttt    1680
   tacacgtaag tgttttgttc tgggctaccg tgtcaacgag gctgctttac aacagcttta    1740
   tttattttta ctttcatgca atttttttac acatcttttg gtggagtaaa cttcaccaca     1800
   tccatgaata aactctcagt tattttgaaa tggcaaattt ctcattattt aagtttggat    1860
   ctggaaagga catgacttct gaaatagccg ctgctgggtt ttaaaagctg aggtctctca    1920
   aagtgtggag gagacgttgc cgtcaggcgg gagccaagtg ccgggaagat gcctattttt    1980
   tttcttgtgt attgaaatgt aaaatcatga tgtttgttat gactgctgat gcgattgttt    2040
   ttgtaaattt tattgtggca tatacagtat tgtcatacag ttgaagagaa acaatgtttc    2100
   ctaatgtaag tgctctgaaa atgttgacac tgtatatata tatatgagga tagtttgttt    2160
   ttttttgttt tgggtttttt tttttttcag attgaaaaat taaaataaat cctactatct     2220
   acc                                                                   2223
<210> 14
   <211> 328
   <212> PRT
   <213> Homo sapiens
<400> 14
   Met Leu Glu Lys Leu Glu Phe Glu Asp Glu Ala Val Glu Asp Ser Glu
   1 5 10 15
   Ser Gly Val Tyr Met Arg Phe Met Arg Ser His Lys Cys Tyr Asp Ile
   20 25 30
   Val Pro Thr Ser Ser Lys Leu Val Val Phe Asp Thr Thr Leu Gln Val
   35 40 45
   Lys Lys Ala Phe Phe Ala Leu Val Ala Asn Gly Val Arg Ala Ala Pro
   50 55 60
   Leu Trp Glu Ser Lys Lys Gln Ser Phe Val Gly Met Leu Thr Ile Thr
   65 70 75 80
   Asp Phe Ile Asn Ile Leu His Arg Tyr Tyr Lys Ser Pro Met Val Gln
   85 90 95
   Ile Tyr Glu Leu Glu Glu His Lys Ile Glu Thr Trp Arg Glu Leu Tyr
   100 105 110
   Leu Gln Glu Thr Phe Lys Pro Leu Val Asn Ile Ser Pro Asp Ala Ser
   115 120 125
   Leu Phe Asp Ala Val Tyr Ser Leu Ile Lys Asn Lys Ile His Arg Leu
   130 135 140
   Pro Val Ile Asp Pro Ile Ser Gly Asn Ala Leu Tyr Ile Leu Thr His
   145 150 155 160
   Lys Arg Ile Leu Lys Phe Leu Gln Leu Phe Met Ser Asp Met Pro Lys
   165 170 175
   Pro Ala Phe Met Lys Gln Asn Leu Asp Glu Leu Gly Ile Gly Thr Tyr
   180 185 190
   His Asn Ile Ala Phe Ile His Pro Asp Thr Pro Ile Ile Lys Ala Leu
   195 200 205
   Asn Ile Phe Val Glu Arg Arg Ile Ser Ala Leu Pro Val Val Asp Glu
   210 215 220
   Ser Gly Lys Val Val Asp Ile Tyr Ser Lys Phe Asp Val Ile Asn Leu
   225 230 235 240
   Ala Ala Glu Lys Thr Tyr Asn Asn Leu Asp Ile Thr Val Thr Gln Ala
   245 250 255
   Leu Gln His Arg Ser Gln Tyr Phe Glu Gly Val Val Lys Cys Asn Lys
   260 265 270
   Leu Glu Ile Leu Glu Thr Ile Val Asp Arg Ile Val Arg Ala Glu Val
   275 280 285
   His Arg Leu Val Val Val Asn Glu Ala Asp Ser Ile Val Gly Ile Ile
   290 295 300
   Ser Leu Ser Asp Ile Leu Gln Ala Leu Ile Leu Thr Pro Ala Gly Ala
   305 310 315 320
   Lys Gln Lys Glu Thr Glu Thr Glu
   325
<210> 15
   <211> 1578
   <212> DNA
   <213> Homo sapiens
<400> 15
   gcgcccttaa agatggtgag ggggctcatg ctctgagtag aaggtggtga cctccaggag 60
   cggtgggatg atgagggccc gggcgcctct tgcaatggag acggtcattt cttcagatag 120
   ctccccagct gtggaaaatg agcatcctca agagacccca gaatccaaca atagcgtgta 180
   tacttccttc atgaagtctc atcgctgcta tgacctgatt cccacaagct ccaaattggt 240
   tgtatttgat acgtccctgc aggtgaagaa agcttttttt gctttggtga ctaacggtgt 300
   acgagctgcc cctttatggg atagtaagaa gcaaagtttt gtgggcatgc tgaccatcac 360
   tgatttcatc aatatcctgc accgctacta taaatcagcc ttggtacaga tctatgagct 420
   agaagaacac aagatagaaa cttggagaga ggtgtatctc caggactcct ttaaaccgct 480
   tgtctgcatt tctcctaatg ccagcttgtt tgatgctgtc tcttcattaa ttcggaacaa 540
   gatccacagg ctgccagtta ttgacccaga atcaggcaat actttgtaca tcctcaccca 600
   caagcgcatt ctgaagttcc tcaaattgtt tatcactgag ttccccaagc cagagttcat 660
   gtccaagtct ctggaagagc tacagattgg cacctatgcc aatattgcta tggttcgcac 720
   taccaccccc gtctatgtgg ctctggggat ttttgtacag catcgagtct cagccctgcc 780
   agtggtggat gagaaggggc gtgtggtgga catctactcc aagtttgatg ttatcaatct 840
   ggcagcagaa aagacctaca acaacctaga tgtatctgtg actaaagcct tgcaacatcg 900
   atcacattac tttgagggtg ttctcaagtg ctacctgcat gagactctgg agaccatcat 960
   caacaggcta gtggaagcag aggttcaccg acttgtagtg gtggatgaaa atgatgtggt 1020
   caagggaatt gtatcactgt ctgacatcct gcaggccctg gtgctcacag gtggagagaa 1080
   gaagccctga gctgggggaa ggggtcatgc agcaccaggg gatatgccca actcactgcc 1140
   tgctggaagc tctgtgggaa tcagatgaaa cttgagggaa ttgtgactct gttccctgtt 1200
   cagggtcccc tgcccttcta tctgggagct agggaaggta tgggggagga aagagaatgg 1260
   atttatagct acccttaccc tcacacatac acttgaaaaa actttcagcc tagccagttc 1320
   tagcccctgt cctcttagat atatccccct ttctgggtga actataggct ctgtgcctct 1380
   cagacaaatt ctgatctcta agagatcccc agacctcact tgcctctgcc tccatcttgg 1440
   ccctgattca accctaagat aatagcacaa caaaattctt cataaagata tttttattca 1500
   cctgttccgt gctatatgga ggaggccaag tccatttagt gacatttctt cccataatgt 1560
   gagtggggag gattgtgg 1578
<210> 16
   <211> 331
   <212> PRT
   <213> Homo sapiens
<400> 16
   Met Glu Thr Val Ile Ser Ser Asp Ser Ser Pro Ala Val Glu Asn Glu
   1 5 10 15
   His Pro Gln Glu Thr Pro Glu Ser Asn Asn Ser Val Tyr Thr Ser Phe
   20 25 30
   Met Lys Ser His Arg Cys Tyr Asp Leu Ile Pro Thr Ser Ser Lys Leu
   35 40 45
   Val Val Phe Asp Thr Ser Leu Gln Val Lys Lys Ala Phe Phe Ala Leu
   50 55 60
   Val Thr Asn Gly Val Arg Ala Ala Pro Leu Trp Asp Ser Lys Lys Gln
   65 70 75 80
   Ser Phe Val Gly Met Leu Thr Ile Thr Asp Phe Ile Asn Ile Leu His
   85 90 95
   Arg Tyr Tyr Lys Ser Ala Leu Val Gln Ile Tyr Glu Leu Glu Glu His
   100 105 110
   Lys Ile Glu Thr Trp Arg Glu Val Tyr Leu Gln Asp Ser Phe Lys Pro
   115 120 125
   Leu Val Cys Ile Ser Pro Asn Ala Ser Leu Phe Asp Ala Val Ser Ser
   130 135 140
   Leu Ile Arg Asn Lys Ile His Arg Leu Pro Val Ile Asp Pro Glu Ser
   145 150 155 160
   Gly Asn Thr Leu Tyr Ile Leu Thr His Lys Arg Ile Leu Lys Phe Leu
   165 170 175
   Lys Leu Phe Ile Thr Glu Phe Pro Lys Pro Glu Phe Met Ser Lys Ser
   180 185 190
   Leu Glu Glu Leu Gln Ile Gly Thr Tyr Ala Asn Ile Ala Met Val Arg
   195 200 205
   Thr Thr Thr Pro Val Tyr Val Ala Leu Gly Ile Phe Val Gln His Arg
   210 215 220
   Val Ser Ala Leu Pro Val Val Asp Glu Lys Gly Arg Val Val Asp Ile
   225 230 235 240
   Tyr Ser Lys Phe Asp Val Ile Asn Leu Ala Ala Glu Lys Thr Tyr Asn
   245 250 255
   Asn Leu Asp Val Ser Val Thr Lys Ala Leu Gln His Arg Ser His Tyr
   260 265 270
   Phe Glu Gly Val Leu Lys Cys Tyr Leu His Glu Thr Leu Glu Thr Ile
   275 280 285
   Ile Asn Arg Leu Val Glu Ala Glu Val His Arg Leu Val Val Val Asp
   290 295 300
   Glu Asn Asp Val Val Lys Gly Ile Val Ser Leu Ser Asp Ile Leu Gln
   305 310 315 320
   Ala Leu Val Leu Thr Gly Gly Glu Lys Lys Pro
   325 330
<210> 17
   <211> 6989
   <212> DNA
   <213> Homo sapiens
<400> 17
   aagcgggggc ggcagcggtg gtagcagctg gtacctcccg ccgcctctgt tcggagggtc 60
   gcggggcacc gaggtgcttt ccggccgccc tctggtcggc cacccaaagc cgcgggcgct 120
   gatgatgggt gaggaggggg cggcaagatt tcgggcgccc ctgccctgaa cgccctcagc 180
   tgctgccgcc ggggccgctc cagtgcctgc gaactctgag gagccgaggc gccggtgaga 240
   gcaaggacgc tgcaaacttg cgcagcgcgg gggctgggat tcacgcccag aagttcagca 300
   ggcagacagt ccgaagcctt cccgcagcgg agagatagct tgagggtgcg caagacggca 360
   gcctccgccc tcggttcccg cccagaccgg gcagaagagc ttggaggagc cacaaggaac 420
   gcaaaaggcg gccaggacag cgtgcagcag ctgggagccg ccgttctcag ccttaaaagt 480
   tgcagagatt ggaggctgcc ccgagagggg acagacccca gctccgactg cggggggcag 540
   gagaggacgg tacccaactg ccacctccct tcaaccatag tagttcctct gtaccgagcg 600
   cagcgagcta cagacggggg cgcggcactc ggcgcggaga gcgggaggct caaggtccca 660
   gccagtgagc ccagtgtgct tgagtgtctc tggactcgcc cctgagcttc caggtctgtt 720
   tcatttagac tcctgctcgc ctccgtgcag ttgggggaaa gcaagagact tgcgcgcacg 780
   cacagtcctc tggagatcag gtggaaggag ccgctgggta ccaaggactg ttcagagcct 840
   cttcccatct cggggagagc gaagggtgag gctgggcccg gagagcagtg taaacggcct 900
   cctccggcgg gatgggagcc atcgggctcc tgtggctgct gccgctgctg ctttccacgg 960
   cagctgtggg ctccgggatg gggaccggcc agcgcgcggg ctccccagct gcggggccgc 1020
   cgctgcagcc ccgggagcca ctcagctact cgcgcctgca gaggaagagt ctggcagttg 1080
   acttcgtggt gccctcgctc ttccgtgtct acgcccggga cctactgctg ccaccatcct 1140
   cctcggagct gaaggctggc aggcccgagg cccgcggctc gctagctctg gactgcgccc 1200
   cgctgctcag gttgctgggg ccggcgccgg gggtctcctg gaccgccggt tcaccagccc 1260
   cggcagaggc ccggacgctg tccagggtgc tgaagggcgg ctccgtgcgc aagctccggc 1320
   gtgccaagca gttggtgctg gagctgggcg aggaggcgat cttggagggt tgcgtcgggc 1380
   cccccgggga ggcggctgtg gggctgctcc agttcaatct cagcgagctg ttcagttggt 1440
   ggattcgcca aggcgaaggg cgactgagga tccgcctgat gcccgagaag aaggcgtcgg 1500
   aagtgggcag agagggaagg ctgtccgcgg caattcgcgc ctcccagccc cgccttctct 1560
   tccagatctt cgggactggt catagctcct tggaatcacc aacaaacatg ccttctcctt 1620
   ctcctgatta ttttacatgg aatctcacct ggataatgaa agactccttc cctttcctgt 1680
   ctcatcgcag ccgatatggt ctggagtgca gctttgactt cccctgtgag ctggagtatt 1740
   cccctccact gcatgacctc aggaaccaga gctggtcctg gcgccgcatc ccctccgagg 1800
   aggcctccca gatggacttg ctggatgggc ctggggcaga gcgttctaag gagatgccca 1860
   gaggctcctt tctccttctc aacacctcag ctgactccaa gcacaccatc ctgagtccgt 1920
   ggatgaggag cagcagtgag cactgcacac tggccgtctc ggtgcacagg cacctgcagc 1980
   cctctggaag gtacattgcc cagctgctgc cccacaacga ggctgcaaga gagatcctcc 2040
   tgatgcccac tccagggaag catggttgga cagtgctcca gggaagaatc gggcgtccag 2100
   acaacccatt tcgagtggcc ctggaataca tctccagtgg aaaccgcagc ttgtctgcag 2160
   tggacttctt tgccctgaag aactgcagtg aaggaacatc cccaggctcc aagatggccc 2220
   tgcagagctc cttcacttgt tggaatggga cagtcctcca gcttgggcag gcctgtgact 2280
   tccaccagga ctgtgcccag ggagaagatg agagccagat gtgccggaaa ctgcctgtgg 2340
   gtttttactg caactttgaa gatggcttct gtggctggac ccaaggcaca ctgtcacccc 2400
   acactcctca gtggcaggtc aggaccctaa aggatgcccg gttccaggac caccaagacc 2460
   atgctctatt gctcagtacc actgatgtcc ccgcttctga aagtgctaca gtgaccagtg 2520
   ctacgtttcc tgcaccgatc aagagctctc catgtgagct ccgaatgtcc tggctcattc 2580
   gtggagtctt gaggggaaac gtgtccttgg tgctagtgga gaacaaaacc gggaaggagc 2640
   aaggcaggat ggtctggcat gtcgccgcct atgaaggctt gagcctgtgg cagtggatgg 2700
   tgttgcctct cctcgatgtg tctgacaggt tctggctgca gatggtcgca tggtggggac 2760
   aaggatccag agccatcgtg gcttttgaca atatctccat cagcctggac tgctacctca 2820
   ccattagcgg agaggacaag atcctgcaga atacagcacc caaatcaaga aacctgtttg 2880
   agagaaaccc aaacaaggag ctgaaacccg gggaaaattc accaagacag acccccatct 2940
   ttgaccctac agttcattgg ctgttcacca catgtggggc cagcgggccc catggcccca 3000
   cccaggcaca gtgcaacaac gcctaccaga actccaacct gagcgtggag gtggggagcg 3060
   agggccccct gaaaggcatc cagatctgga aggtgccagc caccgacacc tacagcatct 3120
   cgggctacgg agctgctggc gggaaaggcg ggaagaacac catgatgcgg tcccacggcg 3180
   tgtctgtgct gggcatcttc aacctggaga aggatgacat gctgtacatc ctggttgggc 3240
   agcagggaga ggacgcctgc cccagtacaa accagttaat ccagaaagtc tgcattggag 3300
   agaacaatgt gatagaagaa gaaatccgtg tgaacagaag cgtgcatgag tgggcaggag 3360
   gcggaggagg agggggtgga gccacctacg tatttaagat gaaggatgga gtgccggtgc 3420
   ccctgatcat tgcagccgga ggtggtggca gggcctacgg ggccaagaca gacacgttcc 3480
   acccagagag actggagaat aactcctcgg ttctagggct aaacggcaat tccggagccg 3540
   caggtggtgg aggtggctgg aatgataaca cttccttgct ctgggccgga aaatctttgc 3600
   aggagggtgc caccggagga cattcctgcc cccaggccat gaagaagtgg gggtgggaga 3660
   caagaggggg tttcggaggg ggtggagggg ggtgctcctc aggtggagga ggcggaggat 3720
   atataggcgg caatgcagcc tcaaacaatg accccgaaat ggatggggaa gatggggttt 3780
   ccttcatcag tccactgggc atcctgtaca ccccagcttt aaaagtgatg gaaggccacg 3840
   gggaagtgaa tattaagcat tatctaaact gcagtcactg tgaggtagac gaatgtcaca 3900
   tggaccctga aagccacaag gtcatctgct tctgtgacca cgggacggtg ctggctgagg 3960
   atggcgtctc ctgcattgtg tcacccaccc cggagccaca cctgccactc tcgctgatcc 4020
   tctctgtggt gacctctgcc ctcgtggccg ccctggtcct ggctttctcc ggcatcatga 4080
   ttgtgtaccg ccggaagcac caggagctgc aagccatgca gatggagctg cagagccctg 4140
   agtacaagct gagcaagctc cgcacctcga ccatcatgac cgactacaac cccaactact 4200
   gctttgctgg caagacctcc tccatcagtg acctgaagga ggtgccgcgg aaaaacatca 4260
   ccctcattcg gggtctgggc catggcgcct ttggggaggt gtatgaaggc caggtgtccg 4320
   gaatgcccaa cgacccaagc cccctgcaag tggctgtgaa gacgctgcct gaagtgtgct 4380
   ctgaacagga cgaactggat ttcctcatgg aagccctgat catcagcaaa ttcaaccacc 4440
   agaacattgt tcgctgcatt ggggtgagcc tgcaatccct gccccggttc atcctgctgg 4500
   agctcatggc ggggggagac ctcaagtcct tcctccgaga gacccgccct cgcccgagcc 4560
   agccctcctc cctggccatg ctggaccttc tgcacgtggc tcgggacatt gcctgtggct 4620
   gtcagtattt ggaggaaaac cacttcatcc accgagacat tgctgccaga aactgcctct 4680
   tgacctgtcc aggccctgga agagtggcca agattggaga cttcgggatg gcccgagaca 4740
   tctacagggc gagctactat agaaagggag gctgtgccat gctgccagtt aagtggatgc 4800
   ccccagaggc cttcatggaa ggaatattca cttctaaaac agacacatgg tcctttggag 4860
   tgctgctatg ggaaatcttt tctcttggat atatgccata ccccagcaaa agcaaccagg 4920
   aagttctgga gtttgtcacc agtggaggcc ggatggaccc acccaagaac tgccctgggc 4980
   ctgtataccg gataatgact cagtgctggc aacatcagcc tgaagacagg cccaactttg 5040
   ccatcatttt ggagaggatt gaatactgca cccaggaccc ggatgtaatc aacaccgctt 5100
   tgccgataga atatggtcca cttgtggaag aggaagagaa agtgcctgtg aggcccaagg 5160
   accctgaggg ggttcctcct ctcctggtct ctcaacaggc aaaacgggag gaggagcgca 5220
   gcccagctgc cccaccacct ctgcctacca cctcctctgg caaggctgca aagaaaccca 5280
   cagctgcaga ggtctctgtt cgagtcccta gagggccggc cgtggaaggg ggacacgtga 5340
   atatggcatt ctctcagtcc aaccctcctt cggagttgca cagggtccac ggatccagaa 5400
   ataagcccac cagcttgtgg aacccaacgt acggctcctg gtttacagag aaacccacca 5460
   aaaagaataa tcctatagca aagaaggagc cacacgagag gggtaacctg gggctggagg 5520
   gaagctgtac tgtcccacct aacgttgcaa ctgggagact tccgggggcc tcactgctcc 5580
   tagagccctc ttcgctgact gccaatatga aggaggtacc tctgttcagg ctacgtcact 5640
   tcccttgtgg gaatgtcaat tacggctacc agcaacaggg cttgccctta gaagccgcta 5700
   ctgcccctgg agctggtcat tacgaggata ccattctgaa aagcaagaat agcatgaacc 5760
   agcctgggcc ctgagctcgg tcgcacactc acttctcttc cttgggatcc ctaagaccgt 5820
   ggaggagaga gaggcaatca atggctcctt tcacaaacca gagaccaaat gtcacgtttt    5880
   gttttgtgcc aacctatttt gaagtaccac caaaaaagct gtattttgaa aatgctttag    5940
   aaaggttttg agcatgggtt catcctattc tttcgaaaga agaaaatatc ataaaaatga    6000
   gtgataaata caaggccaga tgtggttgca taaggttttt atgcatgttt gttgtactct    6060
   gttcgagtcc ctagagggcc ggccgtggaa gggggacacg tgaatatggc attctctcag    6120
   tccaaccctc cttcggagtt gcacaaggtc cacggatcca gaaacaagcc caccagcttg    6180
   tggaacccaa cgtacggctc ctggtttaca gagaaaccca ccaaaaagaa taatcctata    6240
   gcaaagaagg agccacacga caggggtaac ctggggctgg agggaagctg tactgtccca    6300
   cctaacgttg caactgggag acttccgggg gcctcactgc tcctagagcc ctcttcgctg    6360
   actgccaata tgaaggaggt acctctgttc aggctacgtc acttcccttg tgggaatgtc    6420
   aattacggct accagcaaca gggcttgccc ttagaagccg ctactgcccc tggagctggt    6480
   cattacgagg ataccattct gaaaagcaag aatagcatga accagcctgg gccctgagct    6540
   cggtcgcaca ctcacttctc ttccttggga tccctaagac cgtggaggag agagaggcaa    6600
   tggctccttc acaaaccaga gaccaaatgt cacgttttgt tttgtgccaa cctattttga    6660
   agtaccacca aaaaagctgt attttgaaaa tgctttagaa aggttttgag catgggttca    6720
   tcctattctt tcgaaagaag aaaatatcat aaaaatgagt gataaataca aggcccagat    6780
   gtggttgcat aaggttttta tgcatgtttg ttgtatactt ccttatgctt cttttaaatt    6840
   gtgtgtgctc tgcttcaatg tagtcagaat tagctgcttc tatgtttcat agttggggtc    6900
   atagatgttt ccttgccttg ttgatgtgga catgagccat ttgaggggag agggaacgga    6960
   aataaaggag ttatttgtaa tgactaaaa                                      6989
<210> 18
   <211> 1620
   <212> PRT
   <213> Homo sapiens
<400> 18
<210> 19
   <211> 5182
   <212> DNA
   <213> Homo sapiens
<400> 19
   accttcagtt cctccaagtc ctggaagact gagacaaggg gaagaaagca agataagtgc 60
   gatggatgca tctccacgga atatttctcc aggacttcag aatggagaaa aagaggatcg 120
   cttcttaaca actttgtcca gccagagctc caccagttct actcatctcc aattgcctac 180
   gccacctgaa gtcatgtctg aacagtcagt gggagggccc cctgagctag atacagccag 240
   cagttccgaa gatgtgtttg atgggcattt gctgggatcc acagacagcc aagtgaagga 300
   aaagtcaacc atgaaagcca tctttgcaaa tttgcttcca ggaaatagct ataatcctat 360
   tccatttcct tttgatccag ataaacacta cttaatgtat gaacatgaac gagtgcccat 420
   tgcagtctgc gagaaggaac ccagctccat cattgctttt gctctcagtt gtaaagaata 480
   ccgaaatgcc ttagaggaat tgtctaaagc gactcagtgg aacagtgccg aagaagggct 540
   tccaacaaat agtacttcag atagcagacc aaagagtagc agccctatca gattacctga 600
   aatgagtgga ggacagacaa atcgtacaac agaaacagaa ccacaaccaa ccaaaaaggc 660
   ttctggaatg ttgtccttct tcagagggac agcagggaaa agccccgatc tctcttccca 720
   gaagagagag accttacgtg gagcagatag tgcttactac caggttgggc agacaggcaa 780
   ggaggggacc gagaatcaag gcgttgagcc tcaagatgaa gtagatggag gagatacgca 840
   aaagaagcaa ctcataaatc ctcatgtgga acttcaattt tcagatgcta atgccaagtt 900
   ttactgtcgg ctctactatg cgggagagtt tcataagatg cgtgaagtga ttctggacag 960
   cagtgaggaa gatttcattc gttccctctc ccactcatca ccctggcagg cccggggagg 1020
   caaatcagga gctgccttct atgcaactga ggatgataga tttattttga agcaaatgcc 1080
   tcgtctggaa gtccagtcct tcctcgactt tgcaccacat tacttcaatt atattacaaa 1140
   tgctgttcaa caaaagaggc ccacggcgtt ggccaaaatt cttggagttt acagaattgg 1200
   ttataagaac tctcagaaca acactgagaa gaagttagat ctccttgtca tggaaaatct 1260
   tttctacggg agaaagatgg cacaggtttt tgatttgaag ggctctctta ggaatcggaa 1320
   tgtaaaaact gacactggaa aagagagttg tgatgtggtc ctgctagatg aaaatctcct 1380
   aaagatggtt cgagacaacc ctctatatat tcgttctcat tccaaagctg tgctgagaac 1440
   ctcgatccat agtgactccc atttcctttc tagccacctc attatagatt attctttgct 1500
   ggttgggcga gatgatacta gcaatgagct agtagttgga attatagatt atattcgaac 1560
   atttacatgg gacaaaaagc ttgagatggt tgtgaaatca acaggaattt taggtggaca 1620
   aggtaaaatg ccaacagtgg tgtctccgga gttgtacagg actaggtttt gtgaggcaat 1680
   ggacaagtat ttcctaatgg taccagacca ctggacaggc ttgggtctga attgctgaaa 1740
   tcaagcacat attttgaaat ggactgtgaa ggaaaagggg acaggaacaa aggaccaaaa 1800
   ataagctaca tgttttattt cttcatcgtg ttcaccactg tatgccaagg cttttcagtt 1860
   ctgtgtctgt ttagactgtc cgtaatggaa tggtaaaact ccatgaattt gcactttggt 1920
   ttttgatacc tgtggagctg tctgtacgtt gggaagtggc atgaaaattt tcttaagcta 1980
   aaatacagac atgtttcaaa gggctaaagt tggagatgag tagatagggt gaaaaatggg 2040
   ttaaatttgc tagcttaatt gttttaagaa gaaaacagtg tctcataaat tgactatcct 2100
   ggcatcacat ttaacatgtt atctacttag aaagcatttg tagagctgct gaatttgttt 2160
   tgtgtttttc tgtaataatt taatgttact tattatcaga atttctgaaa cctttacaaa 2220
   aattctgatt tattccatta atggccagtt aaacacgtgg gcatttattg ttttattgag 2280
   gaatttgact taaactggga atcctgtcat gttgtttatc tttccagctt gcctgttttt 2340
   gagtatgttt gatgttttta aaattttgtc ttctctgtgg atgacaggag gctacagcaa 2400
   ttaactttaa gcctcctttt agagatattt ttaaagcttg tttaaaattt ttgtgcaatt 2460
   catatattaa attgcactta cttgcatacg ctcatattct agggtttttt ctctattttt 2520
   agggtatcat agtaaatcat tagtaaatga gtctgtagtt actaaaccct aatggaataa 2580
   ttattaatga aagatttttg aaatataaaa aataaattag gcccaatcca agaaattgag 2640
   tgagaaggaa acacttgttt tattcacaga ggtaaagtgt cttttcaata taaccagcaa 2700
   tttaggtggc atctataaaa taaaaaattt ctactgtgga catccccttt tccaactttc 2760
   tacataatgg ctagttctga ctactaagaa atgttaagaa ataggccaag tgcggtggct 2820
   cacgcctata atcctatcac tttgggaggc ctaggcaggc agatcacctg aggtcaggag 2880
   ttcaagacca gcctggccaa catgaggaaa ccccatctct actaaaaaga caaaaaatta 2940
   gctgggcatg gtggcatata cctgtaaccc caactacttg ggtggctgag gcaggagaat 3000
   tgtttgaacc tgggaggcgg aggctgcagt gagctgggat tacaccattg cattccagcc 3060
   tgggcaacag agcaagactg tctcaaaaaa caaaacgaaa caaaaaaaga aagttattct 3120
   tagtaaggaa cttcttgttt aatagcattt ttgtttattt taaaaagtga tcagaagtag 3180
   taaactatct ttgaggaaat actgtaaccc cagaatattt cctcttgact tctttttgta 3240
   acaaggataa tttagggatt tataaagttg taaggatttc actgttttcg gactgcctat 3300
   aataatagca cattaacctt cacataataa gaaatctgga caagttcagt tacacagtat 3360
   gatgaatact tgaattagga acattgtgga aaatttgctt tagagaatca aggcagtagt 3420
   ttggtatttg gtgcttatta aaaatgtggt ttgttttgaa ctggaagcaa gttgaccaag 3480
   gacttatgac taatgtgatg ctaagttcca cttggcccct tttaaaaacg tgtatgtgcc 3540
   ttttgaagat acacaaaaca ctgaggattt tagttttgaa atcaaagact attaaaggag 3600
   ctgtacagag gtaaaaaaat aaatgtggaa cattattaac ttattttgtg tctaggaaca 3660
   atggattttg tatctgattt aaaatgccaa cactgttttg tctctgttca ttttttctgt 3720
   gaggatactt aaggttatta ttcctgtctg tttcctgtac tcccctagtc atgagcactt 3780
   gaagtacaag gtgtctcccc ctaggtgcaa ttaggttgtt tctttgtttt tagtttcaat 3840
   tctatgtgca tagcaggaat gctccacagg aatggcttct gacaataatc tgtcctgttg 3900
   attttgtttt ccttgcccat gacttgaaca actgtgtttt aaagtactgt agtctagtag 3960
   gtaactttgt ggcaaaaatt ttcaatataa tacattctga aacaatagtt gctgccttgc 4020
   aaaggtaatc tctcatttta aaattggaca gtattaatga aggggaaata tacaatttat 4080
   ttctattgag tggtagaact atatgtctgg tcccttgctg ctcttgttta ggccactatc 4140
   atagatatat ttcaaatatt gtactactca gtgttaagta ttgaatgact gtttcccttt 4200
   ccttcaaggc ctagagtata ttctgaaaat ttaggaatga ggaagaaatc ttaatacttc 4260
   cttccttaac atacaacatg agtcccgaga ataattgata gtagcaagag aaaactatgt 4320
   cagtaacatg ttgctttgta taaaaatctt atttataaat gtgaagcttt ttgatgccat 4380
   caaaacttat taaaaaatag gatttacttt tttctaattc tgacctaaga aaaataatga 4440
   gaacaagctg ttgcaagctc ttttgtagtc tattgaatat tttatagata ttcaaaattt 4500
   cctacaaact ataatttttt ccatgattta gcagtgagtg attttctagc tttggctctt 4560
   attaggtatt gtaaatagta gggttatatc gatatcagct tttgtgatgg cattgtggtc 4620
   atcagcttca tgacatttta cccatttgca gtgatcctgt gtaaaactgc caaggaaagt 4680
   aattacctgt aggagtttgc tgagcttgaa gagtgaaaac tgttgtgaat gagcctgatc 4740
   ataaaacgga ccaggccatt cattattcct caagtgttaa tatactgact tatgcagtat 4800
   tcaaaccatc tagtgcaatg tttttgtttt tgtttttttt ttttttggta acacaggtgc 4860
   agtgtattat agaaaaaata aaaactacaa tcattagcag ttttaatact gctgtgtcag 4920
   ttttgtaaaa aatgtacatt atgtcttttg acatgttgaa ttttaaacta gggaaatgac 4980
   attgtaaatc atagtagcct cttttaattt aatatgaaaa atgccactat attgaaagta 5040
   cttaatgtat tgtatatatt tctctacttt ggttctagct attttatatg attgacatgt 5100
   tatttaaaag ataactgcct tgaacttttg gagacttgta ctgtaaataa agaaatctta 5160
   acaataaact cagaatctac tt 5182
<210> 20
   <211> 578
   <212> PRT
   <213> Homo sapiens
<400> 20
   Pro Ser Val Pro Pro Ser Pro Gly Arg Leu Arg Gln Gly Glu Glu Ser
   1 5 10 15
   Lys Ile Ser Ala Met Asp Ala Ser Pro Arg Asn Ile Ser Pro Gly Leu
   20 25 30
   Gln Asn Gly Glu Lys Glu Asp Arg Phe Leu Thr Thr Leu Ser Ser Gln
   35 40 45
   Ser Ser Thr Ser Ser Thr His Leu Gln Leu Pro Thr Pro Pro Glu Val
   50 55 60
   Met Ser Glu Gln Ser Val Gly Gly Pro Pro Glu Leu Asp Thr Ala Ser
   65 70 75 80
   Ser Ser Glu Asp Val Phe Asp Gly His Leu Leu Gly Ser Thr Asp Ser
   85 90 95
   Gln Val Lys Glu Lys Ser Thr Met Lys Ala Ile Phe Ala Asn Leu Leu
   100 105 110
   Pro Gly Asn Ser Tyr Asn Pro Ile Pro Phe Pro Phe Asp Pro Asp Lys
   115 120 125
   His Tyr Leu Met Tyr Glu His Glu Arg Val Pro Ile Ala Val Cys Glu
   130 135 140
   Lys Glu Pro Ser Ser Ile Ile Ala Phe Ala Leu Ser Cys Lys Glu Tyr
   145 150 155 160
   Arg Asn Ala Leu Glu Glu Leu Ser Lys Ala Thr Gln Trp Asn Ser Ala
   165 170 175
   Glu Glu Gly Leu Pro Thr Asn Ser Thr Ser Asp Ser Arg Pro Lys Ser
   180 185 190
   Ser Ser Pro Ile Arg Leu Pro Glu Met Ser Gly Gly Gln Thr Asn Arg
   195 200 205
   Thr Thr Glu Thr Glu Pro Gln Pro Thr Lys Lys Ala Ser Gly Met Leu
   210 215 220
   Ser Phe Phe Arg Gly Thr Ala Gly Lys Ser Pro Asp Leu Ser Ser Gln
   225 230 235 240
   Lys Arg Glu Thr Leu Arg Gly Ala Asp Ser Ala Tyr Tyr Gln Val Gly
   245 250 255
   Gln Thr Gly Lys Glu Gly Thr Glu Asn Gln Gly Val Glu Pro Gln Asp
   260 265 270
   Glu Val Asp Gly Gly Asp Thr Gln Lys Lys Gln Leu Ile Asn Pro His
   275 280 285
   Val Glu Leu Gln Phe Ser Asp Ala Asn Ala Lys Phe Tyr Cys Arg Leu
   290 295 300
   Tyr Tyr Ala Gly Glu Phe His Lys Met Arg Glu Val Ile Leu Asp Ser
   305 310 315 320
   Ser Glu Glu Asp Phe Ile Arg Ser Leu Ser His Ser Ser Pro Trp Gln
   325 330 335
   Ala Arg Gly Gly Lys Ser Gly Ala Ala Phe Tyr Ala Thr Glu Asp Asp
   340 345 350
   Arg Phe Ile Leu Lys Gln Met Pro Arg Leu Glu Val Gln Ser Phe Leu
   355 360 365
   Asp Phe Ala Pro His Tyr Phe Asn Tyr Ile Thr Asn Ala Val Gln Gln
       370                 375                 380
   Lys Arg Pro Thr Ala Leu Ala Lys Ile Leu Gly Val Tyr Arg Ile Gly
   385                 390                 395                 400
   Tyr Lys Asn Ser Gln Asn Asn Thr Glu Lys Lys Leu Asp Leu Leu Val
                   405                 410                 415
   Met Glu Asn Leu Phe Tyr Gly Arg Lys Met Ala Gln Val Phe Asp Leu
               420                 425                 430
   Lys Gly Ser Leu Arg Asn Arg Asn Val Lys Thr Asp Thr Gly Lys Glu
           435                 440                 445
   Ser Cys Asp Val Val Leu Leu Asp Glu Asn Leu Leu Lys Met Val Arg
       450                 455                 460
   Asp Asn Pro Leu Tyr Ile Arg Ser His Ser Lys Ala Val Leu Arg Thr
   465                 470                 475                 480
   Ser Ile His Ser Asp Ser His Phe Leu Ser Ser His Leu Ile Ile Asp
                   485                 490                 495
   Tyr Ser Leu Leu Val Gly Arg Asp Asp Thr Ser Asn Glu Leu Val Val
               500                 505                 510
   Gly Ile Ile Asp Tyr Ile Arg Thr Phe Thr Trp Asp Lys Lys Leu Glu
           515                 520                 525
   Met Val Val Lys Ser Thr Gly Ile Leu Gly Gly Gln Gly Lys Met Pro
       530                 535                 540
   Thr Val Val Ser Pro Glu Leu Tyr Arg Thr Arg Phe Cys Glu Ala Met
   545                 550                 555                 560
   Asp Lys Tyr Phe Leu Met Val Pro Asp His Trp Thr Gly Leu Gly Leu
                   565                 570                 575
   Asn Cys
<210> 21
   <211> 6297
   <212> DNA
   <213> Mus musculus
<400> 21
   gctgggcttc tcggcgcgac cgccggctgc ctgagaaagc cggcctgtgc ttggcctcgg 60
   cttcccggcc gagagtggtg aggcggaggc gggaggcggg aggccccggg ggcggggcgg 120
   gaggcgaaga ctcatgaaat ggccacagat gacaagagtt ccccgacact ggactctgct 180
   aatgatttgc ctcgctctcc tgccagtcct tctcacctca ctcactttaa acccttgact 240
   cctgaccagg atgagccccc cttcaagtca gcatatagtt cttttgtaaa cctttttcgt 300
   tttaacaaag agcgaggaga agggggccaa ggagagcagc agtctccgag ttcaagttgg 360
   gccagccctc agatcccttc aagaacacag tctgtgaggt cgcctgtacc ttataaaaaa 420
   cagcttaatg aggagctcca ccggcgctct tcagtgttag agaacacttt gccacatcct 480
   caggagagca cagactccag aaggaaagca gaaccagcct gtggaggtca tgacccacgt 540
   acagctgttc agcttcgaag cctcagcaca gtattgaaac gcctcaaaga aatcatggaa 600
   ggaaaaagcc aggacagtga cctgaagcaa tattggatgc cagatagcca gtgtaaagag 660
   tgctatgact gcagtgaaaa gtttacaaca tttaggcgca gacaccattg cagactgtgt 720
   gggcagattt tctgcagtcg ttgttgtaat caagaaatcc ctggaaaatt tatgggctat 780
   acaggagacc tccgagcatg cacctactgt agaaaaatag ccttaagtta tgctcattct 840
   acagacagta attccattgg ggaagacttg aatgctcttt cagattcaac ttgctctgtg 900
   tctatacttg atccaagcga acctcggaca ccagttggga gtagaaaagc cagtcgtaac 960
   atattcttag aggatgattt agcctggcaa agcttgattc atccggattc ctcaaatagt 1020
   gctctttcaa caagactcgt atctgttcaa gaggatgctg ggaagtctcc tgctcgaaac 1080
   agatcagcca gcattactaa tctgtcactg gatcggtctg gttctcctat ggttccttca 1140
   tatgagacat ctgtcagtcc ccaggctaac cgaaactaca ttaggacaga gacgactgag 1200
   gatgaacgca aaattcttct ggacagtgct cagttaaagg atctgtggaa gaaaatctgc 1260
   catcacacca gtgggatgga atttcaagat caccgttact ggttgagaac acatcccaac 1320
   tgcattgtag ggaaggaatt agtcaactgg ctaatcagaa atggacacat cgctacaagg 1380
   gcacaagcta tagcaattgg acaagcaatg gttgatggac gttggttgga ttgtgttagt 1440
   catcatgatc agcttttcag ggacgaatat gcgttgtata gaccacttca gagtacagaa 1500
   ttttctgaga caccttctcc agacagtgac tctgtgaact ctgtggaagg acactccgag 1560
   ccatcctggt ttaaagacat aaaatttgat gacagtgaca cagaacagat tgctgaagaa 1620
   ggtgacgata atttggctaa gtatttggtt tctgacactg gaggacagca gctctcaata 1680
   agtgatgcct tcatcaaaga gtccttattt aatcgacgag tagaggaaaa atccaaagag 1740
   ctgcctttta cccctttggg ctggcatcat aacaacctgg aactcttgcg agaggagaat 1800
   gaggagaagc aagccatgga aaggctgctt tcagctaatc ataaccacat gatggcccta 1860
   ctccagcagt tgcttcaaaa cgagtcattg tcatcgtctt ggagggacat cattgtgtca 1920
   ctagtctgcc aggttgttca gacagtccga cctgatgtca agcaccagga tgatgacatg 1980
   gatatccgtc agtttgtcca tatcaagaag atcccaggtg gaaagaaatt tgactctgtg 2040
   gttgtcaatg gctttgtttg taccaagaac attgcacata aaaagatgaa ttcctgtatt 2100
   aaaaacccca aaatccttct gttgaagtgt tctattgagt atctctatag agaagaaact 2160
   aagtttacct gcattgatcc tattgtgctt caggaaaggg aattcttgaa gaattatgtt 2220
   caacgaatag ttgatgttcg acccacattg gttcttgttg agaaaacagt gtctcggatt 2280
   gctcaggaca tgttactgga acatggcatt actctggtca ttaatgtaaa gtcacaagtt 2340
   ttagaaagaa tcagtcgaat gacccaaggt gatttagtgg tgtccatgga ccagctgctc 2400
   accaaacccc acttgggcac ttgccacaaa ttttatatgc agatatttca gctgcctaat 2460
   gaacaaacca aaacactgat gttttttgaa ggttgtccac agcatctagg ctgcacaatc 2520
   aagctcagag gaggctctga ttatgagctg gctcgagtta aggagatcct aatatttatg 2580
   atctgtgtag cttatcattc tcagctagaa atctcttttc tcatggatga gttcgctatg 2640
   cctccaacat taatgcaaag cccttcattc catcttctga cggagggacg aggtgaagag 2700
   ggagcctctc aggagcaggt cagtggcagc tcccttcctc aggatcctga gtgccctcgt 2760
   gaggccctgt cttctgagga tagcactttg ttggaatcaa ggactgtgct agagaagggt 2820
   gaactagaca ataaaagtat tccacaagct gttgcctctt tgaagcatca agattatacc 2880
   acccccactt gcccagcagg tattccctgt gctctttttg cattggtacc agagtcattg 2940
   ttgcctctcc atatggatca acaggatgcc gtaggaaatg aacaccgaga gacttcacag 3000
   caaacggatg agcaacagga tcccaaaagc cagatgaaag cttttagaga ccctttacag 3060
   gatgacactg gaatgtacgt tactgaggaa gtcacctcct ctgaagatca acgaaagact 3120
   tatgccttga catttaaaca ggagttaaaa gatgtaatcc tctgtatctc tccagttatt 3180
   acattccgtg aacctttcct tttaactgaa aaggggatga gatgctcaac tcgagattat 3240
   tttccagagc agatttactg gtctcctctt ctcaacaaag aggtgaagga aatggagagc 3300
   aggaggaaga aacagctgct cagggatctc tctggacttc agggcatgaa tggcagtgtt 3360
   caggccaagt ctattcaagt cttaccctca catgagctag tgagcaccag gattgctgaa 3420
   catgtgggtg acagccagac cttgggtaga atgctagctg attatcgagc tagaggagga 3480
   gaattcagtc aaaacatttg gaaccccttt gtccattcaa aagatgacat catgtacttc 3540
   aggtggcaaa tcagggaaac aaaactgaga gtgatgaaag agaggggatt gattccaagt 3600
   gatgtaatat ggccaacaaa ggtggactgt ctgaaccctg ctaaccacca gaggctctgt 3660
   gtgctcttca gcagctcttc tgcccagtcc agcaatgctc ccagtgcttg tgtcagtcct 3720
   tggattgtaa caatggagtt ttatggaaag aatgacctta cactggggat atttttagaa 3780
   agatactgtt tcaggtactc ttaccagtgt ccgagcatgt tctgtgacac ccccatggtt 3840
   catcacattc gacgctttgt tcatggccaa ggctgtgtac agataattct gaaggagttg 3900
   gattctccag tgcctggata tcaacataca attctcacat attcctggtg cagaatctgc 3960
   aaacaagtaa caccagttgt tgctctttca aatgaatcct ggtctatgtc atttgcaaag 4020
   taccttgaac ttcgatttta tggccaccag tacacacgca gagccaacgc tgagccctgc 4080
   ggtcactcta tccaccatga ttatcaccag tatttctctt ataaccagat ggtggcatct 4140
   ttcagttact ctcctattcg gcttcttgaa gtatgtgttc cactaccaaa aatattcatt 4200
   aagcgtcaag ccccactgaa ggtatctctt cttcaggacc tcaaagactt ttttcagaag 4260
   gtttcacagg tgtacctagc tgttgatgag agacttgcat ccttgaaaac ggatacattt 4320
   agcaaaacta gagaggaaaa gatggaagat atctttgcac aaaaggagat ggaagagggt 4380
   gagtttaaga actggacaga gaagatgcaa gcaaggctca tgtcttcctc tgtggatacc 4440
   cctcagcaac tgcagtccat ttttgagtca ctgattgcca agaagcaaag cctctgtgag 4500
   gtgctccagg cgtggaacag caggttgcag gacctcttcc agcaggaaaa aggtagaaag 4560
   aggccttcag ttcctcccag tcctgggaga ctgagacaag gtgaagaaag caagataaat 4620
   gcaatggaca catctccaag gaatatttct ccaggacttt cacaatggag aaaaagaaga 4680
   tcgcttcttg acaaccctgt ccagccagct acgagctcca cccacctcca gctgcccact 4740
   cctcccgagg ccctggccga gcaggtagtg ggagggccga ctgatctgga ttcagccagt 4800
   ggctctgaag atgtatttga tggtcatttg ctgggatcca cagacagcca ggtgaaggaa 4860
   aagtcaacca tgaaagccat ctttgctaat ttgcttccag gaaacagcta caatcccatt 4920
   ccatttcctt ttgatccaga taaacactac ttaatgtatg aacatgaacg ggtgcccatt 4980
   gctgtctgtg agaaagagcc cagctccatc attgcttttg cactcagttg taaagaatac 5040
   cgcaatgcct tagaggaatt gtccaaagca actctgcgga acagtgctga agaagggctc 5100
   ccagccaata gtgctttaga taacagacct aagagtagca gccctattag actacctgaa 5160
   atcagtggag gacagacaaa ccgcacagta gaagcagaac ctcagccaac caaaaaagct 5220
   tcaggaatgt tgtccttctt cagaggaaca gcagggaaga gccctgatct gtcttcccag 5280
   aagagggaga ccttgcgagg ggcagacagt gcttactacc aggttgggca ggccggcaag 5340
   gaggggttgg agagtcaagg cctggagcct caagatgaag tagatggagg agatacacag 5400
   aagaaacaac tcacaaatcc tcatgtggaa cttcaatttt ctgatgctaa tgccaagttt 5460
   tactgtcggc tgtactacgc gggagagttc cacaagatgc gtgaagtgat tctgggcagc 5520
   agtgaggagg aattcatccg ttccctttct cactcatctc cctggcaggc ccggggaggc 5580
   aagtcaggag ctgctttcta tgccaccgaa gatgatagat tcattctgaa gcaaatgcct 5640
   cgtttggaag tccagtcttt ccttgacttt gcaccacact acttcaatta tatcacaaat 5700
   gctgttcaac aaaagaggcc caccgccttg gctaaaattc ttggagttta cagaattggt 5760
   tataagaact ctcagaacaa cactgagaag aagttagatc tccttgtcat ggaaaatctt 5820
   ttctatggga gaaagatggc acaggttttt gatttgaagg gttcacttag gaatcgaaat 5880
   gtaaaaactg acactgggaa agagagctgt gatgtggttc tgttggatga aaacctccta 5940
   aagatggttc gagacaaccc tctctatatt cgttcccatt ccaaatctga gctgagaacc 6000
   tccatccaca gcgacgccca tttcctttcc agccacctca ttatagacta ttctctgctg 6060
   gttgggcgag atgacactag caatgagctt gtggttggca tcatagatta cattcgaaca 6120
   tttacatggg acaaaaaact tgagatggtt gtgaagtcaa caggaatttt aggaggacaa 6180
   ggtaaaatgc caactgtggt ctctccagag ttgtatagga ctagattttg tgaagcaatg 6240
   gacaagtatt tcttgatggt gccagaccac tggacagggt tggatctgaa ttgctga 6297
<210> 22
   <211> 2052
   <212> PRT
   <213> Mus musculus
<400> 22
   Met Ala Thr Asp Asp Lys Ser Ser Pro Thr Leu Asp Ser Ala Asn Asp
   1 5 10 15
   Leu Pro Arg Ser Pro Ala Ser Pro Ser His Leu Thr His Phe Lys Pro
   20 25 30
   Leu Thr Pro Asp Gln Asp Glu Pro Pro Phe Lys Ser Ala Tyr Ser Ser
   35 40 45
   Phe Val Asn Leu Phe Arg Phe Asn Lys Glu Arg Gly Glu Gly Gly Gln
   50 55 60
   Gly Glu Gln Gln Ser Pro Ser Ser Ser Trp Ala Ser Pro Gln Ile Pro
   65 70 75 80
   Ser Arg Thr Gln Ser Val Arg Ser Pro Val Pro Tyr Lys Lys Gln Leu
   85 90 95
   Asn Glu Glu Leu His Arg Arg Ser Ser Val Leu Glu Asn Thr Leu Pro
   100 105 110
   His Pro Gln Glu Ser Thr Asp Ser Arg Arg Lys Ala Glu Pro Ala Cys
   115 120 125
   Gly Gly His Asp Pro Arg Thr Ala Val Gln Leu Arg Ser Leu Ser Thr
   130 135 140
   Val Leu Lys Arg Leu Lys Glu Ile Met Glu Gly Lys Ser Gln Asp Ser
   145 150 155 160
   Asp Leu Lys Gln Tyr Trp Met Pro Asp Ser Gln Cys Lys Glu Cys Tyr
   165 170 175
   Asp Cys Ser Glu Lys Phe Thr Thr Phe Arg Arg Arg His His Cys Arg
   180 185 190
   Leu Cys Gly Gln Ile Phe Cys Ser Arg Cys Cys Asn Gln Glu Ile Pro
   195 200 205
   Gly Lys Phe Met Gly Tyr Thr Gly Asp Leu Arg Ala Cys Thr Tyr Cys
   210 215 220
   Arg Lys Ile Ala Leu Ser Tyr Ala His Ser Thr Asp Ser Asn Ser Ile
   225 230 235 240
   Gly Glu Asp Leu Asn Ala Leu Ser Asp Ser Thr Cys Ser Val Ser Ile
   245 250 255
   Leu Asp Pro Ser Glu Pro Arg Thr Pro Val Gly Ser Arg Lys Ala Ser
   260 265 270
   Arg Asn Ile Phe Leu Glu Asp Asp Leu Ala Trp Gln Ser Leu Ile His
   275 280 285
   Pro Asp Ser Ser Asn Ser Ala Leu Ser Thr Arg Leu Val Ser Val Gln
   290 295 300
   Glu Asp Ala Gly Lys Ser Pro Ala Arg Asn Arg Ser Ala Ser Ile Thr
   305 310 315 320
   Asn Leu Ser Leu Asp Arg Ser Gly Ser Pro Met Val Pro Ser Tyr Glu
   325 330 335
   Thr Ser Val Ser Pro Gln Ala Asn Arg Asn Tyr Ile Arg Thr Glu Thr
   340 345 350
   Thr Glu Asp Glu Arg Lys Ile Leu Leu Asp Ser Ala Gln Leu Lys Asp
   355 360 365
   Leu Trp Lys Lys Ile Cys His His Thr Ser Gly Met Glu Phe Gln Asp
       370                 375                 380
   His Arg Tyr Trp Leu Arg Thr His Pro Asn Cys Ile Val Gly Lys Glu
   385                 390                 395                 400
   Leu Val Asn Trp Leu Ile Arg Asn Gly His Ile Ala Thr Arg Ala Gln
                   405                 410                 415
   Ala Ile Ala Ile Gly Gln Ala Met Val Asp Gly Arg Trp Leu Asp Cys
               420                 425                 430
   Val Ser His His Asp Gln Leu Phe Arg Asp Glu Tyr Ala Leu Tyr Arg
           435                 440                 445
   Pro Leu Gln Ser Thr Glu Phe Ser Glu Thr Pro Ser Pro Asp Ser Asp
       450                 455                 460
   Ser Val Asn Ser Val Glu Gly His Ser Glu Pro Ser Trp Phe Lys Asp
   465                 470                 475                 480
   Ile Lys Phe Asp Asp Ser Asp Thr Glu Gln Ile Ala Glu Glu Gly Asp
                   485                 490                 495
   Asp Asn Leu Ala Lys Tyr Leu Val Ser Asp Thr Gly Gly Gln Gln Leu
               500                 505                 510
   Ser Ile Ser Asp Ala Phe Ile Lys Glu Ser Leu Phe Asn Arg Arg Val
           515                 520                 525
   Glu Glu Lys Ser Lys Glu Leu Pro Phe Thr Pro Leu Gly Trp His His
       530                 535                 540
   Asn Asn Leu Glu Leu Leu Arg Glu Glu Asn Glu Glu Lys Gln Ala Met
   545                 550                 555                 560
   Glu Arg Leu Leu Ser Ala Asn His Asn His Met Met Ala Leu Leu Gln
                   565                 570                 575
   Gln Leu Leu Gln Asn Glu Ser Leu Ser Ser Ser Trp Arg Asp Ile Ile
               580                 585                 590
   Val Ser Leu Val Cys Gln Val Val Gln Thr Val Arg Pro Asp Val Lys
           595                 600                 605
   His Gln Asp Asp Asp Met Asp Ile Arg Gln Phe Val His Ile Lys Lys
   610 615 620
   Ile Pro Gly Gly Lys Lys Phe Asp Ser Val Val Val Asn Gly Phe Val
   625 630 635 640
   Cys Thr Lys Asn Ile Ala His Lys Lys Met Asn Ser Cys Ile Lys Asn
   645 650 655
   Pro Lys Ile Leu Leu Leu Lys Cys Ser Ile Glu Tyr Leu Tyr Arg Glu
   660 665 670
   Glu Thr Lys Phe Thr Cys Ile Asp Pro Ile Val Leu Gln Glu Arg Glu
   675 680 685
   Phe Leu Lys Asn Tyr Val Gln Arg Ile Val Asp Val Arg Pro Thr Leu
   690 695 700
   Val Leu Val Glu Lys Thr Val Ser Arg Ile Ala Gln Asp Met Leu Leu
   705 710 715 720
   Glu His Gly Ile Thr Leu Val Ile Asn Val Lys Ser Gln Val Leu Glu
   725 730 735
   Arg Ile Ser Arg Met Thr Gln Gly Asp Leu Val Val Ser Met Asp Gln
   740 745 750
   Leu Leu Thr Lys Pro His Leu Gly Thr Cys His Lys Phe Tyr Met Gln
   755 760 765
   Ile Phe Gln Leu Pro Asn Glu Gln Thr Lys Thr Leu Met Phe Phe Glu
   770 775 780
   Gly Cys Pro Gln His Leu Gly Cys Thr Ile Lys Leu Arg Gly Gly Ser
   785 790 795 800
   Asp Tyr Glu Leu Ala Arg Val Lys Glu Ile Leu Ile Phe Met Ile Cys
   805 810 815
   Val Ala Tyr His Ser Gln Leu Glu Ile Ser Phe Leu Met Asp Glu Phe
   820 825 830
   Ala Met Pro Pro Thr Leu Met Gln Ser Pro Ser Phe His Leu Leu Thr
   835 840 845
   Glu Gly Arg Gly Glu Glu Gly Ala Ser Gln Glu Gln Val Ser Gly Ser
   850 855 860
   Ser Leu Pro Gln Asp Pro Glu Cys Pro Arg Glu Ala Leu Ser Ser Glu
   865 870 875 880
   Asp Ser Thr Leu Leu Glu Ser Arg Thr Val Leu Glu Lys Gly Glu Leu
   885 890 895
   Asp Asn Lys Ser Ile Pro Gln Ala Val Ala Ser Leu Lys His Gln Asp
   900 905 910
   Tyr Thr Thr Pro Thr Cys Pro Ala Gly Ile Pro Cys Ala Leu Phe Ala
   915 920 925
   Leu Val Pro Glu Ser Leu Leu Pro Leu His Met Asp Gln Gln Asp Ala
   930 935 940
   Val Gly Asn Glu His Arg Glu Thr Ser Gln Gln Thr Asp Glu Gln Gln
   945 950 955 960
   Asp Pro Lys Ser Gln Met Lys Ala Phe Arg Asp Pro Leu Gln Asp Asp
   965 970 975
   Thr Gly Met Tyr Val Thr Glu Glu Val Thr Ser Ser Glu Asp Gln Arg
   980 985 990
   Lys Thr Tyr Ala Leu Thr Phe Lys Gln Glu Leu Lys Asp Val Ile Leu
   995 1000 1005
   Cys Ile Ser Pro Val Ile Thr Phe Arg Glu Pro Phe Leu Leu Thr
   1010 1015 1020
   Glu Lys Gly Met Arg Cys Ser Thr Arg Asp Tyr Phe Pro Glu Gln
   1025 1030 1035
   Ile Tyr Trp Ser Pro Leu Leu Asn Lys Glu Val Lys Glu Met Glu
   1040 1045 1050
   Ser Arg Arg Lys Lys Gln Leu Leu Arg Asp Leu Ser Gly Leu Gln
   1055 1060 1065
   Gly Met Asn Gly Ser Val Gln Ala Lys Ser Ile Gln Val Leu Pro
   1070 1075 1080
   Ser His Glu Leu Val Ser Thr Arg Ile Ala Glu His Val Gly Asp
   1085 1090 1095
   Ser Gln Thr Leu Gly Arg Met Leu Ala Asp Tyr Arg Ala Arg Gly
   1100 1105 1110
   Gly Glu Phe Ser Gln Asn Ile Trp Asn Pro Phe Val His Ser Lys
   1115 1120 1125
   Asp Asp Ile Met Tyr Phe Arg Trp Gln Ile Arg Glu Thr Lys Leu
   1130 1135 1140
   Arg Val Met Lys Glu Arg Gly Leu Ile Pro Ser Asp Val Ile Trp
   1145 1150 1155
   Pro Thr Lys Val Asp Cys Leu Asn Pro Ala Asn His Gln Arg Leu
   1160 1165 1170
   Cys Val Leu Phe Ser Ser Ser Ser Ala Gln Ser Ser Asn Ala Pro
   1175 1180 1185
   Ser Ala Cys Val Ser Pro Trp Ile Val Thr Met Glu Phe Tyr Gly
   1190 1195 1200
   Lys Asn Asp Leu Thr Leu Gly Ile Phe Leu Glu Arg Tyr Cys Phe
   1205 1210 1215
   Arg Tyr Ser Tyr Gln Cys Pro Ser Met Phe Cys Asp Thr Pro Met
   1220 1225 1230
   Val His His Ile Arg Arg Phe Val His Gly Gln Gly Cys Val Gln
   1235 1240 1245
   Ile Ile Leu Lys Glu Leu Asp Ser Pro Val Pro Gly Tyr Gln His
   1250 1255 1260
   Thr Ile Leu Thr Tyr Ser Trp Cys Arg Ile Cys Lys Gln Val Thr
   1265 1270 1275
   Pro Val Val Ala Leu Ser Asn Glu Ser Trp Ser Met Ser Phe Ala
   1280 1285 1290
   Lys Tyr Leu Glu Leu Arg Phe Tyr Gly His Gln Tyr Thr Arg Arg
   1295 1300 1305
   Ala Asn Ala Glu Pro Cys Gly His Ser Ile His His Asp Tyr His
   1310 1315 1320
   Gln Tyr Phe Ser Tyr Asn Gln Met Val Ala Ser Phe Ser Tyr Ser
   1325 1330 1335
   Pro Ile Arg Leu Leu Glu Val Cys Val Pro Leu Pro Lys Ile Phe
   1340 1345 1350
   Ile Lys Arg Gln Ala Pro Leu Lys Val Ser Leu Leu Gln Asp Leu
   1355 1360 1365
   Lys Asp Phe Phe Gln Lys Val Ser Gln Val Tyr Leu Ala Val Asp
   1370 1375 1380
   Glu Arg Leu Ala Ser Leu Lys Thr Asp Thr Phe Ser Lys Thr Arg
   1385 1390 1395
   Glu Glu Lys Met Glu Asp Ile Phe Ala Gln Lys Glu Met Glu Glu
   1400 1405 1410
   Gly Glu Phe Lys Asn Trp Thr Glu Lys Met Gln Ala Arg Leu Met
   1415 1420 1425
   Ser Ser Ser Val Asp Thr Pro Gln Gln Leu Gln Ser Ile Phe Glu
   1430 1435 1440
   Ser Leu Ile Ala Lys Lys Gln Ser Leu Cys Glu Val Leu Gln Ala
   1445 1450 1455
   Trp Asn Ser Arg Leu Gln Asp Leu Phe Gln Gln Glu Lys Gly Arg
   1460 1465 1470
   Lys Arg Pro Ser Val Pro Pro Ser Pro Gly Arg Leu Arg Gln Gly
   1475 1480 1485
   Glu Glu Ser Lys Ile Asn Ala Met Asp Thr Ser Pro Arg Asn Ile
   1490 1495 1500
   Ser Pro Gly Leu Ser Gln Trp Arg Lys Arg Arg Ser Leu Leu Asp
   1505 1510 1515
   Asn Pro Val Gln Pro Ala Thr Ser Ser Thr His Leu Gln Leu Pro
   1520 1525 1530
   Thr Pro Pro Glu Ala Leu Ala Glu Gln Val Val Gly Gly Pro Thr
   1535 1540 1545
   Asp Leu Asp Ser Ala Ser Gly Ser Glu Asp Val Phe Asp Gly His
   1550 1555 1560
   Leu Leu Gly Ser Thr Asp Ser Gln Val Lys Glu Lys Ser Thr Met
   1565 1570 1575
   Lys Ala Ile Phe Ala Asn Leu Leu Pro Gly Asn Ser Tyr Asn Pro
   1580 1585 1590
   Ile Pro Phe Pro Phe Asp Pro Asp Lys His Tyr Leu Met Tyr Glu
   1595 1600 1605
   His Glu Arg Val Pro Ile Ala Val Cys Glu Lys Glu Pro Ser Ser
   1610 1615 1620
   Ile Ile Ala Phe Ala Leu Ser Cys Lys Glu Tyr Arg Asn Ala Leu
   1625 1630 1635
   Glu Glu Leu Ser Lys Ala Thr Leu Arg Asn Ser Ala Glu Glu Gly
   1640 1645 1650
   Leu Pro Ala Asn Ser Ala Leu Asp Asn Arg Pro Lys Ser Ser Ser
   1655 1660 1665
   Pro Ile Arg Leu Pro Glu Ile Ser Gly Gly Gln Thr Asn Arg Thr
   1670 1675 1680
   Val Glu Ala Glu Pro Gln Pro Thr Lys Lys Ala Ser Gly Met Leu
   1685 1690 1695
   Ser Phe Phe Arg Gly Thr Ala Gly Lys Ser Pro Asp Leu Ser Ser
   1700 1705 1710
   Gln Lys Arg Glu Thr Leu Arg Gly Ala Asp Ser Ala Tyr Tyr Gln
   1715 1720 1725
   Val Gly Gln Ala Gly Lys Glu Gly Leu Glu Ser Gln Gly Leu Glu
   1730 1735 1740
   Pro Gln Asp Glu Val Asp Gly Gly Asp Thr Gln Lys Lys Gln Leu
   1745 1750 1755
   Thr Asn Pro His Val Glu Leu Gln Phe Ser Asp Ala Asn Ala Lys
   1760 1765 1770
   Phe Tyr Cys Arg Leu Tyr Tyr Ala Gly Glu Phe His Lys Met Arg
   1775 1780 1785
   Glu Val Ile Leu Gly Ser Ser Glu Glu Glu Phe Ile Arg Ser Leu
   1790 1795 1800
   Ser His Ser Ser Pro Trp Gln Ala Arg Gly Gly Lys Ser Gly Ala
   1805 1810 1815
   Ala Phe Tyr Ala Thr Glu Asp Asp Arg Phe Ile Leu Lys Gln Met
   1820 1825 1830
   Pro Arg Leu Glu Val Gln Ser Phe Leu Asp Phe Ala Pro His Tyr
   1835 1840 1845
   Phe Asn Tyr Ile Thr Asn Ala Val Gln Gln Lys Arg Pro Thr Ala
   1850 1855 1860
   Leu Ala Lys Ile Leu Gly Val Tyr Arg Ile Gly Tyr Lys Asn Ser
   1865 1870 1875
   Gln Asn Asn Thr Glu Lys Lys Leu Asp Leu Leu Val Met Glu Asn
   1880 1885 1890
   Leu Phe Tyr Gly Arg Lys Met Ala Gln Val Phe Asp Leu Lys Gly
   1895 1900 1905
   Ser Leu Arg Asn Arg Asn Val Lys Thr Asp Thr Gly Lys Glu Ser
   1910 1915 1920
   Cys Asp Val Val Leu Leu Asp Glu Asn Leu Leu Lys Met Val Arg
   1925 1930 1935
   Asp Asn Pro Leu Tyr Ile Arg Ser His Ser Lys Ser Glu Leu Arg
   1940 1945 1950
   Thr Ser Ile His Ser Asp Ala His Phe Leu Ser Ser His Leu Ile
   1955 1960 1965
   Ile Asp Tyr Ser Leu Leu Val Gly Arg Asp Asp Thr Ser Asn Glu
   1970 1975 1980
   Leu Val Val Gly Ile Ile Asp Tyr Ile Arg Thr Phe Thr Trp Asp
   1985 1990 1995
   Lys Lys Leu Glu Met Val Val Lys Ser Thr Gly Ile Leu Gly Gly
   2000 2005 2010
   Gln Gly Lys Met Pro Thr Val Val Ser Pro Glu Leu Tyr Arg Thr
   2015 2020 2025
   Arg Phe Cys Glu Ala Met Asp Lys Tyr Phe Leu Met Val Pro Asp
   2030 2035 2040
   His Trp Thr Gly Leu Asp Leu Asn Cys
   2045 2050
<210> 23
   <211> 2617
   <212> DNA
   <213> Homo sapiens
<400> 23
   gcgatctgca ggtaggggtg cgcgcgaccg ctccccggcg ggagccagcg aaggtttcca      60
   tgtcagaggc cgatggagaa ctgaagattg ccacctacgc acaaaggcca ttgagacact     120
   tcgtgtagct ggaagacacc aacttcctga caggagcttt atttcatttg ggatttcaag     180
   tttacagatg gtatcttctc aaaagttgga aaaacctata gagatgggca gtagcgaacc     240
   ccttcccatc gcagatggtg acaggaggag gaagaagaag cggaggggcc gggccactga     300
   ctccttgcca ggaaagtttg aagatatgta caagctgacc tctgaattgc ttggagaggg     360
   agcctatgcc aaagttcaag gtgccgtgag cctacagaat ggcaaagagt atgccgtcaa     420
   aatcatcgag aaacaagcag ggcacagtcg gagtagggtg tttcgagagg tggagacgct     480
   gtatcagtgt cagggaaaca agaacatttt ggagctgatt gagttctttg aagatgacac     540
   aaggttttac ttggtctttg agaaattgca aggaggttcc atcttagccc acatccagaa     600
   gcaaaagcac ttcaatgagc gagaagccag ccgagtggtg cgggacgttg ctgctgccct     660
   tgacttcctg cataccaaag gcattgctca tcgtgatctg aaaccagaaa atatattgtg     720
   tgaatctcca gaaaaggtgt ctccagtgaa aatctgtgac tttgacttgg gcagtgggat     780
   gaaactgaac aactcctgta cccccataac cacaccagag ctgaccaccc catgtggctc     840
   tgcagaatac atggcccctg aggtagtgga ggtcttcacg gaccaggcca cattctacga     900
   caagcgctgt gacctgtgga gcctgggcgt ggtcctctac atcatgctga gtggctaccc     960
   acccttcgtg ggtcactgcg gggccgactg tggctgggac cggggcgagg tctgcagggt    1020
   gtgccagaac aagctgtttg aaagcatcca ggaaggcaag tatgagtttc ctgacaagga    1080
   ctgggcacac atctccagtg aagccaaaga cctcatctcc aagctcctgg tgcgagatgc    1140
   aaagcagaga cttagcgccg cccaagttct gcagcaccca tgggtgcagg ggcaagctcc    1200
   agaaaaggga ctccccacgc cgcaagtcct ccagaggaac agcagcacaa tggacctgac    1260
   gctcttcgca gctgaggcca tcgcccttaa ccgccagcta tctcagcacg aagagaacga    1320
   actagcagag gagccagagg cactagctga tggcctctgc tccatgaagc tttcccctcc    1380
   ctgcaagtca cgcctggccc ggagacgggc cctggcccag gcaggccgtg gtgaagacag    1440
   gagcccgccc acagcactct gaaatgctcc agtcacacct tataggccct aggcctggcc    1500
   aggcattgtc ccctggaaac ctgtgtggct aaagtctgct gagcaggcag cagcctctgc    1560
   tctgtggctc cattcaggct ttttcatcta cgaaggccct gaggttccca tcaaccccca    1620
   tttccctagg gtcctggagg aaaaagcttt ttccaaaggg gttgtctttg aaaaggaaag    1680
   caatcacttc tcactttgca taattgcctg cagcaggaac atctcttcac tgggctccac    1740
   ctgctcaccc gcctgcagat ctgggatcca gcctgctctc accgctgtag ctgtggcggc    1800
   tggggctgca gcctgcaggg agaagcaaga agcatcagtt gacagaggct gccgacacgt    1860
   gcctcttccc tctcttctct gtcaccctcc tctggcggtc cttccacctt cctctgtcct    1920
   ccggatgtcc tctttgcccg tcttctccct tggctgagca aagccatccc ctcaattcag    1980
   ggaagggcaa ggagccttcc tcattcagga aatcaaatca gtcttccggt ctgcagcacg    2040
   gaaaagcaca taatctttct ttgctgtgac tgaaatgtat ccctcgttta tcatcccctt    2100
   tgtttgtgat tgctgctaaa gtcagtagta tcgttttttt aaaaaaaaag tttggtgttt    2160
   ttaaccatct gttccagcaa agatgatacc ttaaactccc actgcaagcc catgaacttc    2220
   ccagagagtg gaacggcttg ctcttctttc tagaatgtcc atgcacttgg gttttaatca    2280
   gcagttccct attattctga ttttaagctg ttcctgtgat gaacttagag acagcatcgg    2340
   tgtctgctgc tgtgtcccca ggtcttgtgt gggtggcaca gatctgggca gttagatagt    2400
   gctctgtgcc taaggtgaag ccacactagg gtgaagcctc acttccctgt ttgagcaatg    2460
   cagtgcctgc tgcccgtgtg catgaaggta cagccattca tataagtgga actattgagt    2520
   tacataaaga aaatagattt gcatttgtca ggcagacgtt tatacaacac cacggtgctt    2580
   ttatacattg tgcttatttt aataaaactg aaattct                             2617
<210> 24
   <211> 424
   <212> PRT
   <213> Homo sapiens
<400> 24
   Met Val Ser Ser Gln Lys Leu Glu Lys Pro Ile Glu Met Gly Ser Ser
   1               5                   10                  15
   Glu Pro Leu Pro Ile Ala Asp Gly Asp Arg Arg Arg Lys Lys Lys Arg
               20                  25                  30
   Arg Gly Arg Ala Thr Asp Ser Leu Pro Gly Lys Phe Glu Asp Met Tyr
           35                  40                  45
   Lys Leu Thr Ser Glu Leu Leu Gly Glu Gly Ala Tyr Ala Lys Val Gln
       50                  55                  60
   Gly Ala Val Ser Leu Gln Asn Gly Lys Glu Tyr Ala Val Lys Ile Ile
   65 70 75 80
   Glu Lys Gln Ala Gly His Ser Arg Ser Arg Val Phe Arg Glu Val Glu
   85 90 95
   Thr Leu Tyr Gln Cys Gln Gly Asn Lys Asn Ile Leu Glu Leu Ile Glu
   100 105 110
   Phe Phe Glu Asp Asp Thr Arg Phe Tyr Leu Val Phe Glu Lys Leu Gln
   115 120 125
   Gly Gly Ser Ile Leu Ala His Ile Gln Lys Gln Lys His Phe Asn Glu
   130 135 140
   Arg Glu Ala Ser Arg Val Val Arg Asp Val Ala Ala Ala Leu Asp Phe
   145 150 155 160
   Leu His Thr Lys Gly Ile Ala His Arg Asp Leu Lys Pro Glu Asn Ile
   165 170 175
   Leu Cys Glu Ser Pro Glu Lys Val Ser Pro Val Lys Ile Cys Asp Phe
   180 185 190
   Asp Leu Gly Ser Gly Met Lys Leu Asn Asn Ser Cys Thr Pro Ile Thr
   195 200 205
   Thr Pro Glu Leu Thr Thr Pro Cys Gly Ser Ala Glu Tyr Met Ala Pro
   210 215 220
   Glu Val Val Glu Val Phe Thr Asp Gln Ala Thr Phe Tyr Asp Lys Arg
   225 230 235 240
   Cys Asp Leu Trp Ser Leu Gly Val Val Leu Tyr Ile Met Leu Ser Gly
   245 250 255
   Tyr Pro Pro Phe Val Gly His Cys Gly Ala Asp Cys Gly Trp Asp Arg
   260 265 270
   Gly Glu Val Cys Arg Val Cys Gln Asn Lys Leu Phe Glu Ser Ile Gln
   275 280 285
   Glu Gly Lys Tyr Glu Phe Pro Asp Lys Asp Trp Ala His Ile Ser Ser
   290 295 300
   Glu Ala Lys Asp Leu Ile Ser Lys Leu Leu Val Arg Asp Ala Lys Gln
   305 310 315 320
   Arg Leu Ser Ala Ala Gln Val Leu Gln His Pro Trp Val Gln Gly Gln
   325 330 335
   Ala Pro Glu Lys Gly Leu Pro Thr Pro Gln Val Leu Gln Arg Asn Ser
   340 345 350
   Ser Thr Met Asp Leu Thr Leu Phe Ala Ala Glu Ala Ile Ala Leu Asn
   355 360 365
   Arg Gln Leu Ser Gln His Glu Glu Asn Glu Leu Ala Glu Glu Pro Glu
   370 375 380
   Ala Leu Ala Asp Gly Leu Cys Ser Met Lys Leu Ser Pro Pro Cys Lys
   385 390 395 400
   Ser Arg Leu Ala Arg Arg Arg Ala Leu Ala Gln Ala Gly Arg Gly Glu
   405 410 415
   Asp Arg Ser Pro Pro Thr Ala Leu
   420
<210> 25
   <211> 2745
   <212> DNA
   <213> Homo sapiens
<400> 25
   ggcacgaggg cgaccgctcc ccggcgggag ccagcgaagg tttccatgtc agaggccgat 60
   ggagaactga agattgccac ctacgcacaa aggccattga gacacttcgt gtagctggaa 120
   gacaccaact tcctgacagg agctttattt catttgggat ttcaagttta cagatggtat 180
   cttctcaaaa gttggaaaaa cctatagaga tgggcagtag cgaacccctt cccatcgcag 240
   atggtgacag gaggaggaag aagaagcgga ggggccgggc cactgactcc ttgccaggaa 300
   agtttgaaga tatgtacaag ctgacctctg aattgcttgg agagggagcc tatgccaaag 360
   ttcaaggtgc cgtgagccta cagaatggca aagagtatgc cgtcaaaatc atcgagaaac 420
   aagcagggca cagtcggagt agggtgtttc gagaggtgga gacgctgtat cagtgtcagg 480
   gaaacaagaa cattttggag ctgattgagt tctttgaaga tgacacaagg ttttacttgg 540
   tctttgagaa attgcaagga ggttccatct tagcccacat ccagaagcaa aagcacttca 600
   atgagcgaga agccagccga gtggtgcggg acgttgctgc tgcccttgac ttcctgcata 660
   ccaaagacaa agtctctctc tgtcacctag gctggagtgc tatggcgcca tcagggctca 720
   ctgcagcccc aacctccctg ggctccagtg atcctcccac ctcagcctcc caagtagctg 780
   ggactacagg cattgctcat cgtgatctga aaccagaaaa tatattgtgt gaatctccag 840
   aaaaggtgtc tccagtgaaa atctgtgact ttgacttggg cagtgggatg aaactgaaca 900
   actcctgtac ccccataacc acaccagagc tgaccacccc atgtggctct gcagaataca 960
   tggcccctga ggtagtggag gtcttcacgg accaggccac attctacgac aagcgctgtg 1020
   acctgtggag cctgggcgtg gtcctctaca tcatgctgag tggctaccca cccttcgtgg 1080
   gtcactgcgg ggccgactgt ggctgggacc ggggcgaggt ctgcagggtg tgccagaaca 1140
   agctgtttga aagcatccag gaaggcaagt atgagtttcc tgacaaggac tgggcacaca 1200
   tctccagtga agccaaagac ctcatctcca agctcctggt gcgagatgca aagcagagac 1260
   ttagcgccgc ccaagttctg cagcacccat gggtgcaggg gcaagctcca gaaaagggac 1320
   tccccacgcc gcaagtcctc cagaggaaca gcagcacaat ggacctgacg ctcttcgcag 1380
   ctgaggccat cgcccttaac cgccagctat ctcagcacga agagaacgaa ctagcagagg 1440
   agccagaggc actagctgat ggcctctgct ccatgaagct ttcccctccc tgcaagtcac 1500
   gcctggcccg gagacgggcc ctggcccagg caggccgtgg tgaagacagg agcccgccca 1560
   cagcactctg aaatgctcca gtcacacctt ataggcccta ggcctggcca ggcattgtcc 1620
   cctggaaacc tgtgtggcta aagtctgctg agcaggcagc agcctctgct ctgtggctcc 1680
   attcaggctt tttcatctac gaaggccctg aggttcccat caacccccat ttccctaggg 1740
   tcctggagga aaaagctttt tccaaagggg ttgtctttga aaaggaaagc aatcacttct 1800
   cactttgcat aattgcctgc agcaggaaca tctcttcact gggctccacc tgctcacccg 1860
   cctgcagatc tgggatccag cctgctctca ccgctgtagc tgtggcggct ggggctgcag 1920
   cctgcaggga gaagcaagaa gcatcagttg acagaggctg ccgacacgtg cctcttccct 1980
   ctcttctctg tcaccctcct ctggcggtcc ttccaccttc ctctgtcctc cggatgtcct 2040
   ctttgcccgt cttctccctt ggctgagcaa agccatcccc tcaattcagg gaagggcaag 2100
   gagccttcct cattcaggaa atcaaatcag tcttccggtc tgcagcacgg aaaagcacat 2160
   aatctttctt tgctgtgact gaaatgtatc cctcgtttat catccccttt gtttgtgatt 2220
   gctgctaaag tcagtagtat cgttttttta aaaaaaaagt ttggtgtttt taaccatgct 2280
   gttccagcaa agatgatacc ttaaactccc actgcaagcc catgaacttc ccagagagtg 2340
   gaacggcttg ctcttctttc tagaatgtcc atgcacttgg gttttaatca gcagttccct 2400
   attattctga ttttaagctg ttcctgtgat gaacttagag acagcatcgg tgtctgctgc 2460
   tgtgtcccca ggtcttgtgt gggtggcaca gatctgggca gttagatagt gctctgtgcc 2520
   taaggtgaag ccacactagg gtgaagcctc acttccctgt ttgagcaatg cagtgcctgc 2580
   tgcccgtgtg catgaaggta cagccattca gataagtgga actattgagt tacataaaga 2640
   aaatagattt gcatttgtca ggcagacgtt tatacaacac cacggtgctt ttatacattg 2700
   tgcttatttt aataaaactg aaattctaaa aaaaaaaaaa aaaaa 2745
<210> 26
   <211> 465
   <212> PRT
   <213> Homo sapiens
<400> 26
   Met Val Ser Ser Gln Lys Leu Glu Lys Pro Ile Glu Met Gly Ser Ser
   1 5 10 15
   Glu Pro Leu Pro Ile Ala Asp Gly Asp Arg Arg Arg Lys Lys Lys Arg
   20 25 30
   Arg Gly Arg Ala Thr Asp Ser Leu Pro Gly Lys Phe Glu Asp Met Tyr
   35 40 45
   Lys Leu Thr Ser Glu Leu Leu Gly Glu Gly Ala Tyr Ala Lys Val Gln
   50 55 60
   Gly Ala Val Ser Leu Gln Asn Gly Lys Glu Tyr Ala Val Lys Ile Ile
   65 70 75 80
   Glu Lys Gln Ala Gly His Ser Arg Ser Arg Val Phe Arg Glu Val Glu
   85 90 95
   Thr Leu Tyr Gln Cys Gln Gly Asn Lys Asn Ile Leu Glu Leu Ile Glu
   100 105 110
   Phe Phe Glu Asp Asp Thr Arg Phe Tyr Leu Val Phe Glu Lys Leu Gln
   115 120 125
   Gly Gly Ser Ile Leu Ala His Ile Gln Lys Gln Lys His Phe Asn Glu
   130 135 140
   Arg Glu Ala Ser Arg Val Val Arg Asp Val Ala Ala Ala Leu Asp Phe
   145 150 155 160
   Leu His Thr Lys Asp Lys Val Ser Leu Cys His Leu Gly Trp Ser Ala
   165 170 175
   Met Ala Pro Ser Gly Leu Thr Ala Ala Pro Thr Ser Leu Gly Ser Ser
   180 185 190
   Asp Pro Pro Thr Ser Ala Ser Gln Val Ala Gly Thr Thr Gly Ile Ala
           195                 200                 205
   His Arg Asp Leu Lys Pro Glu Asn Ile Leu Cys Glu Ser Pro Glu Lys
       210                 215                 220
   Val Ser Pro Val Lys Ile Cys Asp Phe Asp Leu Gly Ser Gly Met Lys
   225                 230                 235                 240
   Leu Asn Asn Ser Cys Thr Pro Ile Thr Thr Pro Glu Leu Thr Thr Pro
                   245                 250                 255
   Cys Gly Ser Ala Glu Tyr Met Ala Pro Glu Val Val Glu Val Phe Thr
               260                 265                 270
   Asp Gln Ala Thr Phe Tyr Asp Lys Arg Cys Asp Leu Trp Ser Leu Gly
           275                 280                 285
   Val Val Leu Tyr Ile Met Leu Ser Gly Tyr Pro Pro Phe Val Gly His
       290                 295                 300
   Cys Gly Ala Asp Cys Gly Trp Asp Arg Gly Glu Val Cys Arg Val Cys
   305                 310                 315                 320
   Gln Asn Lys Leu Phe Glu Ser Ile Gln Glu Gly Lys Tyr Glu Phe Pro
                   325                 330                 335
   Asp Lys Asp Trp Ala His Ile Ser Ser Glu Ala Lys Asp Leu Ile Ser
               340                 345                 350
   Lys Leu Leu Val Arg Asp Ala Lys Gln Arg Leu Ser Ala Ala Gln Val
           355                 360                 365
   Leu Gln His Pro Trp Val Gln Gly Gln Ala Pro Glu Lys Gly Leu Pro
       370                 375                 380
   Thr Pro Gln Val Leu Gln Arg Asn Ser Ser Thr Met Asp Leu Thr Leu
   385                 390                 395                 400
   Phe Ala Ala Glu Ala Ile Ala Leu Asn Arg Gln Leu Ser Gln His Glu
                   405                 410                 415
   Glu Asn Glu Leu Ala Glu Glu Pro Glu Ala Leu Ala Asp Gly Leu Cys
               420                 425                 430
   Ser Met Lys Leu Ser Pro Pro Cys Lys Ser Arg Leu Ala Arg Arg Arg
           435                 440                 445
   Ala Leu Ala Gln Ala Gly Arg Gly Glu Asp Arg Ser Pro Pro Thr Ala
   450 455 460
   Leu
   465
<210> 27
   <211> 1444
   <212> DNA
   <213> Homo sapiens
<400> 27
   cggtcccctc ccccgctggc ggggcccgga cagaagatgg tgcagaagaa accagccgaa     60
   cttcagggtt tccaccgttc gttcaagggg cagaacccct tcgagctggc cttctcccta    120
   gaccagcccg accacggaga ctctgacttt ggcctgcagt gctcagcccg ccctgacatg    180
   cccgccagcc agcccattga catcccggac gccaagaaga ggggcaagaa gaagaagcgc    240
   ggccgggcca ccgacagctt ctcgggcagg tttgaagacg tctaccagct gcaggaagat    300
   gtgctggggg agggcgctca tgcccgagtg cagacctgca tcaacctgat caccagccag    360
   gagtacgccg tcaagatcat tgagaagcag ccaggccaca ttcggagcag ggttttcagg    420
   gaggtggaga tgctgtacca gtgccaggga cacaggaacg tcctagagct gattgagttc    480
   ttcgaggagg aggaccgctt ctacctggtg tttgagaaga tgcggggagg ctccatcctg    540
   agccacatcc acaagcgccg gcacttcaac gagctggagg ccagcgtggt ggtgcaggac    600
   gtggccagcg ccttggactt tctgcataac aaaggcatcg cccacaggga cctaaagccg    660
   gaaaacatcc tctgtgagca ccccaaccag gtctcccccg tgaagatctg tgacttcgac    720
   ctgggcagcg gcatcaaact caacggggac tgctccccta tctccacccc ggagctgctc    780
   actccgtgcg gctcggcgga gtacatggcc ccggaggtag tggaggcctt cagcgaggag    840
   gctagcatct acgacaagcg ctgcgacctg tggagcctgg gcgtcatctt gtatatccta    900
   ctcagcggct acccgccctt cgtgggccgc tgtggcagcg actgcggctg ggaccgcggc    960
   gaggcctgcc ctgcctgcca gaacatgctg tttgagagca tccaggaggg caagtacgag   1020
   ttccccgaca aggactgggc ccacatctcc tgcgctgcca aagacctcat ctccaagctg   1080
   ctggtccgtg acgccaagca gaggctgagt gccgcccaag tcctgcagca cccctgggtt   1140
   caggggtgcg ccccggagaa caccttgccc actcccatgg tcctgcagag gaacagctgt   1200
   gccaaagacc tcacgtcctt cgcggctgag gccattgcca tgaaccggca gctggcccag   1260
   cacgacgagg acctggctga ggaggaggcc gcggggcagg gccagcccgt cctggtccga   1320
   gctacctcac gctgcctgca gctgtctcca ccctcccagt ccaagctggc gcagcggcgg   1380
   caaagggcca gtctgtcctc ggccccagtg gtcctggtgg gagaccacgc ctgaccctcc   1440
   catc 1444
<210> 28
   <211> 465
   <212> PRT
   <213> Homo sapiens
<400> 28
   Met Val Gln Lys Lys Pro Ala Glu Leu Gln Gly Phe His Arg Ser Phe
   1               5                   10                  15
   Lys Gly Gln Asn Pro Phe Glu Leu Ala Phe Ser Leu Asp Gln Pro Asp
               20                  25                  30
   His Gly Asp Ser Asp Phe Gly Leu Gln Cys Ser Ala Arg Pro Asp Met
           35                  40                  45
   Pro Ala Ser Gln Pro Ile Asp Ile Pro Asp Ala Lys Lys Arg Gly Lys
       50                  55                  60
   Lys Lys Lys Arg Gly Arg Ala Thr Asp Ser Phe Ser Gly Arg Phe Glu
   65                  70                  75                  80
   Asp Val Tyr Gln Leu Gln Glu Asp Val Leu Gly Glu Gly Ala His Ala
                   85                  90                  95
   Arg Val Gln Thr Cys Ile Asn Leu Ile Thr Ser Gln Glu Tyr Ala Val
               100                 105                 110
   Lys Ile Ile Glu Lys Gln Pro Gly His Ile Arg Ser Arg Val Phe Arg
           115                 120                 125
   Glu Val Glu Met Leu Tyr Gln Cys Gln Gly His Arg Asn Val Leu Glu
       130                 135                 140
   Leu Ile Glu Phe Phe Glu Glu Glu Asp Arg Phe Tyr Leu Val Phe Glu
   145                 150                 155                 160
   Lys Met Arg Gly Gly Ser Ile Leu Ser His Ile His Lys Arg Arg His
                   165                 170                 175
   Phe Asn Glu Leu Glu Ala Ser Val Val Val Gln Asp Val Ala Ser Ala
               180                 185                 190
   Leu Asp Phe Leu His Asn Lys Gly Ile Ala His Arg Asp Leu Lys Pro
           195                 200                 205
   Glu Asn Ile Leu Cys Glu His Pro Asn Gln Val Ser Pro Val Lys Ile
   210 215 220
   Cys Asp Phe Asp Leu Gly Ser Gly Ile Lys Leu Asn Gly Asp Cys Ser
   225 230 235 240
   Pro Ile Ser Thr Pro Glu Leu Leu Thr Pro Cys Gly Ser Ala Glu Tyr
   245 250 255
   Met Ala Pro Glu Val Val Glu Ala Phe Ser Glu Glu Ala Ser Ile Tyr
   260 265 270
   Asp Lys Arg Cys Asp Leu Trp Ser Leu Gly Val Ile Leu Tyr Ile Leu
   275 280 285
   Leu Ser Gly Tyr Pro Pro Phe Val Gly Arg Cys Gly Ser Asp Cys Gly
   290 295 300
   Trp Asp Arg Gly Glu Ala Cys Pro Ala Cys Gln Asn Met Leu Phe Glu
   305 310 315 320
   Ser Ile Gln Glu Gly Lys Tyr Glu Phe Pro Asp Lys Asp Trp Ala His
   325 330 335
   Ile Ser Cys Ala Ala Lys Asp Leu Ile Ser Lys Leu Leu Val Arg Asp
   340 345 350
   Ala Lys Gln Arg Leu Ser Ala Ala Gln Val Leu Gln His Pro Trp Val
   355 360 365
   Gln Gly Cys Ala Pro Glu Asn Thr Leu Pro Thr Pro Met Val Leu Gln
   370 375 380
   Arg Asn Ser Cys Ala Lys Asp Leu Thr Ser Phe Ala Ala Glu Ala Ile
   385 390 395 400
   Ala Met Asn Arg Gln Leu Ala Gln His Asp Glu Asp Leu Ala Glu Glu
   405 410 415
   Glu Ala Ala Gly Gln Gly Gln Pro Val Leu Val Arg Ala Thr Ser Arg
   420 425 430
   Cys Leu Gln Leu Ser Pro Pro Ser Gln Ser Lys Leu Ala Gln Arg Arg
   435 440 445
   Gln Arg Ala Ser Leu Ser Ser Ala Pro Val Val Leu Val Gly Asp His
   450 455 460
   Ala
   465
<210> 29
   <211> 2010
   <212> DNA
   <213> Homo sapiens
<400> 29
   gcgcagaggc gtcacgcact ccatggtaac gacgctcggc ccgaagatgg cggccgaatg     60
   gggcggagga gtgggttact cgggctcagg cccgggccgg agccggtggc gctggagcgg    120
   gtcataaata aagttcatct taaggcaaat catgtggtca agagagatgt tgatgagcat    180
   ttaagaatca agactgtcta tgataaaagt gttgaagagt tgctccctga gaaaaagaat    240
   cttgtaaaga acaagctttt cccacaagcg atttcttatt tagagaagac ttttcaggtc    300
   cgtcgacctg cgggcactat cttacttagc agacaatgtg caacaaacca atacctccgg    360
   aaggaaaacg atcctcacag gtactgcacc ggggagtgtg ccgcacacac aaagtgcggc    420
   cccgttattg ttcctgagga acatctccag caatgccggg tctaccgtgg gggtaagtgg    480
   cctcatggag cagtgggtgt gccagaccaa gaaggcatct cagatgcaga ctttgttctt    540
   tacgttggtg ctctggccac cgagagatgc agccatgaaa acatcatctc ttatgcagcc    600
   tattgtcagc aggaagcaaa catggacagg ccaatagcag gatatgctaa cctgtgtcca    660
   aatatgatct ctacccagcc tcaggagttt gttgggatgc tgtccacagt gaaacatgag    720
   gttattcatg ccctgggttt ctctgctggg ctgtttgcat tctaccatga taaagatgga    780
   aatcctctca cttcaagatt tgcagatggc ctcccacctt ttaattatag tctgggatta    840
   tatcaatgga gtgataaagt agttcgaaaa gtggagagat tatgggatgt tcgagataat    900
   aagatagttc gtcacactgt gtatctcctg gtaacgcctc gtgttgttga ggaagcacga    960
   aaacattttg attgtccagt tctagaggga atggaacttg aaaatcaagg tggtgtgggc   1020
   actgagctca accattggga aaaaaggtta ttagagaatg aagcgatgac tggttctcac   1080
   actcagaatc gagtactctc tcgaatcact ctggcattaa tggaggacac tggctggtat   1140
   aaagcaaatt acagcatggc tgagaagtta gactggggcc gaggaatggg ctgtgacttt   1200
   gtcaggaaga gctgtaaatt ctggattgat cagcagagac aaaagagaca gatgctgagc   1260
   ccttactgtg acacgctcag aagtaaccca ctgcagctaa cttgcagaca ggaccagaga   1320
   gcagttgccg tgtgtaattt gcagaagttc cctaagcctt taccacagga atatcagtac   1380
   tttgatgaac tcagtggaat acctgcagaa gatttgcctt attatggtgg ctccgtggaa   1440
   attgctgact actgcccttt cagtcaggaa ttcagttggc atttaagtgg tgaatatcag   1500
   cgcagctcag attgtagaat attggaaaat caaccagaaa tttttaagaa ctatggcgct   1560
   gaaaagtatg gacctcattc cgtttgtcta attcagaaat cagcattcgt tatggagaag   1620
   tgtgagaaga agctgagtta cccagactgg ggaagcggat gctatcaggt ttcttgttct   1680
   cctcaaggtc tgaaagtttg ggtccaagat acttcatatt tgtgtagtcg ggctgggcag   1740
   gtcctccctg tcagtatcca gatgaatggc tggattcacg atggaaacct gctctgccca   1800
   tcatgttggg acttctgtga gctctgtcct ccagaaacag atcctccagc cactaacctg   1860
   acccgagctc tgccacttga tctttgttcc tgttcctcga gcctggtggt caccctctgg   1920
   cttctgctag gcaatctgtt tcctctgctg gctggatttc ttctgtgtat atggcactag   1980
   gaatggaaaa gtggatcttc aagatattct                                   2010
<210> 30
   <211> 640
   <212> PRT
   <213> Homo sapiens
<400> 30
<210> 31
   <211> 2020
   <212> DNA
   <213> Homo sapiens
<400> 31
   gcgcagaggc gtcacgcact ccatggtaac gacgctcggc ccgaagatgg cggccgaatg      60
   gggcggagga gtgggttact cgggctcagg cccgggccgg agccggtggc gctggagcgg     120
   gtctgtgtgg gtccgaagcg ttttactcct gttgggcggg ctccgggcca gcgccacatc     180
   tactcccgtc tccttgggca gttcccctcc ctgccggcac cacgtcccct ctgacactga     240
   ggtcataaat aaagttcatc ttaaggcaaa tcatgtggtc aagagagatg ttgatgagca     300
   tttaagaatc aagactgtct atgataaaag tgttgaagag ttgctccctg agaaaaagaa     360
   tcttgtaaag aacaagcttt tcccacaagc gatttcttat ttagagaaga cttttcaggt     420
   ccgtcgacct gcgggcacta tcttacttag cagacaatgt gcaacaaacc aatacctccg     480
   gaaggaaaac gatcctcaca ggtactgcac cggggagtgt gccgcacaca caaagtgcgg     540
   ccccgttatt gttcctgagg aacatctcca gcaatgccgg gtctaccgtg ggggtaagtg     600
   gcctcatgga gcagtgggtg tgccagacca agaaggcatc tcagatgcag actttgttct     660
   ttacgttggt gctctggcca ccgagagatg cagccatgaa aacatcatct cttatgcagc     720
   ctattgtcag caggaagcaa acatggacag gccaatagca ggatatgcta acctgtgtcc     780
   aaatatgatc tctacccagc ctcaggagtt tgttgggatg ctgtccacag tgaaacatga     840
   ggttattcat gccctgggtt tctctgctgg gctgtttgca ttctaccatg ataaagatgg     900
   aaatcctctc acttcaagat ttgcagatgg cctcccacct tttaattata gtctgggatt     960
   atatcaatgg agtgataaag tagttcgaaa agtggagaga ttatgggatg ttcgagataa    1020
   taagatagtt cgtcacactg tgtatctcct ggtaacgcct cgtgttgttg aggaagcacg    1080
   aaaacatttt gattgtccag ttctagaggg aatggaactt gaaaatcaag gtggtgtggg    1140
   cactgagctc aaccattggg aaaaaaggtt attagagaat gaagcgatga ctggttctca    1200
   cactcagaat cgagtactct ctcgaatcac tctggcatta atggaggaca ctgggagaca    1260
   gatgctgagc ccttactgtg acacgctcag aagtaaccca ctgcagctaa cttgcagaca    1320
   ggaccagaga gcagttgccg tgtgtaattt gcagaagttc cctaagcctt taccacagga    1380
   atatcagtac tttgatgaac tcagtggaat acctgcagaa gatttgcctt attatggtgg    1440
   ctccgtggaa attgctgact actgcccttt cagtcaggaa ttcagttggc atttaagtgg    1500
   tgaatatcag cgcagctcag attgtagaat attggaaaat caaccagaaa tttttaagaa    1560
   ctatggcgct gaaaagtatg gacctcattc cgtttgtcta attcagaaat cagcattcgt    1620
   tatggagaag tgtgagaaga agctgagtta cccagactgg ggaagcggat gctatcaggt    1680
   ttcttgttct cctcaaggtc tgaaagtttg ggtccaagat acttcatatt tgtgtagtcg   1740
   ggctgggcag gtcctccctg tcagtatcca gatgaatggc tggattcacg atggaaacct   1800
   gctctgccca tcatgttggg acttctgtga gctctgtcct ccagaaacag atcctccagc   1860
   cactaacctg acccgagctc tgccacttga tctttgttcc tgttcctcga gcctggtggt   1920
   caccctctgg cttctgctag gcaatctgtt tcctctgctg gctggatttc ttctgtgtat   1980
   atggcactag gaatggaaaa gtggatcttc aagatattct                         2020
<210> 32
   <211> 655
   <212> PRT
   <213> Homo sapiens
<400> 32
   Met Val Thr Thr Leu Gly Pro Lys Met Ala Ala Glu Trp Gly Gly Gly
   1               5                   10                  15
   Val Gly Tyr Ser Gly Ser Gly Pro Gly Arg Ser Arg Trp Arg Trp Ser
               20                  25                  30
   Gly Ser Val Trp Val Arg Ser Val Leu Leu Leu Leu Gly Gly Leu Arg
           35                  40                  45
   Ala Ser Ala Thr Ser Thr Pro Val Ser Leu Gly Ser Ser Pro Pro Cys
       50                  55                  60
   Arg His His Val Pro Ser Asp Thr Glu Val Ile Asn Lys Val His Leu
   65                  70                  75                  80
   Lys Ala Asn His Val Val Lys Arg Asp Val Asp Glu His Leu Arg Ile
                   85                  90                  95
   Lys Thr Val Tyr Asp Lys Ser Val Glu Glu Leu Leu Pro Glu Lys Lys
               100                 105                 110
   Asn Leu Val Lys Asn Lys Leu Phe Pro Gln Ala Ile Ser Tyr Leu Glu
           115                 120                 125
   Lys Thr Phe Gln Val Arg Arg Pro Ala Gly Thr Ile Leu Leu Ser Arg
       130                 135                 140
   Gln Cys Ala Thr Asn Gln Tyr Leu Arg Lys Glu Asn Asp Pro His Arg
   145                 150                 155                 160
   Tyr Cys Thr Gly Glu Cys Ala Ala His Thr Lys Cys Gly Pro Val Ile
   165 170 175
   Val Pro Glu Glu His Leu Gln Gln Cys Arg Val Tyr Arg Gly Gly Lys
   180 185 190
   Trp Pro His Gly Ala Val Gly Val Pro Asp Gln Glu Gly Ile Ser Asp
   195 200 205
   Ala Asp Phe Val Leu Tyr Val Gly Ala Leu Ala Thr Glu Arg Cys Ser
   210 215 220
   His Glu Asn Ile Ile Ser Tyr Ala Ala Tyr Cys Gln Gln Glu Ala Asn
   225 230 235 240
   Met Asp Arg Pro Ile Ala Gly Tyr Ala Asn Leu Cys Pro Asn Met Ile
   245 250 255
   Ser Thr Gln Pro Gln Glu Phe Val Gly Met Leu Ser Thr Val Lys His
   260 265 270
   Glu Val Ile His Ala Leu Gly Phe Ser Ala Gly Leu Phe Ala Phe Tyr
   275 280 285
   His Asp Lys Asp Gly Asn Pro Leu Thr Ser Arg Phe Ala Asp Gly Leu
   290 295 300
   Pro Pro Phe Asn Tyr Ser Leu Gly Leu Tyr Gln Trp Ser Asp Lys Val
   305 310 315 320
   Val Arg Lys Val Glu Arg Leu Trp Asp Val Arg Asp Asn Lys Ile Val
   325 330 335
   Arg His Thr Val Tyr Leu Leu Val Thr Pro Arg Val Val Glu Glu Ala
   340 345 350
   Arg Lys His Phe Asp Cys Pro Val Leu Glu Gly Met Glu Leu Glu Asn
   355 360 365
   Gln Gly Gly Val Gly Thr Glu Leu Asn His Trp Glu Lys Arg Leu Leu
   370 375 380
   Glu Asn Glu Ala Met Thr Gly Ser His Thr Gln Asn Arg Val Leu Ser
   385 390 395 400
   Arg Ile Thr Leu Ala Leu Met Glu Asp Thr Gly Arg Gln Met Leu Ser
   405 410 415
   Pro Tyr Cys Asp Thr Leu Arg Ser Asn Pro Leu Gln Leu Thr Cys Arg
               420                 425                 430
   Gln Asp Gln Arg Ala Val Ala Val Cys Asn Leu Gln Lys Phe Pro Lys
           435                 440                 445
   Pro Leu Pro Gln Glu Tyr Gln Tyr Phe Asp Glu Leu Ser Gly Ile Pro
       450                 455                 460
   Ala Glu Asp Leu Pro Tyr Tyr Gly Gly Ser Val Glu Ile Ala Asp Tyr
   465                 470                 475                 480
   Cys Pro Phe Ser Gln Glu Phe Ser Trp His Leu Ser Gly Glu Tyr Gln
                   485                 490                 495
   Arg Ser Ser Asp Cys Arg Ile Leu Glu Asn Gln Pro Glu Ile Phe Lys
               500                 505                 510
   Asn Tyr Gly Ala Glu Lys Tyr Gly Pro His Ser Val Cys Leu Ile Gln
           515                 520                 525
   Lys Ser Ala Phe Val Met Glu Lys Cys Glu Lys Lys Leu Ser Tyr Pro
       530                 535                 540
   Asp Trp Gly Ser Gly Cys Tyr Gln Val Ser Cys Ser Pro Gln Gly Leu
   545                 550                 555                 560
   Lys Val Trp Val Gln Asp Thr Ser Tyr Leu Cys Ser Arg Ala Gly Gln
                   565                 570                 575
   Val Leu Pro Val Ser Ile Gln Met Asn Gly Trp Ile His Asp Gly Asn
               580                 585                 590
   Leu Leu Cys Pro Ser Cys Trp Asp Phe Cys Glu Leu Cys Pro Pro Glu
           595                 600                 605
   Thr Asp Pro Pro Ala Thr Asn Leu Thr Arg Ala Leu Pro Leu Asp Leu
       610                 615                 620
   Cys Ser Cys Ser Ser Ser Leu Val Val Thr Leu Trp Leu Leu Leu Gly
   625                 630                 635                 640
   Asn Leu Phe Pro Leu Leu Ala Gly Phe Leu Leu Cys Ile Trp His
                   645                 650                 655
<210> 33
   <211> 4083
   <212> DNA
   <213> Homo sapiens
<400> 33
   atggcgagcg actccccggc tcgaagcctg gatgaaatag atctctcggc tctgagggac     60
   cctgcaggga tctttgaatt ggtggaactt gttggaaatg gaacatacgg gcaagtttat    120
   aagggtcgtc atgtcaaaac gggccagctt gcagccatca aggttatgga tgtcacaggg    180
   gatgaagagg aagaaatcaa acaagaaatt aacatgttga agaaatattc tcatcaccgg    240
   aatattgcta catactatgg tgcttttatc aaaaagaacc caccaggcat ggatgaccaa    300
   ctttggttgg tgatggagtt ttgtggtgct ggctctgtca ccgacctgat caagaacaca    360
   aaaggtaaca cgttgaaaga ggagtggatt gcatacatct gcagggaaat cttacggggg    420
   ctgagtcacc tgcaccagca taaagtgatt catcgagata ttaaagggca aaatgtcttg    480
   ctgactgaaa atgcagaagt taaactagtg gactttggag tcagtgctca gcttgatcga    540
   acagtgggca ggaggaatac tttcattgga actccctact ggatggcacc agaagttatt    600
   gcctgtgatg aaaacccaga tgccacatat gatttcaaga gtgacttgtg gtctttgggt    660
   atcaccgcca ttgaaatggc agaaggtgct ccccctctct gtgacatgca ccccatgaga     720
   gctctcttcc tcatcccccg gaatccagcg cctcggctga agtctaagaa gtggtcaaaa    780
   aaattccagt catttattga gagctgcttg gtaaagaatc acagccagcg accagcaaca    840
   gaacaattga tgaagcatcc atttatacga gaccaaccta atgagcgaca ggtccgcatt    900
   caactcaagg accatattga tagaacaaag aagaagcgag gagaaaaaga tgagacagag    960
   tatgagtaca gtggaagtga ggaagaagag gaggagaatg actcaggaga gcccagctcc   1020
   atcctgaatc tgccagggga gtcgacgctg cggagggact ttctgaggct gcagctggcc   1080
   aacaaggagc gttctgaggc cctacggagg cagcagctgg agcagcagca gcgggagaat   1140
   gaggagcaca agcggcagct gctggccgag cgtcagaagc gcatcgagga gcagaaagag   1200
   cagaggcggc ggctggagga gcaacaaagg cgagagaagg agctgcggaa gcagcaggag   1260
   agggagcagc gccggcacta tgaggagcag atgcgccggg aggaggagag gaggcgtgcg   1320
   gagcatgaac aggaatacat caggcgacag ttagaggagg agcagagaca gttagagatc   1380
   ttgcagcagc agctactgca tgaacaagct ctacttctgg aatataagcg caaacaattg   1440
   gaagaacaga gacaagcaga aagactgcag aggcagctaa agcaagaaag agactactta   1500
   gtttcccttc agcatcagcg gcaggagcag aggcctgtgg agaagaagcc actgtaccat   1560
   tacaaagaag gaatgagtcc tagtgagaag ccagcatggg ccaaggaggt agaagaacgg   1620
   tcaaggctca accggcaaag ttcccctgcc atgcctcaca aggttgccaa caggatatct   1680
   gaccccaacc tgcccccaag gtcggagtcc ttcagcatta gtggagttca gcctgctcga   1740
   acacccccca tgctcagacc agtcgatccc cagatcccac atctggtagc tgtaaaatcc 1800
   cagggacctg ccttgaccgc ctcccagtca gtgcacgagc agcccacaaa gggcctctct 1860
   gggtttcagg aggctctgaa cgtgacctcc caccgcgtgg agatgccacg ccagaactca 1920
   gatcccacct cggaaaatcc tcctctcccc actcgcattg aaaagtttga ccgaagctct 1980
   tggttacgac aggaagaaga cattccacca aaggtgcctc aaagaacaac ttctatatcc 2040
   ccagcattag ccagaaagaa ttctcctggg aatggtagtg ctctgggacc cagactagga 2100
   tctcaaccca tcagagcaag caaccctgat ctccggagaa ctgagcccat cttggagagc 2160
   cccttgcaga ggaccagcag tggcagttcc tccagctcca gcacccctag ctcccagccc 2220
   agctcccaag gaggctccca gcctggatca caagcaggat ccagtgaacg caccagagtt 2280
   cgagccaaca gtaagtcaga aggatcacct gtgcttcccc atgagcctgc caaggtgaaa 2340
   ccagaagaat ccagggacat tacccggccc agtcgaccag ctagctacaa aaaagctata 2400
   gatgaggatc tgacggcatt agccaaagaa ctaagagaac tccggattga agaaacaaac 2460
   cgcccaatga agaaggtgac tgattactcc tcctccagtg aggagtcaga aagtagcgag 2520
   gaagaggagg aagatggaga gagcgagacc catgatggga cagtggctgt cagcgacata 2580
   cccagactga taccaacagg agctccaggc agcaacgagc agtacaatgt gggaatggtg 2640
   gggacgcatg ggctggagac ctctcatgcg gacagtttca gcggcagtat ttcaagagaa 2700
   ggaaccttga tgattagaga gacgtctgga gagaagaagc gatctggcca cagtgacagc 2760
   aatggctttg ctggccacat caacctccct gacctggtgc agcagagcca ttctccagct 2820
   ggaaccccga ctgagggact ggggcgcgtc tcaacccatt cccaggagat ggactctggg 2880
   actgaatatg gcatggggag cagcaccaaa gcctccttca ccccctttgt ggaccccaga 2940
   gtataccaga cgtctcccac tgatgaagat gaagaggatg aggaatcatc agccgcagct 3000
   ctgtttacta gcgaacttct taggcaagaa caggccaaac tcaatgaagc aagaaagatt 3060
   tcggtggtaa atgtaaaccc aaccaacatt cggcctcata gcgacacacc agaaatcaga 3120
   aaatacaaga aacgattcaa ctcagaaata ctttgtgcag ctctgtgggg tgtaaacctt 3180
   ctggtgggga ctgaaaatgg cctgatgctt ttggaccgaa gtgggcaagg caaagtctat 3240
   aatctgatca accggaggcg atttcagcag atggatgtgc tagagggact gaatgtcctt 3300
   gtgacaattt caggaaagaa gaataagcta cgagtttact atctttcatg gttaagaaac 3360
   agaatactac ataatgaccc agaagtagaa aagaaacaag gctggatcac tgttggggac 3420
   ttggaaggct gtatacatta taaagttgtt aaatatgaaa ggatcaaatt tttggtgatt 3480
   gccttaaaga atgctgtgga aatatatgct tgggctccta aaccgtatca taaattcatg 3540
   gcatttaagt cttttgcaga tctccagcac aagcctctgc tagttgatct cacggtagaa 3600
   gaaggtcaaa gattaaaggt tatttttggt tcacacactg gtttccatgt aattgatgtt   3660
   gattcaggaa actcttatga tatctacata ccatctcata ttcagggcaa tatcactcct   3720
   catgctattg tcatcttgcc taaaacagat ggaatggaaa tgcttgtttg ctatgaggat   3780
   gagggggtgt atgtaaacac ctatggccgg ataactaagg atgtggtgct ccaatgggga   3840
   gaaatgccca cgtctgtggc ctacattcat tccaatcaga taatgggctg gggcgagaaa   3900
   gctattgaga tccggtcagt ggaaacagga catttggatg gagtatttat gcataagcga   3960
   gctcaaaggt taaagtttct atgtgaaaga aatgataagg tattttttgc atccgtgcga   4020
   tctggaggaa gtagccaagt gtttttcatg accctcaaca gaaattccat gatgaactgg   4080
   taa                                                                   4083
<210> 34
   <211> 1360
   <212> PRT
   <213> Homo sapiens
<400> 34
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Ile Arg
   435 440 445
   Arg Gln Leu Glu Glu Glu Gln Arg Gln Leu Glu Ile Leu Gln Gln Gln
   450 455 460
   Leu Leu His Glu Gln Ala Leu Leu Leu Glu Tyr Lys Arg Lys Gln Leu
   465 470 475 480
   Glu Glu Gln Arg Gln Ala Glu Arg Leu Gln Arg Gln Leu Lys Gln Glu
   485 490 495
   Arg Asp Tyr Leu Val Ser Leu Gln His Gln Arg Gln Glu Gln Arg Pro
   500 505 510
   Val Glu Lys Lys Pro Leu Tyr His Tyr Lys Glu Gly Met Ser Pro Ser
   515 520 525
   Glu Lys Pro Ala Trp Ala Lys Glu Val Glu Glu Arg Ser Arg Leu Asn
   530 535 540
   Arg Gln Ser Ser Pro Ala Met Pro His Lys Val Ala Asn Arg Ile Ser
   545 550 555 560
   Asp Pro Asn Leu Pro Pro Arg Ser Glu Ser Phe Ser Ile Ser Gly Val
   565 570 575
   Gln Pro Ala Arg Thr Pro Pro Met Leu Arg Pro Val Asp Pro Gln Ile
   580 585 590
   Pro His Leu Val Ala Val Lys Ser Gln Gly Pro Ala Leu Thr Ala Ser
   595 600 605
   Gln Ser Val His Glu Gln Pro Thr Lys Gly Leu Ser Gly Phe Gln Glu
   610 615 620
   Ala Leu Asn Val Thr Ser His Arg Val Glu Met Pro Arg Gln Asn Ser
   625 630 635 640
   Asp Pro Thr Ser Glu Asn Pro Pro Leu Pro Thr Arg Ile Glu Lys Phe
   645 650 655
   Asp Arg Ser Ser Trp Leu Arg Gln Glu Glu Asp Ile Pro Pro Lys Val
   660 665 670
   Pro Gln Arg Thr Thr Ser Ile Ser Pro Ala Leu Ala Arg Lys Asn Ser
   675 680 685
   Pro Gly Asn Gly Ser Ala Leu Gly Pro Arg Leu Gly Ser Gln Pro Ile
   690 695 700
   Arg Ala Ser Asn Pro Asp Leu Arg Arg Thr Glu Pro Ile Leu Glu Ser
   705 710 715 720
   Pro Leu Gln Arg Thr Ser Ser Gly Ser Ser Ser Ser Ser Ser Thr Pro
   725 730 735
   Ser Ser Gln Pro Ser Ser Gln Gly Gly Ser Gln Pro Gly Ser Gln Ala
   740 745 750
   Gly Ser Ser Glu Arg Thr Arg Val Arg Ala Asn Ser Lys Ser Glu Gly
   755 760 765
   Ser Pro Val Leu Pro His Glu Pro Ala Lys Val Lys Pro Glu Glu Ser
   770 775 780
   Arg Asp Ile Thr Arg Pro Ser Arg Pro Ala Ser Tyr Lys Lys Ala Ile
   785 790 795 800
   Asp Glu Asp Leu Thr Ala Leu Ala Lys Glu Leu Arg Glu Leu Arg Ile
   805 810 815
   Glu Glu Thr Asn Arg Pro Met Lys Lys Val Thr Asp Tyr Ser Ser Ser
   820 825 830
   Ser Glu Glu Ser Glu Ser Ser Glu Glu Glu Glu Glu Asp Gly Glu Ser
   835 840 845
   Glu Thr His Asp Gly Thr Val Ala Val Ser Asp Ile Pro Arg Leu Ile
   850 855 860
   Pro Thr Gly Ala Pro Gly Ser Asn Glu Gln Tyr Asn Val Gly Met Val
   865 870 875 880
   Gly Thr His Gly Leu Glu Thr Ser His Ala Asp Ser Phe Ser Gly Ser
   885 890 895
   Ile Ser Arg Glu Gly Thr Leu Met Ile Arg Glu Thr Ser Gly Glu Lys
   900 905 910
   Lys Arg Ser Gly His Ser Asp Ser Asn Gly Phe Ala Gly His Ile Asn
   915 920 925
   Leu Pro Asp Leu Val Gln Gln Ser His Ser Pro Ala Gly Thr Pro Thr
   930 935 940
   Glu Gly Leu Gly Arg Val Ser Thr His Ser Gln Glu Met Asp Ser Gly
   945 950 955 960
   Thr Glu Tyr Gly Met Gly Ser Ser Thr Lys Ala Ser Phe Thr Pro Phe
   965 970 975
   Val Asp Pro Arg Val Tyr Gln Thr Ser Pro Thr Asp Glu Asp Glu Glu
   980 985 990
   Asp Glu Glu Ser Ser Ala Ala Ala Leu Phe Thr Ser Glu Leu Leu Arg
   995 1000 1005
   Gln Glu Gln Ala Lys Leu Asn Glu Ala Arg Lys Ile Ser Val Val
   1010 1015 1020
   Asn Val Asn Pro Thr Asn Ile Arg Pro His Ser Asp Thr Pro Glu
   1025 1030 1035
   Ile Arg Lys Tyr Lys Lys Arg Phe Asn Ser Glu Ile Leu Cys Ala
   1040 1045 1050
   Ala Leu Trp Gly Val Asn Leu Leu Val Gly Thr Glu Asn Gly Leu
   1055 1060 1065
   Met Leu Leu Asp Arg Ser Gly Gln Gly Lys Val Tyr Asn Leu Ile
   1070 1075 1080
   Asn Arg Arg Arg Phe Gln Gln Met Asp Val Leu Glu Gly Leu Asn
   1085 1090 1095
   Val Leu Val Thr Ile Ser Gly Lys Lys Asn Lys Leu Arg Val Tyr
   1100 1105 1110
   Tyr Leu Ser Trp Leu Arg Asn Arg Ile Leu His Asn Asp Pro Glu
   1115 1120 1125
   Val Glu Lys Lys Gln Gly Trp Ile Thr Val Gly Asp Leu Glu Gly
   1130 1135 1140
   Cys Ile His Tyr Lys Val Val Lys Tyr Glu Arg Ile Lys Phe Leu
   1145 1150 1155
   Val Ile Ala Leu Lys Asn Ala Val Glu Ile Tyr Ala Trp Ala Pro
   1160 1165 1170
   Lys Pro Tyr His Lys Phe Met Ala Phe Lys Ser Phe Ala Asp Leu
   1175 1180 1185
   Gln His Lys Pro Leu Leu Val Asp Leu Thr Val Glu Glu Gly Gln
   1190 1195 1200
   Arg Leu Lys Val Ile Phe Gly Ser His Thr Gly Phe His Val Ile
   1205 1210 1215
   Asp Val Asp Ser Gly Asn Ser Tyr Asp Ile Tyr Ile Pro Ser His
   1220 1225 1230
   Ile Gln Gly Asn Ile Thr Pro His Ala Ile Val Ile Leu Pro Lys
   1235 1240 1245
   Thr Asp Gly Met Glu Met Leu Val Cys Tyr Glu Asp Glu Gly Val
   1250 1255 1260
   Tyr Val Asn Thr Tyr Gly Arg Ile Thr Lys Asp Val Val Leu Gln
   1265 1270 1275
   Trp Gly Glu Met Pro Thr Ser Val Ala Tyr Ile His Ser Asn Gln
   1280 1285 1290
   Ile Met Gly Trp Gly Glu Lys Ala Ile Glu Ile Arg Ser Val Glu
   1295 1300 1305
   Thr Gly His Leu Asp Gly Val Phe Met His Lys Arg Ala Gln Arg
   1310 1315 1320
   Leu Lys Phe Leu Cys Glu Arg Asn Asp Lys Val Phe Phe Ala Ser
   1325 1330 1335
   Val Arg Ser Gly Gly Ser Ser Gln Val Phe Phe Met Thr Leu Asn
   1340 1345 1350
   Arg Asn Ser Met Met Asn Trp
   1355 1360
<210> 35
   <211> 4863
   <212> DNA
   <213> Homo sapiens
<400> 35
   ggagatagcg cctgtcagtc ggtgggtcgg tcctcgcgcc ggccctcccc ctccccggtc     60
   tccgggggag gcgcggtgga gtccgccccc ggggttctcc gatgggggag aagcggcgac    120
   ggcggcagtg gagtaaccga gccggagcgt gagcggcccc ggtgccccgt tccccacgga    180
   ggccatgggc gacccagccc ccgcccgcag cctggacgac atcgacctgt ccgccctgcg    240
   ggaccctgct gggatctttg agcttgtgga ggtggtcggc aatggaacct acggacaggt    300
   gtacaagggt cggcatgtca agacggggca gctggctgcc atcaaggtca tggatgtcac    360
   ggaggacgag gaggaagaga tcaaacagga gatcaacatg ctgaaaaagt actctcacca    420
   ccgcaacatc gccacctact acggagcctt catcaagaag agccccccgg gaaacgatga    480
   ccagctctgg ctggtgatgg agttctgtgg tgctggttca gtgactgacc tggtaaagaa    540
   cacaaaaggc aacgccctga aggaggactg tatcgcctat atctgcaggg agatcctcag    600
   gggtctggcc catctccatg cccacaaggt gatccatcga gacatcaagg ggcagaatgt    660
   gctgctgaca gagaatgctg aggtcaagct agtggatttt ggggtgagtg ctcagctgga    720
   ccgcaccgtg ggcagacgga acactttcat tgggactccc tactggatgg ctccagaggt    780
   catcgcctgt gatgagaacc ctgatgccac ctatgattac aggagtgata tttggtctct    840
   aggaatcaca gccatcgaga tggcagaggg agccccccct ctgtgtgaca tgcaccccat    900
   gcgagccctc ttcctcattc ctcggaaccc tccgcccagg ctcaagtcca agaagtggtc    960
   taagaagttc attgacttca ttgacacatg tctcatcaag acttacctga gccgcccacc   1020
   cacggagcag ctactgaagt ttcccttcat ccgggaccag cccacggagc ggcaggtccg   1080
   catccagctt aaggaccaca ttgaccgatc ccggaagaag cggggtgaga aagaggagac   1140
   agaatatgag tacagcggca gcgaggagga agatgacagc catggagagg aaggagagcc   1200
   aagctccatc atgaacgtgc ctggagagtc gactctacgc cgggagtttc tccggctcca   1260
   gcaggaaaat aagagcaact cagaggcttt aaaacagcag cagcagctgc agcagcagca   1320
   gcagcgagac cccgaggcac acatcaaaca cctgctgcac cagcggcagc ggcgcataga   1380
   ggagcagaag gaggagcggc gccgcgtgga ggagcaacag cggcgggagc gggagcagcg   1440
   gaagctgcag gagaaggagc agcagcggcg gctggaggac atgcaggctc tgcggcggga   1500
   ggaggagcgg cggcaggcgg agcgcgagca ggaatacaag cggaagcagc tggaggagca   1560
   gcggcagtca gaacgtctcc agaggcagct gcagcaggag catgcctacc tcaagtccct   1620
   gcagcagcag caacagcagc agcagcttca gaaacagcag cagcagcagc tcctgcctgg 1680
   ggacaggaag cccctgtacc attatggtcg gggcatgaat cccgctgaca aaccagcctg 1740
   ggcccgagag gtagaagaga gaacaaggat gaacaagcag cagaactctc ccttggccaa 1800
   gagcaagcca ggcagcacgg ggcctgagcc ccccatcccc caggcctccc cagggccccc 1860
   aggacccctt tcccagactc ctcctatgca gaggccggtg gagccccagg agggaccgca 1920
   caagagcctg gtggcacacc gggtcccact gaagccatat gcagcacctg taccccgatc 1980
   ccagtccctg caggaccagc ccacccgaaa cctggctgcc ttcccagcct cccatgaccc 2040
   cgaccctgcc atccccgcac ccactgccac gcccagtgcc cgaggagctg tcatccgcca 2100
   gaattcagac cccacctctg aaggacctgg ccccagcccg aatcccccag cctgggtccg 2160
   cccagataac gaggccccac ccaaggtgcc tcagaggacc tcatctatcg ccactgccct 2220
   taacaccagt ggggccggag ggtcccggcc agcccaggca gtccgtgcca gtaaccccga 2280
   cctcaggagg agcgaccctg gctgggaacg ctcggacagc gtccttccag cctctcacgg 2340
   gcacctcccc caggctggct cactggagcg gaaccgcgtg ggagtctcct ccaaaccgga 2400
   cagctcccct gtgctctccc ctgggaataa agccaagccc gacgaccacc gctcacggcc 2460
   aggccggccc gcaagctata agcgagcaat tggtgaggac tttgtgttgc tgaaagagcg 2520
   gactctggac gaggcccctc ggcctcccaa gaaggccatg gactactcgt cgtccagcga 2580
   ggaggtggaa agcagtgagg acgacgagga ggaaggcgaa ggcgggccag cagaggggag 2640
   cagagatacc cctgggggcc gcagcgatgg ggatacagac agcgtcagca ccatggtggt 2700
   ccacgacgtc gaggagatca ccgggaccca gcccccatac gggggcggca ccatggtggt 2760
   ccagcgcacc cctgaagagg agcggaacct gctgcatgct gacagcaatg ggtacacaaa 2820
   cctgcctgac gtggtccagc ccagccactc acccaccgag aacagcaaag gccaaagccc 2880
   accctcgaag gatgggagtg gtgactacca gtctcgtggg ctggtaaagg cccctggcaa 2940
   gagctcgttc acgatgtttg tggatctagg gatctaccag cctggaggca gtggggacag 3000
   catccccatc acagccctag tgggtggaga gggcactcgg ctcgaccagc tgcagtacga 3060
   cgtgaggaag ggttctgtgg tcaacgtgaa tcccaccaac acccgggccc acagtgagac 3120
   ccctgagatc cggaagtaca agaagcgatt caactccgag atcctctgtg cagccctttg 3180
   gggggtcaac ctgctggtgg gcacggagaa cgggctgatg ttgctggacc gaagtgggca 3240
   gggcaaggtg tatggactca ttgggcggcg acgcttccag cagatggatg tgctggaggg 3300
   gctcaacctg ctcatcacca tctcagggaa aaggaacaaa ctgcgggtgt attacctgtc 3360
   ctggctccgg aacaagattc tgcacaatga cccagaagtg gagaagaagc agggctggac 3420
   caccgtgggg gacatggagg gctgcgggca ctaccgtgtt gtgaaatacg agcggattaa 3480
   gttcctggtc atcgccctca agagctccgt ggaggtgtat gcctgggccc ccaaacccta 3540
   ccacaaattc atggccttca agtcctttgc cgacctcccc caccgccctc tgctggtcga 3600
   cctgacagta gaggaggggc agcggctcaa ggtcatctat ggctccagtg ctggcttcca 3660
   tgctgtggat gtcgactcgg ggaacagcta tgacatctac atccctgtgc acatccagag 3720
   ccagatcacg ccccatgcca tcatcttcct ccccaacacc gacggcatgg agatgctgct 3780
   gtgctacgag gacgagggtg tctacgtcaa cacgtacggg cgcatcatta aggatgtggt 3840
   gctgcagtgg ggggagatgc ctacttctgt ggcctacatc tgctccaacc agataatggg 3900
   ctggggtgag aaagccattg agatccgctc tgtggagacg ggccacctcg acggggtctt 3960
   catgcacaaa cgagctcaga ggctcaagtt cctgtgtgag cggaatgaca aggtgttttt 4020
   tgcctcagtc cgctctgggg gcagcagcca agtttacttc atgactctga accgtaactg 4080
   catcatgaac tggtgacggg gccctgggct ggggctgtcc cacactggac ccagctctcc 4140
   ccctgcagcc aggcttcccg ggccgcccct ctttcccctc cctgggcttt tgcttttact 4200
   ggtttgattt cactggagcc tgctgggaac gtgacctctg acccctgatg ctttcgtgat 4260
   cacgtgacca tcctcttccc caacatgtcc tcttcccaaa actgtgcctg tccccagctt 4320
   ctggggaggg acacagcttc cccttcccag gaattgagtg ggcctagccc ctcccccctt 4380
   ttctccattt gagaggagag tgcttggggc ttgaacccct taccccactg ctgctgactg 4440
   ggcagggccc tggacccctt tatttgcacg tcaggggagc cggctccccc cttgaatgta 4500
   ccagaccctg gggggggtca ctgggcccta gatttttggg gggtcaccag ccactccagg 4560
   ggcagggacc atttcttcat tttctgaaag cactttaatg attccccttc ccccaaactc 4620
   cagggaatgg aggggggacc ccgccagcca aaacattccc cccattcccg acccccctct 4680
   cctcttctag cccatgccct tccccggtgg agggagggag cagggagccc tcactctcca 4740
   cgccccttgc ttgcatctgt atatagtgtg agcagcaagt aacccttctc cctccccccc 4800
   cacccctcct caatgtagtg gccttggata tcctgtttgt taataaagac aattcaacca 4860
   gct 4863
<210> 36
   <211> 1303
   <212> PRT
   <213> Homo sapiens
<400> 36
   Met Gly Asp Pro Ala Pro Ala Arg Ser Leu Asp Asp Ile Asp Leu Ser
   1 5 10 15
   Ala Leu Arg Asp Pro Ala Gly Ile Phe Glu Leu Val Glu Val Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Glu Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Ser Pro Pro Gly
   85 90 95
   Asn Asp Asp Gln Leu Trp Leu Val Met Glu Phe Cys Gly Ala Gly Ser
   100 105 110
   Val Thr Asp Leu Val Lys Asn Thr Lys Gly Asn Ala Leu Lys Glu Asp
   115 120 125
   Cys Ile Ala Tyr Ile Cys Arg Glu Ile Leu Arg Gly Leu Ala His Leu
   130 135 140
   His Ala His Lys Val Ile His Arg Asp Ile Lys Gly Gln Asn Val Leu
   145 150 155 160
   Leu Thr Glu Asn Ala Glu Val Lys Leu Val Asp Phe Gly Val Ser Ala
   165 170 175
   Gln Leu Asp Arg Thr Val Gly Arg Arg Asn Thr Phe Ile Gly Thr Pro
   180 185 190
   Tyr Trp Met Ala Pro Glu Val Ile Ala Cys Asp Glu Asn Pro Asp Ala
   195 200 205
   Thr Tyr Asp Tyr Arg Ser Asp Ile Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Pro Pro Arg Leu Lys Ser Lys
   245 250 255
   Lys Trp Ser Lys Lys Phe Ile Asp Phe Ile Asp Thr Cys Leu Ile Lys
   260 265 270
   Thr Tyr Leu Ser Arg Pro Pro Thr Glu Gln Leu Leu Lys Phe Pro Phe
   275 280 285
   Ile Arg Asp Gln Pro Thr Glu Arg Gln Val Arg Ile Gln Leu Lys Asp
   290 295 300
   His Ile Asp Arg Ser Arg Lys Lys Arg Gly Glu Lys Glu Glu Thr Glu
   305 310 315 320
   Tyr Glu Tyr Ser Gly Ser Glu Glu Glu Asp Asp Ser His Gly Glu Glu
   325 330 335
   Gly Glu Pro Ser Ser Ile Met Asn Val Pro Gly Glu Ser Thr Leu Arg
   340 345 350
   Arg Glu Phe Leu Arg Leu Gln Gln Glu Asn Lys Ser Asn Ser Glu Ala
   355 360 365
   Leu Lys Gln Gln Gln Gln Leu Gln Gln Gln Gln Gln Arg Asp Pro Glu
   370 375 380
   Ala His Ile Lys His Leu Leu His Gln Arg Gln Arg Arg Ile Glu Glu
   385 390 395 400
   Gln Lys Glu Glu Arg Arg Arg Val Glu Glu Gln Gln Arg Arg Glu Arg
   405 410 415
   Glu Gln Arg Lys Leu Gln Glu Lys Glu Gln Gln Arg Arg Leu Glu Asp
   420 425 430
   Met Gln Ala Leu Arg Arg Glu Glu Glu Arg Arg Gln Ala Glu Arg Glu
   435 440 445
   Gln Glu Tyr Lys Arg Lys Gln Leu Glu Glu Gln Arg Gln Ser Glu Arg
   450 455 460
   Leu Gln Arg Gln Leu Gln Gln Glu His Ala Tyr Leu Lys Ser Leu Gln
   465 470 475 480
   Gln Gln Gln Gln Gln Gln Gln Leu Gln Lys Gln Gln Gln Gln Gln Leu
   485 490 495
   Leu Pro Gly Asp Arg Lys Pro Leu Tyr His Tyr Gly Arg Gly Met Asn
   500 505 510
   Pro Ala Asp Lys Pro Ala Trp Ala Arg Glu Val Glu Glu Arg Thr Arg
   515 520 525
   Met Asn Lys Gln Gln Asn Ser Pro Leu Ala Lys Ser Lys Pro Gly Ser
   530 535 540
   Thr Gly Pro Glu Pro Pro Ile Pro Gln Ala Ser Pro Gly Pro Pro Gly
   545 550 555 560
   Pro Leu Ser Gln Thr Pro Pro Met Gln Arg Pro Val Glu Pro Gln Glu
   565 570 575
   Gly Pro His Lys Ser Leu Val Ala His Arg Val Pro Leu Lys Pro Tyr
   580 585 590
   Ala Ala Pro Val Pro Arg Ser Gln Ser Leu Gln Asp Gln Pro Thr Arg
   595 600 605
   Asn Leu Ala Ala Phe Pro Ala Ser His Asp Pro Asp Pro Ala Ile Pro
   610 615 620
   Ala Pro Thr Ala Thr Pro Ser Ala Arg Gly Ala Val Ile Arg Gln Asn
   625 630 635 640
   Ser Asp Pro Thr Ser Glu Gly Pro Gly Pro Ser Pro Asn Pro Pro Ala
   645 650 655
   Trp Val Arg Pro Asp Asn Glu Ala Pro Pro Lys Val Pro Gln Arg Thr
   660 665 670
   Ser Ser Ile Ala Thr Ala Leu Asn Thr Ser Gly Ala Gly Gly Ser Arg
   675 680 685
   Pro Ala Gln Ala Val Arg Ala Ser Asn Pro Asp Leu Arg Arg Ser Asp
   690 695 700
   Pro Gly Trp Glu Arg Ser Asp Ser Val Leu Pro Ala Ser His Gly His
   705 710 715 720
   Leu Pro Gln Ala Gly Ser Leu Glu Arg Asn Arg Val Gly Val Ser Ser
   725 730 735
   Lys Pro Asp Ser Ser Pro Val Leu Ser Pro Gly Asn Lys Ala Lys Pro
   740 745 750
   Asp Asp His Arg Ser Arg Pro Gly Arg Pro Ala Ser Tyr Lys Arg Ala
   755 760 765
   Ile Gly Glu Asp Phe Val Leu Leu Lys Glu Arg Thr Leu Asp Glu Ala
       770                 775                 780
   Pro Arg Pro Pro Lys Lys Ala Met Asp Tyr Ser Ser Ser Ser Glu Glu
   785                 790                 795                 800
   Val Glu Ser Ser Glu Asp Asp Glu Glu Glu Gly Glu Gly Gly Pro Ala
                   805                 810                 815
   Glu Gly Ser Arg Asp Thr Pro Gly Gly Arg Ser Asp Gly Asp Thr Asp
               820                 825                 830
   Ser Val Ser Thr Met Val Val His Asp Val Glu Glu Ile Thr Gly Thr
           835                 840                 845
   Gln Pro Pro Tyr Gly Gly Gly Thr Met Val Val Gln Arg Thr Pro Glu
       850                 855                 860
   Glu Glu Arg Asn Leu Leu His Ala Asp Ser Asn Gly Tyr Thr Asn Leu
   865                 870                 875                 880
   Pro Asp Val Val Gln Pro Ser His Ser Pro Thr Glu Asn Ser Lys Gly
                   885                 890                 895
   Gln Ser Pro Pro Ser Lys Asp Gly Ser Gly Asp Tyr Gln Ser Arg Gly
               900                 905                 910
   Leu Val Lys Ala Pro Gly Lys Ser Ser Phe Thr Met Phe Val Asp Leu
           915                 920                 925
   Gly Ile Tyr Gln Pro Gly Gly Ser Gly Asp Ser Ile Pro Ile Thr Ala
       930                 935                 940
   Leu Val Gly Gly Glu Gly Thr Arg Leu Asp Gln Leu Gln Tyr Asp Val
   945                 950                 955                 960
   Arg Lys Gly Ser Val Val Asn Val Asn Pro Thr Asn Thr Arg Ala His
                   965                 970                 975
   Ser Glu Thr Pro Glu Ile Arg Lys Tyr Lys Lys Arg Phe Asn Ser Glu
               980                 985                 990
   Ile Leu Cys Ala Ala Leu Trp Gly  Val Asn Leu Leu Val  Gly Thr Glu
           995                 1000                 1005
   Asn Gly  Leu Met Leu Leu Asp  Arg Ser Gly Gln Gly  Lys Val Tyr
   1010 1015 1020
   Gly Leu Ile Gly Arg Arg Arg Phe Gln Gln Met Asp Val Leu Glu
   1025 1030 1035
   Gly Leu Asn Leu Leu Ile Thr Ile Ser Gly Lys Arg Asn Lys Leu
   1040 1045 1050
   Arg Val Tyr Tyr Leu Ser Trp Leu Arg Asn Lys Ile Leu His Asn
   1055 1060 1065
   Asp Pro Glu Val Glu Lys Lys Gln Gly Trp Thr Thr Val Gly Asp
   1070 1075 1080
   Met Glu Gly Cys Gly His Tyr Arg Val Val Lys Tyr Glu Arg Ile
   1085 1090 1095
   Lys Phe Leu Val Ile Ala Leu Lys Ser Ser Val Glu Val Tyr Ala
   1100 1105 1110
   Trp Ala Pro Lys Pro Tyr His Lys Phe Met Ala Phe Lys Ser Phe
   1115 1120 1125
   Ala Asp Leu Pro His Arg Pro Leu Leu Val Asp Leu Thr Val Glu
   1130 1135 1140
   Glu Gly Gln Arg Leu Lys Val Ile Tyr Gly Ser Ser Ala Gly Phe
   1145 1150 1155
   His Ala Val Asp Val Asp Ser Gly Asn Ser Tyr Asp Ile Tyr Ile
   1160 1165 1170
   Pro Val His Ile Gln Ser Gln Ile Thr Pro His Ala Ile Ile Phe
   1175 1180 1185
   Leu Pro Asn Thr Asp Gly Met Glu Met Leu Leu Cys Tyr Glu Asp
   1190 1195 1200
   Glu Gly Val Tyr Val Asn Thr Tyr Gly Arg Ile Ile Lys Asp Val
   1205 1210 1215
   Val Leu Gln Trp Gly Glu Met Pro Thr Ser Val Ala Tyr Ile Cys
   1220 1225 1230
   Ser Asn Gln Ile Met Gly Trp Gly Glu Lys Ala Ile Glu Ile Arg
   1235 1240 1245
   Ser Val Glu Thr Gly His Leu Asp Gly Val Phe Met His Lys Arg
   1250 1255 1260
   Ala Gln Arg Leu Lys Phe Leu Cys Glu Arg Asn Asp Lys Val Phe
   1265 1270 1275
   Phe Ala Ser Val Arg Ser Gly Gly Ser Ser Gln Val Tyr Phe Met
   1280 1285 1290
   Thr Leu Asn Arg Asn Cys Ile Met Asn Trp
   1295 1300
<210> 37
   <211> 3888
   <212> DNA
   <213> Homo sapiens
<400> 37
   atgggcgacc cagcccccgc ccgcagcctg gacgacatcg acctgtccgc cctgcgggac 60
   cctgctggga tctttgagct tgtggaggtg gtcggcaatg gaacctacgg acaggtgtac 120
   aagggtcggc atgtcaagac ggggcagctg gctgccatca aggtcatgga tgtcacggag 180
   gacgaggagg aagagatcaa acaggagatc aacatgctga aaaagtactc tcaccaccgc 240
   aacatcgcca cctactacgg agccttcatc aagaagagcc ccccgggaaa cgatgaccag 300
   ctctggctgg tgatggagtt ctgtggtgct ggttcagtga ctgacctggt aaagaacaca 360
   aaaggcaacg ccctgaagga ggactgtatc gcctatatct gcagggagat cctcaggggt 420
   ctggcccatc tccatgccca caaggtgatc catcgagaca tcaaggggca gaatgtgctg 480
   ctgacagaga atgctgaggt caagctagtg gattttgggg tgagtgctca gctggaccgc 540
   accgtgggca gacggaacac tttcattggg actccctact ggatggctcc agaggtcatc 600
   gcctgtgatg agaaccctga tgccacctat gattacagga gtgatatttg gtctctagga 660
   atcacagcca tcgagatggc agagggagcc ccccctctgt gtgacatgca ccccatgcga 720
   gccctcttcc tcattcctcg gaaccctccg cccaggctca agtccaagaa gtggtctaag 780
   aagttcattg acttcattga cacatgtctc atcaagactt acctgagccg cccacccacg 840
   gagcagctac tgaagtttcc cttcatccgg gaccagccca cggagcggca ggtccgcatc 900
   cagcttaagg accacattga ccgatcccgg aagaagcggg gtgagaaaga ggagacagaa 960
   tatgagtaca gcggcagcga ggaggaagat gacagccatg gagaggaagg agagccaagc 1020
   tccatcatga acgtgcctgg agagtcgact ctacgccggg agtttctccg gctccagcag 1080
   gaaaataaga gcaactcaga ggctttaaaa cagcagcagc agctgcagca gcagcagcag 1140
   cgagaccccg aggcacacat caaacacctg ctgcaccagc ggcagcggcg catagaggag 1200
   cagaaggagg agcggcgccg cgtggaggag caacagcggc gggagcggga gcagcggaag 1260
   ctgcaggaga aggagcagca gcggcggctg gaggacatgc aggctctgcg gcgggaggag 1320
   gagcggcggc aggcggagcg cgagcaggaa tacaagcgga agcagctgga ggagcagcgg 1380
   cagtcagaac gtctccagag gcagctgcag caggagcatg cctacctcaa gtccctgcag 1440
   cagcagcaac agcagcagca gcttcagaaa cagcagcagc agcagctcct gcctggggac 1500
   aggaagcccc tgtaccatta tggtcggggc atgaatcccg ctgacaaacc agcctgggcc 1560
   cgagaggtag aagagagaac aaggatgaac aagcagcaga actctccctt ggccaagagc 1620
   aagccaggca gcacggggcc tgagcccccc atcccccagg cctccccagg gcccccagga 1680
   cccctttccc agactcctcc tatgcagagg ccggtggagc cccaggaggg accgcacaag 1740
   agcctggtgg cacaccgggt cccactgaag ccatatgcag cacctgtacc ccgatcccag 1800
   tccctgcagg accagcccac ccgaaacctg gctgccttcc cagcctccca tgaccccgac 1860
   cctgccatcc ccgcacccac tgccacgccc agtgcccgag gagctgtcat ccgccagaat 1920
   tcagacccca cctctgaagg acctggcccc agcccgaatc ccccagcctg ggtccgccca 1980
   gataacgagg ccccacccaa ggtgcctcag aggacctcat ctatcgccac tgcccttaac 2040
   accagtgggg ccggagggtc ccggccagcc caggcagtcc gtgccagtaa ccccgacctc 2100
   aggaggagcg accctggctg ggaacgctcg gacagcgtcc ttccagcctc tcacgggcac 2160
   ctcccccagg ctggctcact ggagcggaac cgcgtgggag tctcctccaa accggacagc 2220
   tcccctgtgc tctcccctgg gaataaagcc aagcccgacg accaccgctc acggccaggc 2280
   cggcccgcag actttgtgtt gctgaaagag cggactctgg acgaggcccc tcggcctccc 2340
   aagaaggcca tggactactc gtcgtccagc gaggaggtgg aaagcagtga ggacgacgag 2400
   gaggaaggcg aaggcgggcc agcagagggg agcagagata cccctggggg ccgcagcgat 2460
   ggggatacag acagcgtcag caccatggtg gtccacgacg tcgaggagat caccgggacc 2520
   cagcccccat acgggggcgg caccatggtg gtccagcgca cccctgaaga ggagcggaac 2580
   ctgctgcatg ctgacagcaa tgggtacaca aacctgcctg acgtggtcca gcccagccac 2640
   tcacccaccg agaacagcaa aggccaaagc ccaccctcga aggatgggag tggtgactac 2700
   cagtctcgtg ggctggtaaa ggcccctggc aagagctcgt tcacgatgtt tgtggatcta 2760
   gggatctacc agcctggagg cagtggggac agcatcccca tcacagccct agtgggtgga 2820
   gagggcactc ggctcgacca gctgcagtac gacgtgagga agggttctgt ggtcaacgtg 2880
   aatcccacca acacccgggc ccacagtgag acccctgaga tccggaagta caagaagcga 2940
   ttcaactccg agatcctctg tgcagccctt tggggggtca acctgctggt gggcacggag 3000
   aacgggctga tgttgctgga ccgaagtggg cagggcaagg tgtatggact cattgggcgg 3060
   cgacgcttcc agcagatgga tgtgctggag gggctcaacc tgctcatcac catctcaggg   3120
   aaaaggaaca aactgcgggt gtattacctg tcctggctcc ggaacaagat tctgcacaat   3180
   gacccagaag tggagaagaa gcagggctgg accaccgtgg gggacatgga gggctgcggg   3240
   cactaccgtg ttgtgaaata cgagcggatt aagttcctgg tcatcgccct caagagctcc   3300
   gtggaggtgt atgcctgggc ccccaaaccc taccacaaat tcatggcctt caagtccttt   3360
   gccgacctcc cccaccgccc tctgctggtc gacctgacag tagaggaggg gcagcggctc   3420
   aaggtcatct atggctccag tgctggcttc catgctgtgg atgtcgactc ggggaacagc   3480
   tatgacatct acatccctgt gcacatccag agccagatca cgccccatgc catcatcttc   3540
   ctccccaaca ccgacggcat ggagatgctg ctgtgctacg aggacgaggg tgtctacgtc   3600
   aacacgtacg ggcgcatcat taaggatgtg gtgctgcagt ggggggagat gcctacttct   3660
   gtggcctaca tctgctccaa ccagataatg ggctggggtg agaaagccat tgagatccgc   3720
   tctgtggaga cgggccacct cgacggggtc ttcatgcaca aacgagctca gaggctcaag   3780
   ttcctgtgtg agcggaatga caaggtgttt tttgcctcag tccgctctgg gggcagcagc   3840
   caagtttact tcatgactct gaaccgtaac tgcatcatga actggtga                3888
<210> 38
   <211> 1295
   <212> PRT
   <213> Homo sapiens
<400> 38
   Met Gly Asp Pro Ala Pro Ala Arg Ser Leu Asp Asp Ile Asp Leu Ser
   1               5                   10                  15
   Ala Leu Arg Asp Pro Ala Gly Ile Phe Glu Leu Val Glu Val Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Glu Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Ser Pro Pro Gly
                   85                  90                  95
   Asn Asp Asp Gln Leu Trp Leu Val Met Glu Phe Cys Gly Ala Gly Ser
   100 105 110
   Val Thr Asp Leu Val Lys Asn Thr Lys Gly Asn Ala Leu Lys Glu Asp
   115 120 125
   Cys Ile Ala Tyr Ile Cys Arg Glu Ile Leu Arg Gly Leu Ala His Leu
   130 135 140
   His Ala His Lys Val Ile His Arg Asp Ile Lys Gly Gln Asn Val Leu
   145 150 155 160
   Leu Thr Glu Asn Ala Glu Val Lys Leu Val Asp Phe Gly Val Ser Ala
   165 170 175
   Gln Leu Asp Arg Thr Val Gly Arg Arg Asn Thr Phe Ile Gly Thr Pro
   180 185 190
   Tyr Trp Met Ala Pro Glu Val Ile Ala Cys Asp Glu Asn Pro Asp Ala
   195 200 205
   Thr Tyr Asp Tyr Arg Ser Asp Ile Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Pro Pro Arg Leu Lys Ser Lys
   245 250 255
   Lys Trp Ser Lys Lys Phe Ile Asp Phe Ile Asp Thr Cys Leu Ile Lys
   260 265 270
   Thr Tyr Leu Ser Arg Pro Pro Thr Glu Gln Leu Leu Lys Phe Pro Phe
   275 280 285
   Ile Arg Asp Gln Pro Thr Glu Arg Gln Val Arg Ile Gln Leu Lys Asp
   290 295 300
   His Ile Asp Arg Ser Arg Lys Lys Arg Gly Glu Lys Glu Glu Thr Glu
   305 310 315 320
   Tyr Glu Tyr Ser Gly Ser Glu Glu Glu Asp Asp Ser His Gly Glu Glu
   325 330 335
   Gly Glu Pro Ser Ser Ile Met Asn Val Pro Gly Glu Ser Thr Leu Arg
   340 345 350
   Arg Glu Phe Leu Arg Leu Gln Gln Glu Asn Lys Ser Asn Ser Glu Ala
           355                 360                 365
   Leu Lys Gln Gln Gln Gln Leu Gln Gln Gln Gln Gln Arg Asp Pro Glu
       370                 375                 380
   Ala His Ile Lys His Leu Leu His Gln Arg Gln Arg Arg Ile Glu Glu
   385                 390                 395                 400
   Gln Lys Glu Glu Arg Arg Arg Val Glu Glu Gln Gln Arg Arg Glu Arg
                   405                 410                 415
   Glu Gln Arg Lys Leu Gln Glu Lys Glu Gln Gln Arg Arg Leu Glu Asp
               420                 425                 430
   Met Gln Ala Leu Arg Arg Glu Glu Glu Arg Arg Gln Ala Glu Arg Glu
           435                 440                 445
   Gln Glu Tyr Lys Arg Lys Gln Leu Glu Glu Gln Arg Gln Ser Glu Arg
       450                 455                 460
   Leu Gln Arg Gln Leu Gln Gln Glu His Ala Tyr Leu Lys Ser Leu Gln
   465                 470                 475                 480
   Gln Gln Gln Gln Gln Gln Gln Leu Gln Lys Gln Gln Gln Gln Gln Leu
                   485                 490                 495
   Leu Pro Gly Asp Arg Lys Pro Leu Tyr His Tyr Gly Arg Gly Met Asn
               500                 505                 510
   Pro Ala Asp Lys Pro Ala Trp Ala Arg Glu Val Glu Glu Arg Thr Arg
           515                 520                 525
   Met Asn Lys Gln Gln Asn Ser Pro Leu Ala Lys Ser Lys Pro Gly Ser
       530                 535                 540
   Thr Gly Pro Glu Pro Pro Ile Pro Gln Ala Ser Pro Gly Pro Pro Gly
   545                 550                 555                 560
   Pro Leu Ser Gln Thr Pro Pro Met Gln Arg Pro Val Glu Pro Gln Glu
                   565                 570                 575
   Gly Pro His Lys Ser Leu Val Ala His Arg Val Pro Leu Lys Pro Tyr
               580                 585                 590
   Ala Ala Pro Val Pro Arg Ser Gln Ser Leu Gln Asp Gln Pro Thr Arg
   595 600 605
   Asn Leu Ala Ala Phe Pro Ala Ser His Asp Pro Asp Pro Ala Ile Pro
   610 615 620
   Ala Pro Thr Ala Thr Pro Ser Ala Arg Gly Ala Val Ile Arg Gln Asn
   625 630 635 640
   Ser Asp Pro Thr Ser Glu Gly Pro Gly Pro Ser Pro Asn Pro Pro Ala
   645 650 655
   Trp Val Arg Pro Asp Asn Glu Ala Pro Pro Lys Val Pro Gln Arg Thr
   660 665 670
   Ser Ser Ile Ala Thr Ala Leu Asn Thr Ser Gly Ala Gly Gly Ser Arg
   675 680 685
   Pro Ala Gln Ala Val Arg Ala Ser Asn Pro Asp Leu Arg Arg Ser Asp
   690 695 700
   Pro Gly Trp Glu Arg Ser Asp Ser Val Leu Pro Ala Ser His Gly His
   705 710 715 720
   Leu Pro Gln Ala Gly Ser Leu Glu Arg Asn Arg Val Gly Val Ser Ser
   725 730 735
   Lys Pro Asp Ser Ser Pro Val Leu Ser Pro Gly Asn Lys Ala Lys Pro
   740 745 750
   Asp Asp His Arg Ser Arg Pro Gly Arg Pro Ala Asp Phe Val Leu Leu
   755 760 765
   Lys Glu Arg Thr Leu Asp Glu Ala Pro Arg Pro Pro Lys Lys Ala Met
   770 775 780
   Asp Tyr Ser Ser Ser Ser Glu Glu Val Glu Ser Ser Glu Asp Asp Glu
   785 790 795 800
   Glu Glu Gly Glu Gly Gly Pro Ala Glu Gly Ser Arg Asp Thr Pro Gly
   805 810 815
   Gly Arg Ser Asp Gly Asp Thr Asp Ser Val Ser Thr Met Val Val His
   820 825 830
   Asp Val Glu Glu Ile Thr Gly Thr Gln Pro Pro Tyr Gly Gly Gly Thr
   835 840 845
   Met Val Val Gln Arg Thr Pro Glu Glu Glu Arg Asn Leu Leu His Ala
   850 855 860
   Asp Ser Asn Gly Tyr Thr Asn Leu Pro Asp Val Val Gln Pro Ser His
   865 870 875 880
   Ser Pro Thr Glu Asn Ser Lys Gly Gln Ser Pro Pro Ser Lys Asp Gly
   885 890 895
   Ser Gly Asp Tyr Gln Ser Arg Gly Leu Val Lys Ala Pro Gly Lys Ser
   900 905 910
   Ser Phe Thr Met Phe Val Asp Leu Gly Ile Tyr Gln Pro Gly Gly Ser
   915 920 925
   Gly Asp Ser Ile Pro Ile Thr Ala Leu Val Gly Gly Glu Gly Thr Arg
   930 935 940
   Leu Asp Gln Leu Gln Tyr Asp Val Arg Lys Gly Ser Val Val Asn Val
   945 950 955 960
   Asn Pro Thr Asn Thr Arg Ala His Ser Glu Thr Pro Glu Ile Arg Lys
   965 970 975
   Tyr Lys Lys Arg Phe Asn Ser Glu Ile Leu Cys Ala Ala Leu Trp Gly
   980 985 990
   Val Asn Leu Leu Val Gly Thr Glu Asn Gly Leu Met Leu Leu Asp Arg
   995 1000 1005
   Ser Gly Gln Gly Lys Val Tyr Gly Leu Ile Gly Arg Arg Arg Phe
   1010 1015 1020
   Gln Gln Met Asp Val Leu Glu Gly Leu Asn Leu Leu Ile Thr Ile
   1025 1030 1035
   Ser Gly Lys Arg Asn Lys Leu Arg Val Tyr Tyr Leu Ser Trp Leu
   1040 1045 1050
   Arg Asn Lys Ile Leu His Asn Asp Pro Glu Val Glu Lys Lys Gln
   1055 1060 1065
   Gly Trp Thr Thr Val Gly Asp Met Glu Gly Cys Gly His Tyr Arg
   1070 1075 1080
   Val Val Lys Tyr Glu Arg Ile Lys Phe Leu Val Ile Ala Leu Lys
   1085 1090 1095
   Ser Ser Val Glu Val Tyr Ala Trp Ala Pro Lys Pro Tyr His Lys
   1100 1105 1110
   Phe Met Ala Phe Lys Ser Phe Ala Asp Leu Pro His Arg Pro Leu
   1115 1120 1125
   Leu Val Asp Leu Thr Val Glu Glu Gly Gln Arg Leu Lys Val Ile
   1130 1135 1140
   Tyr Gly Ser Ser Ala Gly Phe His Ala Val Asp Val Asp Ser Gly
   1145 1150 1155
   Asn Ser Tyr Asp Ile Tyr Ile Pro Val His Ile Gln Ser Gln Ile
   1160 1165 1170
   Thr Pro His Ala Ile Ile Phe Leu Pro Asn Thr Asp Gly Met Glu
   1175 1180 1185
   Met Leu Leu Cys Tyr Glu Asp Glu Gly Val Tyr Val Asn Thr Tyr
   1190 1195 1200
   Gly Arg Ile Ile Lys Asp Val Val Leu Gln Trp Gly Glu Met Pro
   1205 1210 1215
   Thr Ser Val Ala Tyr Ile Cys Ser Asn Gln Ile Met Gly Trp Gly
   1220 1225 1230
   Glu Lys Ala Ile Glu Ile Arg Ser Val Glu Thr Gly His Leu Asp
   1235 1240 1245
   Gly Val Phe Met His Lys Arg Ala Gln Arg Leu Lys Phe Leu Cys
   1250 1255 1260
   Glu Arg Asn Asp Lys Val Phe Phe Ala Ser Val Arg Ser Gly Gly
   1265 1270 1275
   Ser Ser Gln Val Tyr Phe Met Thr Leu Asn Arg Asn Cys Ile Met
   1280 1285 1290
   Asn Trp
   1295
<210> 39
   <211> 3864
   <212> DNA
   <213> Homo sapiens
<400> 39
   aattcgagga tccgggtacc atggcacaga gcgacagaga catttattgt tatttgtttt 60
   ttggtggcaa aaagggaaaa tggcgaacga ctcccctgca aaaagtctgg tggacatcga 120
   cctctcctcc ctgcgggatc ctgctgggat ttttgagctg gtggaagtgg ttggaaatgg 180
   cacctatgga caagtctata agggtcgaca tgttaaaacg ggtcagttgg cagccatcaa 240
   agttatggat gtcactgagg atgaagagga agaaatcaaa ctggagataa atatgctaaa 300
   gaaatactct catcacagaa acattgcaac atattatggt gctttcatca aaaagagccc 360
   tccaggacat gatgaccaac tctggcttgt tatggagttc tgtggggctg ggtccattac 420
   agaccttgtg aagaacacca aagggaacac actcaaagaa gactggatcg cttacatctc 480
   cagagaaatc ctgaggggac tggcacatct tcacattcat catgtgattc accgggatat 540
   caagggccag aatgtgttgc tgactgagaa tgcagaggtg aaacttgttg actttggtgt 600
   gagtgctcag ctggacagga ctgtggggcg gagaaatacg ttcataggca ctccctactg 660
   gatggctcct gaggtcatcg cctgtgatga gaacccagat gccacctatg attacagaag 720
   tgatctttgg tcttgtggca ttacagccat tgagatggca gaaggtgctc cccctctctg 780
   tgacatgcat ccaatgagag cactgtttct cattcccaga aaccctcctc cccggctgaa 840
   gtcaaaaaaa tggtcgaaga agttttttag ttttatagaa gggtgcctgg tgaagaatta 900
   catgcagcgg ccctctacag agcagctttt gaaacatcct tttataaggg atcagccaaa 960
   tgaaaggcaa gttagaatcc agcttaagga tcatatagat cgtaccagga agaagagagg 1020
   cgagaaagat gaaactgagt atgagtacag tgggagtgag gaagaagagg aggaagtgcc 1080
   tgaacaggaa ggagagccaa gttccattgt gaacgtgcct ggtgagtcta ctcttcgccg 1140
   agatttcctg agactgcagc aggagaacaa ggaacgttcc gaggctcttc ggagacaaca 1200
   gttactacag gagcaacagc tccgggagca ggaagaatat aaaaggcaac tgctggcaga 1260
   gagacagaag cggattgagc agcagaaaga acagaggcga cggctagaag agcaacaaag 1320
   gagagagcgg gaggctagaa ggcagcagga acgtgaacag cgaaggagag aacaagaaga 1380
   aaagaggcgt ctagaggagt tggagagaag gcgcaaagaa gaagaggaga ggagacgggc 1440
   agaagaagaa aagaggagag ttgaaagaga acaggagtat atcaggcgac agctagaaga 1500
   ggagcagcgg cacttggaag tccttcagca gcagctgctc caggagcagg ccatgttact 1560
   gcatgaccat aggaggccgc acccgcagca ctcgcagcag ccgccaccac cgcagcagga 1620
   aaggagcaag ccaagcttcc atgctcccga gcccaaagcc cactacgagc ctgctgaccg 1680
   agcgcgagag gttcctgtga gaacaacatc tcgctcccct gttctgtccc gtcgagattc 1740
   cccactgcag ggcagtgggc agcagaatag ccaggcagga cagagaaact ccaccagtat 1800
   tgagcccagg cttctgtggg agagagtgga gaagctggtg cccagacctg gcagtggcag 1860
   ctcctcaggg tccagcaact caggatccca gcccgggtct caccctgggt ctcagagtgg 1920
   ctccggggaa cgcttcagag tgagatcatc atccaagtct gaaggctctc catctcagcg 1980
   cctggaaaat gcagtgaaaa aacctgaaga taaaaaggaa gttttcagac ccctcaagcc 2040
   tgctggcgaa gtggatctga ccgcactggc caaagagctt cgagcagtgg aagatgtacg 2100
   gccacctcac aaagtaacgg actactcctc atccagtgag gagtcgggga cgacggatga 2160
   ggaggacgac gatgtggagc aggaaggggc tgacgagtcc acctcaggac cagaggacac 2220
   cagagcagcg tcatctctga atttgagcaa tggtgaaacg gaatctgtga aaaccatgat 2280
   tgtccatgat gatgtagaaa gtgagccggc catgacccca tccaaggagg gcactctaat 2340
   cgtccgccag actcagtccg ctagtagcac actccagaaa cacaaatctt cctcctcctt 2400
   tacacctttt atagacccca gattactaca gatttctcca tctagcggaa caacagtgac 2460
   atctgtggtg ggattttcct gtgatgggat gagaccagaa gccataaggc aagatcctac 2520
   ccggaaaggc tcagtggtca atgtgaatcc taccaacact aggccacaga gtgacacccc 2580
   ggagattcgt aaatacaaga agaggtttaa ctctgagatt ctgtgtgctg ccttatgggg 2640
   agtgaatttg ctagtgggta cagagagtgg cctgatgctg ctggacagaa gtggccaagg 2700
   gaaggtctat cctcttatca accgaagacg atttcaacaa atggacgtac ttgagggctt 2760
   gaatgtcttg gtgacaatat ctggcaaaaa ggataagtta cgtgtctact atttgtcctg 2820
   gttaagaaat aaaatacttc acaatgatcc agaagttgag aagaagcagg gatggacaac 2880
   cgtaggggat ttggaaggat gtgtacatta taaagttgta aaatatgaaa gaatcaaatt 2940
   tctggtgatt gctttgaaga gttctgtgga agtctatgcg tgggcaccaa agccatatca 3000
   caaatttatg gcctttaagt catttggaga attggtacat aagccattac tggtggatct 3060
   cactgttgag gaaggccaga ggttgaaagt gatctatgga tcctgtgctg gattccatgc 3120
   tgttgatgtg gattcaggat cagtctatga catttatcta ccaacacatg taagaaagaa 3180
   cccacactct atgatccagt gtagcatcaa accccatgca atcatcatcc tccccaatac 3240
   agatggaatg gagcttctgg tgtgctatga agatgagggg gtttatgtaa acacatatgg 3300
   aaggatcacc aaggatgtag ttctacagtg ggagagatgg cctacatcag tagcatatat 3360
   tcgatccaat cagacaatgg gctggggaga gaaggccata gagatccgat ctgtggaaac 3420
   tggtcacttg gatggtgtgt tcatgcacaa aagggctcaa agactaaaat tcttgtgtga 3480
   acgcaatgac aaggtgttct ttgcctctgt tcggtctggt ggcagcagtc aggtttattt 3540
   catgacctta ggcaggactt ctcttctgag ctggtagaag cagtgtgatc cagggattac 3600
   tggcctccag agtcttcaag atcctgagaa cttggaattc cttgtaactg gagctcggag 3660
   ctgcaccgag ggcaaccagg acagctgtgt gtgcagacct catgtgttgg gttctctccc    3720
   ctccttcctg ttcctcttat ataccagttt atccccattc tttttttttt tcttactcca     3780
   aaataaatca aggctgcaat gcagctggtg ctgttcagat tccaaaaaaa aaaaaaaacc     3840
   atggtacccg gatcctcgaa ttcc                                           3864
<210> 40
   <211> 1165
   <212> PRT
   <213> Homo sapiens
<400> 40
   Met Ala Asn Asp Ser Pro Ala Lys Ser Leu Val Asp Ile Asp Leu Ser
   1               5                   10                  15
   Ser Leu Arg Asp Pro Ala Gly Ile Phe Glu Leu Val Glu Val Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Glu Asp Glu Glu Glu
       50                  55                  60
   Glu Ile Lys Leu Glu Ile Asn Met Leu Lys Lys Tyr Ser His His Arg
   65                  70                  75                  80
   Asn Ile Ala Thr Tyr Tyr Gly Ala Phe Ile Lys Lys Ser Pro Pro Gly
                   85                  90                  95
   His Asp Asp Gln Leu Trp Leu Val Met Glu Phe Cys Gly Ala Gly Ser
               100                 105                 110
   Ile Thr Asp Leu Val Lys Asn Thr Lys Gly Asn Thr Leu Lys Glu Asp
           115                 120                 125
   Trp Ile Ala Tyr Ile Ser Arg Glu Ile Leu Arg Gly Leu Ala His Leu
       130                 135                 140
   His Ile His His Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
   180 185 190
   Tyr Trp Met Ala Pro Glu Val Ile Ala Cys Asp Glu Asn Pro Asp Ala
   195 200 205
   Thr Tyr Asp Tyr Arg Ser Asp Leu Trp Ser Cys Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Pro Pro Arg Leu Lys Ser Lys
   245 250 255
   Lys Trp Ser Lys Lys Phe Phe Ser Phe Ile Glu Gly Cys Leu Val Lys
   260 265 270
   Asn Tyr Met Gln Arg Pro Ser Thr Glu Gln Leu Leu Lys His Pro Phe
   275 280 285
   Ile Arg Asp Gln Pro Asn Glu Arg Gln Val Arg Ile Gln Leu Lys Asp
   290 295 300
   His Ile Asp Arg Thr Arg Lys Lys Arg Gly Glu Lys Asp Glu Thr Glu
   305 310 315 320
   Tyr Glu Tyr Ser Gly Ser Glu Glu Glu Glu Glu Glu Val Pro Glu Gln
   325 330 335
   Glu Gly Glu Pro Ser Ser Ile Val Asn Val Pro Gly Glu Ser Thr Leu
   340 345 350
   Arg Arg Asp Phe Leu Arg Leu Gln Gln Glu Asn Lys Glu Arg Ser Glu
   355 360 365
   Ala Leu Arg Arg Gln Gln Leu Leu Gln Glu Gln Gln Leu Arg Glu Gln
   370 375 380
   Glu Glu Tyr Lys Arg Gln Leu Leu Ala Glu Arg Gln Lys Arg Ile Glu
   385 390 395 400
   Gln Gln Lys Glu Gln Arg Arg Arg Leu Glu Glu Gln Gln Arg Arg Glu
   405 410 415
   Arg Glu Ala Arg Arg Gln Gln Glu Arg Glu Gln Arg Arg Arg Glu Gln
   420 425 430
   Glu Glu Lys Arg Arg Leu Glu Glu Leu Glu Arg Arg Arg Lys Glu Glu
   435 440 445
   Glu Glu Arg Arg Arg Ala Glu Glu Glu Lys Arg Arg Val Glu Arg Glu
   450 455 460
   Gln Glu Tyr Ile Arg Arg Gln Leu Glu Glu Glu Gln Arg His Leu Glu
   465 470 475 480
   Val Leu Gln Gln Gln Leu Leu Gln Glu Gln Ala Met Leu Leu His Asp
   485 490 495
   His Arg Arg Pro His Pro Gln His Ser Gln Gln Pro Pro Pro Pro Gln
   500 505 510
   Gln Glu Arg Ser Lys Pro Ser Phe His Ala Pro Glu Pro Lys Ala His
   515 520 525
   Tyr Glu Pro Ala Asp Arg Ala Arg Glu Val Pro Val Arg Thr Thr Ser
   530 535 540
   Arg Ser Pro Val Leu Ser Arg Arg Asp Ser Pro Leu Gln Gly Ser Gly
   545 550 555 560
   Gln Gln Asn Ser Gln Ala Gly Gln Arg Asn Ser Thr Ser Ile Glu Pro
   565 570 575
   Arg Leu Leu Trp Glu Arg Val Glu Lys Leu Val Pro Arg Pro Gly Ser
   580 585 590
   Gly Ser Ser Ser Gly Ser Ser Asn Ser Gly Ser Gln Pro Gly Ser His
   595 600 605
   Pro Gly Ser Gln Ser Gly Ser Gly Glu Arg Phe Arg Val Arg Ser Ser
   610 615 620
   Ser Lys Ser Glu Gly Ser Pro Ser Gln Arg Leu Glu Asn Ala Val Lys
   625 630 635 640
   Lys Pro Glu Asp Lys Lys Glu Val Phe Arg Pro Leu Lys Pro Ala Gly
   645 650 655
   Glu Val Asp Leu Thr Ala Leu Ala Lys Glu Leu Arg Ala Val Glu Asp
   660 665 670
   Val Arg Pro Pro His Lys Val Thr Asp Tyr Ser Ser Ser Ser Glu Glu
   675 680 685
   Ser Gly Thr Thr Asp Glu Glu Asp Asp Asp Val Glu Gln Glu Gly Ala
   690 695 700
   Asp Glu Ser Thr Ser Gly Pro Glu Asp Thr Arg Ala Ala Ser Ser Leu
   705 710 715 720
   Asn Leu Ser Asn Gly Glu Thr Glu Ser Val Lys Thr Met Ile Val His
   725 730 735
   Asp Asp Val Glu Ser Glu Pro Ala Met Thr Pro Ser Lys Glu Gly Thr
   740 745 750
   Leu Ile Val Arg Gln Thr Gln Ser Ala Ser Ser Thr Leu Gln Lys His
   755 760 765
   Lys Ser Ser Ser Ser Phe Thr Pro Phe Ile Asp Pro Arg Leu Leu Gln
   770 775 780
   Ile Ser Pro Ser Ser Gly Thr Thr Val Thr Ser Val Val Gly Phe Ser
   785 790 795 800
   Cys Asp Gly Met Arg Pro Glu Ala Ile Arg Gln Asp Pro Thr Arg Lys
   805 810 815
   Gly Ser Val Val Asn Val Asn Pro Thr Asn Thr Arg Pro Gln Ser Asp
   820 825 830
   Thr Pro Glu Ile Arg Lys Tyr Lys Lys Arg Phe Asn Ser Glu Ile Leu
   835 840 845
   Cys Ala Ala Leu Trp Gly Val Asn Leu Leu Val Gly Thr Glu Ser Gly
   850 855 860
   Leu Met Leu Leu Asp Arg Ser Gly Gln Gly Lys Val Tyr Pro Leu Ile
   865 870 875 880
   Asn Arg Arg Arg Phe Gln Gln Met Asp Val Leu Glu Gly Leu Asn Val
   885 890 895
   Leu Val Thr Ile Ser Gly Lys Lys Asp Lys Leu Arg Val Tyr Tyr Leu
   900 905 910
   Ser Trp Leu Arg Asn Lys Ile Leu His Asn Asp Pro Glu Val Glu Lys
   915 920 925
   Lys Gln Gly Trp Thr Thr Val Gly Asp Leu Glu Gly Cys Val His Tyr
   930 935 940
   Lys Val Val Lys Tyr Glu Arg Ile Lys Phe Leu Val Ile Ala Leu Lys
   945 950 955 960
   Ser Ser Val Glu Val Tyr Ala Trp Ala Pro Lys Pro Tyr His Lys Phe
   965 970 975
   Met Ala Phe Lys Ser Phe Gly Glu Leu Val His Lys Pro Leu Leu Val
   980 985 990
   Asp Leu Thr Val Glu Glu Gly Gln Arg Leu Lys Val Ile Tyr Gly Ser
   995 1000 1005
   Cys Ala Gly Phe His Ala Val Asp Val Asp Ser Gly Ser Val Tyr
   1010 1015 1020
   Asp Ile Tyr Leu Pro Thr His Val Arg Lys Asn Pro His Ser Met
   1025 1030 1035
   Ile Gln Cys Ser Ile Lys Pro His Ala Ile Ile Ile Leu Pro Asn
   1040 1045 1050
   Thr Asp Gly Met Glu Leu Leu Val Cys Tyr Glu Asp Glu Gly Val
   1055 1060 1065
   Tyr Val Asn Thr Tyr Gly Arg Ile Thr Lys Asp Val Val Leu Gln
   1070 1075 1080
   Trp Gly Glu Met Pro Thr Ser Val Ala Tyr Ile Arg Ser Asn Gln
   1085 1090 1095
   Thr Met Gly Trp Gly Glu Lys Ala Ile Glu Ile Arg Ser Val Glu
   1100 1105 1110
   Thr Gly His Leu Asp Gly Val Phe Met His Lys Arg Ala Gln Arg
   1115 1120 1125
   Leu Lys Phe Leu Cys Glu Arg Asn Asp Lys Val Phe Phe Ala Ser
   1130 1135 1140
   Val Arg Ser Gly Gly Ser Ser Gln Val Tyr Phe Met Thr Leu Gly
   1145 1150 1155
   Arg Thr Ser Leu Leu Ser Trp
   1160 1165
<210> 41
   <211> 1523
   <212> DNA
   <213> Homo sapiens
<400> 41
   ggcacgaggc agcgccatgc tgggctcgcg agccgcgggg ttcgcgcggg gcctgcgggc      60
   tttggcactg gcgtggctgc cgggctggcg gggccgctcc ttcgccctgg cgcgtgcggc     120
   aggcgcgccc cacggtggtg acttgcagcc ccccgcctgt cccgagccgc gcgggcgcca     180
   gctcagtttg tccgcggcgg cggtggtgga ctctgcgccc cgccccctgc agccgtactt     240
   gcgcctcatg cggttggaca agcccattgg aacctggctt ctgtatttac catgtacctg     300
   gagcattggt ttggcagctg aaccaggttg ttttccagat tggtacatgc tctccctctt     360
   tggcactgga gctattctga tgcgtggagc aggctgtact attaatgaca tgtgggacca     420
   ggactatgat aaaaaggtta caagaacagc caatcgtcca atagccgctg gagacatttc     480
   aacttttcag tcctttgttt ttcttggggg acagctaacc ctggcactgg gtgttcttct     540
   gtgtctaaat tactacagta tagctctggg agcaggatcc ttacttcttg tcatcaccta     600
   cccactaatg aaaagaattt catactggcc tcaactagcc ttgggcttga catttaattg     660
   gggagcgtta cttggatggt ctgctatcaa gggttcctgt gatccatctg tttgcctgcc     720
   tctttatttt tctggagtta tgtggacact aatatatgac actatttatg cccatcagga     780
   caaaagagat gatgttttga ttggtcttaa gtcaacggct ctgcggttcg gagaaaatac     840
   caagccgtgg ctcagcggct tcagtgttgc aatgctgggg gcactgagcc tagtgggtgt     900
   gaacagtgga cagactgctc cctactacgc tgccctgggt gctgtaggag cccatctgac     960
   tcaccagatt tacactctag acatccacag acctgaggat tgttggaata aatttatctc    1020
   caaccgaaca ctgggactaa tagttttttt agggattgtc cttgggaatt tgtggaaaga    1080
   aaagaagaca gacaaaacaa agaagggtat agagaataaa atagaaaatt aatgaatgaa    1140
   atttatctag gaatttttaa aacatttttt acaaaatata attagatttg aatacaaaat    1200
   ctgatacaat atgttaaaga attaagaacc tgaagatgaa gatttagagc atatttacct    1260
   ggattttact tatttgctag caaaattccc ccttgtcaca gaaaccaggg actcttcagg    1320
   atttgagatg gccttgagta ttttagttga tacattcttc tgcccattat aattctcacc    1380
   tgaagttatg gggattgcac gggttttggc actttagaaa aagcctgatg tgggtcttac    1440
   ataaatgaat gtctgtataa gaaaatggac tctttttttt agggaaaaat aaaagcaact    1500
   atgggaaaaa aaaaaaaaaa aaa                                            1523
<210> 42
   <211> 371
   <212> PRT
   <213> Homo sapiens
<400> 42
   Met Leu Gly Ser Arg Ala Ala Gly Phe Ala Arg Gly Leu Arg Ala Leu
   1               5                   10                  15
   Ala Leu Ala Trp Leu Pro Gly Trp Arg Gly Arg Ser Phe Ala Leu Ala
               20                  25                  30
   Arg Ala Ala Gly Ala Pro His Gly Gly Asp Leu Gln Pro Pro Ala Cys
           35                  40                  45
   Pro Glu Pro Arg Gly Arg Gln Leu Ser Leu Ser Ala Ala Ala Val Val
       50                  55                  60
   Asp Ser Ala Pro Arg Pro Leu Gln Pro Tyr Leu Arg Leu Met Arg Leu
   65                  70                  75                  80
   Asp Lys Pro Ile Gly Thr Trp Leu Leu Tyr Leu Pro Cys Thr Trp Ser
                   85                  90                  95
   Ile Gly Leu Ala Ala Glu Pro Gly Cys Phe Pro Asp Trp Tyr Met Leu
               100                 105                 110
   Ser Leu Phe Gly Thr Gly Ala Ile Leu Met Arg Gly Ala Gly Cys Thr
           115                 120                 125
   Ile Asn Asp Met Trp Asp Gln Asp Tyr Asp Lys Lys Val Thr Arg Thr
       130                 135                 140
   Ala Asn Arg Pro Ile Ala Ala Gly Asp Ile Ser Thr Phe Gln Ser Phe
   145                 150                 155                 160
   Val Phe Leu Gly Gly Gln Leu Thr Leu Ala Leu Gly Val Leu Leu Cys
                   165                 170                 175
   Leu Asn Tyr Tyr Ser Ile Ala Leu Gly Ala Gly Ser Leu Leu Leu Val
               180                 185                 190
   Ile Thr Tyr Pro Leu Met Lys Arg Ile Ser Tyr Trp Pro Gln Leu Ala
           195                 200                 205
   Leu Gly Leu Thr Phe Asn Trp Gly Ala Leu Leu Gly Trp Ser Ala Ile
       210                 215                 220
   Lys Gly Ser Cys Asp Pro Ser Val Cys Leu Pro Leu Tyr Phe Ser Gly
   225 230 235 240
   Val Met Trp Thr Leu Ile Tyr Asp Thr Ile Tyr Ala His Gln Asp Lys
   245 250 255
   Arg Asp Asp Val Leu Ile Gly Leu Lys Ser Thr Ala Leu Arg Phe Gly
   260 265 270
   Glu Asn Thr Lys Pro Trp Leu Ser Gly Phe Ser Val Ala Met Leu Gly
   275 280 285
   Ala Leu Ser Leu Val Gly Val Asn Ser Gly Gln Thr Ala Pro Tyr Tyr
   290 295 300
   Ala Ala Leu Gly Ala Val Gly Ala His Leu Thr His Gln Ile Tyr Thr
   305 310 315 320
   Leu Asp Ile His Arg Pro Glu Asp Cys Trp Asn Lys Phe Ile Ser Asn
   325 330 335
   Arg Thr Leu Gly Leu Ile Val Phe Leu Gly Ile Val Leu Gly Asn Leu
   340 345 350
   Trp Lys Glu Lys Lys Thr Asp Lys Thr Lys Lys Gly Ile Glu Asn Lys
   355 360 365
   Ile Glu Asn
   370
<210> 43
   <211> 3131
   <212> DNA
   <213> Homo sapiens
<400> 43
   cgagccgtgc ggccagagcg ggaaagagac tcgtctttgc gtccgagttc tggagccgcc 60
   gcaccccgac tcctggggcc gcggcagcgg ctgcgagggg acgggcgtcc gctgtctcct 120
   gggttcccct cgtagcgacc cgcgggatcg gaaaaaaagg agaagatgga ggaggagggt 180
   ggcagcagcg gcggcgccgc ggggaccagc gcggacggcg gcgacggagg agagcagctc 240
   ctcactgtca agcacgagct gcggactgct aatttgacag gacatgctga gaaggtggga 300
   atagaaaatt ttgagctcct gaaggtccta ggaactggag cttatggaaa agtatttcta 360
   gttcgtaaaa taagtggcca tgatactgga aagctgtatg ccatgaaagt tttgaaaaag 420
   gcaacaatcg ttcaaaaggc caaaaccaca gagcatacaa ggacagaacg acaagtcctg 480
   gaacacatta ggcagtcgcc atttttggta acattacatt atgctttcca gacagaaacc 540
   aaacttcatc tcattttaga ttatataaat ggtggtgaac tttttactca tctttctcaa 600
   agagagcgtt tcacagagca tgaggtgcag atttatgttg gagagattgt gcttgccctc 660
   gaacatctcc acaagttggg gattatatat cgtgatatta agcttgagaa tattctactt 720
   gattctaatg gccatgtggt gctgacagat tttggtctga gtaaggagtt tgtggctgat 780
   gaaactgaaa gagcatattc cttttgtgga actattgaat acatggcacc agatattgtc 840
   agagggggag attcaggaca tgacaaggca gttgactggt ggagtttggg tgttctaatg 900
   tatgaattac taactggagc atctcctttc actgttgatg gagaaaaaaa ttcccaagct 960
   gagatatcta ggagaatatt aaaaagtgag cctccatatc cccaagaaat gagtgcttta 1020
   gcgaaagacc taattcagcg tcttttgatg aaagatccca agaagagatt gggatgtggt 1080
   ccacgtgatg cagatgaaat caaagaacat ctcttctttc agaaaataaa ttgggatgat 1140
   ttagccgcca aaaaagtgcc tgcaccattt aagccagtca ttcgagatga attagatgtg 1200
   agtaactttg cagaagagtt cacagaaatg gatcccactt attctcccgc agccctgccc 1260
   cagagttctg agaagctgtt tcagggctat tcctttgttg ctccttccat cctattcaag 1320
   cgtaatgcag ctgtcataga ccctcttcag tttcacatgg gagttgaacg tcctggagtg 1380
   acaaatgttg ccaggagtgc aatgatgaag gactctccat tctatcaaca ctatgaccta 1440
   gatttgaagg acaaacccct gggagaaggt agtttttcaa tttgtcgaaa gtgtgtgcat 1500
   aaaaaaagta accaagcttt tgcagtcaaa ataatcagca aaaggatgga agccaatact 1560
   caaaaggaaa taacagctct gaaactctgt gaaggacacc ccaatattgt gaagttgcat 1620
   gaagtttttc atgatcagct tcacacgttt ctagtgatgg aacttctgaa tggaggagaa 1680
   ctgtttgagc gcattaagaa aaagaagcac ttcagtgaga cggaagccag ctacatcatg 1740
   aggaagcttg tttcagctgt aagccacatg catgatgttg gagtggtgca cagggatctg 1800
   aaacctgaga atttattgtt caccgatgaa aatgacaatt tggaaattaa aataattgat 1860
   tttggatttg cacggctaaa gccaccggat aatcagcccc tgaagactcc atgcttcacc 1920
   cttcattatg ccgccccaga gctcttgaat cagaacggct acgatgagtc ctgtgacctg 1980
   tggagcttgg gcgtcatttt gtacacaatg ttgtcaggac aggttccctt ccaatctcat 2040
   gaccgaagtt tgacgtgtac cagcgcggtg gaaatcatga agaaaattaa aaagggagat 2100
   ttctcctttg aaggagaagc ctggaagaat gtatcccaag aggctaaaga tttgatccaa 2160
   ggacttctca cagtagatcc aaacaaaagg cttaaaatgt ctggcttgag gtacaatgaa 2220
   tggctacaag atggaagtca gctgtcctcc aatcctctga tgactccgga tattctagga 2280
   tcttccggag ctgccgtgca tacctgtgtg aaagcaacct tccacgcctt taacaaatac 2340
   aagagagagg ggttttgcct tcagaatgtt gataaggccc ctttggctaa gagaagaaaa    2400
   atgaaaaaga ctagcaccag taccgagacg cgcagcagtt ccagtgagag ttcccattct    2460
   tcttcctctc attctcacgg taaaactaca cccaccaaga cactgcagcc cagcaatcct    2520
   gccgacagca ataacccgga gaccctcttc cagttctcgg actcagtagc ttaggcatgg    2580
   taggagtgta tcagtgatcc attgcacctt tattccctca gcatatgcct gaggcgatct    2640
   tttatgcttt taaaaatgtt tcccgttggt ctcattggaa tctgcctcct aatgattttt    2700
   tttcaggaaa acctgtttgg ttatcctcat tcaaaagcac tggacagaga atgttactgt    2760
   gaatagagca catattactc tttttagcaa cctagcatga tgccaacaag actatttttg    2820
   aaagagcaaa ggttcctgta aatttaatta gggctagatt tgagctgctt gtaagtcaca    2880
   ggttttccag atgtctgcca acaagaaatg actcatactg tgatgatacc ttttgctttg    2940
   ccttgtggac aatgtgggtt tttgaaattt gcacccttca aacaatgatt tatcagagaa    3000
   aggggtctgt tttcaaaaaa gattctgtaa tgaattttat gtgtggcata tacttatttc    3060
   ttgagagaag attttaactt attgttttta ttttatggtt acatatgatg ataacctgct    3120
   attattaaac t                                                         3131
<210> 44
   <211> 802
   <212> PRT
   <213> Homo sapiens
<400> 44
   Met Glu Glu Glu Gly Gly Ser Ser Gly Gly Ala Ala Gly Thr Ser Ala
   1               5                   10                  15
   Asp Gly Gly Asp Gly Gly Glu Gln Leu Leu Thr Val Lys His Glu Leu
               20                  25                  30
   Arg Thr Ala Asn Leu Thr Gly His Ala Glu Lys Val Gly Ile Glu Asn
           35                  40                  45
   Phe Glu Leu Leu Lys Val Leu Gly Thr Gly Ala Tyr Gly Lys Val Phe
       50                  55                  60
   Leu Val Arg Lys Ile Ser Gly His Asp Thr Gly Lys Leu Tyr Ala Met
   65                  70                  75                  80
   Lys Val Leu Lys Lys Ala Thr Ile Val Gln Lys Ala Lys Thr Thr Glu
                   85                  90                  95
   His Thr Arg Thr Glu Arg Gln Val Leu Glu His Ile Arg Gln Ser Pro
   100 105 110
   Phe Leu Val Thr Leu His Tyr Ala Phe Gln Thr Glu Thr Lys Leu His
   115 120 125
   Leu Ile Leu Asp Tyr Ile Asn Gly Gly Glu Leu Phe Thr His Leu Ser
   130 135 140
   Gln Arg Glu Arg Phe Thr Glu His Glu Val Gln Ile Tyr Val Gly Glu
   145 150 155 160
   Ile Val Leu Ala Leu Glu His Leu His Lys Leu Gly Ile Ile Tyr Arg
   165 170 175
   Asp Ile Lys Leu Glu Asn Ile Leu Leu Asp Ser Asn Gly His Val Val
   180 185 190
   Leu Thr Asp Phe Gly Leu Ser Lys Glu Phe Val Ala Asp Glu Thr Glu
   195 200 205
   Arg Ala Tyr Ser Phe Cys Gly Thr Ile Glu Tyr Met Ala Pro Asp Ile
   210 215 220
   Val Arg Gly Gly Asp Ser Gly His Asp Lys Ala Val Asp Trp Trp Ser
   225 230 235 240
   Leu Gly Val Leu Met Tyr Glu Leu Leu Thr Gly Ala Ser Pro Phe Thr
   245 250 255
   Val Asp Gly Glu Lys Asn Ser Gln Ala Glu Ile Ser Arg Arg Ile Leu
   260 265 270
   Lys Ser Glu Pro Pro Tyr Pro Gln Glu Met Ser Ala Leu Ala Lys Asp
   275 280 285
   Leu Ile Gln Arg Leu Leu Met Lys Asp Pro Lys Lys Arg Leu Gly Cys
   290 295 300
   Gly Pro Arg Asp Ala Asp Glu Ile Lys Glu His Leu Phe Phe Gln Lys
   305 310 315 320
   Ile Asn Trp Asp Asp Leu Ala Ala Lys Lys Val Pro Ala Pro Phe Lys
   325 330 335
   Pro Val Ile Arg Asp Glu Leu Asp Val Ser Asn Phe Ala Glu Glu Phe
   340 345 350
   Thr Glu Met Asp Pro Thr Tyr Ser Pro Ala Ala Leu Pro Gln Ser Ser
   355 360 365
   Glu Lys Leu Phe Gln Gly Tyr Ser Phe Val Ala Pro Ser Ile Leu Phe
   370 375 380
   Lys Arg Asn Ala Ala Val Ile Asp Pro Leu Gln Phe His Met Gly Val
   385 390 395 400
   Glu Arg Pro Gly Val Thr Asn Val Ala Arg Ser Ala Met Met Lys Asp
   405 410 415
   Ser Pro Phe Tyr Gln His Tyr Asp Leu Asp Leu Lys Asp Lys Pro Leu
   420 425 430
   Gly Glu Gly Ser Phe Ser Ile Cys Arg Lys Cys Val His Lys Lys Ser
   435 440 445
   Asn Gln Ala Phe Ala Val Lys Ile Ile Ser Lys Arg Met Glu Ala Asn
   450 455 460
   Thr Gln Lys Glu Ile Thr Ala Leu Lys Leu Cys Glu Gly His Pro Asn
   465 470 475 480
   Ile Val Lys Leu His Glu Val Phe His Asp Gln Leu His Thr Phe Leu
   485 490 495
   Val Met Glu Leu Leu Asn Gly Gly Glu Leu Phe Glu Arg Ile Lys Lys
   500 505 510
   Lys Lys His Phe Ser Glu Thr Glu Ala Ser Tyr Ile Met Arg Lys Leu
   515 520 525
   Val Ser Ala Val Ser His Met His Asp Val Gly Val Val His Arg Asp
   530 535 540
   Leu Lys Pro Glu Asn Leu Leu Phe Thr Asp Glu Asn Asp Asn Leu Glu
   545 550 555 560
   Ile Lys Ile Ile Asp Phe Gly Phe Ala Arg Leu Lys Pro Pro Asp Asn
   565 570 575
   Gln Pro Leu Lys Thr Pro Cys Phe Thr Leu His Tyr Ala Ala Pro Glu
   580 585 590
   Leu Leu Asn Gln Asn Gly Tyr Asp Glu Ser Cys Asp Leu Trp Ser Leu
           595                 600                 605
   Gly Val Ile Leu Tyr Thr Met Leu Ser Gly Gln Val Pro Phe Gln Ser
       610                 615                 620
   His Asp Arg Ser Leu Thr Cys Thr Ser Ala Val Glu Ile Met Lys Lys
   625                 630                 635                 640
   Ile Lys Lys Gly Asp Phe Ser Phe Glu Gly Glu Ala Trp Lys Asn Val
                   645                 650                 655
   Ser Gln Glu Ala Lys Asp Leu Ile Gln Gly Leu Leu Thr Val Asp Pro
               660                 665                 670
   Asn Lys Arg Leu Lys Met Ser Gly Leu Arg Tyr Asn Glu Trp Leu Gln
           675                 680                 685
   Asp Gly Ser Gln Leu Ser Ser Asn Pro Leu Met Thr Pro Asp Ile Leu
       690                 695                 700
   Gly Ser Ser Gly Ala Ala Val His Thr Cys Val Lys Ala Thr Phe His
   705                 710                 715                 720
   Ala Phe Asn Lys Tyr Lys Arg Glu Gly Phe Cys Leu Gln Asn Val Asp
                   725                 730                 735
   Lys Ala Pro Leu Ala Lys Arg Arg Lys Met Lys Lys Thr Ser Thr Ser
               740                 745                 750
   Thr Glu Thr Arg Ser Ser Ser Ser Glu Ser Ser His Ser Ser Ser Ser
           755                 760                 765
   His Ser His Gly Lys Thr Thr Pro Thr Lys Thr Leu Gln Pro Ser Asn
       770                 775                 780
   Pro Ala Asp Ser Asn Asn Pro Glu Thr Leu Phe Gln Phe Ser Asp Ser
   785                 790                 795                 800
   Val Ala
<210> 45
   <211> 3120
   <212> DNA
   <213> Mus musculus
<400> 45
   gtaaccggcg ccagccgtag ccccagccgc gcggccccag cgacccgccg ccatgggaga      60
   cgaggatgag gacgagggct gcgccgtgga gctgcagatc accgaagcca acctcaccgg 120
   gcatgaggag aaggtgagcg tggagaactt cgcgctgctc aaggtgctgg gcacgggagc 180
   ctatgggaag gtgttcctgg tgcggaagac gggtgggcac gacgcgggca agctctatgc 240
   catgaaggtg ctacgcaagg cggcgttggt gcagcgcgcg aagacacagg agcatacccg 300
   caccgaacgc tcggtgctgg agctggttcg ccaagcaccc ttcctggtca cactgcacta 360
   cgccttccag acggatgcca agctgcacct catcctggac tacgtgagcg gtggtgagat 420
   gttcactcac ctctaccagc gccagtactt caaggaggct gaggttcgag tgtatggggg 480
   cgagattgtg ctggccctgg aacacctgca caagctgggt atcatctacc gggacctgaa 540
   gctggagaac gtcttacttg actcagaagg tcacatcgtc cttacagact ttgggctgag 600
   caaggagttc ctgacggagg agaaagagcg gaccttctcc ttctgtggca caatcgagta 660
   catggctccc gaaatcatcc gaagcaaggc tggacatggc aaggctgtgg actggtggag 720
   cctgggtatc ctgctcttcg agctgctgac aggggcctca cccttcacac tggagggaga 780
   gaggaacact caggctgagg tgtcccgacg gatcttgaag tgctcccctc ccttccctct 840
   ccggattggg cctgtggcac aggacctgct acagcggctg ctgtgcaagg accctaagaa 900
   gaggttgggc gcaggtcccc agggtgcgca ggaagtcaag agtcacccct tcttccaggg 960
   tctggactgg tgggctctgg ctgccagaaa gatcccagcc ccattccggc cccagatccg 1020
   ctcagagctg gatgtgggga attttgcgga ggaattcacc cggctggagc ccgtctactc 1080
   ccctgcaggc agccctccac ctggggaccc tcggatcttt cagggatact ccttcgtggc 1140
   tccgtccatc ctctttgacc acaacaatgc agtgatggct gatgtactgc aggcaccggg 1200
   tgccggatac aggcccggca gggcagcagt tgccaggagt gccatgatgc aggactcgcc 1260
   tttcttccag cagtacgaac tggaccttcg ggagccagcg ctggggcagg gcagcttctc 1320
   tgtgtgtcgg agatgtaggc agcgccagag cggccaggag tttgctgtca agatcctcag 1380
   ccgcaggctg gaggagaaca ctcagcgaga ggtggctgct cttcgcctgt gccagtcaca 1440
   ccccaacgtg gtgaatctgc atgaggtgct tcatgaccag ctacacactt acctggtcct 1500
   ggagttgctg cgaggcggag agctattgga acacatccgc aagaagcggc tcttcagcga 1560
   gtcggaggcc agccagatcc ttcggagcct ggtttcggcc gtgagcttca tgcacgagga 1620
   ggcaggcgtg gtgcaccgcg acctgaaacc cgagaacatc ttgtacgcgg acgacactcc 1680
   cggggccccg gtgaagatca tcgacttcgg gttcgcgcga ctgcggcccc agagcccggc 1740
   agagcccatg cagactcctt gcttcacact gcagtacgct gcacccgagc tgctggcaca 1800
   gcagggctac gatgagtcct gcgatctatg gagcctgggt gtcattctgt acatgatgct 1860
   gtctggccag gttcccttcc aaggggcctc cggccagggt ggacagagtc aggcagctga 1920
   gatcatgtgc aagatccgtg aagggcgctt ctccctggac ggggaagcct ggcaaggtgt   1980
   gtcggaggaa gccaaggagc tggtccgagg gctactgaca gtcgaccccg ccaagcggct   2040
   gaagctggag gggctgcgta gcagctcgtg gcttcaggac ggcagcgcgc gctcctcgcc   2100
   cccgctccgc acgccggatg tgctggagtc ctctgggcca gctgtgcgtt ccgggctcaa   2160
   tgccactttc atggcgttca accgaggcaa gcgcgagggc ttctttctca agagtgtaga   2220
   gaatgcgcct ctggccaaga ggcgcaagca gaagctccgg agcgcggccg cctcacgcag   2280
   aggctcccca gtgcctgcct cctccggtcg cctaccagcc tctgccgcta aggggacaac   2340
   tcgccgagcc aacggcccct tgtccccttc ctagctcccg ccactgtgac ccccttacct   2400
   ttaggagctc cgatccggga gcctggtgct gccaccagac ttttgccagc ggctcccact   2460
   ctgatcccta ggaattgccc tcccctccac acccacttcc agacagagca gaagtatttt   2520
   tataagcaga gattttttaa aagtgtctta ctagataaga gttggggaca cagagaagca   2580
   actgttgtga gcaaagtgga gcttggggct ccctcgcccg atcccaatgc tgccccatca   2640
   ggaagagaga aggtcctttc caggagcaac tctgctaaga aagggcatcc tatcctgttc   2700
   ctaagtaccc agttgccatg gagaagaaat caggagcccg tatactgtgc ttccctggga   2760
   ctttgcccct aggaggggac acccatcaat gtcactttat ggaccgtgtg caatgatgtc   2820
   caccaaagac ctgtgttggg aatgagagag agagagaccc tgagggacct ctagagcatt   2880
   tctgagatac atcacttttg tcaacggctc ccctagtggg gttaaggaag aaggcaggtg   2940
   ctccccaggg ggagcccctt tctcactcac ccattcctca tgggcacagc tgcttctggc   3000
   tgcaggtgga gccttagggt tctgggctag gcatccacag tcactgcctc agcccctcta   3060
   ggctgtgcct ttgactttaa aataaaactc cacccagtgc tgcaaaaaaa aaaaaaaaaa   3120
<210> 46
   <211> 773
   <212> PRT
   <213> Mus musculus
<400> 46
   Met Gly Asp Glu Asp Glu Asp Glu Gly Cys Ala Val Glu Leu Gln Ile
   1               5                   10                  15
   Thr Glu Ala Asn Leu Thr Gly His Glu Glu Lys Val Ser Val Glu Asn
               20                  25                  30
   Phe Ala Leu Leu Lys Val Leu Gly Thr Gly Ala Tyr Gly Lys Val Phe
           35                  40                  45
   Leu Val Arg Lys Thr Gly Gly His Asp Ala Gly Lys Leu Tyr Ala Met
   50 55 60
   Lys Val Leu Arg Lys Ala Ala Leu Val Gln Arg Ala Lys Thr Gln Glu
   65 70 75 80
   His Thr Arg Thr Glu Arg Ser Val Leu Glu Leu Val Arg Gln Ala Pro
   85 90 95
   Phe Leu Val Thr Leu His Tyr Ala Phe Gln Thr Asp Ala Lys Leu His
   100 105 110
   Leu Ile Leu Asp Tyr Val Ser Gly Gly Glu Met Phe Thr His Leu Tyr
   115 120 125
   Gln Arg Gln Tyr Phe Lys Glu Ala Glu Val Arg Val Tyr Gly Gly Glu
   130 135 140
   Ile Val Leu Ala Leu Glu His Leu His Lys Leu Gly Ile Ile Tyr Arg
   145 150 155 160
   Asp Leu Lys Leu Glu Asn Val Leu Leu Asp Ser Glu Gly His Ile Val
   165 170 175
   Leu Thr Asp Phe Gly Leu Ser Lys Glu Phe Leu Thr Glu Glu Lys Glu
   180 185 190
   Arg Thr Phe Ser Phe Cys Gly Thr Ile Glu Tyr Met Ala Pro Glu Ile
   195 200 205
   Ile Arg Ser Lys Ala Gly His Gly Lys Ala Val Asp Trp Trp Ser Leu
   210 215 220
   Gly Ile Leu Leu Phe Glu Leu Leu Thr Gly Ala Ser Pro Phe Thr Leu
   225 230 235 240
   Glu Gly Glu Arg Asn Thr Gln Ala Glu Val Ser Arg Arg Ile Leu Lys
   245 250 255
   Cys Ser Pro Pro Phe Pro Leu Arg Ile Gly Pro Val Ala Gln Asp Leu
   260 265 270
   Leu Gln Arg Leu Leu Cys Lys Asp Pro Lys Lys Arg Leu Gly Ala Gly
   275 280 285
   Pro Gln Gly Ala Gln Glu Val Lys Ser His Pro Phe Phe Gln Gly Leu
   290 295 300
   Asp Trp Trp Ala Leu Ala Ala Arg Lys Ile Pro Ala Pro Phe Arg Pro
   305 310 315 320
   Gln Ile Arg Ser Glu Leu Asp Val Gly Asn Phe Ala Glu Glu Phe Thr
   325 330 335
   Arg Leu Glu Pro Val Tyr Ser Pro Ala Gly Ser Pro Pro Pro Gly Asp
   340 345 350
   Pro Arg Ile Phe Gln Gly Tyr Ser Phe Val Ala Pro Ser Ile Leu Phe
   355 360 365
   Asp His Asn Asn Ala Val Met Ala Asp Val Leu Gln Ala Pro Gly Ala
   370 375 380
   Gly Tyr Arg Pro Gly Arg Ala Ala Val Ala Arg Ser Ala Met Met Gln
   385 390 395 400
   Asp Ser Pro Phe Phe Gln Gln Tyr Glu Leu Asp Leu Arg Glu Pro Ala
   405 410 415
   Leu Gly Gln Gly Ser Phe Ser Val Cys Arg Arg Cys Arg Gln Arg Gln
   420 425 430
   Ser Gly Gln Glu Phe Ala Val Lys Ile Leu Ser Arg Arg Leu Glu Glu
   435 440 445
   Asn Thr Gln Arg Glu Val Ala Ala Leu Arg Leu Cys Gln Ser His Pro
   450 455 460
   Asn Val Val Asn Leu His Glu Val Leu His Asp Gln Leu His Thr Tyr
   465 470 475 480
   Leu Val Leu Glu Leu Leu Arg Gly Gly Glu Leu Leu Glu His Ile Arg
   485 490 495
   Lys Lys Arg Leu Phe Ser Glu Ser Glu Ala Ser Gln Ile Leu Arg Ser
   500 505 510
   Leu Val Ser Ala Val Ser Phe Met His Glu Glu Ala Gly Val Val His
   515 520 525
   Arg Asp Leu Lys Pro Glu Asn Ile Leu Tyr Ala Asp Asp Thr Pro Gly
   530 535 540
   Ala Pro Val Lys Ile Ile Asp Phe Gly Phe Ala Arg Leu Arg Pro Gln
   545 550 555 560
   Ser Pro Ala Glu Pro Met Gln Thr Pro Cys Phe Thr Leu Gln Tyr Ala
   565 570 575
   Ala Pro Glu Leu Leu Ala Gln Gln Gly Tyr Asp Glu Ser Cys Asp Leu
   580 585 590
   Trp Ser Leu Gly Val Ile Leu Tyr Met Met Leu Ser Gly Gln Val Pro
   595 600 605
   Phe Gln Gly Ala Ser Gly Gln Gly Gly Gln Ser Gln Ala Ala Glu Ile
   610 615 620
   Met Cys Lys Ile Arg Glu Gly Arg Phe Ser Leu Asp Gly Glu Ala Trp
   625 630 635 640
   Gln Gly Val Ser Glu Glu Ala Lys Glu Leu Val Arg Gly Leu Leu Thr
   645 650 655
   Val Asp Pro Ala Lys Arg Leu Lys Leu Glu Gly Leu Arg Ser Ser Ser
   660 665 670
   Trp Leu Gln Asp Gly Ser Ala Arg Ser Ser Pro Pro Leu Arg Thr Pro
   675 680 685
   Asp Val Leu Glu Ser Ser Gly Pro Ala Val Arg Ser Gly Leu Asn Ala
   690 695 700
   Thr Phe Met Ala Phe Asn Arg Gly Lys Arg Glu Gly Phe Phe Leu Lys
   705 710 715 720
   Ser Val Glu Asn Ala Pro Leu Ala Lys Arg Arg Lys Gln Lys Leu Arg
   725 730 735
   Ser Ala Ala Ala Ser Arg Arg Gly Ser Pro Val Pro Ala Ser Ser Gly
   740 745 750
   Arg Leu Pro Ala Ser Ala Ala Lys Gly Thr Thr Arg Arg Ala Asn Gly
   755 760 765
   Pro Leu Ser Pro Ser
   770
<210> 47
   <211> 1470
   <212> DNA
   <213> Homo sapiens
<400> 47
   gacggtcacc cgttgccagc tctagccttt aaattcccgg ctcggggacc tccacgcacc     60
   gcggctagcg ccgacaacca gctagcgtgc aaggcgccgc ggctcagcgc gtaccggcgg    120
   gtttcgaaac cgcagtcctc cggcgacccc gaactccgct ccggagcctc agccccctgg   180
   aaagtgatcc cggcatcgga gagccaagat gccggcccac ttgctgcagg acgatatctc    240
   tagctcctat accaccacca ccaccattac agcgcctcct ccaggggtcc tgcagaatgg    300
   aggagataag ttggagacga tgcccctcta cttggaagac gacattcgcc ctgatataaa    360
   agatgatata tatgacccca cctacaagga taaggaaggc ccaagcccca aggttgaata    420
   tgtctggaga aacatcatcc ttatgtctct gctacacttg ggagccctgt atgggatcac    480
   tttgattcct acctgcaagt tctacacctg gctttggggg gtattctact attttgtcag    540
   tgccctgggc ataacagcag gagctcatcg tctgtggagc caccgctctt acaaagctcg    600
   gctgccccta cggctctttc tgatcattgc caacacaatg gcattccaga atgatgtcta    660
   tgaatgggct cgtgaccacc gtgcccacca caagttttca gaaacacatg ctgatcctca    720
   taattcccga cgtggctttt tcttctctca cgtgggttgg ctgcttgtgc gcaaacaccc    780
   agctgtcaaa gagaagggga gtacgctaga cttgtctgac ctagaagctg agaaactggt    840
   gatgttccag aggaggtact acaaacctgg cttgctgatg atgtgcttca tcctgcccac    900
   gcttgtgccc tggtatttct ggggtgaaac ttttcaaaac agtgtgttcg ttgccacttt    960
   cttgcgatat gctgtggtgc ttaatgccac ctggctggtg aacagtgctg cccacctctt   1020
   cggatatcgt ccttatgaca agaacattag cccccgggag aatatcctgg tttcacttgg   1080
   agctgtgggt gagggcttcc acaactacca ccactccttt ccctatgact actctgccag   1140
   tgagtaccgc tggcacatca acttcaacac attcttcatt gattggatgg ccgccctcgg   1200
   tctgacctat gaccggaaga aagtctccaa ggccgccatc ttggccagga ttaaaagaac   1260
   cggagatgga aactacaaga gtggctgagt ttggggtccc tcaggttcct ttttcaaaaa   1320
   ccagccaggc agaggtttta atgtctgttt attaactact gaataatgct accaggatgc   1380
   taaagatgat gatgttaacc cattccagta cagtattctt ttaaaattca aaagtattga   1440
   aagccaaaaa aaaaaaaaaa aaaaaaaaaa                                    1470
<210> 48
   <211> 359
   <212> PRT
   <213> Homo sapiens
<400> 48
   Met Pro Ala His Leu Leu Gln Asp Asp Ile Ser Ser Ser Tyr Thr Thr
   1 5 10 15
   Thr Thr Thr Ile Thr Ala Pro Pro Pro Gly Val Leu Gln Asn Gly Gly
   20 25 30
   Asp Lys Leu Glu Thr Met Pro Leu Tyr Leu Glu Asp Asp Ile Arg Pro
   35 40 45
   Asp Ile Lys Asp Asp Ile Tyr Asp Pro Thr Tyr Lys Asp Lys Glu Gly
   50 55 60
   Pro Ser Pro Lys Val Glu Tyr Val Trp Arg Asn Ile Ile Leu Met Ser
   65 70 75 80
   Leu Leu His Leu Gly Ala Leu Tyr Gly Ile Thr Leu Ile Pro Thr Cys
   85 90 95
   Lys Phe Tyr Thr Trp Leu Trp Gly Val Phe Tyr Tyr Phe Val Ser Ala
   100 105 110
   Leu Gly Ile Thr Ala Gly Ala His Arg Leu Trp Ser His Arg Ser Tyr
   115 120 125
   Lys Ala Arg Leu Pro Leu Arg Leu Phe Leu Ile Ile Ala Asn Thr Met
   130 135 140
   Ala Phe Gln Asn Asp Val Tyr Glu Trp Ala Arg Asp His Arg Ala His
   145 150 155 160
   His Lys Phe Ser Glu Thr His Ala Asp Pro His Asn Ser Arg Arg Gly
   165 170 175
   Phe Phe Phe Ser His Val Gly Trp Leu Leu Val Arg Lys His Pro Ala
   180 185 190
   Val Lys Glu Lys Gly Ser Thr Leu Asp Leu Ser Asp Leu Glu Ala Glu
   195 200 205
   Lys Leu Val Met Phe Gln Arg Arg Tyr Tyr Lys Pro Gly Leu Leu Met
   210 215 220
   Met Cys Phe Ile Leu Pro Thr Leu Val Pro Trp Tyr Phe Trp Gly Glu
   225 230 235 240
   Thr Phe Gln Asn Ser Val Phe Val Ala Thr Phe Leu Arg Tyr Ala Val
   245 250 255
   Val Leu Asn Ala Thr Trp Leu Val Asn Ser Ala Ala His Leu Phe Gly
   260 265 270
   Tyr Arg Pro Tyr Asp Lys Asn Ile Ser Pro Arg Glu Asn Ile Leu Val
   275 280 285
   Ser Leu Gly Ala Val Gly Glu Gly Phe His Asn Tyr His His Ser Phe
   290 295 300
   Pro Tyr Asp Tyr Ser Ala Ser Glu Tyr Arg Trp His Ile Asn Phe Asn
   305 310 315 320
   Thr Phe Phe Ile Asp Trp Met Ala Ala Leu Gly Leu Thr Tyr Asp Arg
   325 330 335
   Lys Lys Val Ser Lys Ala Ala Ile Leu Ala Arg Ile Lys Arg Thr Gly
   340 345 350
   Asp Gly Asn Tyr Lys Ser Gly
   355
<210> 49
   <211> 5228
   <212> DNA
   <213> Homo sapiens
<400> 49
   ggatccggat tcggattagc agcccgggaa gagtgccgtg gcacaggcgc cggagggagc 60
   gcgaccctcg gaccccgcct ggcccgcggg gctgggaccc ggccccggcc tgcccgatgg 120
   ggcgcgcggc cccggagatg cgccctcgcc cggccccgcg cccccggccc cgcgcccccg 180
   gcccgcccgc cccggcccgc gcctccgcct gagtcccccg cgccttggcc cgccaccccc 240
   cgccccgcgc ccccggcccg cctgcgccat ggagcccggc cgcggcggca cagagaccgt 300
   gggcaagttc gagttctccc gcaaggacct gatcggccac ggcgccttcg cggtggtctt 360
   caagggccgc caccgcgaga agcacgattt ggaggtcgcc gtcaagtgca ttaacaagaa 420
   gaacctcgcc aagtctcaga cgctgctggg gaaggaaatc aaaatcctga aggaactgaa 480
   acatgaaaac atcgtggccc tgtacgactt ccaggaaatg gctaattctg tctacctggt 540
   tatggagtac tgcaacggtg gggacctggc cgactacctg cacgccatgc gcacgctgag 600
   cgaggacacc atcaggctct tcctgcagca gatcgcgggc gccatgcggc ttctgcacag 660
   caaaggcatc atccaccgcg acctgaaacc gcagaacatc ctgctgtcca accccgccgg 720
   ccgccgcgcc aaccccaaca gcatccgcgt caagatcgct gacttcggct tcgcgcggta 780
   cctccagagc aacatgatgg cggccacact ctgcggctcc cccatgtaca tggcccccga 840
   ggtcatcatg tcccagcact acgacgggaa ggcggacctg tggagcatcg gcaccatcgt 900
   ctaccagtgc ctgacgggga aggcgccctt ccaggccagc agcccccagg acctgcgcct 960
   gttctacgag aagaacaaga cgttggtccc caccatcccc cgggagacct cggccccgct 1020
   gcggcagctg ctcctggccc tactgcaacg caaccacaag gaccgcatgg acttcgatga 1080
   gttttttcat caccctttcc tcgatgccag cccctcggtc aggaaatccc cacccgtgcc 1140
   tgtgccctcg tacccaagct cggggtccgg cagcagctcc agcagcagct ccacctccca 1200
   cctggcctcc ccgccgtccc tgggcgagat gcagcagctg cagaagaccc tggcctcccc 1260
   ggctgacacc gctggcttcc tgcacagctc ccgggactct ggtggcagca aggactcttc 1320
   ctgtgacaca gacgacttcg tcatggtccc cgcgcagttt ccaggtgacc tggtggctga 1380
   ggcgcccagt gccaaacccc cgccagacag cctgatgtgc agtgggagct cactggtggc 1440
   ctctgcgggc ttggagagcc acggccggac cccatctcca tccccaccct gcagcagctc 1500
   ccccagtccc tcaggccggg ctggcccgtt ctccagcagc aggtgcggcg cctctgtccc 1560
   catcccagtc cccacgcagg tgcagaacta ccagcgcatt gagcgaaacc tgcagtcacc 1620
   cacccagttc caaacacctc ggtcctctgc catccgcagg tcaggcagca ccagccccct 1680
   gggctttgca agggccagcc cctcgccccc tgcccacgct gagcatggag gcgtcctggc 1740
   caggaagatg tctctgggtg gaggccggcc ctacacgcca tctcctcaag ttggaaccat 1800
   ccctgagcgg ccaggctgga gcgggacgcc ctccccacag ggagctgaga tgcggggtgg 1860
   caggtcccct cgtccaggct cctctgcacc cgagcactct ccccgcactt ccgggctggg 1920
   ctgccgcctg cacagcgccc ccaacctgtc tgacttgcac gtcgtccgcc ccaagctgcc 1980
   caaacccccc acggaccccc tgggagctgt gttcagccca ccacaggcca gccctcccca 2040
   gccgtcccac ggcctgcagt cctgccggaa cctgcggggc tcacccaagc tgcccgactt 2100
   cctgcagcga aaccccctgc cccccatcct gggctccccc accaaggctg tgccctcctt 2160
   tgacttcccg aagaccccca gctcccagaa cctgctggcc ctcctagccc ggcagggcgt 2220
   ggtgatgacg ccccctcgaa accggacgct gcccgacctc tcggaggtgg gacccttcca 2280
   tggtcagccg ttgggccctg gcctgcggcc aggcgaggac cccaagggcc cctttggccg 2340
   gtctttcagc accagccgcc tcactgacct gctccttaag gcggcgtttg ggacacaagc 2400
   cccggacccg ggcagcacgg agagcctgca ggagaagccc atggagatcg caccctcagc 2460
   tggctttgga gggagcctgc acccaggagc ccgtgctggg ggcaccagca gcccctcccc 2520
   ggtggtcttc accgtgggct ctcccccgag cgggagcacg cccccccagg gcccccgcac 2580
   caggatgttc tcagcgggcc ccactggctc tgccagctct tctgcccgcc acctggtgcc 2640
   tgggccctgc agcgaggccc cagcccctga gctccctgct ccaggacacg gctgcagctt 2700
   tgccgacccc attgctgcga acctggaggg ggctgtgacc ttcgaggccc ccgacctccc 2760
   tgaggagacc ctcatggagc aagagcacac ggagatcctg cgtggcctgc gcttcacgct 2820
   gctgttcgtg cagcacgtcc tggagatcgc agccctgaag ggcagcgcca gtgaggcggc 2880
   ggggggccct gagtaccagc tgcaggagag tgtggtggcc gaccagatca gcctgctgag 2940
   ccgagaatgg ggcttcgcgg aacagctggt gctgtacctg aaggtggccg agctactgtc 3000
   ctccggcctg caaagtgcca tcgaccagat ccgggccggc aagctctgcc tgtcgtccac 3060
   tgtgaagcag gtggtgcgca ggctgaatga gctgtacaag gccagcgtgg tgtcctgcca 3120
   gggcctgagc ctgcggctgc agcgcttctt cctggacaag cagcggctcc tggaccgcat 3180
   tcacagcatc actgccgaga ggctcatctt cagccacgct gtgcagatgg tgcagtcggc 3240
   tgccctggac gagatgttcc agcaccgtga gggctgcgtc ccacgctacc acaaggccct 3300
   gctgctcctg gaggggctgc agcacatgct ctcggaccag gccgacatcg agaacgtcac 3360
   caagtgcaag ctgtgcattg agcggagact ctcggcgctg ctgactggca tctgtgcctg 3420
   acctttctgg cctggctggg ccccccgtcc tgccgagccc tgcagagtgg gctctgtgtg 3480
   ctggctggac tcctcgggac aagcccatgg cgctgatcgc tggtgctgag ccctgccctg 3540
   ggccccacgg acagtcagcc tgccggcctc cctgcagctc acggggcaga accagcacat 3600
   ctggagccac acagcttggg gggtgtctcc catcttttac aggtggggat cacagaattt 3660
   ctgcccctcc agctgcctgg ctcagcaggc gtgggtgcca ccaccctcta gccccagggc 3720
   agccccggag gacaggcaag ggcctgagac cactgccgac tcaaagccaa agcgagctcc 3780
   tgcttagggc aggtcagcag gcactgtgcc caggaagagc ctgcggcctc ggcgtccccc 3840
   agtctccagg agcctctccc tccgagatac ccacccagct ttgtcaatca cccaagcact 3900
   ttatgcatat agagacagaa cctggacctc accagggact gctgggcagc gattcctggc 3960
   agtggcctgg tgtttgtaca tacacatatg cagacacatg ccagggcccc ccaagcccga 4020
   gcaccggacc acgttgctgc ccaggtctgg acctcagcgg gagaactggc tccgggggga 4080
   gtggggccct gcgctagagg cagaggcagt tctttgttca agcgttcctc tggggaccgg 4140
   cagcagaggc accgtgttct ctcagccctg gatacgtctt gtaatctttc acactttatt 4200
   cctaaaacgt gtcttatttt tatgcagctc attttttctt taaaggagaa aacttgtagg 4260
   tgtttaagaa ttggttttgg gagggcgagg actgggccag gttagaggca gatggcacag 4320
   gggcgtgtgg cgggcgggtg aggctgcttt gcacacctgt gttggtggct gtcccctgcc 4380
   gcccctccct gtggcagcag caggacaggt gtgtgcccag caccctccct acctgggcct 4440
   ggaagcagat gaggggaata cttcatgcaa agaaaaaagt aacatgtgca aaagctcccc 4500
   gtccagcttt gacagtcagt tttgatgtca gctcctcggc agggtaggcc tgatgacagc 4560
   cctgtccctc cctgcctccg ccttgcccaa ggccacggag ggcgtctgca gagaggcctg 4620
   ccttccggat tccaggcggg catgccctgc aaaccccgcc tgggcctccc ttggtctgcc 4680
   cagccctcgg ttagccctgc ctgaatcagt agatacttga acgagtcccc agtctgcggg 4740
   aggcagtggt ggggccatgg acccatgcgg ggggttccag ggtcacacgc cacataacag 4800
   acaaaaatac acacacgtgt gtttttcttt gcaatacttg aaatattgcc actgtgcttg 4860
   gacttagaag aagaaaatcc ccgtgacttc ttcctcatca ccttgatggc tttattctca 4920
   ccttgtgggg catgtttgaa tttattgctt catggccgac tggaatcctg agtcctggga 4980
   agctggcact gcggggatct tgcccggtgt cctggtcctc ttgcttccgt cgcggccgca 5040
   tgtgcgtgtg tccaagcagg tcctgggcgc ctcaactgct gcccctggtt gaatgttctc 5100
   ttgatagtgc tggacccttt gtctatttta aagcgaattt tgtgtgattt cctgcccttt 5160
   gcgttatatt gtataatacc aacgtaagga aataaacctt tggaattgtt gaaaaaaaaa 5220
   aaaaaaaa 5228
<210> 50
   <211> 1050
   <212> PRT
   <213> Homo sapiens
<400> 50
   Met Glu Pro Gly Arg Gly Gly Thr Glu Thr Val Gly Lys Phe Glu Phe
   1 5 10 15
   Ser Arg Lys Asp Leu Ile Gly His Gly Ala Phe Ala Val Val Phe Lys
   20 25 30
   Gly Arg His Arg Glu Lys His Asp Leu Glu Val Ala Val Lys Cys Ile
   35 40 45
   Asn Lys Lys Asn Leu Ala Lys Ser Gln Thr Leu Leu Gly Lys Glu Ile
   50 55 60
   Lys Ile Leu Lys Glu Leu Lys His Glu Asn Ile Val Ala Leu Tyr Asp
   65 70 75 80
   Phe Gln Glu Met Ala Asn Ser Val Tyr Leu Val Met Glu Tyr Cys Asn
   85 90 95
   Gly Gly Asp Leu Ala Asp Tyr Leu His Ala Met Arg Thr Leu Ser Glu
   100 105 110
   Asp Thr Ile Arg Leu Phe Leu Gln Gln Ile Ala Gly Ala Met Arg Leu
   115 120 125
   Leu His Ser Lys Gly Ile Ile His Arg Asp Leu Lys Pro Gln Asn Ile
   130 135 140
   Leu Leu Ser Asn Pro Ala Gly Arg Arg Ala Asn Pro Asn Ser Ile Arg
   145 150 155 160
   Val Lys Ile Ala Asp Phe Gly Phe Ala Arg Tyr Leu Gln Ser Asn Met
   165 170 175
   Met Ala Ala Thr Leu Cys Gly Ser Pro Met Tyr Met Ala Pro Glu Val
   180 185 190
   Ile Met Ser Gln His Tyr Asp Gly Lys Ala Asp Leu Trp Ser Ile Gly
   195 200 205
   Thr Ile Val Tyr Gln Cys Leu Thr Gly Lys Ala Pro Phe Gln Ala Ser
   210 215 220
   Ser Pro Gln Asp Leu Arg Leu Phe Tyr Glu Lys Asn Lys Thr Leu Val
   225 230 235 240
   Pro Thr Ile Pro Arg Glu Thr Ser Ala Pro Leu Arg Gln Leu Leu Leu
   245 250 255
   Ala Leu Leu Gln Arg Asn His Lys Asp Arg Met Asp Phe Asp Glu Phe
   260 265 270
   Phe His His Pro Phe Leu Asp Ala Ser Pro Ser Val Arg Lys Ser Pro
   275 280 285
   Pro Val Pro Val Pro Ser Tyr Pro Ser Ser Gly Ser Gly Ser Ser Ser
   290 295 300
   Ser Ser Ser Ser Thr Ser His Leu Ala Ser Pro Pro Ser Leu Gly Glu
   305 310 315 320
   Met Gln Gln Leu Gln Lys Thr Leu Ala Ser Pro Ala Asp Thr Ala Gly
   325 330 335
   Phe Leu His Ser Ser Arg Asp Ser Gly Gly Ser Lys Asp Ser Ser Cys
   340 345 350
   Asp Thr Asp Asp Phe Val Met Val Pro Ala Gln Phe Pro Gly Asp Leu
   355 360 365
   Val Ala Glu Ala Pro Ser Ala Lys Pro Pro Pro Asp Ser Leu Met Cys
   370 375 380
   Ser Gly Ser Ser Leu Val Ala Ser Ala Gly Leu Glu Ser His Gly Arg
   385 390 395 400
   Thr Pro Ser Pro Ser Pro Pro Cys Ser Ser Ser Pro Ser Pro Ser Gly
   405 410 415
   Arg Ala Gly Pro Phe Ser Ser Ser Arg Cys Gly Ala Ser Val Pro Ile
   420 425 430
   Pro Val Pro Thr Gln Val Gln Asn Tyr Gln Arg Ile Glu Arg Asn Leu
   435 440 445
   Gln Ser Pro Thr Gln Phe Gln Thr Pro Arg Ser Ser Ala Ile Arg Arg
   450 455 460
   Ser Gly Ser Thr Ser Pro Leu Gly Phe Ala Arg Ala Ser Pro Ser Pro
   465 470 475 480
   Pro Ala His Ala Glu His Gly Gly Val Leu Ala Arg Lys Met Ser Leu
   485 490 495
   Gly Gly Gly Arg Pro Tyr Thr Pro Ser Pro Gln Val Gly Thr Ile Pro
   500 505 510
   Glu Arg Pro Gly Trp Ser Gly Thr Pro Ser Pro Gln Gly Ala Glu Met
   515 520 525
   Arg Gly Gly Arg Ser Pro Arg Pro Gly Ser Ser Ala Pro Glu His Ser
   530 535 540
   Pro Arg Thr Ser Gly Leu Gly Cys Arg Leu His Ser Ala Pro Asn Leu
   545 550 555 560
   Ser Asp Leu His Val Val Arg Pro Lys Leu Pro Lys Pro Pro Thr Asp
   565 570 575
   Pro Leu Gly Ala Val Phe Ser Pro Pro Gln Ala Ser Pro Pro Gln Pro
   580 585 590
   Ser His Gly Leu Gln Ser Cys Arg Asn Leu Arg Gly Ser Pro Lys Leu
   595 600 605
   Pro Asp Phe Leu Gln Arg Asn Pro Leu Pro Pro Ile Leu Gly Ser Pro
   610 615 620
   Thr Lys Ala Val Pro Ser Phe Asp Phe Pro Lys Thr Pro Ser Ser Gln
   625 630 635 640
   Asn Leu Leu Ala Leu Leu Ala Arg Gln Gly Val Val Met Thr Pro Pro
   645 650 655
   Arg Asn Arg Thr Leu Pro Asp Leu Ser Glu Val Gly Pro Phe His Gly
   660 665 670
   Gln Pro Leu Gly Pro Gly Leu Arg Pro Gly Glu Asp Pro Lys Gly Pro
   675 680 685
   Phe Gly Arg Ser Phe Ser Thr Ser Arg Leu Thr Asp Leu Leu Leu Lys
   690 695 700
   Ala Ala Phe Gly Thr Gln Ala Pro Asp Pro Gly Ser Thr Glu Ser Leu
   705 710 715 720
   Gln Glu Lys Pro Met Glu Ile Ala Pro Ser Ala Gly Phe Gly Gly Ser
   725 730 735
   Leu His Pro Gly Ala Arg Ala Gly Gly Thr Ser Ser Pro Ser Pro Val
   740 745 750
   Val Phe Thr Val Gly Ser Pro Pro Ser Gly Ser Thr Pro Pro Gln Gly
   755 760 765
   Pro Arg Thr Arg Met Phe Ser Ala Gly Pro Thr Gly Ser Ala Ser Ser
   770 775 780
   Ser Ala Arg His Leu Val Pro Gly Pro Cys Ser Glu Ala Pro Ala Pro
   785 790 795 800
   Glu Leu Pro Ala Pro Gly His Gly Cys Ser Phe Ala Asp Pro Ile Ala
   805 810 815
   Ala Asn Leu Glu Gly Ala Val Thr Phe Glu Ala Pro Asp Leu Pro Glu
   820 825 830
   Glu Thr Leu Met Glu Gln Glu His Thr Glu Ile Leu Arg Gly Leu Arg
   835 840 845
   Phe Thr Leu Leu Phe Val Gln His Val Leu Glu Ile Ala Ala Leu Lys
   850 855 860
   Gly Ser Ala Ser Glu Ala Ala Gly Gly Pro Glu Tyr Gln Leu Gln Glu
   865 870 875 880
   Ser Val Val Ala Asp Gln Ile Ser Leu Leu Ser Arg Glu Trp Gly Phe
   885 890 895
   Ala Glu Gln Leu Val Leu Tyr Leu Lys Val Ala Glu Leu Leu Ser Ser
   900 905 910
   Gly Leu Gln Ser Ala Ile Asp Gln Ile Arg Ala Gly Lys Leu Cys Leu
   915 920 925
   Ser Ser Thr Val Lys Gln Val Val Arg Arg Leu Asn Glu Leu Tyr Lys
   930 935 940
   Ala Ser Val Val Ser Cys Gln Gly Leu Ser Leu Arg Leu Gln Arg Phe
   945 950 955 960
   Phe Leu Asp Lys Gln Arg Leu Leu Asp Arg Ile His Ser Ile Thr Ala
   965 970 975
   Glu Arg Leu Ile Phe Ser His Ala Val Gln Met Val Gln Ser Ala Ala
   980 985 990
   Leu Asp Glu Met Phe Gln His Arg Glu Gly Cys Val Pro Arg Tyr His
   995 1000 1005
   Lys Ala Leu Leu Leu Leu Glu Gly Leu Gln His Met Leu Ser Asp
   1010 1015 1020
   Gln Ala Asp Ile Glu Asn Val Thr Lys Cys Lys Leu Cys Ile Glu
   1025 1030 1035
   Arg Arg Leu Ser Ala Leu Leu Thr Gly Ile Cys Ala
   1040 1045 1050
<210> 51
   <211> 1875
   <212> DNA
   <213> Homo sapiens
<400> 51
   tgagtgctaa agcttccaac aagactgatg ctagctcgtg tcaccaggaa gatgctacgt 60
   catgccaagt gttttcagcg cctagcaatt tttggttctg tgagggcact gcataaagat 120
   aatagaacag caacccctca gaatttctcc aactatgaat ccatgaaaca ggacttcaaa 180
   ctggggattc cagagtattt caactttgct aaagatgtcc tggaccaatg gactgataag 240
   gaaaaggctg gaaagaaacc ttcaaatcca gccttctggt ggatcaacag aaatggagaa 300
   gagatgcgat ggagttttga ggaactggga tctctgtcca gaaaatttgc caatatactt 360
   tcagaagcct gttccctaca aagaggagat cgggtaattc tgattctgcc cagggtcccg 420
   agatggtggc ttgcaaatgt ggcctgtctg cgaacaggga cagttttaat tccaggaacc 480
   actcagctga cccagaaaga cattctctac agactacaat cttcaaaagc aaactgcatt 540
   atcaccaatg atgttttagc cccagcagta gacgctgttg catccaaatg tgaaaatctg 600
   cactccaagc tgattgtatc agagaactcc agagaggggt gggggaacct caaggagttg 660
   atgaaacatg ccagtgacag ccacacctgt gtgaagacaa aacacaatga gatcatggcc 720
   atattcttta ccagtgcaac aagtggatat ccgaaaatgt ctgcacacac ccacagcagt 780
   tttggtttag gtttatctgt aaatggaagg ttctggctag atttgacacc ctcagatgtg 840
   atgtggaata cctcagatac gggctgggca aagtctgcat ggagtagtgt tttttctccg 900
   tggatccagg gagcatgtgt attcacacac catttacccc gttttgagcc gacttctatc 960
   ttgcaaacac tctccaagta ccccatcaca gtcttctgtt cagcaccaac tgtataccga 1020
   atgcttgtac agaatgatat aaccagctat aagtttaaaa gcttaaagca ctgtgtgagt 1080
   gctggggaac caattacccc tgacgtgact gaaaaatgga gaaacaagac gggcctggat 1140
   atctacgaag gatatggaca gactgaaacg gtgctaatct gtggaaattt taagggaatg 1200
   aaaattaaac ctggctcaat gggaaaacct tctcctgctt tcgatgttaa gattgtagat 1260
   gtaaatggca atgttctacc tcctggacaa gaaggagata ttggcattca agttctaccc 1320
   aaccgaccat ttggcctttt tactcattac gtagataatc cttcaaaaac agcttcaact 1380
   ctacgaggca atttctatat cactggggac agagcatata tggataaaga tgggtatttc 1440
   tggtttgttg caagagcaga tgatgtcata ttatcctctg gctatcgaat tggaccattt 1500
   gaggtagaaa atgccctgaa tgaacaccct tcagttgcag agtcagctgt tgtcagcagc 1560
   ccagacccca tcagaggaga ggtagtaaag gcttttgtcg ttctaaatcc tgattacaag 1620
   tcacatgatc aagaacaact aataaaggag attcaggagc atgttaaaaa aactacagca 1680
   ccttacaaat atcccagaaa ggtagaattt attcaagagc tgccaaagac tgtcagtggg 1740
   aagacaaaaa gaaatgaact gaggaagaaa gaatggaaga caatttaaag ttgtttcatt 1800
   aattaccata tctataaaca aacatagtat ctgtcaatct ctagagcggc cgccaccgcg 1860
   gtggagctcc aattc 1875
<210> 52
   <211> 578
   <212> PRT
   <213> Homo sapiens
<400> 52
<210> 53
   <211> 1225
   <212> DNA
   <213> Homo sapiens
<400> 53
   ccgcagcgcc ggagtcaaac ggttcccggc ccagtcccgt cctgcagcag tctgcctcct 60
   ctttcaacat gacagatgcc gctgtgtcct tcgccaagga cttcctggca ggtggagtgg 120
   ccgcagccat ctccaagacg gcggtagcgc ccatcgagcg ggtcaagctg ctgctgcagg 180
   tgcagcatgc cagcaagcag atcactgcag ataagcaata caaaggcatt atagactgcg 240
   tggtccgtat tcccaaggag cagggagttc tgtccttctg gcgcggtaac ctggccaatg 300
   tcatcagata cttccccacc caggctctta acttcgcctt caaagataaa tacaagcaga 360
   tcttcctggg tggtgtggac aagagaaccc agttttggcg ctactttgca gggaatctgg 420
   catcgggtgg tgccgcaggg gccacatccc tgtgttttgt gtaccctctt gattttgccc 480
   gtacccgtct agcagctgat gtgggtaaag ctggagctga aagggaattc cgaggcctcg 540
   gtgactgcct ggttaagatc tacaaatctg atgggattaa gggcctgtac caaggcttta 600
   acgtgtctgt gcagggtatt atcatctacc gagccgccta cttcggtatc tatgacactg 660
   caaagggaat gcttccggat cccaagaaca ctcacatcgt catcagctgg atgatcgcac 720
   agactgtcac tgctgttgcc gggttgactt cctatccatt tgacaccgtt cgccgccgca 780
   tgatgatgca gtcagggcgc aaaggaactg acatcatgta cacaggcacg cttgactgct 840
   ggcggaagat tgctcgtgat gaaggaggca aagctttttt caagggtgca tggtccaatg 900
   ttctcagagg catgggtggt gcttttgtgc ttgtcttgta tgatgaaatc aagaagtaca     960
   cataagttat ttcctaggat ttttccccct gtgaacaggc atgttgtatt ctataacaca    1020
   atcttgagca ttcttgacag actcctggct gtcagtttct cagtggcaac tactttactg    1080
   gttgaaaatg ggaagcaata atattcatct gaccagtttt cctctaaagc catttccatg    1140
   atgatgatga tgggactcaa ttgtattttt tatttcagtc actcctgata aataacaaat    1200
   ttggagaaat aaaaatatct aaaat                                           1225
<210> 54
   <211> 298
   <212> PRT
   <213> Homo sapiens
<400> 54
   Met Thr Asp Ala Ala Val Ser Phe Ala Lys Asp Phe Leu Ala Gly Gly
   1               5                   10                  15
   Val Ala Ala Ala Ile Ser Lys Thr Ala Val Ala Pro Ile Glu Arg Val
               20                  25                  30
   Lys Leu Leu Leu Gln Val Gln His Ala Ser Lys Gln Ile Thr Ala Asp
           35                  40                  45
   Lys Gln Tyr Lys Gly Ile Ile Asp Cys Val Val Arg Ile Pro Lys Glu
       50                  55                  60
   Gln Gly Val Leu Ser Phe Trp Arg Gly Asn Leu Ala Asn Val Ile Arg
   65                  70                  75                  80
   Tyr Phe Pro Thr Gln Ala Leu Asn Phe Ala Phe Lys Asp Lys Tyr Lys
                   85                  90                  95
   Gln Ile Phe Leu Gly Gly Val Asp Lys Arg Thr Gln Phe Trp Arg Tyr
               100                 105                 110
   Phe Ala Gly Asn Leu Ala Ser Gly Gly Ala Ala Gly Ala Thr Ser Leu
           115                 120                 125
   Cys Phe Val Tyr Pro Leu Asp Phe Ala Arg Thr Arg Leu Ala Ala Asp
       130                 135                 140
   Val Gly Lys Ala Gly Ala Glu Arg Glu Phe Arg Gly Leu Gly Asp Cys
   145                 150                 155                 160
   Leu Val Lys Ile Tyr Lys Ser Asp Gly Ile Lys Gly Leu Tyr Gln Gly
   165 170 175
   Phe Asn Val Ser Val Gln Gly Ile Ile Ile Tyr Arg Ala Ala Tyr Phe
   180 185 190
   Gly Ile Tyr Asp Thr Ala Lys Gly Met Leu Pro Asp Pro Lys Asn Thr
   195 200 205
   His Ile Val Ile Ser Trp Met Ile Ala Gln Thr Val Thr Ala Val Ala
   210 215 220
   Gly Leu Thr Ser Tyr Pro Phe Asp Thr Val Arg Arg Arg Met Met Met
   225 230 235 240
   Gln Ser Gly Arg Lys Gly Thr Asp Ile Met Tyr Thr Gly Thr Leu Asp
   245 250 255
   Cys Trp Arg Lys Ile Ala Arg Asp Glu Gly Gly Lys Ala Phe Phe Lys
   260 265 270
   Gly Ala Trp Ser Asn Val Leu Arg Gly Met Gly Gly Ala Phe Val Leu
   275 280 285
   Val Leu Tyr Asp Glu Ile Lys Lys Tyr Thr
   290 295
<210> 55
   <211> 2549
   <212> DNA
   <213> Homo sapiens
<400> 55
   cctccccggc ttccgagcac agcatggcgg tcaaggtgca gacaactaag cgaggggatc    60
   ctcatgagtt aagaaacata tttctacagt atgccagtac tgaggttgat ggagagcgtt   120
   acatgacccc agaagacttt gttcagcgct atcttggact gtataatgat ccaaatagta   180
   acccaaagat cgtgcagctc ttggcaggag tagctgatca aaccaaggat gggttgatct   240
   cctatcaaga gtttttggca tttgaatctg ttttatgtgc tccagattcc atgttcatag   300
   tggctttcca gttgtttgac aagagtggaa atggagaggt gacatttgaa aatgtcaaag   360
   aaatttttgg acagactatt attcatcatc atatcccttt taactgggat tgtgaattta   420
   tccgactgca ttttgggcat aaccggaaga agcatcttaa ctacacagaa ttcacgcagt   480
   ttctccagga gctgcaattg gaacatgcaa gacaagcctt tgcactcaaa gacaaaagca   540
   aaagtggcat gatttctggt ctggatttca gtgacatcat ggttaccatt agatctcaca   600
   tgcttactcc ttttgtggag gagaacttag tttcagcagc tggaggaagt atctcacacc   660
   aggttagctt ctcctacttc aatgcattta actcgttact gaataacatg gagcttgttc 720
   gtaagatata tagcactcta gctggcacaa ggaaagatgt tgaagtcaca aaggaggaat 780
   ttgcccagag tgccatacgc tatggacaag tcacaccact agaaattgat attctatatc 840
   agcttgcaga cttatataat gcttcagggc gcttgacttt ggcagatatt gagagaatag 900
   ccccattggc tgagggggcc ttaccttaca acctggcaga acttcagaga cagcagtctc 960
   ctgggttagg caggcctatc tggctccaga ttgccgagtc tgcttacaga ttcactctgg 1020
   gctcagttgc tggagctgtg ggagccactg cagtgtatcc tatagatctg gtgaagaccc 1080
   gaatgcaaaa ccagcgtggc tctggctctg ttgttgggga gctaatgtac aaaaacagct 1140
   ttgactgttt taagaaagtc ttgcgttatg agggcttctt tggactctac aggggtctga 1200
   taccacaact tataggggtt gctccagaaa aggccattaa actgactgtt aatgattttg 1260
   ttcgggacaa atttaccaga agagatggct ctgttccact tccagcagaa gttcttgctg 1320
   gaggctgtgc tggaggctct caggtcattt ttaccaaccc attggagata gtgaagattc 1380
   gtctgcaagt agctggagag atcaccacgg gacccagagt cagcgccctg aatgtgctcc 1440
   gggacttggg aatttttggt ctgtataagg gtgccaaagc gtgtttcctc cgagacattc 1500
   ccttctctgc aatctatttt cctgtttatg ctcattgcaa actacttctg gctgatgaaa 1560
   atggacacgt gggaggttta aatcttcttg cagctggagc catggcaggt gtcccagctg 1620
   catctctggt gacccctgct gatgtcatca agacaagact gcaggtggct gcccgcgctg 1680
   gccagacgac atacagtggt gtcatcgact gtttcaggaa gattctccgg gaagaagggc 1740
   cctcagcatt ttggaaaggg actgcagctc gagtgtttcg atcctctccc cagtttggtg 1800
   ttaccttggc tcattatgaa gttctccagc ggtggtttta cattgatttt ggaggcctca 1860
   aacccgctgg ttcagaacca acacctaagt cacgcattgc agaccttcct cctgccaacc 1920
   ctgatcacat cggtggatac agactcgcca cagccacgtt tgcaggcatc gaaaacaaat 1980
   ttggccttta tctcccgaaa tttaagtctc ctagtgttgc tgtggttcag ccaaaggcag 2040
   cagtggcagc cactcagtga tgagacaact gttgagtgtg gcaaaatggc gccttgaaga 2100
   aagagcctag gagagcagcc ctgtaatgta tccagtcagc gcatggtact gacgagctga 2160
   ggagtcaaac tcttctttct gtatgtcata tacatatact tgtttataaa ataatcattt 2220
   gcccagggaa aaaaccacaa tgctgtttca agctttagtc ttatgtgttg aaatgttttt 2280
   gtaagccttg gcatgaatta gtgttctaga ctctgctttg cacagcttgc acttacgtga 2340
   ttacatattg tacatctttg tacagagaca tcttggcacc tcatcccaac aaatcacatt 2400
   tgtagaaatg taatgcggtt ctgagtggct tgaaatgtac agaatgtttt gaaagtgttt 2460
   tattaagaat cacacaaaaa taaatgtatt aaaattaaat tcattctctt attggtgact 2520
   tatggaaata aagcatcaat attggatgt 2549
<210> 56
   <211> 678
   <212> PRT
   <213> Homo sapiens
<400> 56
   Met Ala Val Lys Val Gln Thr Thr Lys Arg Gly Asp Pro His Glu Leu
   1               5                   10                  15
   Arg Asn Ile Phe Leu Gln Tyr Ala Ser Thr Glu Val Asp Gly Glu Arg
               20                  25                  30
   Tyr Met Thr Pro Glu Asp Phe Val Gln Arg Tyr Leu Gly Leu Tyr Asn
           35                  40                  45
   Asp Pro Asn Ser Asn Pro Lys Ile Val Gln Leu Leu Ala Gly Val Ala
       50                  55                  60
   Asp Gln Thr Lys Asp Gly Leu Ile Ser Tyr Gln Glu Phe Leu Ala Phe
   65                  70                  75                  80
   Glu Ser Val Leu Cys Ala Pro Asp Ser Met Phe Ile Val Ala Phe Gln
                   85                  90                  95
   Leu Phe Asp Lys Ser Gly Asn Gly Glu Val Thr Phe Glu Asn Val Lys
               100                 105                 110
   Glu Ile Phe Gly Gln Thr Ile Ile His His His Ile Pro Phe Asn Trp
           115                 120                 125
   Asp Cys Glu Phe Ile Arg Leu His Phe Gly His Asn Arg Lys Lys His
       130                 135                 140
   Leu Asn Tyr Thr Glu Phe Thr Gln Phe Leu Gln Glu Leu Gln Leu Glu
   145                 150                 155                 160
   His Ala Arg Gln Ala Phe Ala Leu Lys Asp Lys Ser Lys Ser Gly Met
                   165                 170                 175
   Ile Ser Gly Leu Asp Phe Ser Asp Ile Met Val Thr Ile Arg Ser His
               180                 185                 190
   Met Leu Thr Pro Phe Val Glu Glu Asn Leu Val Ser Ala Ala Gly Gly
           195                 200                 205
   Ser Ile Ser His Gln Val Ser Phe Ser Tyr Phe Asn Ala Phe Asn Ser
       210                 215                 220
   Leu Leu Asn Asn Met Glu Leu Val Arg Lys Ile Tyr Ser Thr Leu Ala
   225                 230                 235                 240
   Gly Thr Arg Lys Asp Val Glu Val Thr Lys Glu Glu Phe Ala Gln Ser
                   245                 250                 255
   Ala Ile Arg Tyr Gly Gln Val Thr Pro Leu Glu Ile Asp Ile Leu Tyr
               260                 265                 270
   Gln Leu Ala Asp Leu Tyr Asn Ala Ser Gly Arg Leu Thr Leu Ala Asp
           275                 280                 285
   Ile Glu Arg Ile Ala Pro Leu Ala Glu Gly Ala Leu Pro Tyr Asn Leu
       290                 295                 300
   Ala Glu Leu Gln Arg Gln Gln Ser Pro Gly Leu Gly Arg Pro Ile Trp
   305                 310                 315                 320
   Leu Gln Ile Ala Glu Ser Ala Tyr Arg Phe Thr Leu Gly Ser Val Ala
                   325                 330                 335
   Gly Ala Val Gly Ala Thr Ala Val Tyr Pro Ile Asp Leu Val Lys Thr
               340                 345                 350
   Arg Met Gln Asn Gln Arg Gly Ser Gly Ser Val Val Gly Glu Leu Met
           355                 360                 365
   Tyr Lys Asn Ser Phe Asp Cys Phe Lys Lys Val Leu Arg Tyr Glu Gly
       370                 375                 380
   Phe Phe Gly Leu Tyr Arg Gly Leu Ile Pro Gln Leu Ile Gly Val Ala
   385                 390                 395                 400
   Pro Glu Lys Ala Ile Lys Leu Thr Val Asn Asp Phe Val Arg Asp Lys
                   405                 410                 415
   Phe Thr Arg Arg Asp Gly Ser Val Pro Leu Pro Ala Glu Val Leu Ala
               420                 425                 430
   Gly Gly Cys Ala Gly Gly Ser Gln Val Ile Phe Thr Asn Pro Leu Glu
           435                 440                 445
   Ile Val Lys Ile Arg Leu Gln Val Ala Gly Glu Ile Thr Thr Gly Pro
   450 455 460
   Arg Val Ser Ala Leu Asn Val Leu Arg Asp Leu Gly Ile Phe Gly Leu
   465 470 475 480
   Tyr Lys Gly Ala Lys Ala Cys Phe Leu Arg Asp Ile Pro Phe Ser Ala
   485 490 495
   Ile Tyr Phe Pro Val Tyr Ala His Cys Lys Leu Leu Leu Ala Asp Glu
   500 505 510
   Asn Gly His Val Gly Gly Leu Asn Leu Leu Ala Ala Gly Ala Met Ala
   515 520 525
   Gly Val Pro Ala Ala Ser Leu Val Thr Pro Ala Asp Val Ile Lys Thr
   530 535 540
   Arg Leu Gln Val Ala Ala Arg Ala Gly Gln Thr Thr Tyr Ser Gly Val
   545 550 555 560
   Ile Asp Cys Phe Arg Lys Ile Leu Arg Glu Glu Gly Pro Ser Ala Phe
   565 570 575
   Trp Lys Gly Thr Ala Ala Arg Val Phe Arg Ser Ser Pro Gln Phe Gly
   580 585 590
   Val Thr Leu Ala His Tyr Glu Val Leu Gln Arg Trp Phe Tyr Ile Asp
   595 600 605
   Phe Gly Gly Leu Lys Pro Ala Gly Ser Glu Pro Thr Pro Lys Ser Arg
   610 615 620
   Ile Ala Asp Leu Pro Pro Ala Asn Pro Asp His Ile Gly Gly Tyr Arg
   625 630 635 640
   Leu Ala Thr Ala Thr Phe Ala Gly Ile Glu Asn Lys Phe Gly Leu Tyr
   645 650 655
   Leu Pro Lys Phe Lys Ser Pro Ser Val Ala Val Val Gln Pro Lys Ala
   660 665 670
   Ala Val Ala Ala Thr Gln
   675
<210> 57
   <211> 3150
   <212> DNA
   <213> Homo sapiens
<400> 57
   ggccaggaac gcacgctgcc tggccgtatc gccgccccca ccgacgccgc cgccgggact      60
   agaagtgagc cgcccgggtc ccaaacgcca gccagccagt cagtgggtcc cgcagtcgcc     120
   cgcaaccggg gcgaatcatg gcggccgcca aggtggcttt aaccaagaga gcagatccag     180
   ctgagcttag aacaatattt ttgaagtatg caagcattga gaaaaacggt gaatttttca     240
   tgtcccccaa tgactttgtc actcgatact tgaacatttt tggagaaagc cagcctaatc     300
   caaagactgt ggaactttta agtggagtgg tggatcagac caaagatgga ttaatatctt     360
   ttcaagaatt tgttgccttt gaatctgtcc tgtgtgcccc tgatgctttg tttatggtag     420
   cctttcagct gtttgacaaa gctggcaaag gagaagtaac ttttgaggat gttaagcaag     480
   tttttggaca gaccacaatt catcaacata ttccatttaa ctgggattca gaatttgtgc     540
   aactacattt tggaaaagaa agaaaaagac acctgacata tgcggaattt actcagtttt     600
   tattggaaat acaactggag cacgcaaagc aagcctttgt gcaacgggac aatgctagga     660
   ctgggagagt cacagccatc gacttccgag acatcatggt caccatccgc ccccatgtct     720
   tgactccttt tgtagaagaa tgtctagtag ctgctgctgg aggtaccaca tcccatcaag     780
   ttagtttctc ctattttaat ggatttaatt cgctccttaa caacatggaa ctcattagaa     840
   agatctatag cactctggct ggcaccagga aagatgttga agtgactaag gaggagtttg     900
   ttctggcagc tcagaaattt ggtcaggtta cacccatgga agttgacatc ttgtttcagt     960
   tagcagattt atatgagcca aggggacgta tgaccttagc agacattgaa cggattgctc    1020
   ctctggaaga gggaactctg ccctttaact tggctgaggc ccagaggcag aaggcctcag    1080
   gtgattcagc tcgaccagtt cttctacaag ttgcagagtc ggcctacagg tttggtctgg    1140
   gttctgttgc tggagctgtt ggagccactg ctgtgtatcc tatcgatctt gtaaaaactc    1200
   gaatgcagaa ccaacgatca actggctctt ttgtgggaga actcatgtat aaaaacagct    1260
   ttgactgttt taagaaagtg ctacgctatg aaggcttctt tggactgtat agaggtctgt    1320
   tgccacagtt attgggagtt gccccagaga aggccataaa acttacagtg aacgattttg    1380
   tgagggataa atttatgcac aaagatggtt cggtcccact tgcagcagaa attcttgctg    1440
   gaggctgcgc tggaggctcc caggtgattt tcacaaatcc tttagaaatc gtcaagatcc    1500
   gtttgcaagt ggcaggagaa atcaccactg gtcctcgagt cagtgctctg tctgtcgtgc    1560
   gggacctggg gttttttggg atctacaagg gtgccaaagc atgctttctg cgggacattc    1620
   ctttctcggc catctacttt ccgtgctatg ctcatgtgaa ggcttccttt gcaaatgaag    1680
   atgggcaggt tagcccagga agcctgctct tagctggtgc catagctggt atgcctgcag    1740
   catctttagt gacccctgct gatgttatca agacgagatt acaggtggct gcccgggctg    1800
   gccaaaccac ttacagcgga gtgatagact gctttagaaa gatactgcgt gaagaaggac   1860
   caaaagctct gtggaaggga gctggtgctc gtgtatttcg atcctcaccc cagtttggtg   1920
   taactttgct gacttacgaa ttgctacagc gatggttcta cattgatttt ggaggagtaa   1980
   aacccatggg atcagagcca gttcctaaat ccaggatcaa cctgcctgcc ccgaatcctg   2040
   atcacgttgg gggctacaaa ctggcagttg ctacatttgc agggattgaa aacaaatttg   2100
   gactttacct acctctcttc aagccatcag tatctacctc aaaggctatt ggtggaggcc   2160
   cataggaaga tcagccctgg gatagtgctg tctttttgtg ggtactgcag taaagaacat   2220
   ccctcctggg aatgaagcaa tgcttcatcc cttttacgtc catctcttgt ttaaattcaa   2280
   gtccaggctt ttttatcatg tgaaatcatt cattttctgg gtgttttctt aaccagatca   2340
   ttgtgaaatt attcataatt attatttggc cctctgccca gaaacctttg tttgcatctg   2400
   aaaattgatg ggatttggtc aacactaaca tgatttgggg aaaggagcaa gtcagaatag   2460
   aaattagtac tcccctcctt gaactaggat tgtagtccca aagaggctac tgtaaggcaa   2520
   tcatggtgct cagagcagtg tttcgtgtgt gttttaaact ggtaggaaac taggtgcata   2580
   tttataaaaa taaaaaacac tgggagaaat gaaaaaatat atatcaaata tattcagcct   2640
   ggcttcaaat tgtaagcatg cacaaattct gtctctggat tatattatga agcttttatg   2700
   tgaaacatgt ttctttgtaa tgaaaaccac attggagatg tttagtaatc atattgttac   2760
   tggtaccaag actactaggg aaatgccttt gtactttagg gaagtacttt tggcatttta   2820
   ctgtacagac agaaaaaact gagatgtagc ccctctcctg gaagtgctaa ttttgaaaaa   2880
   ctgctcatat gatgtacatg tactgattac tgcctatttt aataaacact cttgaaaaat   2940
   gcatgttgcc ctgttgctgc ctgccctatt ctcctcatct ccccatcatt ggtacccact   3000
   tgcttttaaa atccacttta tcttgaataa tgtaagacaa atatgttctg acataagtat   3060
   ttaattcatg ttgccttgca taatggtcag aggcgcatga atttgtgaag gtggaaataa   3120
   actatttgta aagtgaaaaa aaaaaaaaaa                                    3150
<210> 58
   <211> 674
   <212> PRT
   <213> Homo sapiens
<400> 58
   Met Ala Ala Ala Lys Val Ala Leu Thr Lys Arg Ala Asp Pro Ala Glu
   1               5                   10                  15
   Leu Arg Thr Ile Phe Leu Lys Tyr Ala Ser Ile Glu Lys Asn Gly Glu
               20                  25                  30
   Phe Phe Met Ser Pro Asn Asp Phe Val Thr Arg Tyr Leu Asn Ile Phe
           35                  40                  45
   Gly Glu Ser Gln Pro Asn Pro Lys Thr Val Glu Leu Leu Ser Gly Val
       50                  55                  60
   Val Asp Gln Thr Lys Asp Gly Leu Ile Ser Phe Gln Glu Phe Val Ala
   65                  70                  75                  80
   Phe Glu Ser Val Leu Cys Ala Pro Asp Ala Leu Phe Met Val Ala Phe
                   85                  90                  95
   Gln Leu Phe Asp Lys Ala Gly Lys Gly Glu Val Thr Phe Glu Asp Val
               100                 105                 110
   Lys Gln Val Phe Gly Gln Thr Thr Ile His Gln His Ile Pro Phe Asn
           115                 120                 125
   Trp Asp Ser Glu Phe Val Gln Leu His Phe Gly Lys Glu Arg Lys Arg
       130                 135                 140
   His Leu Thr Tyr Ala Glu Phe Thr Gln Phe Leu Leu Glu Ile Gln Leu
   145                 150                 155                 160
   Glu His Ala Lys Gln Ala Phe Val Gln Arg Asp Asn Ala Arg Thr Gly
                   165                 170                 175
   Arg Val Thr Ala Ile Asp Phe Arg Asp Ile Met Val Thr Ile Arg Pro
               180                 185                 190
   His Val Leu Thr Pro Phe Val Glu Glu Cys Leu Val Ala Ala Ala Gly
           195                 200                 205
   Gly Thr Thr Ser His Gln Val Ser Phe Ser Tyr Phe Asn Gly Phe Asn
       210                 215                 220
   Ser Leu Leu Asn Asn Met Glu Leu Ile Arg Lys Ile Tyr Ser Thr Leu
   225                 230                 235                 240
   Ala Gly Thr Arg Lys Asp Val Glu Val Thr Lys Glu Glu Phe Val Leu
                   245                 250                 255
   Ala Ala Gln Lys Phe Gly Gln Val Thr Pro Met Glu Val Asp Ile Leu
               260                 265                 270
   Phe Gln Leu Ala Asp Leu Tyr Glu Pro Arg Gly Arg Met Thr Leu Ala
   275 280 285
   Asp Ile Glu Arg Ile Ala Pro Leu Glu Glu Gly Thr Leu Pro Phe Asn
   290 295 300
   Leu Ala Glu Ala Gln Arg Gln Lys Ala Ser Gly Asp Ser Ala Arg Pro
   305 310 315 320
   Val Leu Leu Gln Val Ala Glu Ser Ala Tyr Arg Phe Gly Leu Gly Ser
   325 330 335
   Val Ala Gly Ala Val Gly Ala Thr Ala Val Tyr Pro Ile Asp Leu Val
   340 345 350
   Lys Thr Arg Met Gln Asn Gln Arg Ser Thr Gly Ser Phe Val Gly Glu
   355 360 365
   Leu Met Tyr Lys Asn Ser Phe Asp Cys Phe Lys Lys Val Leu Arg Tyr
   370 375 380
   Glu Gly Phe Phe Gly Leu Tyr Arg Gly Leu Leu Pro Gln Leu Leu Gly
   385 390 395 400
   Val Ala Pro Glu Lys Ala Ile Lys Leu Thr Val Asn Asp Phe Val Arg
   405 410 415
   Asp Lys Phe Met His Lys Asp Gly Ser Val Pro Leu Ala Ala Glu Ile
   420 425 430
   Leu Ala Gly Gly Cys Ala Gly Gly Ser Gln Val Ile Phe Thr Asn Pro
   435 440 445
   Leu Glu Ile Val Lys Ile Arg Leu Gln Val Ala Gly Glu Ile Thr Thr
   450 455 460
   Gly Pro Arg Val Ser Ala Leu Ser Val Val Arg Asp Leu Gly Phe Phe
   465 470 475 480
   Gly Ile Tyr Lys Gly Ala Lys Ala Cys Phe Leu Arg Asp Ile Pro Phe
   485 490 495
   Ser Ala Ile Tyr Phe Pro Cys Tyr Ala His Val Lys Ala Ser Phe Ala
   500 505 510
   Asn Glu Asp Gly Gln Val Ser Pro Gly Ser Leu Leu Leu Ala Gly Ala
   515 520 525
   Ile Ala Gly Met Pro Ala Ala Ser Leu Val Thr Pro Ala Asp Val Ile
   530 535 540
   Lys Thr Arg Leu Gln Val Ala Ala Arg Ala Gly Gln Thr Thr Tyr Ser
   545 550 555 560
   Gly Val Ile Asp Cys Phe Arg Lys Ile Leu Arg Glu Glu Gly Pro Lys
   565 570 575
   Ala Leu Trp Lys Gly Ala Gly Ala Arg Val Phe Arg Ser Ser Pro Gln
   580 585 590
   Phe Gly Val Thr Leu Leu Thr Tyr Glu Leu Leu Gln Arg Trp Phe Tyr
   595 600 605
   Ile Asp Phe Gly Gly Val Lys Pro Met Gly Ser Glu Pro Val Pro Lys
   610 615 620
   Ser Arg Ile Asn Leu Pro Ala Pro Asn Pro Asp His Val Gly Gly Tyr
   625 630 635 640
   Lys Leu Ala Val Ala Thr Phe Ala Gly Ile Glu Asn Lys Phe Gly Leu
   645 650 655
   Tyr Leu Pro Leu Phe Lys Pro Ser Val Ser Thr Ser Lys Ala Ile Gly
   660 665 670
   Gly Gly
<210> 59
   <211> 2213
   <212> DNA
   <213> Homo sapiens
<400> 59
   gccttctgat tgacagcgga ggggctcagt gaaaaaatga gcgagctaga ggaagacttt 60
   gccaagattc tcatgctcaa ggaggagagg atcaaagagc tggagaagcg gctgtcagag 120
   aaggaggaag aaattcagga gctgaagagg aaactccaca aatgccagtc ggtgctccca 180
   gtgccctcga cccacatcgg cccccggacc acccgggcgc agggcatctc ggccgagccg 240
   cagacgtaca ggtccttcca cgacctccga caggcattcc ggaagttcac caagtccgaa 300
   aggtccaagg atcttataaa ggaagctatc cttgacaatg actttatgaa gaacttggag 360
   ctgtcgcaga tccaggagat tgtggattgt atgtacccgg tggagtatgg caaggacagt 420
   tgcatcatca aagaaggaga cgtggggtca ctggtgtatg tcatggaaga tggtaaggtt 480
   gaagttacaa aagaaggtgt gaagttgtgt accatgggtc caggaaaagt gtttggggaa 540
   ttggctattc tttacaactg tacccggaca gcgaccgtca agactcttgt aaatgtaaaa 600
   ctctgggcca ttgatcgaca atgttttcaa acaataatga tgaggacagg actcatcaag 660
   cataccgagt atatggaatt tttaaaaagc gttccaacat tccagagcct tcctgaagag 720
   atcctcagca agcttgctga tgtccttgaa gagacccact atgaaaatgg agaatatatt 780
   atcaggcaag gtgcaagagg ggacaccttc tttatcatca gcaaaggaac ggtaaatgtc 840
   actcgtgaag actcaccgag tgaagaccca gtctttctta gaactttagg aaaaggagac 900
   tggtttggag agaaagcctt gcagggggaa gatgtgagaa cagcaaacgt aattgctgca 960
   gaagctgtaa cctgccttgt gattgacaga gactctttta aacatttgat tggagggctg 1020
   gatgatgttt ctaataaagc atatgaagat gcagaagcta aagcaaaata tgaagctgaa 1080
   gcggctttct tcgccaacct gaagctgtct gatttcaaca tcattgatac ccttggagtt 1140
   ggaggtttcg gacgagtaga actggtccag ttgaaaagtg aagaatccaa aacgtttgca 1200
   atgaagattc tcaagaaacg tcacattgtg gacacaagac agcaggagca catccgctca 1260
   gagaagcaga tcatgcaggg ggctcattcc gatttcatag tgagactgta cagaacattt 1320
   aaggacagca aatatttgta tatgttgatg gaagcttgtc taggtggaga gctctggacc 1380
   attctcaggg atagaggttc gtttgaagat tctacaacca gattttacac agcatgtgtg 1440
   gtagaagctt ttgcctatct gcattccaaa ggaatcattt acagggacct caagccagaa 1500
   aatctcatcc tagatcaccg aggttatgcc aaactggttg attttggctt tgcaaagaaa 1560
   ataggatttg gaaagaaaac atggactttt tgtgggactc cagagtatgt agccccagag 1620
   atcatcctga acaaaggcca tgacatttca gccgactact ggtcactggg aatcctaatg 1680
   tatgaactcc tgactggcag cccacctttc tcaggcccag atcctatgaa aacctataac 1740
   atcatattga gggggattga catgatagaa tttccaaaga agattgccaa aaatgctgct 1800
   aatttaatta aaaaactatg cagggacaat ccatcagaaa gattagggaa tttgaaaaat 1860
   ggagtaaaag acattcaaaa gcacaaatgg tttgagggct ttaactggga aggcttaaga 1920
   aaaggtacct tgacacctcc tataatacca agtgttgcat cacccacaga cacaagtaat 1980
   tttgacagtt tccctgagga caacgatgaa ccaccacctg atgacaactc aggatgggat 2040
   atagacttct aatgtatttc tcttacctgc ttctgccttg ctgaagacag ctttttctga 2100
   gacacagctg ccagcaaacc tgagggaaag agagaagatt agtgctcggg gtcaccatga 2160
   tgcctttgat cgatgctgct ccagtaacta cagtggcatt aggacttatc gct 2213
<210> 60
   <211> 671
   <212> PRT
   <213> Homo sapiens
<400> 60
   Met Ser Glu Leu Glu Glu Asp Phe Ala Lys Ile Leu Met Leu Lys Glu
   1               5                   10                  15
   Glu Arg Ile Lys Glu Leu Glu Lys Arg Leu Ser Glu Lys Glu Glu Glu
               20                  25                  30
   Ile Gln Glu Leu Lys Arg Lys Leu His Lys Cys Gln Ser Val Leu Pro
           35                  40                  45
   Val Pro Ser Thr His Ile Gly Pro Arg Thr Thr Arg Ala Gln Gly Ile
       50                  55                  60
   Ser Ala Glu Pro Gln Thr Tyr Arg Ser Phe His Asp Leu Arg Gln Ala
   65                  70                  75                  80
   Phe Arg Lys Phe Thr Lys Ser Glu Arg Ser Lys Asp Leu Ile Lys Glu
                   85                  90                  95
   Ala Ile Leu Asp Asn Asp Phe Met Lys Asn Leu Glu Leu Ser Gln Ile
               100                 105                 110
   Gln Glu Ile Val Asp Cys Met Tyr Pro Val Glu Tyr Gly Lys Asp Ser
           115                 120                 125
   Cys Ile Ile Lys Glu Gly Asp Val Gly Ser Leu Val Tyr Val Met Glu
       130                 135                 140
   Asp Gly Lys Val Glu Val Thr Lys Glu Gly Val Lys Leu Cys Thr Met
   145                 150                 155                 160
   Gly Pro Gly Lys Val Phe Gly Glu Leu Ala Ile Leu Tyr Asn Cys Thr
                   165                 170                 175
   Arg Thr Ala Thr Val Lys Thr Leu Val Asn Val Lys Leu Trp Ala Ile
               180                 185                 190
   Asp Arg Gln Cys Phe Gln Thr Ile Met Met Arg Thr Gly Leu Ile Lys
           195                 200                 205
   His Thr Glu Tyr Met Glu Phe Leu Lys Ser Val Pro Thr Phe Gln Ser
       210                 215                 220
   Leu Pro Glu Glu Ile Leu Ser Lys Leu Ala Asp Val Leu Glu Glu Thr
   225 230 235 240
   His Tyr Glu Asn Gly Glu Tyr Ile Ile Arg Gln Gly Ala Arg Gly Asp
   245 250 255
   Thr Phe Phe Ile Ile Ser Lys Gly Thr Val Asn Val Thr Arg Glu Asp
   260 265 270
   Ser Pro Ser Glu Asp Pro Val Phe Leu Arg Thr Leu Gly Lys Gly Asp
   275 280 285
   Trp Phe Gly Glu Lys Ala Leu Gln Gly Glu Asp Val Arg Thr Ala Asn
   290 295 300
   Val Ile Ala Ala Glu Ala Val Thr Cys Leu Val Ile Asp Arg Asp Ser
   305 310 315 320
   Phe Lys His Leu Ile Gly Gly Leu Asp Asp Val Ser Asn Lys Ala Tyr
   325 330 335
   Glu Asp Ala Glu Ala Lys Ala Lys Tyr Glu Ala Glu Ala Ala Phe Phe
   340 345 350
   Ala Asn Leu Lys Leu Ser Asp Phe Asn Ile Ile Asp Thr Leu Gly Val
   355 360 365
   Gly Gly Phe Gly Arg Val Glu Leu Val Gln Leu Lys Ser Glu Glu Ser
   370 375 380
   Lys Thr Phe Ala Met Lys Ile Leu Lys Lys Arg His Ile Val Asp Thr
   385 390 395 400
   Arg Gln Gln Glu His Ile Arg Ser Glu Lys Gln Ile Met Gln Gly Ala
   405 410 415
   His Ser Asp Phe Ile Val Arg Leu Tyr Arg Thr Phe Lys Asp Ser Lys
   420 425 430
   Tyr Leu Tyr Met Leu Met Glu Ala Cys Leu Gly Gly Glu Leu Trp Thr
   435 440 445
   Ile Leu Arg Asp Arg Gly Ser Phe Glu Asp Ser Thr Thr Arg Phe Tyr
   450 455 460
   Thr Ala Cys Val Val Glu Ala Phe Ala Tyr Leu His Ser Lys Gly Ile
   465 470 475 480
   Ile Tyr Arg Asp Leu Lys Pro Glu Asn Leu Ile Leu Asp His Arg Gly
   485 490 495
   Tyr Ala Lys Leu Val Asp Phe Gly Phe Ala Lys Lys Ile Gly Phe Gly
   500 505 510
   Lys Lys Thr Trp Thr Phe Cys Gly Thr Pro Glu Tyr Val Ala Pro Glu
   515 520 525
   Ile Ile Leu Asn Lys Gly His Asp Ile Ser Ala Asp Tyr Trp Ser Leu
   530 535 540
   Gly Ile Leu Met Tyr Glu Leu Leu Thr Gly Ser Pro Pro Phe Ser Gly
   545 550 555 560
   Pro Asp Pro Met Lys Thr Tyr Asn Ile Ile Leu Arg Gly Ile Asp Met
   565 570 575
   Ile Glu Phe Pro Lys Lys Ile Ala Lys Asn Ala Ala Asn Leu Ile Lys
   580 585 590
   Lys Leu Cys Arg Asp Asn Pro Ser Glu Arg Leu Gly Asn Leu Lys Asn
   595 600 605
   Gly Val Lys Asp Ile Gln Lys His Lys Trp Phe Glu Gly Phe Asn Trp
   610 615 620
   Glu Gly Leu Arg Lys Gly Thr Leu Thr Pro Pro Ile Ile Pro Ser Val
   625 630 635 640
   Ala Ser Pro Thr Asp Thr Ser Asn Phe Asp Ser Phe Pro Glu Asp Asn
   645 650 655
   Asp Glu Pro Pro Pro Asp Asp Asn Ser Gly Trp Asp Ile Asp Phe
   660 665 670
<210> 61
   <211> 3740
   <212> DNA
   <213> Homo sapiens
<400> 61
   aggaagcctc aagacgcgga gcagcggcag gaaggagccc ccggcagccc ggaggagcat 60
   gggcaccttg cgggatttac agtacgcgct ccaggagaag atcgaggagc tgaggcagcg 120
   ggatgctctc atcgacgagc tggagctgga gttggatcag aaggacgaac tgatccagaa 180
   gctgcagaac gagctggaca agtaccgctc ggtgatccga ccagccaccc agcaggcgca 240
   gaagcagagc gcgagcacct tgcagggcga gccgcgcacc aagcggcagg cgatctccgc 300
   cgagcccacc gccttcgaca tccaggatct cagccatgtg accctgccct tctaccccaa 360
   gagcccacag tccaaggatc ttataaagga agctatcctt gacaatgact ttatgaagaa 420
   cttggagctg tcgcagatcc aggagattgt ggattgtatg tacccggtgg agtatggcaa 480
   ggacagttgc atcatcaaag aaggagacgt ggggtcactg gtgtatgtca tggaagatgg 540
   taaggttgaa gttacaaaag aaggtgtgaa gttgtgtacc atgggtccag gaaaagtgtt 600
   tggggaattg gctattcttt acaactgtac ccggacagcg accgtcaaga ctcttgtaaa 660
   tgtaaaactc tgggccattg atcgacaatg ttttcaaaca ataatgatga ggacaggact 720
   catcaagcat accgagtata tggaattttt aaaaagcgtt ccaacattcc agagccttcc 780
   tgaagagatc ctcagcaagc ttgctgatgt ccttgaagag acccactatg aaaatggaga 840
   atatattatc aggcaaggtg caagagggga caccttcttt atcatcagca aaggaacggt 900
   aaatgtcact cgtgaagact caccgagtga agacccagtc tttcttagaa ctttaggaaa 960
   aggagactgg tttggagaga aagccttgca gggggaagat gtgagaacag caaacgtaat 1020
   tgctgcagaa gctgtaacct gccttgtgat tgacagagac tcttttaaac atttgattgg 1080
   agggctggat gatgtttcta ataaagcata tgaagatgca gaagctaaag caaaatatga 1140
   agctgaagcg gctttcttcg ccaacctgaa gctgtctgat ttcaacatca ttgataccct 1200
   tggagttgga ggtttcggac gagtagaact ggtccagttg aaaagtgaag aatccaaaac 1260
   gtttgcaatg aagattctca agaaacgtca cattgtggac acaagacagc aggagcacat 1320
   ccgctcagag aagcagatca tgcagggggc tcattccgat ttcatagtga gactgtacag 1380
   aacatttaag gacagcaaat atttgtatat gttgatggaa gcttgtctag gtggagagct 1440
   ctggaccatt ctcagggata gaggttcgtt tgaagattct acaaccagat tttacacagc 1500
   atgtgtggta gaagcttttg cctatctgca ttccaaagga atcatttaca gggacctcaa 1560
   gccagaaaat ctcatcctag atcaccgagg ttatgccaaa ctggttgatt ttggctttgc 1620
   aaagaaaata ggatttggaa agaaaacatg gactttttgt gggactccag agtatgtagc 1680
   cccagagatc atcctgaaca aaggccatga catttcagcc gactactggt cactgggaat 1740
   cctaatgtat gaactcctga ctggcagccc acctttctca ggcccagatc ctatgaaaac 1800
   ctataacatc atattgaggg ggattgacat gatagaattt ccaaagaaga ttgccaaaaa 1860
   tgctgctaat ttaattaaaa aactatgcag ggacaatcca tcagaaagat tagggaattt 1920
   gaaaaatgga gtaaaagaca ttcaaaagca caaatggttt gagggcttta actgggaagg 1980
   cttaagaaaa ggtaccttga cacctcctat aataccaagt gttgcatcac ccacagacac 2040
   aagtaatttt gacagtttcc ctgaggacaa cgatgaacca ccacctgatg acaactcagg 2100
   atgggatata gacttctaat gtatttctct tacctgcttc tgccttgctg aagacagctt 2160
   tttctgagac acagctgcca gcaaacctga gggaaagaga gaagattagt gctcggggtc 2220
   accatgatgc ctttgatcga tgctgctcca gtaactacag tggcattagg acttatcgct 2280
   tagatgacaa tagtgctctt tacatgtttt ctgtttgaac ctaaaatagc agttgacatg 2340
   gtggtcctga agcaaagcct ttcaccagta aagagatgtt ttctattgtt gcaatgacct 2400
   tgctttgctc tgattataat ttgaaagact gtaggaaaca cttcaatgta gtataagagt 2460
   ctgtaccttg ctggaatatt caagaagatg aaagaataat atattgggta caatagatta 2520
   ctatggtaca gaaactgggc tattcccttt cttcaagtga aggctgtggg atctattaca 2580
   gctgcaggcc ggtgtatata ccatacaaaa gaggaccaca catctgttgg tcacagagtt 2640
   catgtcacac cagtgctaga agtttcatga ttttatttcc cagcagtgct gatgacaaga 2700
   ctgaatgtta ccttttcttt ctgacagatt ttaaaaattg atatgataaa agcacaactg 2760
   ctatagattc tgctgagacc tctcatagta ggtatatatg agttttcaca gaagactgaa 2820
   aaataatgca tgatatttgt ttgttttttt tgataaattg gcatgacaga gtggggaaaa 2880
   aaagcaattc acaaaaccat ttcatatttt ttaaaatatt gtgcttaaag atggtcctgg 2940
   aagtaaatga ctagcagcca attggtttta cttaacatac cctcaaactg aggcttaaag 3000
   tattcccttt tataaaaata aatgcttggg gtagggtgga gtggggaggg attaaaaccc 3060
   atccaaaaaa taaataaaaa ctatataggt gctatgtata tctttcatct gtaaatgtca 3120
   gtgtctgaac agcaacacaa attcaaatca ttatacgtgt agccagaaac tcaagcattt 3180
   tcactaaagt tattaaacca aactcctgtc caatttgact tatacaacat agtcagtcta 3240
   gagttgagag acaaaggtaa ttataaacct atttgaacta gcttcttgtc ttaggcctga 3300
   accaaaaaac aacaaacaaa caaaaaacaa gaatgaaaaa cagaaataaa agaagtagaa 3360
   aagacaaaga aagaaagccc aaagtcaaag ttgttaatat ttacaggttt accagatctg 3420
   gaacattact tatttgaggt cagagaacaa aacaagaacc tggccaggtg ttgattacct 3480
   tttagtgaat aagctgagtc catatacttg tctaactaag aaagcagtac agaggaaaac 3540
   aggaacctga tttttttaaa ataaatttta aataaaatag aattactaca attctgcaat 3600
   ttcatactac ctaaaaaaga ctagatttga aaatgtcaag ctgatttact ttattcacat 3660
   ggagaaaaga atccacaaat taaactgagt ccttcactgg catgccagtt gactattatt 3720
   agctgtcata agtaaccccg 3740
<210> 62
   <211> 686
   <212> PRT
   <213> Homo sapiens
<400> 62
<210> 63
   <211> 5892
   <212> DNA
   <213> Homo sapiens
<400> 63
   attctgcacc tcctgacgta accacgtaca cctcagagca ctcgatacag gtggagagac      60
   cgcagggttc cacggggtcc cggacagcgc ccaagtacgg caacgccgag ctcatggaga     120
   ccggggatgg agtaccagta agtagccggg tgtcagcaaa aatccagcag cttgtcaata     180
   ccctcaaacg accgaaacga ccacctttac gagaattctt tgtcgatgac tttgaagaat 240
   tattagaagt tcaacaaccg gatccgaacc aaccaaagcc ggagggggcc cagatgctgg 300
   ccatgcgcgg agagcagctg ggcgtggtca cgaactggcc gccgtcgctg gaggccgcac 360
   tgcagaggtg gggcaccatc tcgcccaagg cgccctgcct gaccaccatg gacaccaacg 420
   ggaagcccct ctacatcctc acttacggca agctgtggac aagaagtatg aaggtcgctt 480
   acagcattct acacaaatta ggcacaaagc aggaacccat ggtccggcct ggagataggg 540
   tggcactggt gttccccaac aatgatccgg ctgccttcat ggcggctttc tacggctgcc 600
   tgctggccga ggtggtcccc gtgcccatcg aggtgccact caccaggaag gacgcaggga 660
   gccagcagat aggtttcttg cttggaagct gtggagttac tgtagccttg actagtgacg 720
   cctgccataa aggacttcca aaaagcccaa cgggagagat cccacagttt aaaggttggc 780
   caaagctgct gtggtttgtc acagagtcta aacatctctc caaaccgccc cgagactggt 840
   tcccacacat taaagatgcc aataacgaca ctgcgtatat tgagtacaag acgtgtaagg 900
   atggcagtgt gctgggtgtg acggtgacga ggactgcgct gctgacacac tgccaggccc 960
   tgacgcaggc gtgtggctac acggaagctg aaaccattgt gaatgtgctg gatttcaaga 1020
   aggacgtcgg gctctggcat ggcatcctga caagcgtcat gaacatgatg catgtgatca 1080
   gcatcccgta ctcgctgatg aaggtgaacc ctctctcctg gatccagaag gtctgccagt 1140
   acaaagcaaa agtggcgtgt gtgaaatcga gggatatgca ttgggcatta gtagcacaca 1200
   gagatcagag agacatcaac ctctcctctc tgcgaatgct gatagtggcg gacggcgcga 1260
   acccctggtc tatttcttct tgcgatgcat ttctcaatgt cttccaaagt aaaggccttc 1320
   gacaggaggt catctgtcct tgtgccagct cgccagaggc cctcactgtg gccatccgga 1380
   ggcccacgga tgacagtaac cagcccccgg gccggggtgt cctctccatg catggactga 1440
   cctatggggt cattcgtgtg gactcggaag agaagctgtc cgtgctcacc gtgcaggatg 1500
   tcggcctcgt gatgcctgga gccatcatgt gttcagtgaa gccagacggg gttcctcagc 1560
   tgtgcagaac ggatgagatc ggggagctgt gtgtgtgtgc agttgcgacg ggcacgtcct 1620
   actatggcct ctctggcatg accaagaaca cctttgaggt gtttcccatg acaagctccg 1680
   gggctccgat cagtgaatac ccattcataa ggacaggctt gctggggttc gtgggtcccg 1740
   gaggcctcgt cttcgtggtg ggcaagatgg atggcctcat ggtggtcagc gggcgcaggc 1800
   acaacgccga cgacatcgtg gccactgcgc tggccgtaga acccatgaag tttgtctacc 1860
   ggggaaggat agccgtgttc tcggtgaccg tgctgcacga cgagaggatc gtgatcgtgg 1920
   ctgagcagag gcctgactcc acggaagagg acagtttcca gtggatgagc cgtgtgctgc 1980
   aggcgattga cagtatacat caagttggag tttattgcct ggccttggtg ccagcaaaca 2040
   ccctccccaa aaccccgctt ggtgggatcc atttatcaga aacaaaacag ctttttctgg 2100
   agggctctct gcacccctgc aatgtcctaa tgtgccccca cacctgcgtc acaaacttgc 2160
   ctaagcctcg acagaagcag ccagaaatcg gccctgcctc tgtgatggtg gggaacctgg 2220
   tctctgggaa gagaatcgcc caggccagtg gcagagacct gggtcagatc gaagataacg 2280
   accaggcacg caagttcctg ttcctctcag aggtcttgca gtggagagca cagaccaccc 2340
   cggaccacat cctctacacg ctgctcaact gtcggggtgc gatagcgaac tcgctgacct 2400
   gcgtgcagct gcacaagaga gctgagaaga tcgccgtgat gctgatggag aggggccacc 2460
   ttcaggacgg cgaccacgtg gccttggtct accccccagg aatagacctg atagcagcgt 2520
   tttatggttg cctgtacgca ggctgtgtgc caataaccgt ccgtcccccg cacccacaga 2580
   acatcgcgac gacgttgcct accgtcaaga tgattgtgga ggtgagtcgc tctgcctgtc 2640
   tgatgacgac acagctgatc tgtaagttgc tgcggtccag ggaggcggcg gcggctgtgg 2700
   acgtcaggac gtggcccctc atcctggaca cagatgattt gccaaagaag cggcctgccc 2760
   agatctgcaa accttgcaac ccagacactc ttgcatatct cgacttcagc gtgtccacaa 2820
   ctgggatgct agctggcgta aagatgtctc acgcagccac cagtgccttc tgccgttcca 2880
   ttaagctgca gtgtgaactt tacccctcta gagaagtggc catctgcctg gacccttact 2940
   gtggactggg atttgtcctc tggtgcctct gcagtgtgta ttctgggcac cagtccatcc 3000
   tgatcccgcc ctctgagctg gaaaccaacc ccgccttgtg gcttcttgcc gtgagtcagt 3060
   acaaagtccg agacacgttt tgctcctact ccgtgatgga gctgtgcacc aaggggctgg 3120
   gctcgcaaac agagtccctc aaggcgcgag ggctggactt gtcccgagtg aggacctgcg 3180
   tggttgtggc ggaagagagg cctcggatcg cactcacaca gtcgttctca aagctgttta 3240
   aggacctggg ccttcacccg cgggccgtca gcacctcgtt cggttgcagg gtgaacctgg 3300
   cgatttgctt gcagggaacc tcaggacctg acccaaccac tgtctacgtg gacatgagag 3360
   ccctgagaca cgacagagtc cgcttagtgg aaagaggatc ccctcatagt ctgcccctga 3420
   tggaatcggg aaagatactt ccaggggttc ggattataat tgccaaccca gaaacaaaag 3480
   gaccgctggg ggactcacac cttggagaga tttgggttca cagtgcccac aatgccagcg 3540
   gttatttcac tatttacgga gacgaatccc tccagtcaga tcacttcaac tcaagactaa 3600
   gttttggaga cacccagacc atctgggcac gcacaggcta cttggggttc ctgcggagaa 3660
   ctgagctcac agatgcaaat ggagagcgcc atgatgccct ctacgtggta ggggcactgg 3720
   acgaagccat ggagctgcgg ggcatgcggt accacccaat cgacattgag acctcggtca 3780
   tcagagccca taaaagcgtt acggaatgtg ctgtgtttac ctggacaaat ttgttggtgg 3840
   ttgtggttga gctggatggg tcggaacaag aagccttgga cctggttccc ttggtgacca 3900
   acgtggtcct ggaggagcac tacctgatcg tcggagtggt ggtcgtggtg gacatcggcg 3960
   tcatccccat caactcccgt ggggagaagc agcgcatgca cctgcgagac gggtttttgg 4020
   cagaccagct agaccccatc tatgtggcct acaacatgta gtctcgtctc ttggcttcca 4080
   tggacttttc tagagatgta gacattgttc tccgtgtcca ctgaagcgtg cagacacagg 4140
   gcaacactca ccagaataca gccatttgtg gtgagagtgg aggaggaaga ggaggaggaa 4200
   gaggacttct cacagcagcc acgattggca tgggggtgaa atgtgaattt accactgaat 4260
   ttcgctcaga aggactttgg attactgcct tcagtttgtt ggaaaagccc atttcaaaaa 4320
   ctttcttttc ttttctttct tttttaatta ttggataata agtgctttct tcgtaaatgt 4380
   ggtattttgt taagccgaaa tagcaattaa aaaaatatcc tgccctccag atgggttctt 4440
   ttaaacaatt tatgtagtgt gacaaagaat tgttttctct gttttaatgt gtcatgaaat 4500
   cttaatgaca tggatctgtt actaatttaa gccattgcta gatctcatcc ttttaggaaa 4560
   gtttgaggta cgagaaaacc ttccaaatag caccttccaa ttagataata gctttctttg 4620
   tcagaaatgt gctgaagaaa caaaggctgg tatacggcct tcgaagttag tatagaatga 4680
   gaagaaatta taaataaggt gtatttcggc aattatcttg caaatatctt tgtactaaac 4740
   taaaaagata aaataagtta acttcctcaa tatgtaatta tgtacaaaac gtttaattta 4800
   ttttgatctc tttagaacta taaaagagaa aaacattcaa gaatattaaa gtcttgtaat 4860
   gtttgctaat ataaaaaagt gttgtattat cttgcgtgga tagtatcaca acaaatatat 4920
   atatatgaaa tataaattca ctaatgaaca aaggagattt taaagtttaa gatgcagaac 4980
   ttgtcacttg catggtgtgc cccccgtact cacatacact ctgctgttgc cagcagtcgc 5040
   agaccgcagg agccctgtct aaaagtttct tctagaacca gagaccagca agtgaaatta 5100
   ttgccatctc aaggatggca aaagaattca aagctcaatg tgcactattt ttttctttgc 5160
   tgtgggacaa cagtgaatgt gtttatgcca gcgtgtgctg atgatactga ggggctttag 5220
   gttggcaaat agcactgttt tcttagctgc aagaattcat tgcacaatgt ttttcatcat 5280
   ttttgttaat gtcatctttt tttggtcctt gctacgaaaa ggaatgcgat tctgtggtca 5340
   ttcgcactgg gttgcattga ttccccctct gatggccaat gtggagtgga caaagtgtcc 5400
   ggaactcaca tcggtgatcg tcccctcgtc ttaagaccca gcccgctctg tgtgagcctc 5460
   tggggctccc tcgctcagtg agcacagttc cccgggggtt catgccagag ctccggctga 5520
   agcaagaagt cctccagctg cgtcgtttgc cgcctgtgga cgagtgcgcc ccagtttctg 5580
   ccctggcagc tcctggccac accttctcag agctcacctg tgcacttcta aattgaattg 5640
   gcccacggtg tccaaccaag aaggagcatc tgcactccga gaaagatgtg ttctgtaact 5700
   gccccagtgt gaccccgcag tggctctcgg tgctagatat gcatgactaa gattgatgct 5760
   gggcaaaatg tagatgatct ttcattatgt tgtgggcagc gtctttctct gcctttgcta 5820
   tatgcagtca gcagtaagcc ttttgctaaa agagttttgt ttgacttctg agatccaagg 5880
   ctgattgttg tt 5892
<210> 64
   <211> 1352
   <212> PRT
   <213> Homo sapiens
<400> 64
   Ser Ala Pro Pro Asp Val Thr Thr Tyr Thr Ser Glu His Ser Ile Gln
   1 5 10 15
   Val Glu Arg Pro Gln Gly Ser Thr Gly Ser Arg Thr Ala Pro Lys Tyr
   20 25 30
   Gly Asn Ala Glu Leu Met Glu Thr Gly Asp Gly Val Pro Val Ser Ser
   35 40 45
   Arg Val Ser Ala Lys Ile Gln Gln Leu Val Asn Thr Leu Lys Arg Pro
   50 55 60
   Lys Arg Pro Pro Leu Arg Glu Phe Phe Val Asp Asp Phe Glu Glu Leu
   65 70 75 80
   Leu Glu Val Gln Gln Pro Asp Pro Asn Gln Pro Lys Pro Glu Gly Ala
   85 90 95
   Gln Met Leu Ala Met Arg Gly Glu Gln Leu Gly Val Val Thr Asn Trp
   100 105 110
   Pro Pro Ser Leu Glu Ala Ala Leu Gln Arg Trp Gly Thr Ile Ser Pro
   115 120 125
   Lys Ala Pro Cys Leu Thr Thr Met Asp Thr Asn Gly Lys Pro Leu Tyr
   130 135 140
   Ile Leu Thr Tyr Gly Lys Leu Trp Thr Arg Ser Met Lys Val Ala Tyr
   145 150 155 160
   Ser Ile Leu His Lys Leu Gly Thr Lys Gln Glu Pro Met Val Arg Pro
   165 170 175
   Gly Asp Arg Val Ala Leu Val Phe Pro Asn Asn Asp Pro Ala Ala Phe
   180 185 190
   Met Ala Ala Phe Tyr Gly Cys Leu Leu Ala Glu Val Val Pro Val Pro
   195 200 205
   Ile Glu Val Pro Leu Thr Arg Lys Asp Ala Gly Ser Gln Gln Ile Gly
   210 215 220
   Phe Leu Leu Gly Ser Cys Gly Val Thr Val Ala Leu Thr Ser Asp Ala
   225 230 235 240
   Cys His Lys Gly Leu Pro Lys Ser Pro Thr Gly Glu Ile Pro Gln Phe
   245 250 255
   Lys Gly Trp Pro Lys Leu Leu Trp Phe Val Thr Glu Ser Lys His Leu
   260 265 270
   Ser Lys Pro Pro Arg Asp Trp Phe Pro His Ile Lys Asp Ala Asn Asn
   275 280 285
   Asp Thr Ala Tyr Ile Glu Tyr Lys Thr Cys Lys Asp Gly Ser Val Leu
   290 295 300
   Gly Val Thr Val Thr Arg Thr Ala Leu Leu Thr His Cys Gln Ala Leu
   305 310 315 320
   Thr Gln Ala Cys Gly Tyr Thr Glu Ala Glu Thr Ile Val Asn Val Leu
   325 330 335
   Asp Phe Lys Lys Asp Val Gly Leu Trp His Gly Ile Leu Thr Ser Val
   340 345 350
   Met Asn Met Met His Val Ile Ser Ile Pro Tyr Ser Leu Met Lys Val
   355 360 365
   Asn Pro Leu Ser Trp Ile Gln Lys Val Cys Gln Tyr Lys Ala Lys Val
   370 375 380
   Ala Cys Val Lys Ser Arg Asp Met His Trp Ala Leu Val Ala His Arg
   385 390 395 400
   Asp Gln Arg Asp Ile Asn Leu Ser Ser Leu Arg Met Leu Ile Val Ala
   405 410 415
   Asp Gly Ala Asn Pro Trp Ser Ile Ser Ser Cys Asp Ala Phe Leu Asn
   420 425 430
   Val Phe Gln Ser Lys Gly Leu Arg Gln Glu Val Ile Cys Pro Cys Ala
   435 440 445
   Ser Ser Pro Glu Ala Leu Thr Val Ala Ile Arg Arg Pro Thr Asp Asp
   450 455 460
   Ser Asn Gln Pro Pro Gly Arg Gly Val Leu Ser Met His Gly Leu Thr
   465 470 475 480
   Tyr Gly Val Ile Arg Val Asp Ser Glu Glu Lys Leu Ser Val Leu Thr
   485 490 495
   Val Gln Asp Val Gly Leu Val Met Pro Gly Ala Ile Met Cys Ser Val
   500 505 510
   Lys Pro Asp Gly Val Pro Gln Leu Cys Arg Thr Asp Glu Ile Gly Glu
   515 520 525
   Leu Cys Val Cys Ala Val Ala Thr Gly Thr Ser Tyr Tyr Gly Leu Ser
   530 535 540
   Gly Met Thr Lys Asn Thr Phe Glu Val Phe Pro Met Thr Ser Ser Gly
   545 550 555 560
   Ala Pro Ile Ser Glu Tyr Pro Phe Ile Arg Thr Gly Leu Leu Gly Phe
   565 570 575
   Val Gly Pro Gly Gly Leu Val Phe Val Val Gly Lys Met Asp Gly Leu
   580 585 590
   Met Val Val Ser Gly Arg Arg His Asn Ala Asp Asp Ile Val Ala Thr
   595 600 605
   Ala Leu Ala Val Glu Pro Met Lys Phe Val Tyr Arg Gly Arg Ile Ala
   610 615 620
   Val Phe Ser Val Thr Val Leu His Asp Glu Arg Ile Val Ile Val Ala
   625 630 635 640
   Glu Gln Arg Pro Asp Ser Thr Glu Glu Asp Ser Phe Gln Trp Met Ser
   645 650 655
   Arg Val Leu Gln Ala Ile Asp Ser Ile His Gln Val Gly Val Tyr Cys
   660 665 670
   Leu Ala Leu Val Pro Ala Asn Thr Leu Pro Lys Thr Pro Leu Gly Gly
   675 680 685
   Ile His Leu Ser Glu Thr Lys Gln Leu Phe Leu Glu Gly Ser Leu His
       690                 695                 700
   Pro Cys Asn Val Leu Met Cys Pro His Thr Cys Val Thr Asn Leu Pro
   705                 710                 715                 720
   Lys Pro Arg Gln Lys Gln Pro Glu Ile Gly Pro Ala Ser Val Met Val
                   725                 730                 735
   Gly Asn Leu Val Ser Gly Lys Arg Ile Ala Gln Ala Ser Gly Arg Asp
               740                 745                 750
   Leu Gly Gln Ile Glu Asp Asn Asp Gln Ala Arg Lys Phe Leu Phe Leu
           755                 760                 765
   Ser Glu Val Leu Gln Trp Arg Ala Gln Thr Thr Pro Asp His Ile Leu
       770                 775                 780
   Tyr Thr Leu Leu Asn Cys Arg Gly Ala Ile Ala Asn Ser Leu Thr Cys
   785                 790                 795                 800
   Val Gln Leu His Lys Arg Ala Glu Lys Ile Ala Val Met Leu Met Glu
                   805                 810                 815
   Arg Gly His Leu Gln Asp Gly Asp His Val Ala Leu Val Tyr Pro Pro
               820                 825                 830
   Gly Ile Asp Leu Ile Ala Ala Phe Tyr Gly Cys Leu Tyr Ala Gly Cys
           835                 840                 845
   Val Pro Ile Thr Val Arg Pro Pro His Pro Gln Asn Ile Ala Thr Thr
       850                 855                 860
   Leu Pro Thr Val Lys Met Ile Val Glu Val Ser Arg Ser Ala Cys Leu
   865                 870                 875                 880
   Met Thr Thr Gln Leu Ile Cys Lys Leu Leu Arg Ser Arg Glu Ala Ala
                   885                 890                 895
   Ala Ala Val Asp Val Arg Thr Trp Pro Leu Ile Leu Asp Thr Asp Asp
               900                 905                 910
   Leu Pro Lys Lys Arg Pro Ala Gln Ile Cys Lys Pro Cys Asn Pro Asp
           915                 920                 925
   Thr Leu Ala Tyr Leu Asp Phe Ser Val Ser Thr Thr Gly Met Leu Ala
   930 935 940
   Gly Val Lys Met Ser His Ala Ala Thr Ser Ala Phe Cys Arg Ser Ile
   945 950 955 960
   Lys Leu Gln Cys Glu Leu Tyr Pro Ser Arg Glu Val Ala Ile Cys Leu
   965 970 975
   Asp Pro Tyr Cys Gly Leu Gly Phe Val Leu Trp Cys Leu Cys Ser Val
   980 985 990
   Tyr Ser Gly His Gln Ser Ile Leu Ile Pro Pro Ser Glu Leu Glu Thr
   995 1000 1005
   Asn Pro Ala Leu Trp Leu Leu Ala Val Ser Gln Tyr Lys Val Arg
   1010 1015 1020
   Asp Thr Phe Cys Ser Tyr Ser Val Met Glu Leu Cys Thr Lys Gly
   1025 1030 1035
   Leu Gly Ser Gln Thr Glu Ser Leu Lys Ala Arg Gly Leu Asp Leu
   1040 1045 1050
   Ser Arg Val Arg Thr Cys Val Val Val Ala Glu Glu Arg Pro Arg
   1055 1060 1065
   Ile Ala Leu Thr Gln Ser Phe Ser Lys Leu Phe Lys Asp Leu Gly
   1070 1075 1080
   Leu His Pro Arg Ala Val Ser Thr Ser Phe Gly Cys Arg Val Asn
   1085 1090 1095
   Leu Ala Ile Cys Leu Gln Gly Thr Ser Gly Pro Asp Pro Thr Thr
   1100 1105 1110
   Val Tyr Val Asp Met Arg Ala Leu Arg His Asp Arg Val Arg Leu
   1115 1120 1125
   Val Glu Arg Gly Ser Pro His Ser Leu Pro Leu Met Glu Ser Gly
   1130 1135 1140
   Lys Ile Leu Pro Gly Val Arg Ile Ile Ile Ala Asn Pro Glu Thr
   1145 1150 1155
   Lys Gly Pro Leu Gly Asp Ser His Leu Gly Glu Ile Trp Val His
   1160 1165 1170
   Ser Ala  His Asn Ala Ser Gly  Tyr Phe Thr Ile Tyr  Gly Asp Glu
       1175                 1180                 1185
   Ser Leu  Gln Ser Asp His Phe  Asn Ser Arg Leu Ser  Phe Gly Asp
       1190                 1195                 1200
   Thr Gln  Thr Ile Trp Ala Arg  Thr Gly Tyr Leu Gly  Phe Leu Arg
       1205                 1210                 1215
   Arg Thr  Glu Leu Thr Asp Ala  Asn Gly Glu Arg His  Asp Ala Leu
       1220                 1225                 1230
   Tyr Val  Val Gly Ala Leu Asp  Glu Ala Met Glu Leu  Arg Gly Met
       1235                 1240                 1245
   Arg Tyr  His Pro Ile Asp Ile  Glu Thr Ser Val Ile  Arg Ala His
       1250                 1255                 1260
   Lys Ser  Val Thr Glu Cys Ala  Val Phe Thr Trp Thr  Asn Leu Leu
       1265                 1270                 1275
   Val Val  Val Val Glu Leu Asp  Gly Ser Glu Gln Glu  Ala Leu Asp
       1280                 1285                 1290
   Leu Val  Pro Leu Val Thr Asn  Val Val Leu Glu Glu  His Tyr Leu
       1295                 1300                 1305
   Ile Val  Gly Val Val Val Val  Val Asp Ile Gly Val  Ile Pro Ile
       1310                 1315                 1320
   Asn Ser  Arg Gly Glu Lys Gln  Arg Met His Leu Arg  Asp Gly Phe
       1325                 1330                 1335
   Leu Ala  Asp Gln Leu Asp Pro  Ile Tyr Val Ala Tyr  Asn Met
       1340                 1345                 1350
<210> 65
   <211> 4639
   <212> DNA
   <213> Homo sapiens
<400> 65
<210> 66
   <211> 863
   <212> PRT
   <213> Homo sapiens
<400> 66
   Glu Lys Leu Ser Val Leu Thr Val Gln Asp Val Gly Gln Val Met Pro
   1               5                   10                  15
   Gly Ala Asn Val Cys Val Val Lys Leu Glu Gly Thr Pro Tyr Leu Cys
               20                  25                  30
   Lys Thr Asp Glu Val Gly Glu Ile Cys Val Ser Ser Ser Ala Thr Gly
           35                  40                  45
   Thr Ala Tyr Tyr Gly Leu Leu Gly Ile Thr Lys Asn Val Phe Glu Ala
       50                  55                  60
   Val Pro Val Thr Thr Gly Gly Ala Pro Ile Phe Asp Arg Pro Phe Thr
   65                  70                  75                  80
   Arg Thr Gly Leu Leu Gly Phe Ile Gly Pro Asp Asn Leu Val Phe Ile
                   85                  90                  95
   Val Gly Lys Leu Asp Gly Leu Met Val Thr Gly Val Arg Arg His Asn
               100                 105                 110
   Ala Asp Asp Val Val Ala Thr Ala Leu Ala Val Glu Pro Met Lys Phe
   115 120 125
   Val Tyr Arg Gly Arg Ile Ala Val Phe Ser Val Thr Val Leu His Asp
   130 135 140
   Asp Arg Ile Val Leu Val Ala Glu Gln Arg Pro Asp Ala Ser Glu Glu
   145 150 155 160
   Asp Ser Phe Gln Trp Met Ser Arg Val Leu Gln Ala Ile Asp Ser Ile
   165 170 175
   His Gln Val Gly Val Tyr Cys Leu Ala Leu Val Pro Ala Asn Thr Leu
   180 185 190
   Pro Lys Ala Pro Leu Gly Gly Ile His Ile Ser Glu Thr Lys Gln Arg
   195 200 205
   Phe Leu Glu Gly Thr Leu His Pro Cys Asn Val Leu Met Cys Pro His
   210 215 220
   Thr Cys Val Thr Asn Leu Pro Lys Pro Arg Gln Lys Gln Pro Glu Val
   225 230 235 240
   Gly Pro Ala Ser Met Ile Val Gly Asn Leu Val Ala Gly Lys Arg Ile
   245 250 255
   Ala Gln Ala Ser Gly Arg Glu Leu Ala His Leu Glu Asp Ser Asp Gln
   260 265 270
   Ala Arg Lys Phe Leu Phe Leu Ala Asp Val Leu Gln Trp Arg Ala His
   275 280 285
   Thr Thr Pro Asp His Pro Leu Phe Leu Leu Leu Asn Ala Lys Gly Thr
   290 295 300
   Val Thr Ser Thr Ala Thr Cys Val Gln Leu His Lys Arg Ala Glu Arg
   305 310 315 320
   Val Ala Ala Ala Leu Met Glu Lys Gly Arg Leu Ser Val Gly Asp His
   325 330 335
   Val Ala Leu Val Tyr Pro Pro Gly Val Asp Leu Ile Ala Ala Phe Tyr
   340 345 350
   Gly Cys Leu Tyr Cys Gly Cys Val Pro Val Thr Val Arg Pro Pro His
   355 360 365
   Pro Gln Asn Leu Gly Thr Thr Leu Pro Thr Val Lys Met Ile Val Glu
   370 375 380
   Val Ser Lys Ser Ala Cys Val Leu Thr Thr Gln Ala Val Thr Arg Leu
   385 390 395 400
   Leu Arg Ser Lys Glu Ala Ala Ala Ala Val Asp Ile Arg Thr Trp Pro
   405 410 415
   Thr Ile Leu Asp Thr Asp Asp Ile Pro Lys Lys Lys Ile Ala Ser Val
   420 425 430
   Phe Arg Pro Pro Ser Pro Asp Val Leu Ala Tyr Leu Asp Phe Ser Val
   435 440 445
   Ser Thr Thr Gly Ile Leu Ala Gly Val Lys Met Ser His Ala Ala Thr
   450 455 460
   Ser Ala Leu Cys Arg Ser Ile Lys Leu Gln Cys Glu Leu Tyr Pro Ser
   465 470 475 480
   Arg Gln Ile Ala Ile Cys Leu Asp Pro Tyr Cys Gly Leu Gly Phe Ala
   485 490 495
   Leu Trp Cys Leu Cys Ser Val Tyr Ser Gly His Gln Ser Val Leu Val
   500 505 510
   Pro Pro Leu Glu Leu Glu Ser Asn Val Ser Leu Trp Leu Ser Ala Val
   515 520 525
   Ser Gln Tyr Lys Ala Arg Val Thr Phe Cys Ser Tyr Ser Val Met Glu
   530 535 540
   Met Cys Thr Lys Gly Leu Gly Ala Gln Thr Gly Val Leu Arg Met Lys
   545 550 555 560
   Gly Val Asn Leu Ser Cys Val Arg Thr Cys Met Val Val Ala Glu Glu
   565 570 575
   Arg Pro Arg Ile Ala Leu Thr Gln Ser Phe Ser Lys Leu Phe Lys Asp
   580 585 590
   Leu Gly Leu Pro Ala Arg Ala Val Ser Thr Thr Phe Gly Cys Arg Val
   595 600 605
   Asn Val Ala Ile Cys Leu Gln Gly Thr Ala Gly Pro Asp Pro Thr Thr
   610 615 620
   Val Tyr Val Asp Met Arg Ala Leu Arg His Asp Arg Val Arg Leu Val
   625 630 635 640
   Glu Arg Gly Ser Pro His Ser Leu Pro Leu Met Glu Ser Gly Lys Ile
   645 650 655
   Leu Pro Gly Val Lys Val Ile Ile Ala His Thr Glu Thr Lys Gly Pro
   660 665 670
   Leu Gly Asp Ser His Leu Gly Glu Ile Trp Val Ser Ser Pro His Asn
   675 680 685
   Ala Thr Gly Tyr Tyr Thr Val Tyr Gly Glu Glu Ala Leu His Ala Asp
   690 695 700
   His Phe Ser Ala Arg Leu Ser Phe Gly Asp Thr Gln Thr Ile Trp Ala
   705 710 715 720
   Arg Thr Gly Tyr Leu Gly Phe Leu Arg Arg Thr Glu Leu Thr Asp Ala
   725 730 735
   Ser Gly Gly Arg His Asp Ala Leu Tyr Val Val Gly Ser Leu Asp Glu
   740 745 750
   Thr Leu Glu Leu Arg Gly Met Arg Tyr His Pro Ile Asp Ile Glu Thr
   755 760 765
   Ser Val Ile Arg Ala His Arg Ser Ile Ala Glu Cys Ala Val Phe Thr
   770 775 780
   Trp Thr Asn Leu Leu Val Val Val Val Glu Leu Asp Gly Leu Glu Gln
   785 790 795 800
   Asp Ala Leu Asp Leu Val Ala Leu Val Thr Asn Val Val Leu Glu Glu
   805 810 815
   His Tyr Leu Val Val Gly Val Val Val Ile Val Asp Pro Gly Val Ile
   820 825 830
   Pro Ile Asn Ser Arg Gly Glu Lys Gln Arg Met His Leu Arg Asp Gly
   835 840 845
   Phe Leu Ala Asp Gln Leu Asp Pro Ile Tyr Val Ala Tyr Asn Met
   850 855 860
<210> 67
   <211> 7389
   <212> DNA
   <213> Homo sapiens
<400> 67
   gtttctctct ctggtcggaa gcggcggtaa tggcggatgg tgggttgtgg cgccggcggc      60
   ggctgctgtg agggacgatg agtgcctcct tcgtgccgaa cggggccagc ctggaagatt     120
   gtcactgtaa cctcttctgc ctggctgact tgacaggaat taagtggaaa aaatatgtat     180
   ggcaaggccc aacttctgcc cctattctgt ttcctgtgac agaagaagac cccattttga     240
   gcagttttag tcgctgcctt aaggcagatg tacttggtgt ttggcggcga gatcaaagac     300
   ctggaagaag agaattgtgg atattttggt ggggtgaaga cccagttttg ctgaccttat     360
   tcaccatgac ttatcagaag aagaagatgg aatgtgggag aatggacttt cctatgaatg     420
   ccgtactctg cttttccaaa gcagttcaca atctattgga acggtgttta atgaacagga     480
   attttgtacg tattggcaag tggtttgtaa agccttatga aaaagatgaa aaacctataa     540
   ataaaagtga acacttgtcc tgctccttca cctttttctt gcatggagac agcaatgttt     600
   gtaccagtgt ggaaattaac caacatcaac ctgtatacct tctcagtgaa gagcatatca     660
   cccttgctca acagtctaat agcccatttc aagttatctt atgcccattt ggactaaatg     720
   gcactctcac aggacaggca ttcaagatgt ctgattcagc tacaaaaaaa ttaattggtg     780
   aatggaaaca gttctatcct atctcatgtt gcttgaagga gatgtctgaa gaaaaacagg     840
   aagatatgga ttgggaagat gattctttag ctgcagtaga agttcttgtt gctggtgtcc     900
   gaatgatcta cccagcatgc tttgttctag tccctcagtc agacattcct actcctagcc     960
   ctgtgggatc cactcactgt tcatcttctt gcttgggtgt ccaccaagtg cctgcttcca    1020
   caagagatcc tgctatgtct tcggttacgc ttacaccacc tacgtctcct gaggaagtcc    1080
   aaacagttga tcctcagtct gtccagaagt gggtcaaatt ttcttcagta tctgatggct    1140
   tcaactccga tagtactagc caccatggtg ggaaaatacc cagaaaatta gcaaatcatg    1200
   tggtggatag agtttggcaa gaatgcaata tgaacagagc acagaacaag aagaagtatt    1260
   ctgcttcatc aggtggtcta tgcgaagaag cgacagctgc taaagtggca tcctgggatt    1320
   ttgttgaagc cacacaaaga acaaattgca gttgtttgag gcacaaaaat ctcaagtcaa    1380
   gaaatgctgg acaacaagga caggcaccat ctttaggtca gcaacaacaa atacttccta    1440
   agcacaagac caatgagaag caagaaaaga gtgaagagcc acagaaacgc cccttgactc    1500
   cttttcacca tcgtgtgtct gttagtgatg atgttggcat ggacgcagat tcagccagcc    1560
   aaagacttgt gatctctgct ccagacagtc aagtgagatt ttcaaatatc cgaactaatg    1620
   atgtagcaaa gactcctcag atgcatggca ccgaaatggc aaattcacct caaccacccc    1680
   cacttagtcc tcacccttgt gatgtggttg atgaaggagt gactaaaaca ccttcaactc 1740
   ctcagagtca acatttttat caaatgccaa caccagatcc cttggttcct tctaaaccaa 1800
   tggaagatag gatagacagt ttgtcccagt ctttcccacc tcaatatcag gaagctgtag 1860
   aacctacagt atatgttggt acagcagtaa acttggaaga agatgaagcc aatatagcct 1920
   ggaagtatta caagttccca aagaaaaaag atgtagagtt tttaccacct caacttccaa 1980
   gtgataaatt caaggatgat ccagttggac cttttggaca ggaaagtgta acatcagtta 2040
   cagagttaat ggtgcaatgt aagaaacctt taaaagtttc tgatgaatta gtgcagcaat 2100
   atcaaattaa aaaccagtgt ctttcagcaa tagcatctga tgcagaacaa gaacctaaaa 2160
   ttgatccata tgcatttgtt gaaggagatg aggaattcct ttttcctgat aaaaaagata 2220
   gacaaaatag tgagagagaa gctggaaaaa aacacaaggt agaagatggg acatctagtg 2280
   taacagtgtt atcacatgaa gaagatgcta tgtcattatt tagtccctct atcaagcaag 2340
   atgctccacg ccctactagt catgcccgtc ctccatcaac aagtttgatt tatgactcag 2400
   acctggctgt ctcttatact gaccttgata atctcttcaa ttctgatgaa gatgaactaa 2460
   cacctggatc taaaagatca gcaaatggat cagatgataa agccagctgc aaggaatcaa 2520
   agacaggaaa tctggacccg ttatcttgca taagcactgc agatcttcat aaaatgtatc 2580
   ctacaccacc atcattggaa caacatatta tgggattttc cccaatgaat atgaataata 2640
   aagaatatgg tagtatggat acaacacctg gaggaactgt tctagaagga aatagttcta 2700
   gtataggagc gcagttcaaa attgaggttg atgagggatt ctgtagcccc aaaccttctg 2760
   aaattaaaga tttttcttat gtctataagc ctgaaaattg tcaaattcta gtgggatgtt 2820
   ccatgtttgc acctctaaaa actctaccaa gccaatatct gccccttatc aaattgccag 2880
   aagagtgtat ttaccgtcag agttggactg ttggaaaatt ggaattgctt tcttcagggc 2940
   cttcaatgcc attcatcaaa gagggtgatg gaagtaatat ggatcaagaa tatggcactg 3000
   cttatacacc tcaaactcat acttcttgtg ggatgcctcc tagcagtgca cctcctagta 3060
   acagcggagc aggaattctt ccttctccat ccacccctcg gtttccaact ccaaggactc 3120
   caaggactcc tcggactcct cgtggagctg gtggacctgc tagtgctcaa ggttcagtca 3180
   aatatgaaaa ttcagacttg tattcaccag cttctacccc atctacatgc agacccctta 3240
   attctgttga acctgcaact gtcccttcca tccctgaagc acacagtctt tatgtaaacc 3300
   tcatcctttc agaatcagtt atgaatttgt ttaaagactg taactctgat agttgttgca 3360
   tctgtgtttg caacatgaac atcaagggtg ccgatgttgg agtttacatt ccagatccaa 3420
   cgcaggaagc acaatatagg tgtacctgtg gcttcagtgc tgtcatgaac agaaaatttg 3480
   gaaacaattc aggattattt cttgaagatg aactagatat cataggacgc aatacagact 3540
   gtggcaaaga agcagaaaaa cgttttgaag ctctcagggc tacctctgct gaacatgtta 3600
   atggaggact aaaggaatct gaaaaattat ctgatgattt gatattattg ctacaagatc 3660
   agtgcactaa tttattttca ccctttggag cagcagacca agatcctttt cctaaaagtg 3720
   gtgtaattag caattgggta cgtgttgaag agcgtgactg ttgcaatgac tgctaccttg 3780
   cattagaaca tgggcgtcag ttcatggata acatgtcagg aggaaaagtt gatgaagcac 3840
   ttgtgaaaag ttcatgctta cacccctggt ccaaaagaaa cgatgtgagt atgcagtgct 3900
   cacaggatat acttcgaatg ctcctctctc ttcagccagt tcttcaggat gccattcaga 3960
   aaaaaagaac agtaagacct tggggtgttc agggtcctct cacttggcaa caatttcata 4020
   aaatggctgg ccgaggctct tatggaactg atgaatcccc agaaccactg ccaatcccca 4080
   catttttgtt gggttatgat tatgattatc tggtgctttc tccatttgct cttccttatt 4140
   gggagagact tatgctggaa ccctatggat ctcaaagaga tatagcctat gttgtactgt 4200
   gtccagaaaa tgaagccttg ttaaatggag caaaaagctt ttttagagat cttactgcaa 4260
   tatatgagtc ctgtcgatta ggtcaacata gacctgtttc tcgactgtta acagatggga 4320
   tcatgagagt tggatctact gcatcaaaga aactatcaga aaagttggta gcagaatggt 4380
   tttctcaggc agctgatggt aacaatgaag cattttctaa actcaagctt tatgcacaag 4440
   tctgcagata tgacctaggt ccttatcttg cttccctgcc attggacagc tctctacttt 4500
   cccagccaaa tttagttgcc cctacaagtc agtctttgat tactccacct cagatgacaa 4560
   atactggaaa tgctaatact ccatctgcca ccttagcatc tgcagcgagc agcactatga 4620
   cagtgacttc aggtgttgcc atatctactt cagttgccac agctaattca actttgacca 4680
   cagcttcaac ttcatcttca tcatcctcca acttgaatag tggagtatca tcaaataaac 4740
   taccttcgtt tccacccttt ggcagtatga acagtaatgc tgcaggatcc atgtctacac 4800
   aagcaaatac agttcagagt ggtcagctag gagggcaaca gacatcagct ctacagacag 4860
   ctgggatttc tggagaatca tcttcacttc ccactcagcc gcatcctgat gtgtctgaaa 4920
   gcacgatgga tcgggataaa gtgggaatcc ccacagatgg tgattcacat gcagtcacgt 4980
   atccacctgc aattgttgtt tatataattg atccttttac atacgaaaat acagacgaga 5040
   gcactaactc ttctagtgtg tggacattgg ggctacttcg atgctttcta gaaatggtcc 5100
   agactcttcc tcctcatatc aagagtactg tttctgtaca gattattcct tgtcagtacc 5160
   tgttgcaacc tgtgaagcat gaagatagag aaatctatcc ccagcattta aaatccctgg 5220
   ctttttcggc ctttacccag tgtcggaggc cacttccaac atcaaccaat gtgaaaacat 5280
   tgactggctt tggtccaggt ttagccatgg aaactgccct tagaagtcct gatagaccag 5340
   agtgtattcg actttatgca cctcctttta ttctggctcc agtgaaggac aaacagacag 5400
   agctaggaga aacatttgga gaagctggac agaaatataa tgttcttttt gtgggatact 5460
   gtttatcaca tgatcaaagg tggattcttg catcttgcac agatctatat ggagaacttt 5520
   tagaaacttg tatcattaac atcgatgttc caaatagggc tcgtcggaaa aaaagttctg 5580
   ctagaaaatt tggtctacag aaactttggg agtggtgctt aggacttgta caaatgagtt 5640
   cattgccatg gagagttgta attggtcgtc taggaaggat tggtcatgga gaattgaaag 5700
   attggagctg tttgctgagt cgtcgaaact tgcagtctct aagtaaaagg ctcaaagaca 5760
   tgtgtagaat gtgtggtata tctgctgcag actcccctag cattctcagt gcttgcttgg 5820
   tggcaatgga gccgcaaggc tcttttgtta ttatgccaga ttctgtgtca actggttctg 5880
   tatttggaag aagcacgact ctaaatatgc agacatctca gctaaatacc ccacaggata 5940
   catcatgtac tcatatactt gtgtttccta cttctgcttc tgtgcaagta gcttcagcta 6000
   cttataccac tgaaaatttg gatttagctt tcaatcccaa caatgatgga gcagatggaa 6060
   tgggtatctt tgatttgtta gacacaggag atgatcttga ccctgatatc attaatatcc 6120
   ttcctgcttc tccaactggt tctcctgtac attctccagg atctcattac ccccatggag 6180
   gtgatgcggg caagggtcag agtactgatc ggctactatc aacagaacct catgaggaag 6240
   tacctaatat tcttcagcaa ccattggccc ttggttactt tgtatcaact gccaaagcag 6300
   gtccattacc tgactggttc tggtcagcat gtcctcaagc acaatatcag tgtccccttt 6360
   ttcttaaggc ctctttgcac ctccacgtgc cttcagtgca atctgacgag ctgcttcaca 6420
   gtaaacactc ccacccactt gactcaaatc agacttcaga tgtcctcagg tttgttttgg 6480
   aacagtacaa tgcactctcc tggctaacct gtgaccctgc aacccaggac agacgctcat 6540
   gtctcccaat tcattttgtg gtgctgaatc agttatataa ctttattatg aatatgctgt 6600
   gatcttcatt tgatggaact gtgcaagaaa agaacaagga aaaatggatg tttcgctgca 6660
   ggattaagtt acaattatct tctcagtgaa ggtcatttgt gatggggtct aattcttatt 6720
   acttcaacaa atattgtttt gacttggggg gaggggctat aaccctgcta tttttcattg 6780
   actctattga actctttagg atgatgactg atcatacaaa acgtattata acattttcgt 6840
   agcaaaatta accttttttt tttccagtca cagtatttgt gaaaagtaat gagccatagt 6900
   acccagtcat gttaaatgaa tattaaaagc atggagagga aacatgagga acaatgaatt 6960
   tcaacatatg gcttcagaac atgaagatgt tcttgtatgg attatagtat ctagtattca 7020
   aaaatgcctg catctcttct cttatttatt gtaagttttt aaatgtataa attgtcttat 7080
   atttcttaac ctcttttata aaaattttcc tagaaggttt atactgcctt cttgctttaa 7140
   agcaattggt ctaaaatata tgtaatcgtc ttaattaaaa agttgcagta gggttgcttt 7200
   tagagtatta tttttttgta agggggtggg tgggacagta aatttgtatt gtctcgatgt 7260
   acagtttaac ggggatagag ggggaataat gtccatacca ttgtgtgtgg aggatttaca 7320
   gctaagctgt agttgcagag tacatgtaca gtaatgaagt tcactgtgtt tataaattga 7380
   aaaggtacc 7389
<210> 68
   <211> 2174
   <212> PRT
   <213> Homo sapiens
<400> 68
   Met Ser Ala Ser Phe Val Pro Asn Gly Ala Ser Leu Glu Asp Cys His
   1 5 10 15
   Cys Asn Leu Phe Cys Leu Ala Asp Leu Thr Gly Ile Lys Trp Lys Lys
   20 25 30
   Tyr Val Trp Gln Gly Pro Thr Ser Ala Pro Ile Leu Phe Pro Val Thr
   35 40 45
   Glu Glu Asp Pro Ile Leu Ser Ser Phe Ser Arg Cys Leu Lys Ala Asp
   50 55 60
   Val Leu Gly Val Trp Arg Arg Asp Gln Arg Pro Gly Arg Arg Glu Leu
   65 70 75 80
   Trp Ile Phe Trp Trp Gly Glu Asp Pro Val Leu Leu Thr Leu Phe Thr
   85 90 95
   Met Thr Tyr Gln Lys Lys Lys Met Glu Cys Gly Arg Met Asp Phe Pro
   100 105 110
   Met Asn Ala Val Leu Cys Phe Ser Lys Ala Val His Asn Leu Leu Glu
   115 120 125
   Arg Cys Leu Met Asn Arg Asn Phe Val Arg Ile Gly Lys Trp Phe Val
   130 135 140
   Lys Pro Tyr Glu Lys Asp Glu Lys Pro Ile Asn Lys Ser Glu His Leu
   145 150 155 160
   Ser Cys Ser Phe Thr Phe Phe Leu His Gly Asp Ser Asn Val Cys Thr
   165 170 175
   Ser Val Glu Ile Asn Gln His Gln Pro Val Tyr Leu Leu Ser Glu Glu
   180 185 190
   His Ile Thr Leu Ala Gln Gln Ser Asn Ser Pro Phe Gln Val Ile Leu
   195 200 205
   Cys Pro Phe Gly Leu Asn Gly Thr Leu Thr Gly Gln Ala Phe Lys Met
   210 215 220
   Ser Asp Ser Ala Thr Lys Lys Leu Ile Gly Glu Trp Lys Gln Phe Tyr
   225 230 235 240
   Pro Ile Ser Cys Cys Leu Lys Glu Met Ser Glu Glu Lys Gln Glu Asp
   245 250 255
   Met Asp Trp Glu Asp Asp Ser Leu Ala Ala Val Glu Val Leu Val Ala
   260 265 270
   Gly Val Arg Met Ile Tyr Pro Ala Cys Phe Val Leu Val Pro Gln Ser
   275 280 285
   Asp Ile Pro Thr Pro Ser Pro Val Gly Ser Thr His Cys Ser Ser Ser
   290 295 300
   Cys Leu Gly Val His Gln Val Pro Ala Ser Thr Arg Asp Pro Ala Met
   305 310 315 320
   Ser Ser Val Thr Leu Thr Pro Pro Thr Ser Pro Glu Glu Val Gln Thr
   325 330 335
   Val Asp Pro Gln Ser Val Gln Lys Trp Val Lys Phe Ser Ser Val Ser
   340 345 350
   Asp Gly Phe Asn Ser Asp Ser Thr Ser His His Gly Gly Lys Ile Pro
   355 360 365
   Arg Lys Leu Ala Asn His Val Val Asp Arg Val Trp Gln Glu Cys Asn
   370 375 380
   Met Asn Arg Ala Gln Asn Lys Lys Lys Tyr Ser Ala Ser Ser Gly Gly
   385 390 395 400
   Leu Cys Glu Glu Ala Thr Ala Ala Lys Val Ala Ser Trp Asp Phe Val
   405 410 415
   Glu Ala Thr Gln Arg Thr Asn Cys Ser Cys Leu Arg His Lys Asn Leu
   420 425 430
   Lys Ser Arg Asn Ala Gly Gln Gln Gly Gln Ala Pro Ser Leu Gly Gln
   435 440 445
   Gln Gln Gln Ile Leu Pro Lys His Lys Thr Asn Glu Lys Gln Glu Lys
   450 455 460
   Ser Glu Glu Pro Gln Lys Arg Pro Leu Thr Pro Phe His His Arg Val
   465 470 475 480
   Ser Val Ser Asp Asp Val Gly Met Asp Ala Asp Ser Ala Ser Gln Arg
   485 490 495
   Leu Val Ile Ser Ala Pro Asp Ser Gln Val Arg Phe Ser Asn Ile Arg
   500 505 510
   Thr Asn Asp Val Ala Lys Thr Pro Gln Met His Gly Thr Glu Met Ala
   515 520 525
   Asn Ser Pro Gln Pro Pro Pro Leu Ser Pro His Pro Cys Asp Val Val
   530 535 540
   Asp Glu Gly Val Thr Lys Thr Pro Ser Thr Pro Gln Ser Gln His Phe
   545 550 555 560
   Tyr Gln Met Pro Thr Pro Asp Pro Leu Val Pro Ser Lys Pro Met Glu
   565 570 575
   Asp Arg Ile Asp Ser Leu Ser Gln Ser Phe Pro Pro Gln Tyr Gln Glu
   580 585 590
   Ala Val Glu Pro Thr Val Tyr Val Gly Thr Ala Val Asn Leu Glu Glu
   595 600 605
   Asp Glu Ala Asn Ile Ala Trp Lys Tyr Tyr Lys Phe Pro Lys Lys Lys
   610 615 620
   Asp Val Glu Phe Leu Pro Pro Gln Leu Pro Ser Asp Lys Phe Lys Asp
   625 630 635 640
   Asp Pro Val Gly Pro Phe Gly Gln Glu Ser Val Thr Ser Val Thr Glu
   645 650 655
   Leu Met Val Gln Cys Lys Lys Pro Leu Lys Val Ser Asp Glu Leu Val
   660 665 670
   Gln Gln Tyr Gln Ile Lys Asn Gln Cys Leu Ser Ala Ile Ala Ser Asp
   675 680 685
   Ala Glu Gln Glu Pro Lys Ile Asp Pro Tyr Ala Phe Val Glu Gly Asp
   690 695 700
   Glu Glu Phe Leu Phe Pro Asp Lys Lys Asp Arg Gln Asn Ser Glu Arg
   705 710 715 720
   Glu Ala Gly Lys Lys His Lys Val Glu Asp Gly Thr Ser Ser Val Thr
   725 730 735
   Val Leu Ser His Glu Glu Asp Ala Met Ser Leu Phe Ser Pro Ser Ile
   740 745 750
   Lys Gln Asp Ala Pro Arg Pro Thr Ser His Ala Arg Pro Pro Ser Thr
   755 760 765
   Ser Leu Ile Tyr Asp Ser Asp Leu Ala Val Ser Tyr Thr Asp Leu Asp
   770 775 780
   Asn Leu Phe Asn Ser Asp Glu Asp Glu Leu Thr Pro Gly Ser Lys Arg
   785 790 795 800
   Ser Ala Asn Gly Ser Asp Asp Lys Ala Ser Cys Lys Glu Ser Lys Thr
   805 810 815
   Gly Asn Leu Asp Pro Leu Ser Cys Ile Ser Thr Ala Asp Leu His Lys
   820 825 830
   Met Tyr Pro Thr Pro Pro Ser Leu Glu Gln His Ile Met Gly Phe Ser
   835 840 845
   Pro Met Asn Met Asn Asn Lys Glu Tyr Gly Ser Met Asp Thr Thr Pro
   850 855 860
   Gly Gly Thr Val Leu Glu Gly Asn Ser Ser Ser Ile Gly Ala Gln Phe
   865 870 875 880
   Lys Ile Glu Val Asp Glu Gly Phe Cys Ser Pro Lys Pro Ser Glu Ile
   885 890 895
   Lys Asp Phe Ser Tyr Val Tyr Lys Pro Glu Asn Cys Gln Ile Leu Val
   900 905 910
   Gly Cys Ser Met Phe Ala Pro Leu Lys Thr Leu Pro Ser Gln Tyr Leu
   915 920 925
   Pro Leu Ile Lys Leu Pro Glu Glu Cys Ile Tyr Arg Gln Ser Trp Thr
   930 935 940
   Val Gly Lys Leu Glu Leu Leu Ser Ser Gly Pro Ser Met Pro Phe Ile
   945 950 955 960
   Lys Glu Gly Asp Gly Ser Asn Met Asp Gln Glu Tyr Gly Thr Ala Tyr
   965 970 975
   Thr Pro Gln Thr His Thr Ser Cys Gly Met Pro Pro Ser Ser Ala Pro
   980 985 990
   Pro Ser Asn Ser Gly Ala Gly Ile Leu Pro Ser Pro Ser Thr Pro Arg
   995 1000 1005
   Phe Pro Thr Pro Arg Thr Pro Arg Thr Pro Arg Thr Pro Arg Gly
   1010 1015 1020
   Ala Gly Gly Pro Ala Ser Ala Gln Gly Ser Val Lys Tyr Glu Asn
   1025 1030 1035
   Ser Asp Leu Tyr Ser Pro Ala Ser Thr Pro Ser Thr Cys Arg Pro
   1040 1045 1050
   Leu Asn Ser Val Glu Pro Ala Thr Val Pro Ser Ile Pro Glu Ala
   1055 1060 1065
   His Ser Leu Tyr Val Asn Leu Ile Leu Ser Glu Ser Val Met Asn
   1070 1075 1080
   Leu Phe Lys Asp Cys Asn Ser Asp Ser Cys Cys Ile Cys Val Cys
   1085 1090 1095
   Asn Met Asn Ile Lys Gly Ala Asp Val Gly Val Tyr Ile Pro Asp
   1100 1105 1110
   Pro Thr Gln Glu Ala Gln Tyr Arg Cys Thr Cys Gly Phe Ser Ala
   1115 1120 1125
   Val Met Asn Arg Lys Phe Gly Asn Asn Ser Gly Leu Phe Leu Glu
   1130 1135 1140
   Asp Glu Leu Asp Ile Ile Gly Arg Asn Thr Asp Cys Gly Lys Glu
   1145 1150 1155
   Ala Glu Lys Arg Phe Glu Ala Leu Arg Ala Thr Ser Ala Glu His
   1160 1165 1170
   Val Asn Gly Gly Leu Lys Glu Ser Glu Lys Leu Ser Asp Asp Leu
   1175 1180 1185
   Ile Leu Leu Leu Gln Asp Gln Cys Thr Asn Leu Phe Ser Pro Phe
   1190 1195 1200
   Gly Ala Ala Asp Gln Asp Pro Phe Pro Lys Ser Gly Val Ile Ser
   1205 1210 1215
   Asn Trp Val Arg Val Glu Glu Arg Asp Cys Cys Asn Asp Cys Tyr
   1220 1225 1230
   Leu Ala Leu Glu His Gly Arg Gln Phe Met Asp Asn Met Ser Gly
   1235 1240 1245
   Gly Lys Val Asp Glu Ala Leu Val Lys Ser Ser Cys Leu His Pro
   1250 1255 1260
   Trp Ser Lys Arg Asn Asp Val Ser Met Gln Cys Ser Gln Asp Ile
   1265 1270 1275
   Leu Arg Met Leu Leu Ser Leu Gln Pro Val Leu Gln Asp Ala Ile
   1280 1285 1290
   Gln Lys Lys Arg Thr Val Arg Pro Trp Gly Val Gln Gly Pro Leu
   1295 1300 1305
   Thr Trp Gln Gln Phe His Lys Met Ala Gly Arg Gly Ser Tyr Gly
   1310 1315 1320
   Thr Asp Glu Ser Pro Glu Pro Leu Pro Ile Pro Thr Phe Leu Leu
   1325 1330 1335
   Gly Tyr Asp Tyr Asp Tyr Leu Val Leu Ser Pro Phe Ala Leu Pro
   1340 1345 1350
   Tyr Trp Glu Arg Leu Met Leu Glu Pro Tyr Gly Ser Gln Arg Asp
   1355 1360 1365
   Ile Ala Tyr Val Val Leu Cys Pro Glu Asn Glu Ala Leu Leu Asn
   1370 1375 1380
   Gly Ala Lys Ser Phe Phe Arg Asp Leu Thr Ala Ile Tyr Glu Ser
   1385 1390 1395
   Cys Arg Leu Gly Gln His Arg Pro Val Ser Arg Leu Leu Thr Asp
   1400 1405 1410
   Gly Ile Met Arg Val Gly Ser Thr Ala Ser Lys Lys Leu Ser Glu
   1415 1420 1425
   Lys Leu Val Ala Glu Trp Phe Ser Gln Ala Ala Asp Gly Asn Asn
   1430 1435 1440
   Glu Ala Phe Ser Lys Leu Lys Leu Tyr Ala Gln Val Cys Arg Tyr
   1445 1450 1455
   Asp Leu Gly Pro Tyr Leu Ala Ser Leu Pro Leu Asp Ser Ser Leu
   1460 1465 1470
   Leu Ser Gln Pro Asn Leu Val Ala Pro Thr Ser Gln Ser Leu Ile
   1475 1480 1485
   Thr Pro Pro Gln Met Thr Asn Thr Gly Asn Ala Asn Thr Pro Ser
   1490 1495 1500
   Ala Thr Leu Ala Ser Ala Ala Ser Ser Thr Met Thr Val Thr Ser
   1505 1510 1515
   Gly Val Ala Ile Ser Thr Ser Val Ala Thr Ala Asn Ser Thr Leu
   1520 1525 1530
   Thr Thr Ala Ser Thr Ser Ser Ser Ser Ser Ser Asn Leu Asn Ser
   1535 1540 1545
   Gly Val Ser Ser Asn Lys Leu Pro Ser Phe Pro Pro Phe Gly Ser
   1550 1555 1560
   Met Asn Ser Asn Ala Ala Gly Ser Met Ser Thr Gln Ala Asn Thr
   1565 1570 1575
   Val Gln Ser Gly Gln Leu Gly Gly Gln Gln Thr Ser Ala Leu Gln
   1580 1585 1590
   Thr Ala Gly Ile Ser Gly Glu Ser Ser Ser Leu Pro Thr Gln Pro
   1595 1600 1605
   His Pro Asp Val Ser Glu Ser Thr Met Asp Arg Asp Lys Val Gly
   1610 1615 1620
   Ile Pro Thr Asp Gly Asp Ser His Ala Val Thr Tyr Pro Pro Ala
   1625 1630 1635
   Ile Val Val Tyr Ile Ile Asp Pro Phe Thr Tyr Glu Asn Thr Asp
   1640 1645 1650
   Glu Ser Thr Asn Ser Ser Ser Val Trp Thr Leu Gly Leu Leu Arg
   1655 1660 1665
   Cys Phe Leu Glu Met Val Gln Thr Leu Pro Pro His Ile Lys Ser
   1670 1675 1680
   Thr Val Ser Val Gln Ile Ile Pro Cys Gln Tyr Leu Leu Gln Pro
   1685 1690 1695
   Val Lys His Glu Asp Arg Glu Ile Tyr Pro Gln His Leu Lys Ser
   1700 1705 1710
   Leu Ala Phe Ser Ala Phe Thr Gln Cys Arg Arg Pro Leu Pro Thr
   1715 1720 1725
   Ser Thr Asn Val Lys Thr Leu Thr Gly Phe Gly Pro Gly Leu Ala
   1730 1735 1740
   Met Glu Thr Ala Leu Arg Ser Pro Asp Arg Pro Glu Cys Ile Arg
   1745 1750 1755
   Leu Tyr Ala Pro Pro Phe Ile Leu Ala Pro Val Lys Asp Lys Gln
   1760 1765 1770
   Thr Glu Leu Gly Glu Thr Phe Gly Glu Ala Gly Gln Lys Tyr Asn
   1775 1780 1785
   Val Leu Phe Val Gly Tyr Cys Leu Ser His Asp Gln Arg Trp Ile
   1790 1795 1800
   Leu Ala Ser Cys Thr Asp Leu Tyr Gly Glu Leu Leu Glu Thr Cys
   1805 1810 1815
   Ile Ile Asn Ile Asp Val Pro Asn Arg Ala Arg Arg Lys Lys Ser
   1820 1825 1830
   Ser Ala Arg Lys Phe Gly Leu Gln Lys Leu Trp Glu Trp Cys Leu
   1835 1840 1845
   Gly Leu Val Gln Met Ser Ser Leu Pro Trp Arg Val Val Ile Gly
   1850 1855 1860
   Arg Leu Gly Arg Ile Gly His Gly Glu Leu Lys Asp Trp Ser Cys
   1865 1870 1875
   Leu Leu Ser Arg Arg Asn Leu Gln Ser Leu Ser Lys Arg Leu Lys
   1880 1885 1890
   Asp Met Cys Arg Met Cys Gly Ile Ser Ala Ala Asp Ser Pro Ser
   1895 1900 1905
   Ile Leu Ser Ala Cys Leu Val Ala Met Glu Pro Gln Gly Ser Phe
   1910 1915 1920
   Val Ile Met Pro Asp Ser Val Ser Thr Gly Ser Val Phe Gly Arg
   1925 1930 1935
   Ser Thr Thr Leu Asn Met Gln Thr Ser Gln Leu Asn Thr Pro Gln
   1940 1945 1950
   Asp Thr Ser Cys Thr His Ile Leu Val Phe Pro Thr Ser Ala Ser
   1955 1960 1965
   Val Gln Val Ala Ser Ala Thr Tyr Thr Thr Glu Asn Leu Asp Leu
   1970 1975 1980
   Ala Phe Asn Pro Asn Asn Asp Gly Ala Asp Gly Met Gly Ile Phe
   1985 1990 1995
   Asp Leu Leu Asp Thr Gly Asp Asp Leu Asp Pro Asp Ile Ile Asn
   2000 2005 2010
   Ile Leu Pro Ala Ser Pro Thr Gly Ser Pro Val His Ser Pro Gly
   2015 2020 2025
   Ser His Tyr Pro His Gly Gly Asp Ala Gly Lys Gly Gln Ser Thr
   2030 2035 2040
   Asp Arg Leu Leu Ser Thr Glu Pro His Glu Glu Val Pro Asn Ile
   2045 2050 2055
   Leu Gln Gln Pro Leu Ala Leu Gly Tyr Phe Val Ser Thr Ala Lys
   2060 2065 2070
   Ala Gly Pro Leu Pro Asp Trp Phe Trp Ser Ala Cys Pro Gln Ala
   2075 2080 2085
   Gln Tyr Gln Cys Pro Leu Phe Leu Lys Ala Ser Leu His Leu His
   2090 2095 2100
   Val Pro Ser Val Gln Ser Asp Glu Leu Leu His Ser Lys His Ser
   2105 2110 2115
   His Pro Leu Asp Ser Asn Gln Thr Ser Asp Val Leu Arg Phe Val
   2120 2125 2130
   Leu Glu Gln Tyr Asn Ala Leu Ser Trp Leu Thr Cys Asp Pro Ala
   2135 2140 2145
   Thr Gln Asp Arg Arg Ser Cys Leu Pro Ile His Phe Val Val Leu
   2150 2155 2160
   Asn Gln Leu Tyr Asn Phe Ile Met Asn Met Leu
   2165 2170
<210> 69
   <211> 6131
   <212> DNA
   <213> Homo sapiens
<400> 69
   cactgccagt ggtcaagggt ctgttaagta tgatagcacc gatcaaggat caccagcctc 60
   caccccctct actacacggc ccctcaactc tgtggagccc gccaccatgc agccaattcc 120
   cgaagcccac agcctctatg ttaccctgat tctctccgat tccgtgatga atatctttaa 180
   agacagaaac tttgacagct gttgcatctg tgcctgcaac atgaacatca aaggggcgga 240
   tgtcgggctt tacatccccg attcttccaa tgaggaccag taccgctgta cctgtgggtt 300
   tagtgcgatt atgaaccgca aacttggcta caattcagga ctcttccttg aagatgagtt 360
   ggatattttt gggaagaatt ctgatattgg tcaggctgca gagaggcgct taatgatgtg 420
   tcagtccacc ttccttcctc aggtggaagg aaccaaaaaa ccccaggagc cacccataag 480
   ccttctcctc ctcctccaga atcaacacac acaacctttt gcttcactga atttcctgga 540
   ctacatttcc tctaacaatc gccaaactct tccctgtgta agctggagtt atgaccgggt 600
   gcaagcagat aataatgatt actggacgga atgctttaat gcgttggagc aggggcggca 660
   gtatgtggat aaccccactg gtggaaaagt ggacgaagct ctggtgagaa gtgccactgt 720
   gcactcttgg cctcacagca atgtgctgga catcagcatg ctctcctccc aggatgtggt 780
   tcgtatgctg ttgtccctgc agccctttct ccaagatgcc atccaaaaga agcgcacggg 840
   caggacctgg gagaacatcc agcatgtgca gggaccactc acttggcagc agttccataa 900
   aatggcagga cggggaacct acggttcgga agaatctcct gagccgttgc ccatccccac 960
   tctgctggta ggctatgaca aggatttcct caccatctcg ccattctcct tgccgttttg 1020
   ggagaggctc ttgttggacc catatggggg ccaccgtgat gttgcctata ttgtggtgtg 1080
   tccagaaaat gaggccttgc tcgaaggagc caaaactttc ttcagggact tgagtgctgt 1140
   atacgagatg tgtaggcttg ggcagcacaa gcccatctgc aaagtgctac gtgacgggat 1200
   catgcgcgtg ggaaaaactg tggcacagaa gctgacagat gagcttgtga gtgagtggtt 1260
   taaccagcct tggagcggcg aggagaatga caatcattcc agactcaaac tttatgcgca 1320
   agtttgccgc catcacctag caccttattt agccactctg cagcttgata gcagcctatt 1380
   gataccacct aaataccaga ccccaccagc agcagcacag ggacaagcta cgccagggaa 1440
   tgctgggccc ttagctccaa atggatcagc agctccccca gctggcagtg catttaatcc 1500
   cacctcgaat agtagttcta caaatcctgc agcaagtagt tctgcatctg gttcctctgt 1560
   gccaccggtc tcatcgtctg cctctgctcc tggtattagc cagataagca ctacctcttc 1620
   ttcaggattc agtggtagtg ttggagggca gaaccccagc actgggggca tttctgcgga 1680
   tagaacgcaa gggaacatag gctgtggtgg agacactgac cctgggcaga gctcttctca 1740
   gccctcacag gatggacaag agagtgttac agaaagggag agaataggaa ttcccacgga 1800
   gcctgactct gcagacagcc atgcccaccc tccagctgtt gtcatttaca tggtggaccc 1860
   gttcacgtat gctgcagagg aggactccac ttctgggaac ttttggctgt tgagcttgat 1920
   gcgctgctac acagaaatgc tggataattt acctgagcat atgagaaatt ctttcattct 1980
   ccagattgtg ccttgccagt acatgctgca gacaatgaag gatgagcaag ttttctacat 2040
   tcaatacttg aagtccatgg cattttcagt gtactgccag tgcaggcgac cactgcctac 2100
   acagatccac attaaatccc tcacgggatt tgggcctgca gccagcattg agatgaccct 2160
   caagaaccct gagcggccca gcccaatcca gctttactcc cctcccttta tattggcccc 2220
   aatcaaagac aagcagacag agctgggaga gacgtttggt gaggcgagcc agaaatacaa 2280
   tgtgctcttc gtgggctatt gtctgtctca cgaccagcgc tggcttttgg cttcctgcac 2340
   tgacctccat ggggaattat tagagacctg cgttgtaaat attgctttac caaacaggtc 2400
   acggaggagt aaagtatctg cacgtaaaat tggactacag aagttatggg agtggtgcat 2460
   agggattgtc caaatgacat ctctaccctg gagagttgta atcgggcgac ttgggcgtct 2520
   tggccatggg gagcttaaag attggagtat cctccttgga gaatgttcac tacagacaat 2580
   cagcaaaaag ctcaaggatg tgtgccggat gtgtggaatc tctgccgcag actctccttc 2640
   tatccttagt gcctgcctgg ttgccatgga gccccagggg tcctttgtag tgatgccaga 2700
   tgctgtcaca atgggctctg tttttggccg aagtactgca ctgaacatgc agtcatctca 2760
   gctcaacacc cctcaagatg cttcttgtac acacatcttg gtgttcccaa catcatcaac 2820
   catccaggtg gctccagcca actaccccaa tgaagatggg tttagcccca acaatgatga 2880
   tatgtttgtt gaccttccat tcccagatga tatggacaat gatattggca tattaatgac 2940
   tgggaacctc cattcctctc ccaactcttc cccagtaccc tccccaggct ctccttctgg 3000
   aattggtgtg ggctctcact tccagcatag tcggagccag ggtgagcgtc ttctttctag 3060
   agaagcacca gaggagctaa agcagcagcc cctggccctt gggtattttg tatcaactgc 3120
   caaagctgag aatcttcccc agtggttttg gtcatcgtgt ccccaggctc aaaaccagtg 3180
   ccctctcttc ttaaaggctt cgctgcatca ccacatttca gtagcacaga cagacgaact 3240
   tctgcctgcc aggaattctc agcgggttcc acaccctctt gactccaaaa ccacgtcgga 3300
   tgttttaagg tttgttttgg agcagtacaa cgctctgtcc tggctcacgt gcaatccggc 3360
   cacccaggac cgtacttcct gccttcccgt ccactttgtg gtgctcactc agttgtacaa 3420
   tgccatcatg aatatacttt aattggaaaa gcacttgttc tctctggctc agttccttct 3480
   ccctgcaacc tcagtccaag gaacctgcta cactctgcaa ataacccaca tccttttctt 3540
   cagaccactc tccacagtcc tgcactgtga ttccttctca gcaggcacat gtcaattctg 3600
   cagtgttcat taccagagtg actccttgac acttctctca tggacctgga aacttccata 3660
   agtggtgact ttcagccagt gcggtggtgt gtgtagcccc aaccactggt ccccaggaag 3720
   tggtggtggt tgatggcttt tcagcgggaa acagaagaga cagtgtcctt ttgcacaaga 3780
   gtctgtgttt tcagcctctg tatacaattg agggcagtct agccctttgg atgaaatcct 3840
   cttagttact ggtgtatggc ctgtgggtta cctgaactcc ataatcgggg actttttaaa 3900
   aataagaacc agctcaagta catggtttca tactggggtt tctgtctccc tagtgttccc 3960
   atccagatta gcatgagtgc tttggttgac ttcaaacctg tgtgtcaatg cagaaggtct 4020
   ggagacagct tcattttgtt tatttatttt aatttgtttt gtcatatggt ttttgtgact 4080
   ttattttttt aattcacaag gaccaggtac agtagctgaa acccaattca gatccaccat 4140
   aggattcttt gactacatac ctctgtccta gaagccggaa aaggagtaaa aacacattgg 4200
   ggagatcatg cctaaaagta atatattcaa aaccacccag cagtaggttt tgttaacaac 4260
   aaactggatt ttaaaagttc tgccatgtta agtggccagc atttcatgaa ggataacatt 4320
   tttatacaga aggcagtcaa gctcaactca gagccatgga ggcaagtacc ttaattagtt 4380
   ttatatagtc acaacggaaa tatattttct agtgaattct tattggaagc caggtctctc 4440
   ctctcattag atcaaaaggg acttatgtac atacaacaat tgaaagtgtt tgctcatgaa 4500
   atcagttata aatatggtga atttttttct ggaccatagg aatattattt caaagaaata 4560
   ttacaactta accattaaat tagtacttga agttgagcct ttgtggtggg actttttaaa 4620
   aaaatgcctt tttaaagcat taatggctaa ttgaagtatt ttatgactcc tcattcctgg 4680
   cccagagggt tgtctttgaa accctgtttc taacccttgt gttgtgtgtt tctgtctgag 4740
   gacagtgggt gtgtactggc ctcccgggag ccactgtgac caggcctttg agctcttgtc 4800
   atctgtggag agaatcatgc aaattttaaa agttcttcca agagacttcc atgtcctggt   4860
   tattaacaaa aaagaaaaaa tgtaataatt gatatgattt tgtaaaagta tttttcttga   4920
   aataatctaa agtttaaaac attatattaa aaaaaaagtt gtgtggtggg aatgtgaaag   4980
   cagagaaata acttgtaaat ggataatttt gttctctgta ccaccagttg aagggggggt    5040
   tgactttcgc aatgtatagg ataaaaaatc tgatatatca aaccatttgt atctaatgtg   5100
   tacagtgtaa aattgacttt aaaaatattg cagtgctatt ttttcttaat cagaaaggaa   5160
   aattctcaag gccttttgaa gagcataaga agatgaagat tgtaaacttg tataaaatta  5220
   tcttggtgag aagacaaatt gtaaagtaga tatttgtaat cttttaccac tttggggttg   5280
   cttttttccc ggaattcatc agaactttga attttttttt taaatgggct gtttttaatg   5340
   caggggcttt tcttccctag aaacccaatt ctaagcagaa aaagaaaaaa aacacaaaaa   5400
   ataaaaaacc cctacaaaaa aactttaaaa aaaatggcag caaagggtag ttttcatctg   5460
   gtgtctttta tttaagtttt ttaagttaag aaaagctggt gacatattta tacgtttttg   5520
   tgcaaaaata aatgaatggc aatagatttt aaaaaatctt attatgtact tctgtgtgaa   5580
   aaagtctgta taatatttcc cttaaatatg cattatttta cttgtgagtt ttttactgaa   5640
   ttaatctgaa atgtacaagc cctggatttg ctacagagtg agaagttatt ttattttttt   5700
   ttatttttaa ttttggaaat tctgcagaaa tcagaactct taccatggtt tgaacaaaaa   5760
   aaggggaaat ggggagggga aaagggtggg attgtccagc atgcttgtat gtatatttca   5820
   gaaccttttt taaatgtaaa agctgtacat ttctgggaag ttctgaattt cttttgtttc   5880
   cttttttcct tcaagcattt tgcagtgagc ttcttttata tatagcaaac aatttgaaag   5940
   aatacaaaaa tatgtgaagt tcatttaaaa aaataactac agtatagcgc tggtacagta   6000
   cactaaaaga ctttgataaa aagaaacaat aataaaaggc ctccatttta aatgtcattc   6060
   atatatacct tgtggatgag agctatatac ttttacacac ttttttagag gaataaatta   6120
   ttgaattact g                                                        6131
<210> 70
   <211> 1146
   <212> PRT
   <213> Homo sapiens
<400> 70
   Thr Ala Ser Gly Gln Gly Ser Val Lys Tyr Asp Ser Thr Asp Gln Gly
   1               5                   10                  15
   Ser Pro Ala Ser Thr Pro Ser Thr Thr Arg Pro Leu Asn Ser Val Glu
               20                  25                  30
   Pro Ala Thr Met Gln Pro Ile Pro Glu Ala His Ser Leu Tyr Val Thr
   35 40 45
   Leu Ile Leu Ser Asp Ser Val Met Asn Ile Phe Lys Asp Arg Asn Phe
   50 55 60
   Asp Ser Cys Cys Ile Cys Ala Cys Asn Met Asn Ile Lys Gly Ala Asp
   65 70 75 80
   Val Gly Leu Tyr Ile Pro Asp Ser Ser Asn Glu Asp Gln Tyr Arg Cys
   85 90 95
   Thr Cys Gly Phe Ser Ala Ile Met Asn Arg Lys Leu Gly Tyr Asn Ser
   100 105 110
   Gly Leu Phe Leu Glu Asp Glu Leu Asp Ile Phe Gly Lys Asn Ser Asp
   115 120 125
   Ile Gly Gln Ala Ala Glu Arg Arg Leu Met Met Cys Gln Ser Thr Phe
   130 135 140
   Leu Pro Gln Val Glu Gly Thr Lys Lys Pro Gln Glu Pro Pro Ile Ser
   145 150 155 160
   Leu Leu Leu Leu Leu Gln Asn Gln His Thr Gln Pro Phe Ala Ser Leu
   165 170 175
   Asn Phe Leu Asp Tyr Ile Ser Ser Asn Asn Arg Gln Thr Leu Pro Cys
   180 185 190
   Val Ser Trp Ser Tyr Asp Arg Val Gln Ala Asp Asn Asn Asp Tyr Trp
   195 200 205
   Thr Glu Cys Phe Asn Ala Leu Glu Gln Gly Arg Gln Tyr Val Asp Asn
   210 215 220
   Pro Thr Gly Gly Lys Val Asp Glu Ala Leu Val Arg Ser Ala Thr Val
   225 230 235 240
   His Ser Trp Pro His Ser Asn Val Leu Asp Ile Ser Met Leu Ser Ser
   245 250 255
   Gln Asp Val Val Arg Met Leu Leu Ser Leu Gln Pro Phe Leu Gln Asp
   260 265 270
   Ala Ile Gln Lys Lys Arg Thr Gly Arg Thr Trp Glu Asn Ile Gln His
   275 280 285
   Val Gln Gly Pro Leu Thr Trp Gln Gln Phe His Lys Met Ala Gly Arg
   290 295 300
   Gly Thr Tyr Gly Ser Glu Glu Ser Pro Glu Pro Leu Pro Ile Pro Thr
   305 310 315 320
   Leu Leu Val Gly Tyr Asp Lys Asp Phe Leu Thr Ile Ser Pro Phe Ser
   325 330 335
   Leu Pro Phe Trp Glu Arg Leu Leu Leu Asp Pro Tyr Gly Gly His Arg
   340 345 350
   Asp Val Ala Tyr Ile Val Val Cys Pro Glu Asn Glu Ala Leu Leu Glu
   355 360 365
   Gly Ala Lys Thr Phe Phe Arg Asp Leu Ser Ala Val Tyr Glu Met Cys
   370 375 380
   Arg Leu Gly Gln His Lys Pro Ile Cys Lys Val Leu Arg Asp Gly Ile
   385 390 395 400
   Met Arg Val Gly Lys Thr Val Ala Gln Lys Leu Thr Asp Glu Leu Val
   405 410 415
   Ser Glu Trp Phe Asn Gln Pro Trp Ser Gly Glu Glu Asn Asp Asn His
   420 425 430
   Ser Arg Leu Lys Leu Tyr Ala Gln Val Cys Arg His His Leu Ala Pro
   435 440 445
   Tyr Leu Ala Thr Leu Gln Leu Asp Ser Ser Leu Leu Ile Pro Pro Lys
   450 455 460
   Tyr Gln Thr Pro Pro Ala Ala Ala Gln Gly Gln Ala Thr Pro Gly Asn
   465 470 475 480
   Ala Gly Pro Leu Ala Pro Asn Gly Ser Ala Ala Pro Pro Ala Gly Ser
   485 490 495
   Ala Phe Asn Pro Thr Ser Asn Ser Ser Ser Thr Asn Pro Ala Ala Ser
   500 505 510
   Ser Ser Ala Ser Gly Ser Ser Val Pro Pro Val Ser Ser Ser Ala Ser
   515 520 525
   Ala Pro Gly Ile Ser Gln Ile Ser Thr Thr Ser Ser Ser Gly Phe Ser
   530 535 540
   Gly Ser Val Gly Gly Gln Asn Pro Ser Thr Gly Gly Ile Ser Ala Asp
   545 550 555 560
   Arg Thr Gln Gly Asn Ile Gly Cys Gly Gly Asp Thr Asp Pro Gly Gln
   565 570 575
   Ser Ser Ser Gln Pro Ser Gln Asp Gly Gln Glu Ser Val Thr Glu Arg
   580 585 590
   Glu Arg Ile Gly Ile Pro Thr Glu Pro Asp Ser Ala Asp Ser His Ala
   595 600 605
   His Pro Pro Ala Val Val Ile Tyr Met Val Asp Pro Phe Thr Tyr Ala
   610 615 620
   Ala Glu Glu Asp Ser Thr Ser Gly Asn Phe Trp Leu Leu Ser Leu Met
   625 630 635 640
   Arg Cys Tyr Thr Glu Met Leu Asp Asn Leu Pro Glu His Met Arg Asn
   645 650 655
   Ser Phe Ile Leu Gln Ile Val Pro Cys Gln Tyr Met Leu Gln Thr Met
   660 665 670
   Lys Asp Glu Gln Val Phe Tyr Ile Gln Tyr Leu Lys Ser Met Ala Phe
   675 680 685
   Ser Val Tyr Cys Gln Cys Arg Arg Pro Leu Pro Thr Gln Ile His Ile
   690 695 700
   Lys Ser Leu Thr Gly Phe Gly Pro Ala Ala Ser Ile Glu Met Thr Leu
   705 710 715 720
   Lys Asn Pro Glu Arg Pro Ser Pro Ile Gln Leu Tyr Ser Pro Pro Phe
   725 730 735
   Ile Leu Ala Pro Ile Lys Asp Lys Gln Thr Glu Leu Gly Glu Thr Phe
   740 745 750
   Gly Glu Ala Ser Gln Lys Tyr Asn Val Leu Phe Val Gly Tyr Cys Leu
   755 760 765
   Ser His Asp Gln Arg Trp Leu Leu Ala Ser Cys Thr Asp Leu His Gly
   770 775 780
   Glu Leu Leu Glu Thr Cys Val Val Asn Ile Ala Leu Pro Asn Arg Ser
   785 790 795 800
   Arg Arg Ser Lys Val Ser Ala Arg Lys Ile Gly Leu Gln Lys Leu Trp
   805 810 815
   Glu Trp Cys Ile Gly Ile Val Gln Met Thr Ser Leu Pro Trp Arg Val
   820 825 830
   Val Ile Gly Arg Leu Gly Arg Leu Gly His Gly Glu Leu Lys Asp Trp
   835 840 845
   Ser Ile Leu Leu Gly Glu Cys Ser Leu Gln Thr Ile Ser Lys Lys Leu
   850 855 860
   Lys Asp Val Cys Arg Met Cys Gly Ile Ser Ala Ala Asp Ser Pro Ser
   865 870 875 880
   Ile Leu Ser Ala Cys Leu Val Ala Met Glu Pro Gln Gly Ser Phe Val
   885 890 895
   Val Met Pro Asp Ala Val Thr Met Gly Ser Val Phe Gly Arg Ser Thr
   900 905 910
   Ala Leu Asn Met Gln Ser Ser Gln Leu Asn Thr Pro Gln Asp Ala Ser
   915 920 925
   Cys Thr His Ile Leu Val Phe Pro Thr Ser Ser Thr Ile Gln Val Ala
   930 935 940
   Pro Ala Asn Tyr Pro Asn Glu Asp Gly Phe Ser Pro Asn Asn Asp Asp
   945 950 955 960
   Met Phe Val Asp Leu Pro Phe Pro Asp Asp Met Asp Asn Asp Ile Gly
   965 970 975
   Ile Leu Met Thr Gly Asn Leu His Ser Ser Pro Asn Ser Ser Pro Val
   980 985 990
   Pro Ser Pro Gly Ser Pro Ser Gly Ile Gly Val Gly Ser His Phe Gln
   995 1000 1005
   His Ser Arg Ser Gln Gly Glu Arg Leu Leu Ser Arg Glu Ala Pro
   1010 1015 1020
   Glu Glu Leu Lys Gln Gln Pro Leu Ala Leu Gly Tyr Phe Val Ser
   1025 1030 1035
   Thr Ala Lys Ala Glu Asn Leu Pro Gln Trp Phe Trp Ser Ser Cys
   1040 1045 1050
   Pro Gln Ala Gln Asn Gln Cys Pro Leu Phe Leu Lys Ala Ser Leu
   1055 1060 1065
   His His His Ile Ser Val Ala Gln Thr Asp Glu Leu Leu Pro Ala
   1070 1075 1080
   Arg Asn Ser Gln Arg Val Pro His Pro Leu Asp Ser Lys Thr Thr
   1085 1090 1095
   Ser Asp Val Leu Arg Phe Val Leu Glu Gln Tyr Asn Ala Leu Ser
   1100 1105 1110
   Trp Leu Thr Cys Asn Pro Ala Thr Gln Asp Arg Thr Ser Cys Leu
   1115 1120 1125
   Pro Val His Phe Val Val Leu Thr Gln Leu Tyr Asn Ala Ile Met
   1130 1135 1140
   Asn Ile Leu
   1145
<210> 71
   <211> 4829
   <212> DNA
   <213> Homo sapiens
<400> 71
   accgatcaag gatcaccagc ctccaccccc tctactacac ggcccctcaa ctctgtggag 60
   cccgccacca tgcagccaat tcccgaagcc cacagcctct atgttaccct gattctctcc 120
   gattccgtga tgaatatctt taaagacaga aactttgaca gctgttgcat ctgtgcctgc 180
   aacatgaaca tcaaaggggc ggatgtcggg ctttacatcc ccgattcttc caatgaggac 240
   cagtaccgct gtacctgtgg gtttagtgcg attatgaacc gcaaacttgg ctacaattca 300
   ggactcttcc ttgaagatga gttggatatt tttgggaaga attctgatat tggtcaggct 360
   gcagagaggc gcttaatgat gtgtcagtcc accttccttc ctcaggtgga aggaaccaaa 420
   aaaccccagg agccacccat aagccttctc ctcctcctcc agaatcaaca cacacaacct 480
   tttgcttcac tgaatttcct ggactacatt tcctctaaca atcgccaaac tcttccctgt 540
   gtaagctgga gttatgaccg ggtgcaagca gataataatg attactggac ggaatgcttt 600
   aatgcgttgg agcaggggcg gcagtatgtg gataacccca ctggtggaaa agtggacgaa 660
   gctctggtga gaagtgccac tgtgcactct tggcctcaca gcaatgtgct ggacatcagc 720
   atgctctcct cccaggatgt ggttcgtatg ctgttgtccc tgcagccctt tctccaagat 780
   gccatccaaa agaagcgcac gggcaggacc tgggagaaca tccagcatgt gcagggacca 840
   ctcacttggc agcagttcca taaaatggca ggacggggaa cctacggttc ggaagaatct 900
   cctgagccgt tgcccatccc cactctgctg gtaggctatg acaaggattt cctcaccatc 960
   tcgccattct ccttgccgtt ttgggagagg ctcttgttgg acccatatgg gggccaccgt 1020
   gatgttgcct atattgtggt gtgtccagaa aatgaggcct tgctcgaagg agccaaaact 1080
   ttcttcaggg acttgagtgc tgtatacgag atgtgtaggc ttgggcagca caagctcatc 1140
   tgcaaagtgc tacgtgacgg gatcatgcgc gtgggaaaaa ctgtggcaca gaagctgaca 1200
   gatgagcttg tgagtgagtg gtttaaccag ccttggagcg gcgaggagaa tgacaatcat 1260
   tccagactca aactttatgc gcaagtttgc cgccatcacc tagcacctta tttagccact 1320
   ctgcagcttg atagcagcct attgatacca cctaaatacc agaccccacc agcagcagca 1380
   cagggacaag ctacgccagg gaatgctggg cccttagctc caaatggatc agcagctccc 1440
   ccagctggca gtgcatttaa tcccacctcg aatagtagtt ctacaaatcc tgcagcaagt 1500
   aattctgcat ctggttcctc tgtgccaccg gtctcatcgt ctgcctctgc tcctggtatt 1560
   agccagataa gcactacctc ttcttcagga ttcagtggta gtgttggagg gcagaacccc 1620
   agcactgggg gcatttctgc ggatagaacg caagggaaca taggctgtgg tggagacact 1680
   gaccctgggc agagctcttc tcagccctca caggatggac aagagagtgt tacagaaagg 1740
   gagagaatag gaattcccac ggagcctgac tctgcagaca gccatgccca ccctccagct 1800
   gttgtcattt acatggtgga cccgttcacg tatgctgcag aggaggactc cacttctggg 1860
   aacttttggc tgttgagcat gatgcgctgc tacacagaaa tgctggataa tttacctgag 1920
   catatgagaa attctttcat tctccagatt gtgccttgcc agtacatgct gcagacaatg 1980
   aaggatgagc aagttttcta cattcaatac ttgaagtcca tggcattttc agtgtactgc 2040
   cagtgcaggc gaccactgcc tacacagatc cacattaaat ccctcacggg atttgggcct 2100
   gcagccagca ttgagatgac cctcaagaac cctgagcggc ccagcccaat ccagctttac 2160
   tcccctccct ttatattggc cccaatcaaa gacaagcaga cagagctggg agagacgttt 2220
   ggtgaggcga gccagaaata caatgtgctc ttcgtgggct attgtctgtc tcacgaccag 2280
   cgctggcttt tggcttcctg cactgacctc catggggaat tattagagac ctgcgttgta 2340
   aatattgctt taccaaacag gtcacggagg agtaaagtat ctgcacgtaa aattggacta 2400
   cagaagttat gggagtggtg catagggatt gtccaaatga catctctacc ctggagagtt 2460
   gtaatcgggc gacttgggcg tcttggccat ggggagctta aagattggag tatcctcctt 2520
   ggagaatgtt cactacagac aatcagcaaa aagctcaagg atgtgtgccg gatgtgtgga 2580
   atctctgccg cagactctcc ttctatcctt agtgcctgcc tggttgccat ggagccccag 2640
   gggtcctttg tagtgatgcc agatgctgtc acaatgggct cttttttggg ccgaagtact 2700
   gcactgaaca tgcagtcatc tcagctcaac acccctcaag atgcttcttg tacacacatc 2760
   ttggtgttcc caacatcatc aaccatccag gtggctccag ccaactaccc caatgaagat 2820
   gggtttagcc ccaacaatga tgatatgttt gttgaccttc cattcccaga tgatatggac 2880
   aatgatattg gcatattaat gactgggaac ctccattcct ctcccaactc ttccccagta 2940
   ccctccccag gctctccttc tggaattggt gtgggctctc acttccagca tagtcggagc 3000
   cagggtgagc gtcttctttc tagagaagca ccagaggagc taaagcagca gcccctggcc 3060
   cttgggtatt ttgtatcaac tgccaaagct gagaatcttc cccagtggtt ttggtcatcg 3120
   tgtccccagg ctcaaaacca gtgccctctc ttcttaaagg cttcgctgca tcaccacatt 3180
   tcagtagcac agacagacga acttctgcct gccaggaatt ctcagcgggt tccacaccct 3240
   cttgactcca aaaccacgtc ggatgtttta aggtttgttt tggagcagta caacgctctg 3300
   tcctggctca cgtgcaatcc ggccacccag gaccgtactt cctgccttcc cgcccacttt 3360
   gtggtgctca ctcagttgta caatgccatc atgaatatac tttaattgga aaagcacttg 3420
   ttctctctgg ctcagttctt tctccctgca acctctgtcc aaggaacctg ctacactctg 3480
   caaataaccc acatcctttt cttcagacca ctctccacag tcctgcactg tgattccttc 3540
   tcagcaggca catgtcaatt ctgcagtgtt cattaccaga gtgactcctt gacacttctc 3600
   tcatggacct ggaaacttcc ataagtggtg actttcagcc agtgcggtgg tgtgtgtagc 3660
   cccaaccact ggtccccagg aagtggtggt ggttgatggc ttttcagcgg gaaacagaag 3720
   agacagtgtc cttttgcaca agagtctgtg ttttcagccc ctgtatacaa ttgagggcag 3780
   tctagccctt tggatgaaat cctcttagtt actggtgtat ggcctgtggg ttacctgaac 3840
   tccataatct gggacttttt aaaaataaga accagctcaa gtacatggtt tcatactggg 3900
   gtttctgtct ccctagtgtt cccatccaga ttagcatgag tgctttggtt gacttcaaac 3960
   ctgtgtgtca atgcagaagg tctggagaca gcttcatttt gtttatttat tttaatttgt 4020
   tttgtcatat ggtttttgtg accttatttt tttaattcac aaggaccagg tacagtagct 4080
   gaaacccaat tcagatccac cataggattc tttgactaca tacctctgtc ctagaagccg 4140
   gaaaaggagt aaaaacacat tggggagatc atgcctaaaa gtaatatatt caaaaccacc 4200
   cagcagtagg ttttgttaac aacaaactgg attttaaaag ttctgccatg ttaagtggcc 4260
   agcatttcat gaaggataac atttttatac agaaggcagt caagctcaac tcagagccat 4320
   ggaggcaagt accttaatta gttttatata gtcacaacgg aaatatattt tctagtgaat    4380
   tcttattgga agccaggtct ctcctctcat tagatcaaaa gggacttatg tacatacaac    4440
   aattgaaagt gtttgctcat gaaatcagtt ataaatatgg tgaatttttt ctggaccata    4500
   ggaatattat ttcaaagaaa tattacaact taaccattaa attagtactt gaagttgagc    4560
   ctttgtggtg ggacttttta aaaaaatgcc tttttaaagc attaatggct aattgaagta    4620
   ttttatgact cctcattcct ggcccagagg gttgtctttg aaaccctgtt tctaaccctt    4680
   gtgttgtgtg tttctgtctg aggacagtgg gtgtgtactg gcctcccggg agccactgtg    4740
   accaggcctt tgagctcttg tcatctgtgg agagaatcat gcaaatttta aaagttcttc    4800
   caagagactt ccatgtcctg gttattaac                                        4829
<210> 72
   <211> 1111
   <212> PRT
   <213> Homo sapiens
<400> 72
<210> 73
   <211> 3869
   <212> DNA
   <213> Homo sapiens
<400> 73
<210> 74
   <211> 660
   <212> PRT
   <213> Homo sapiens
<400> 74
   Met Phe Ser Leu Ser Ser Thr Val Gln Pro Gln Val Thr Val Pro Leu
   1               5                   10                  15
   Ser His Leu Ile Asn Ala Phe His Thr Pro Lys Asn Thr Ser Val Ser
               20                  25                  30
   Leu Ser Gly Val Ser Val Ser Gln Asn Gln His Arg Asp Val Val Pro
           35                  40                  45
   Glu His Glu Ala Pro Ser Ser Glu Pro Ser Leu Asn Leu Arg Asp Leu
       50                  55                  60
   Gly Leu Ser Glu Leu Lys Ile Gly Gln Ile Asp Gln Leu Val Glu Asn
   65                  70                  75                  80
   Leu Leu Pro Gly Phe Cys Lys Gly Lys Asn Ile Ser Ser His Trp His
                   85                  90                  95
   Thr Ser His Val Ser Ala Gln Ser Phe Phe Glu Asn Lys Tyr Gly Asn
               100                 105                 110
   Leu Asp Ile Phe Ser Thr Leu Arg Ser Ser Cys Leu Tyr Arg His His
           115                 120                 125
   Ser Arg Ala Leu Gln Ser Ile Cys Ser Asp Leu Gln Tyr Trp Pro Val
       130                 135                 140
   Phe Ile Gln Ser Arg Gly Phe Lys Thr Leu Lys Ser Arg Thr Arg Arg
   145                 150                 155                 160
   Leu Gln Ser Thr Ser Glu Arg Leu Ala Glu Thr Gln Asn Ile Ala Pro
                   165                 170                 175
   Ser Phe Val Lys Gly Phe Leu Leu Arg Asp Arg Gly Ser Asp Val Glu
   180 185 190
   Ser Leu Asp Lys Leu Met Lys Thr Lys Asn Ile Pro Glu Ala His Gln
   195 200 205
   Asp Ala Phe Lys Thr Gly Phe Ala Glu Gly Phe Leu Lys Ala Gln Ala
   210 215 220
   Leu Thr Gln Lys Thr Asn Asp Ser Leu Arg Arg Thr Arg Leu Ile Leu
   225 230 235 240
   Phe Val Leu Leu Leu Phe Gly Ile Tyr Gly Leu Leu Lys Asn Pro Phe
   245 250 255
   Leu Ser Val Arg Phe Arg Thr Thr Thr Gly Leu Asp Ser Ala Val Asp
   260 265 270
   Pro Val Gln Met Lys Asn Val Thr Phe Glu His Val Lys Gly Val Glu
   275 280 285
   Glu Ala Lys Gln Glu Leu Gln Glu Val Val Glu Phe Leu Lys Asn Pro
   290 295 300
   Gln Lys Phe Thr Ile Leu Gly Gly Lys Leu Pro Lys Gly Ile Leu Leu
   305 310 315 320
   Val Gly Pro Pro Gly Thr Gly Lys Thr Leu Leu Ala Arg Ala Val Ala
   325 330 335
   Gly Glu Ala Asp Val Pro Phe Tyr Tyr Ala Ser Gly Ser Glu Phe Asp
   340 345 350
   Glu Met Phe Val Gly Val Gly Ala Ser Arg Ile Arg Asn Leu Phe Arg
   355 360 365
   Glu Ala Lys Ala Asn Ala Pro Cys Val Ile Phe Ile Asp Glu Leu Asp
   370 375 380
   Ser Val Gly Gly Lys Arg Ile Glu Ser Pro Met His Pro Tyr Ser Arg
   385 390 395 400
   Gln Thr Ile Asn Gln Leu Leu Ala Glu Met Asp Gly Phe Lys Pro Asn
   405 410 415
   Glu Gly Val Ile Ile Ile Gly Ala Thr Asn Phe Pro Glu Ala Leu Asp
   420 425 430
   Asn Ala Leu Ile Arg Pro Gly Arg Phe Asp Met Gln Val Thr Val Pro
           435                 440                 445
   Arg Pro Asp Val Lys Gly Arg Thr Glu Ile Leu Lys Trp Tyr Leu Asn
       450                 455                 460
   Lys Ile Lys Phe Asp Gln Ser Val Asp Pro Glu Ile Ile Ala Arg Gly
   465                 470                 475                 480
   Thr Val Gly Phe Ser Gly Ala Glu Leu Glu Asn Leu Val Asn Gln Ala
                   485                 490                 495
   Ala Leu Lys Ala Ala Val Asp Gly Lys Glu Met Val Thr Met Lys Glu
               500                 505                 510
   Leu Glu Phe Ser Lys Asp Lys Ile Leu Met Gly Pro Glu Arg Arg Ser
           515                 520                 525
   Val Glu Ile Asp Asn Lys Asn Lys Thr Ile Thr Ala Tyr His Glu Ser
       530                 535                 540
   Gly His Ala Ile Ile Ala Tyr Tyr Thr Lys Asp Ala Met Pro Ile Asn
   545                 550                 555                 560
   Lys Ala Thr Ile Met Pro Arg Gly Pro Thr Leu Gly His Val Ser Leu
                   565                 570                 575
   Leu Pro Glu Asn Asp Arg Trp Asn Glu Thr Arg Ala Gln Leu Leu Ala
               580                 585                 590
   Gln Met Asp Val Ser Met Gly Gly Arg Val Ala Glu Glu Leu Ile Phe
           595                 600                 605
   Gly Thr Asp His Ile Thr Thr Gly Ala Ser Ser Asp Phe Asp Asn Ala
       610                 615                 620
   Thr Lys Ile Ala Lys Arg Met Val Thr Lys Phe Gly Met Ser Glu Lys
   625                 630                 635                 640
   Leu Gly Val Met Thr Tyr Ser Asp Thr Gly Lys Leu Ser Pro Glu Thr
                   645                 650                 655
   Gln Ser Ala Ile
               660
<210> 75
   <211> 1922
   <212> DNA
   <213> Homo sapiens
<400> 75
   cgacgtctcc agtctacctc cgagagatta gctgaaacac agaatatagc gccatcattc      60
   gtgaagggtt tcttttgcgg gacagaggat cagatgttga gagtttggac aaactcatga     120
   aaaccaaaaa tatacctgaa gctcaccaag atgcatttaa aactggtttt gcggaaggtt     180
   ttctgaaagc tcaagcactc acacaaaaaa ccaatgattc cctaaggcga acccgtctga     240
   ttctcttcgt tctgctgcta ttcggcattt atggacttct aaaaaaccca tttttatctg     300
   tccgcttccg gacaacaaca gggcttgatt ctgcagtaga tcctgtccag atgaaaaatg     360
   tcacctttga acatgttaaa ggggtggagg aagctaaaca agaattacag gaagttgttg     420
   aattcttgaa aaatccacaa aaatttacta ttcttggagg taaacttcca aaaggaattc     480
   ttttagttgg acccccaggg actggaaaga cacttcttgc ccgagctgtg gcgggagaag     540
   ctgatgttcc tttttattat gcttctggat ccgaatttga tgagatgttt gtgggtgtgg     600
   gagccagccg tatcagaaat ctttttaggg aagcaaaggc gaatgctcct tgtgttatat     660
   ttattgatga attagattct gttggtggga agagaattga atctccaatg catccatatt     720
   caaggcagac cataaatcaa cttcttgctg aaatggatgg ttttaaaccc aatgaaggag     780
   ttatcataat aggagccaca aacttcccag aggcattaga taatgcctta atacgtcctg     840
   gtcgttttga catgcaagtt acagttccaa ggccagatgt aaaaggtcga acagaaattt     900
   tgaaatggta tctcaataaa ataaagtttg atcaatccgt tgatccagaa attatagctc     960
   gaggtactgt tggcttttcc ggagcagagt tggagaatct tgtgaaccag gctgcattaa    1020
   aagcagctgt tgatggaaaa gaaatggtta ccatgaagga gctggagttt tccaaagaca    1080
   aaattctaat ggggcctgaa agaagaagtg tggaaattga taacaaaaac aaaaccatca    1140
   cagcatatca tgaatctggt catgccatta ttgcatatta cacaaaagat gcaatgccta    1200
   tcaacaaagc tacaatcatg ccacgggggc caacacttgg acatgtgtcc ctgttacctg    1260
   agaatgacag atggaatgaa actagagccc agctgcttgc acaaatggat gttagtatgg    1320
   gaggaagagt ggcagaggag cttatatttg gaaccgacca tattacaaca ggtgcttcca    1380
   gtgattttga taatgccact aaaatagcaa agcggatggt taccaaattt ggaatgagtg    1440
   aaaagcttgg agttatgacc tacagtgata cagggaaact aagtccagaa acccaatctg    1500
   ccatcgaaca agaaataaga atccttctaa gggactcata tgaacgagca aaacatatct    1560
   tgaaaactca tgcaaaggag cataagaatc tcgcagaagc tttattgacc tatgagactt    1620
   tgatgccaaa gagattcaaa ttgttcttga ggggaaaaag ttggaagtga gatgataact    1680
   ctcttgatat ggatgcttgc tggttttatt gcaagaatat aagtagcatt gcagtagtct    1740
   acttttacaa cgctttcccc tcattcttga tgtggtgtaa ttgaagggtg tgaaatgctt   1800
   tgtcaatcat ttgtcacatt tatccagttt gggttattct cattatgaca cctattgcaa   1860
   attagcatcc catggcaaat atattttgaa aaaataaaga actatcagga ttgaaaaaaa   1920
   aa                                                                  1922
<210> 76
   <211> 517
   <212> PRT
   <213> Homo sapiens
<400> 76
   Met Lys Thr Lys Asn Ile Pro Glu Ala His Gln Asp Ala Phe Lys Thr
   1               5                   10                  15
   Gly Phe Ala Glu Gly Phe Leu Lys Ala Gln Ala Leu Thr Gln Lys Thr
               20                  25                  30
   Asn Asp Ser Leu Arg Arg Thr Arg Leu Ile Leu Phe Val Leu Leu Leu
           35                  40                  45
   Phe Gly Ile Tyr Gly Leu Leu Lys Asn Pro Phe Leu Ser Val Arg Phe
       50                  55                  60
   Arg Thr Thr Thr Gly Leu Asp Ser Ala Val Asp Pro Val Gln Met Lys
   65                  70                  75                  80
   Asn Val Thr Phe Glu His Val Lys Gly Val Glu Glu Ala Lys Gln Glu
                   85                  90                  95
   Leu Gln Glu Val Val Glu Phe Leu Lys Asn Pro Gln Lys Phe Thr Ile
               100                 105                 110
   Leu Gly Gly Lys Leu Pro Lys Gly Ile Leu Leu Val Gly Pro Pro Gly
           115                 120                 125
   Thr Gly Lys Thr Leu Leu Ala Arg Ala Val Ala Gly Glu Ala Asp Val
       130                 135                 140
   Pro Phe Tyr Tyr Ala Ser Gly Ser Glu Phe Asp Glu Met Phe Val Gly
   145                 150                 155                 160
   Val Gly Ala Ser Arg Ile Arg Asn Leu Phe Arg Glu Ala Lys Ala Asn
                   165                 170                 175
   Ala Pro Cys Val Ile Phe Ile Asp Glu Leu Asp Ser Val Gly Gly Lys
   180 185 190
   Arg Ile Glu Ser Pro Met His Pro Tyr Ser Arg Gln Thr Ile Asn Gln
   195 200 205
   Leu Leu Ala Glu Met Asp Gly Phe Lys Pro Asn Glu Gly Val Ile Ile
   210 215 220
   Ile Gly Ala Thr Asn Phe Pro Glu Ala Leu Asp Asn Ala Leu Ile Arg
   225 230 235 240
   Pro Gly Arg Phe Asp Met Gln Val Thr Val Pro Arg Pro Asp Val Lys
   245 250 255
   Gly Arg Thr Glu Ile Leu Lys Trp Tyr Leu Asn Lys Ile Lys Phe Asp
   260 265 270
   Gln Ser Val Asp Pro Glu Ile Ile Ala Arg Gly Thr Val Gly Phe Ser
   275 280 285
   Gly Ala Glu Leu Glu Asn Leu Val Asn Gln Ala Ala Leu Lys Ala Ala
   290 295 300
   Val Asp Gly Lys Glu Met Val Thr Met Lys Glu Leu Glu Phe Ser Lys
   305 310 315 320
   Asp Lys Ile Leu Met Gly Pro Glu Arg Arg Ser Val Glu Ile Asp Asn
   325 330 335
   Lys Asn Lys Thr Ile Thr Ala Tyr His Glu Ser Gly His Ala Ile Ile
   340 345 350
   Ala Tyr Tyr Thr Lys Asp Ala Met Pro Ile Asn Lys Ala Thr Ile Met
   355 360 365
   Pro Arg Gly Pro Thr Leu Gly His Val Ser Leu Leu Pro Glu Asn Asp
   370 375 380
   Arg Trp Asn Glu Thr Arg Ala Gln Leu Leu Ala Gln Met Asp Val Ser
   385 390 395 400
   Met Gly Gly Arg Val Ala Glu Glu Leu Ile Phe Gly Thr Asp His Ile
   405 410 415
   Thr Thr Gly Ala Ser Ser Asp Phe Asp Asn Ala Thr Lys Ile Ala Lys
   420 425 430
   Arg Met Val Thr Lys Phe Gly Met Ser Glu Lys Leu Gly Val Met Thr
           435                 440                 445
   Tyr Ser Asp Thr Gly Lys Leu Ser Pro Glu Thr Gln Ser Ala Ile Glu
       450                 455                 460
   Gln Glu Ile Arg Ile Leu Leu Arg Asp Ser Tyr Glu Arg Ala Lys His
   465                 470                 475                 480
   Ile Leu Lys Thr His Ala Lys Glu His Lys Asn Leu Ala Glu Ala Leu
                   485                 490                 495
   Leu Thr Tyr Glu Thr Leu Met Pro Lys Arg Phe Lys Leu Phe Leu Arg
               500                 505                 510
   Gly Lys Ser Trp Lys
           515

## Description

This application claims priority to U.S. Provisional Application Nos.: 60/261,335, 60/261,532, 60/261,590, 60/261,361, 60/261,531, 60/261,457, 60/261,694, 60/261,226, 60/261,304, 60/261,459, 60/261,456, 601261,589, 60/261,461, 60/261,697, 60/261,458, 60/261,695, 60/261,336, 60/261,518, and 60/261,303, filed on January 12, 2001.

### BACKGROUND OF THE INVENTION

Insulin is the central hormone governing metabolism in vertebrates (reviewed in Steiner et al., 1989, In Endocrinology, DeGrroot, eds. Philadelphia, Saunders: 1263-1289). In humans, insulin is secreted by the beta cells of the pancreas in response to elevated blood glucose levels, which normally occur following a meal. The immediate effect of insulin secretion is to induce the uptake of glucose by muscle, adipose tissue, and the liver. A longer-term effect of insulin is to increase the activity of enzymes that synthesize glycogen in the liver and triglycerides in adipose tissue. Insulin can exert other actions beyond these "classic" metabolic activities, including increasing potassium transport in muscle, promoting cellular differentiation of adipocytes, increasing renal retention of sodium, and promoting production of androgens by the ovary. Defects in the secretion and/or response to insulin are responsible for the disease diabetes mellitus, which is of enormous economic significance. Within the United States, diabetes mellitus is the fourth most common reason for physician visits by patients; it is the leading cause of end-stage renal disease, non-traumatic limb amputations, and blindness in individuals of working age (Warram et al., 1995, In Joslin's Diabetes Mellitus, Kahn and Weir, eds., Philadelphia, Lea & Febiger, pp. 201-215; Kahn et al., 1996, Annu. Rev. Med. 47:509-531; Kahn, 1998, Cell 92:593-596). Beyond its role in diabetes mellitus, the phenomenon of insulin resistance has been linked to other pathogenic disorders including obesity, ovarian hyperandrogenism, and hypertension.

Within the pharmaceutical industry, there is interest in understanding the molecular mechanisms that connect lipid defects and insulin resistance. Hyperlipidemia and elevation of free fatty acid levels correlate with "Metabolic Syndrome," defined as the linkage between several diseases, including obesity and insulin resistance, which often occur in the same patients and which are major risk factors for development of Type 2 diabetes and cardiovascular disease. Current research suggests that the control of lipid levels, in addition to glucose levels, may be required to treat Type 2 Diabetes, heart disease, and other manifestations of Metabolic Syndrome (Santomauro AT et al., Diabetes (1999) 48:1836-1841).

The ability to manipulate and screen the genomes of model organisms such as *Drosophila* and *C. elegans* provides a powerful means to analyze biochemical processes that, due to significant evolutionary conservation of genes, pathways, and cellular processes, have direct relevance to more complex vertebrate organisms. Identification of novel functions of genes involved in particular pathways in such model organisms can directly contribute to the understanding of the correlative pathways in mammals and of methods of modulating them (see *e.g.*, Miklos GL and Rubin GM, Cell 1996, 86:521-529). While *Drosophila* and *C. elegans* are not susceptible to human pathologies, various experimental models can mimic the pathological states. A correlation between the pathology model and the modified expression of a *Drosophila* or *C. elegans* gene can identify the association of the human ortholog with the human disease.

In one example, a genetic screen is performed in an invertebrate model organism displaying a mutant (generally visible or selectable) phenotype due to mis-expression - generally reduced, enhanced or ectopic expression - of a known gene (the "genetic entry point"). Additional genes are mutated in a random or targeted manner. When an additional gene mutation changes the original mutant phenotype, this gene is identified as a "modifier" that directly or indirectly interacts with the genetic entry point and its associated pathway. If the genetic entry point is an ortholog of a human gene associated with a human pathology, such as lipid metabolic disorders, the screen can identify modifier genes that are candidate targets for novel therapeutics.

Genetic screens may utilize RNA interference (RNAi) techniques, whereby introduction of exogenous double stranded (ds) RNA disrupts the activity of genes containing homologous sequences and induce specific loss-of-function phenotypes (Fire et al., 1998, Nature391:806-811). Suitable methods for introduction of dsRNA include injection, feeding, and bathing (Tabara et al, 1998, Science 282:430-431).

Genetic screens may also utilize mis-expression techniques to identify genes that, when over- or misexpressed in a pattern of interest, give a specific phenotype or modulate an existing mutant phenotype. An exemplary mis-expression screen is the *Drosophila* "EP" screen, which uses *Drosophila* lines carrying essentially random insertion of a P-element containing gene regulatory sequences oriented to act on flanking genomic sequences (Rorth P, 1996, Proc Natl Acad Sci U S A 93:12418-22; Rorth P et al., Development (1998) 125:1049-1057; WO0015843).

The insulin receptor (INR) signaling pathway has been extensively studied in *C. elegans* and *Drosophila.* For instance, it has been found that signaling through *daf-2,* the *C. elegans* INR ortholog, mediates various events, including reproductive growth and normal adult life span (see, e.g., US PAT NO 6,225,120; Tissenbaum HA and Ruvkun G, 1998, Genetics 148:703-17; Ogg S and Ruvkun G, 1998, Mol Cell 2:887-93; Lin K et al, 2001, Nat Genet 28:139-45).

Modifiers of INR signaling were identified in genetic screens using *Drosophila* or *C. elegans.*

US 5,726,027 describes a screening method for identifying compositions which affect the binding of protein tyrosine phosphatase 1B to phosphorylated insulin receptor. Shisheva et al, Mol. Cell. Biol. 19, 1999, p. 623-634 describes the cloning and characterization of p235.

### SUMMARY OF THE INVENTION

The invention provides a method of identifying candidate INR signaling enhancing agents using a non-human assay system comprising an ISM polypeptide or nucleic acid; wherein said ISM polypeptide is a full-length ISM protein consisting of amino acid sequence SEQ ID NO: 22, or a fragment thereof that has kinase activity and comprises at least 50 contiguous amino acids of the ISM protein, wherein said ISM nucleic acid is a DNA or RNA molecule that encodes said ISM polypeptide; contacting the assay system with a test agent under conditions whereby, but for the presence of the test agent, the system provides a reference activity; and detecting reduced activity or expression of the ISM polypeptide or nucleic acid, wherein the reduced activity or expression of the ISM polypeptide or nucleic acid between the presence or absence of the test agent identifies the test agent as a candidate INR signaling enhancing agent. Candidate test agents include small molecule modulators, antibodies, and nucleic acid modulators such as antisense oligomers and PMOs, among others.

In one embodiment of the invention, the assay system comprises cultured cells expressing ISM, and the assay system detects an agent-biased change in INR signaling.

In certain embodiments, candidate INR signaling modulating agents are identified in cell-free or cell-based assays, and a second assay system that detects an agent-biased change in an activity associated with INR signaling is used to confirm the INR signaling modulating activity of the candidate agent. In a preferred embodiment, the second assay detects an agent-biased change in an activity associated with INR signaling. Preferred second assay systems are carried out in cultured cells.

The invention further provides *an in vitro* method of enhancing INR signaling in a mammalian cell as defined in claim 17. Preferred agents include small molecule modulators, nucleic acid modulators, or antibodies.

### DETAILED DESCRIPTION OF THE INVENTION

We identified novel invertebrate modifiers of insulin receptor (INR) signaling and their human orthologs. The human orthologs of these genes (nucleic acids and polypeptides) are collectively referred to as ISMs (Insulin receptor Signaling Modifiers).

A reverse genetics screen was performed using a *C. elegans* model for defective INR function, with the goal of identifying genes that when inactivated result in restoration of INR signaling. The *C. elegans* INR signaling pathway negatively regulates entry into a dauer state, an alternate developmental fate that normally occurs only in adverse conditions (Riddle DL and PS Albert. 1997. In C. elegans II, DL Riddle, T. Blumenthal, BJ Meyer, and JR Priess, eds., p. 739-768). When *daf-2,* the C. *elegans* insulin receptor, is inactivated by mutation, animals enter the dauer state regardless of the environmental conditions (Kimura KD, et al,. 1997, Science 277:942). We used an RNAi-based screen to identify modifiers (suppressors) of the dauer-formation phenotype of *daf*-2 loss-of-function mutations. The screen used two worm strains, each containing a missense mutation in the ligand-binding domain of *daf-*2, and involved RNAi treatment of these strains with dsRNA derived from cDNA or exon-rich genomic fragments of worm genes in order to cause reduction-of-function of these genes. Potential suppressors were identified as those genes that, when knocked down by RNAi treatment, allowed growth of the insulin-receptor mutant strains rather than larval arrest. Standard sequence analysis was used to determine the human (or mouse) orthologs of these genes, which are candidate targets for therapeutics designed to treat pathologies related to insulin signaling. Since many of the symptoms of diabetes are a result of decreased insulin signaling, candidate targets for antagonist therapies are those genes that, when mutated, restore INR signaling.

The *C. elegans* screen identified the genes whose amino acid sequences are provided in SEQ ID NOs: 2, 4, 6, 8, 10, 12, 14, 16, 18, 20 and 22.

An overexpression screen was performed in *Drosophila.* Signaling through the *Drosophila* insulin receptor, designated Dinr (GI 1362614), has been implicated in controlling cell size and cell number (Fernandez R, et al, 1995, EMBO J 14:3373-3384; Brogiolo W et al., Stocker H and Hafen E, 2000, Curr Opin Genet Dev 10:529-35; Chen C et al, 1996, Endocrinology 137:846-56). Mis-expression of a dominant negative form of Dinr in the *Drosophila* eye results in small eye phenotype. The overexpression screen used a collection of *Drosophila* lines carrying insertions of a P-element vector ("EP element") comprising a GAL4-regulated promoter oriented to transcribe flanking genomic sequences (Rorth P, 1996, Proc Natl Acad Sci U S A 93:12418-22; Rorth P et al., 1998, Development 125:1049-57). Each EP line was crossed to lines expressing the dominant negative Dinr, as well as GAL4, in the eye. Resulting progeny were examined for a change in the small eye phenotype. Sequence information surrounding the P insertion site was used to identify the overexpressed genes.

The *Drosophila* screen identified the genes whose amino acid sequences are provided in SEQ ID NOS: 24, 26, 28, 30, 32, 34, 36, 38, 40, 42, 44, 46, 48, 50, 52, 54, 56, 58, 60, 62, 64, 66, 68, 70, 72, 74 and 76

The invertebrate screens identified novel associations of ISM proteins and INR signaling. To applicants' knowledge, associations between diabetes (i.e., type II diabetes), glucose metabolism and/or INR signaling and the ISM proteins presented in SEQ ID NOs: 18, 30, 32, 34, 36, 38, 40, 50, 52, 54, 56, 58, 64, 66, 68, 70, 72, 74 and 76 have not previously been disclosed. For other ISM proteins, including those presented in SEQ ID NOs: 2, 4, 6, 8, 10, 12, 14, 16, 20, 22, 24, 26, 28, 42, 44, 46, 48, 60, and 62, there have been previous reports correlating activity of these proteins to diabetes, glucose metabolism, or another output of INR signaling. However, to applicants' knowledge, the methods described herein present the first evidence that these proteins may directly influence and/or interact with INR signaling to regulate the metabolic effects of insulin..

ISM genes (i.e., nucleic acids and polypeptides) are attractive drug targets for the treatment of disorders related to INR signaling. In a preferred example, treatment involves increasing signaling through INR in order to treat pathologies related to diabetes and/or metabolic syndrome.

Described herein are *in vitro* and *in vivo* methods of assessing ISM function, and methods of modulating (generally inhibiting or agonizing) ISM activity, which are useful for further elucidating INR signaling and for developing diagnostic and therapeutic modalities for pathologies associated with INR signaling. As used herein, pathologies associated with INR signaling encompass pathologies where INR signaling contributes to maintaining the healthy state, as well as pathologies whose course may be altered by modulation of the INR signaling.

### ISM nucleic acids and polypeptides

Human ISM nucleic acid (cDNA) sequences are provided in SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63,65, 67, 69, 71,73, and 75. Corresponding protein sequences are provided in SEQ ID NOs:. 2,4,6, 8,10,12,14,16,18,20,22,24,26,28,30, 32, 34,36,38,40,42, 44, 46, 48, 50, 52, 54, 56, 58, 60, 62, 64, 66, 68, 70, 72, 74 and 76. ISM proteins of this invention include several categories of proteins with particular function domains. ISMs are identified as enzymes belonging to kinase (SEQ ID NOs: 2, 4, 6, 8, 10, 12, 14, 16, 18, 20, 22, 24, 26,28,34, 36, 38, 40, 44, 46, 50, 60, and 62), protease (SEQ ID NOs: 30, 32, 74, and 76), desaturase (SEQ ID NO:48), and biosynthetic enzyme (SEQ ID NOs:42 and 52) classes. Other classes include mitochondrial transport proteins (SEQ ID NOs:54, 56 and 58), specific binding proteins (SEQ ID NOs:64 and 66), and transcriptional proteins (SEQ ID NOs: 68, 70, 72).

The term "ISM polypeptide" refers to a full-length ISM protein or a fragment or derivative thereof that is "functionally active," meaning that the ISM protein derivative or fragment exhibits one or more functional activities associated with a full-length, wild-type ISM protein. As one example, a fragment or derivative may have antigenicity such that it can be used in immunoassays, for immunization, for generation of inhibitory antibodies, *etc,* as discussed further below. Preferably, a functionally active ISM fragment or derivative displays one or more biological activities associated with ISM proteins such as enzymatic activity, signaling activity, ability to bind natural cellular substrates, etc. For those ISM proteins that are identified as enzymes, preferred ISM polypeptides display enzymatic (kinase, protease, desaturase, biosynthetic, etc.) activity. Other preferred ISM polypeptides display transport, binding, or transcriptional activity, according to the particular protein class. In one embodiment, a functionally active ISM polypeptide is an ISM derivative capable of rescuing defective endogenous ISM activity, such as in cell based or animal assays; the rescuing derivative may be from the same or a different species. If ISM fragments are used in assays to identify modulating agents, the fragments preferably comprise a ISM domain, such as a C- or N-terminal or catalytic domain, among others, and comprise at least 50 contiguous amino acids of a ISM protein. A preferred ISM fragment comprises a catalytic domain, such as a kinase domain, for those ISMs identified as kinases. Other preferred ISM fragments comprise specific binding domains. Functional domains can be identified using the PFAM program (Bateman A et al., 1999 Nucleic Acids Res 27:260-262; website at pfam.wustl.edu).

The term "ISM nucleic acid" refers to a DNA or RNA molecule that encodes a ISM polypeptide. Preferably, the ISM polypeptide or nucleic acid or fragment thereof is from a human, but it can be an ortholog or derivative thereof with at least 70%, preferably with at least 80%, preferably 85%, still more preferably 90%, and most preferably at least 95% sequence identity with a human ISM. As used herein, "percent (%) sequence identity" with respect to a specified subject sequence, or a specified portion thereof, is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program WU-BLAST-2.0a19 (Altschul et al., J. Mol. Biol. (1997) 215:403-410; http://blast.wustl.edu/blast/README.html) with search parameters set to default values. The HSP S and HSP S2 parameters are dynamic values and are established by the program itself depending upon the composition of the particular sequence and composition of the particular database against which the sequence of interest is being searched. A "% identity value" is determined by the number of matching identical nucleotides or amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation. A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine and glycine.

Alternatively, an alignment for nucleic acid sequences is provided by the local homology algorithm of Smith and Waterman (Smith and Waterman, 1981, Advances in Applied Mathematics 2:482-489; database: European Bioinformatics Institute wwwz.ebi.ac.uk/bic.sub.-- sw/; Smith and Waterman, 1981, J. of Molec.Biol., 147:195-197; Nicholas et al., 1998, "A Tutorial on Searching Sequence Databases and Sequence Scoring Methods" (www.psc.edu) and references cited therein.; W.R. Pearson, 1991, Genomics 11:635-650). This algorithm can be applied to amino acid sequences by using the scoring matrix developed by Dayhoff (Dayhoff: Atlas of Protein Sequences and Structure, M. O. Dayhoff ed., 5 suppl. 3:353-358, National Biomedical Research Foundation, Washington, D.C., USA), and normalized by Gribskov (Gribskov 1986 Nucl. Acids Res. 14(6):6745-6763). The Smith-Waterman algorithm is used to search databases for sequences similar to a query sequence. Smith-Waterman uses dynamic programming to determine how an optimal alignment between the query sequence and a database sequence can be produced. This alignment is obtained by determining what transformations the query sequence would need to undergo to match the database sequence. Transformations include substituting one character for another and inserting or deleting a string of characters. A score is assigned for each character-to-character comparison--positive scores for exact matches and some substitutions, negative scores for other substitutions and insertions/deletions. The first character in an insertion or deletion gap is scored with a gap open penalty and subsequent characters are scored with a gap extension penalty. Scores are obtained from statistically-derived scoring matrices. The combination of transformations that results in the highest score is used to generate an alignment between the query sequence and database sequence. Smith-Waterman algorithm may be employed where default parameters are used for scoring (for example, gap open penalty of 12, gap extension penalty of two). From the data generated the "Match" value reflects "sequence identity."

Derivative nucleic acid molecules of the subject nucleic acid molecules include sequences that hybridize to an ISM nucleic acid sequence. The stringency of hybridization can be controlled by temperature, ionic strength, pH, and the presence of denaturing agents such as formamide during hybridization and washing. Conditions routinely used are set out in readily available procedure texts (*e.g.*, Current Protocol in Molecular Biology, Vol. 1, Chap. 2.10, John Wiley & Sons, Publishers (1994); Sambrook et al., Molecular Cloning, Cold Spring Harbor (1989)). In some embodiments, a nucleic acid molecule of the invention is capable of hybridizing to a nucleic acid molecule containing the an ISM nucleotide under stringent hybridization conditions that comprise: prehybridization of filters containing nucleic acid for 8 hours to overnight at 65° C in a solution comprising 6X single strength citrate (SSC) (1X SSC is 0.15 MNaCl, 0.015 M Na citrate; pH 7.0), 5X Denhardt's solution, 0.05% sodium pyrophosphate and 100 µg/ml hening sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1 h in a solution containing 0.2X SSC and 0.1% SDS (sodium dodecyl sulfate). In other embodiments, moderately stringent hybridization conditions are used that comprise: pretreatment of filters containing nucleic acid for 6 h at 40° C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA; hybridization for 18-20 h at 40° C in a solution containing 35% formamide, 5X SSC, 50 mM Tris-HCl (pH 7.5), 5 mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1 % SDS. Alternatively, low stringency conditions can be used that comprise: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

In some embodiments, the ISM is an ortholog of human ISM. Methods of identifying the human orthologs of these genes are known in the art. Normally, orthologs in different species retain the same function, due to presence of one or more protein motifs and/or 3-dimensional structures. Orthologs are generally identified by sequence homology analysis, such as BLAST analysis, usually using protein bait sequences. Sequences are assigned as a potential ortholog if the best hit sequence from the forward BLAST result retrieves the original query sequence in the reverse BLAST (Huynen MA and Bork P, Proc Natl Acad Sci (1998) 95:5849-5856; Huynen MA et al., Genome Research (2000) 10:1204-1210). Programs for multiple sequence alignment, such as CLUSTAL (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species. Structural threading or other analysis of protein folding (e.g., using software by ProCeryon, Biosciences, Salzburg, Austria) may also identify potential orthologs. In evolution, when a gene duplication event follows speciation, a single gene in one species, such as *Drosophila*, may correspond to multiple genes (paralogs) in another, such as human. As used herein, the term "orthologs" encompasses paralogs.

### Isolation, Production, Expression, and Mis-expression of ISM Nucleic Acids and Polypeptides

ISM nucleic acids and polypeptides are useful for identifying and testing agents that modulate ISM function and for other applications related to the involvement of ISM in INR signaling. ISM nucleic acids may be obtained using any available method. For instance, techniques for isolating cDNA or genomic DNA sequences of interest by screening DNA libraries or by using polymerase chain reaction (PCR) are well known in the art.

A wide variety of methods are available for obtaining ISM polypeptides. In general, the intended use for the polypeptide will dictate the particulars of expression, production, and purification methods. For instance, production of polypeptides for use in screening for modulating agents may require methods that preserve specific biological activities of these proteins, whereas production of polypeptides for antibody generation may require structural integrity of particular epitopes. Expression of polypeptides to be purified for screening or antibody production may require the addition of specific tags (*i.e.*, generation of fusion proteins). Overexpression of a ISM polypeptide for cell-based assays used to assess ISM function, such as involvement in tubulogenesis, may require expression in eukaryotic cell lines capable of these cellular activities. Techniques for the expression, production, and purification of proteins are well known in the art; any suitable means therefor may be used (e.g., Higgins SJ and Hames BD (eds.) Protein Expression: A Practical Approach, Oxford University Press Inc., New York 1999; Stanbury PF et al., Principles of Fermentation Technology, 2nd edition, Elsevier Science, New York, 1995; Doonan S (ed.) Protein Purification Protocols, Humana Press, New Jersey, 1996; Coligan JE et al, Current Protocols in Protein Science (eds.), 1999, John Wiley & Sons, New York; U.S. Pat. No. 6,165,992).

The nucleotide sequence encoding an ISM polypeptide can be inserted into any appropriate vector for expression of the inserted protein-coding sequence. The necessary transcriptional and translational signals, including promoter/enhancer element, can derive from the native ISM gene and/or its flanking regions or can be heterologous. A variety of host-vector expression systems may be utilized, such as mammalian cell systems infected with virus (*e.g.* vaccinia virus, adenovirus, *etc.*); insect cell systems infected with virus (*e.g.* baculovirus); microorganisms such as yeast containing yeast vectors, or bacteria transformed with bacteriophage, plasmid, or cosmid DNA. A host cell strain that modulates the expression of, modifies, and/or specifically processes the gene product may be used.

The ISM polypeptide may be optionally expressed as a fusion or chimeric product, joined via a peptide bond to a heterologous protein sequence. In one application the heterologous sequence encodes a transcriptional reporter gene (e.g., GFP or other fluorescent proteins, luciferase, beta-galactosidase, etc.). A chimeric product can be made by ligating the appropriate nucleic acid sequences encoding the desired amino acid sequences to each other in the proper coding frame using standard methods and expressing the chimeric product. A chimeric product may also be made by protein synthetic techniques, *e.g.* by use of a peptide synthesizer (Hunkapiller et al., Nature (1984) 310:105-111).

An ISM polypeptide can be isolated and purified using standard methods (*e.g.* ion exchange, affinity, and gel exclusion chromatography; centrifugation; differential solubility; electrophoresis). Alternatively, native ISM proteins can be purified from natural sources, by standard methods (*e.g.* immunoaffinity purification). Once a protein is obtained, it may be quantified and its activity measured by appropriate methods, such as immunoassay, bioassay, or other measurements of physical properties, such as crystallography.

The methods of this invention may also use cells that have been engineered for altered expression (mis-expression) of ISM or other genes associated with INR signaling. As used herein, mis-expression encompasses ectopic expression, over-expression, under-expression, and non-expression (*e.g.* by gene knock-out or blocking expression that would otherwise normally occur).

### Genetically modified animals

The methods of this invention may use non-human animals that have been genetically modified to alter expression of ISM and/or other genes known to be involved in INR signaling. Preferred genetically modified animals are mammals, particularly mice or rats. Preferred non-mammalian species include Zebrafish, *C. elegans*, and *Drosophila.* Preferably, the altered ISM or other gene expression results in a detectable phenotype, such as modified levels of INR signaling, modified levels of plasma glucose or insulin, or modified lipid profile as compared to control animals having normal expression of the altered gene. The genetically modified animals can be used to further elucidate INR signaling, in animal models of pathologies associated with INR signaling, and for *in vivo* testing of candidate therapeutic agents, as described below.

Preferred genetically modified animals are transgenic, at least a portion of their cells harboring non-native nucleic acid that is present either as a stable genomic insertion or as an extra-chromosomal element, which is typically mosaic. Preferred transgenic animals have germ-line insertions that are stably transmitted to all cells of progeny animals.

Non-native nucleic acid is introduced into host animals by any expedient method. Methods of making transgenic non-human animals are well-known in the art (for mice see Brinster et al., Proc. Nat. Acad. Sci. USA 1985, 82:4438-42; U.S. Pat. Nos. 4,736,866, 4,870,009, 4,873,191, 6,127,598; Hogan, B., Manipulating the Mouse Embryo, Cold Spring Harbor Laboratory Press, Cold Spring Harbor, N.Y., (1986); for homologous recombination see Capecchi, Science1989, 244:1288-1292; Joyner et al., Nature 1989, 338:153-156; for particle bombardment see U.S. Pat. No., 4,945,050; for *Drosophila* see Rubin and Spradling, Science (1982) 218:348-53, U.S. Pat. No. 4,670,388; for transgenic insects see Berghammer A.J. et al., Nature 1999, 402:370-371; for Zebrafish see Lin S. Methods Mol Biol. (2000);136:375-3830; for fish, amphibians and birds see Houdebine and Chourrout, Experientia (1991) 47:897-905; for rats see Hammer et al., Cell (1990)63:1099- 1112; for embryonic stem (ES) cells see Teratocarcinomas and Embryonic Stem Cells, A Practical Approach, E. J. Robertson, ed., IRL Press (1987); for livestock see Pursel et al., Science (1989) 244:1281-1288; for nonhuman animal clones see Wilmut, I. et al. (1997) Nature 385:810-813, PCT Publication Nos. WO 97/07668 and WO 97/07669; for recombinase systems for regulated transgene expression see, Lakso et al., PNAS (1992) 89:6232-6236; U.S. Pat. No. 4,959,317 [for cre.loxP] and O'Gorman et al. (1991) Science 251:1351-1355; U.S. Pat. No. 5,654,182 [for FLP/FRT]).

Homozygous or heterozygous alterations in the genomes of transgenic animals may result in mis-expression of native genes, including ectopic expression, over-expression (*e.g.* by multiple gene copies), under-expression, and non-expression (*e.g.* by gene knock-out or blocking expression that would otherwise normally occur). In one application, a "knock-out" animal is generated, typically using homologous recombination, in which an alteration in an endogenous gene causes a decrease in that gene's function, preferably such that gene expression is undetectable or insignificant.

### ISM Modulating agents

The invention provides methods to identify agents that interact with and/or modulate the function of ISM and/or INR signaling. Such agents are useful in a variety of diagnostic and therapeutic applications associated with INR signaling, as well as in further analysis of the ISM protein and its contribution to INR signaling. Accordingly, also described herein are methods for modulating INR signaling comprising the step of specifically modulating ISM activity by administering an ISM-interacting or -modulating agent.

ISM-modulating agents may inhibit or enhance ISM activity or otherwise affect normal ISM function, including transcription, protein expression, protein localization, and cellular or extra-cellular activity. The candidate INR signaling- modulating agent may specifically modulate the function of the ISM. The phrases "specific modulating agent", "specifically modulate", etc., are used herein to refer to modulating agents that directly bind to the ISM polypeptide or nucleic acid, and preferably inhibit, enhance, or otherwise alter the function of the ISM. The term also encompasses modulating agents that alter the interaction of ISM with a binding partner or substrate (e.g. by binding to a binding partner of an ISM, or to a protein/binding partner complex, and inhibiting function).

ISM-modulating agents may include small molecule chemical agents, ISM-interacting proteins, including antibodies and other biotherapeutics, and nucleic acid modulators, including antisense oligomers and RNA. The modulating agents may be formulated in pharmaceutical compositions, for example, as compositions that may comprise other active ingredients, as in combination therapy, and/or suitable carriers or excipients. Techniques for formulation and administration of the compounds may be found in "Remington's Pharmaceutical Sciences" Mack Publishing Co., Easton, PA, 19th edition.

### Small Molecule Modulators

Chemical agents, referred to in the art as "small molecule" compounds are typically organic, non-peptide molecules, having a molecular weight less than 10,000, preferably less than 5,000, more preferably less than 1,000, and most preferably less than 500. This class of modulators includes chemically synthesized molecules, for instance, compounds from combinatorial chemical libraries. Synthetic compounds may be rationally designed or identified based on known or inferred properties of the ISM protein or may be identified by screening compound libraries. Alternative appropriate modulators of this class are natural products, particularly secondary metabolites from organisms such as plants or fungi, which can also be identified by screening compound libraries for ISM-modulating activity. Methods for generating and obtaining compounds are well known in the art (Schreiber SL, Science (2000) 151: 1964-1969; Radmann J and Gunther J, Science (2000) 151:1947-1948).

Small molecule modulators identified from screening assays, as described below, ,can be used as lead compounds from which candidate clinical compounds may be designed, optimized, and synthesized. Such clinical compounds may have utility in treating pathologies associated with INR signaling. The activity of candidate small molecule modulating agents may be improved several-fold through iterative secondary functional validation, as further described below, structure determination, and candidate modulator modification and testing. Additionally, candidate clinical compounds are generated with specific regard to clinical and pharmacological properties. For example, the reagents may be derivatized and re-screened using *in vitro* and *in vivo* assays to optimize activity and minimize toxicity for pharmaceutical development.

### Protein Modulators

An ISM-interacting protein may be endogenous, *i.e.* one that normally interacts genetically or biochemically with an ISM, such as a member of the ISM pathway that modulates ISM expression, localization, and/or activity. ISM-modulators include dominant negative forms of ISM-interacting proteins and of ISM proteins themselves. Yeast two-hybrid and variant screens offer preferred methods for identifying endogenous ISM-interacting (Finley, R. L. et al. (1996) in DNA Cloning-Expression Systems: A Practical Approach, eds. Glover D. & Hames B. D (Oxford University Press, Oxford, England), pp. 169-203; Fashema SF et al., Gene (2000) 250:1-14; Drees BL Curr Opin Chem Biol (1999) 3:64-70; Vidal M and Legrain P Nucleic Acids Res (1999) 27:919-29; and U.S. Pat. No. 5,928,868). Mass spectrometry offers alternative preferred methods for the elucidation of protein complexes (reviewed in, *e.g.*, Pandley A and Mann M, Nature (2000) 405:837-846; Yates JR 3rd, Trends Genet (2000) 16:5-8).

### Specific biotherapeutics

An ISM-interacting protein may have biotherapeutic applications. Biotherapeutic agents formulated in pharmaceutically acceptable carriers and dosages may be used to activate or inhibit signal transduction pathways. This modulation may be accomplished by binding a ligand, thus inhibiting the activity of the pathway; or by binding a receptor, either to inhibit activation of, or to activate, the receptor. Alternatively, the biotherapeutic may itself be a ligand capable of activating or inhibiting a receptor. Biotherapeutic agents and methods of producing them are described in detail in U.S. Pat. No. 6,146,628.

### Antibody Modulators

In a preferred embodiment, the ISM-interacting protein is an antibody. In a further preferred embodiment, antibody agonists or antagonists are produced that have therapeutic utility.

Antibodies that specifically bind ISM polypeptides can be generated using known methods. Preferably the antibody is specific to a mammalian ISM polypeptide, and more preferably, a human ISM. Antibodies may be polyclonal, monoclonal (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Monoclonal antibodies with affinities of 10⁸ M⁻¹ preferably 10⁹ M⁻¹ to 10¹⁰ M⁻¹, or stronger can be made by standard procedures as described (Harlow E and Lane D, 1988, Antibodies: A Laboratory Manual, CSH Laboratory Press; (Harlow E and Lane D, 1999 Using Antibodies: A Laboratory Manual, CSH Laboratory Press; Goding (1986) Monoclonal Antibodies: Principles and Practice (2d ed) Academic Press, New York; and U.S. Pat. Nos. 4,381,292; 4,451,570; and 4,618,577). Antibodies may be generated against crude cell extracts of ISM or substantially purified fragments thereof. If ISM fragments are used, they preferably comprise at least 10, and more preferably, at least 20 contiguous amino acids of an ISM protein. In a particular embodiment, ISM-specific antigens and/or immunogens are coupled to carrier proteins that stimulate the immune response. For example, the subject polypeptides are covalently coupled to the keyhole limpet hemocyanin (KLH) carrier, and the conjugate is emulsified in Freund's complete adjuvant, which enhances the immune response. An appropriate immune system such as a laboratory rabbit or mouse is immunized according to conventional protocols.

Chimeric antibodies specific to ISM polypeptides can be made that contain different portions from different animal species. For instance, a human immunoglobulin constant region may be linked to a variable region of a murine mAb, such that the antibody derives its biological activity from the human antibody, and its binding specificity from the murine fragment. Chimeric antibodies are produced by splicing together genes that encode the appropriate regions from each species (Morrison et al., Proc. Natl. Acad. Sci. (1984) 81:6851-6855; Neuberger et al., Nature (1984) 312:604-608; Takeda et al., Nature (1985) 31:452-454). Humanized antibodies, which are a form of chimeric antibodies, can be generated by grafting complementary-determining regions (CDRs) (Carlos, T. M., J. M. Harlan. 1994. Blood 84:2068-2101) of mouse antibodies into a background of human framework regions and constant regions by recombinant DNA technology (Riechmann LM, et al., 1988 Nature 323: 323-327). Humanized antibodies contain ~10% murine sequences and ~90% human sequences, and thus further reduce or eliminate immunogenicity, while retaining the antibody specificities (Co MS, and Queen C. 1991 Nature 351: 501-501; Morrison SL. 1992 Ann. Rev. Immun. 10:239-265). Humanized antibodies and methods of their production are well-known in the art (US PAT NO: 5,530,101; US PAT NO:5,585,089; US PAT NO:5,693,762, and US PAT NO:6,180,370).

ISM-specific single chain antibodies, which are recombinant, single chain polypeptides formed by linking the heavy and light chain fragments of the Fv regions via an amino acid bridge, can be produced (U.S. Pat. No. 4,946,778; Bird, Science (1988) 242:423-426; Huston et al., Proc. Natl. Acad. Sci. USA (1988) 85:5879-5883; and Ward et al., Nature (1989) 334:544-546).

Other suitable techniques for antibody production involve in vitro exposure of lymphocytes to the antigenic polypeptides or alternatively to selection of libraries of antibodies in phage or similar vectors (Huse et al., Science (1989) 246:1275-1281).

As used herein, T-cell antigen receptors are included within the scope of antibody modulators (Harlow and Lane, 1988, *supra*).

The polypeptides and antibodies may be used with or without modification. Frequently, the polypeptides and antibodies will be labeled by joining, either covalently or non-covalently, a substance that provides for a detectable signal, or that is toxic to cells that express the targeted protein (Menard S, et al., Int J. Biol Markers (1989) 4:131-134). A wide variety of labels and conjugation techniques are known and are reported extensively in both the scientific and patent literature. Suitable labels include radionuclides, enzymes, substrates, cofactors, inhibitors, fluorescent moieties, fluorescent emitting lanthanide metals, chemiluminescent moieties, bioluminescent moieties, magnetic particles, and the like (U.S. Pat. Nos. 3,817,837; 3,850,752; 3,939,350; 3,996,345; 4,277,437; 4,275,149; and 4,366,241). Also, recombinant immunoglobulins may be produced (U.S. Pat. No. 4,816,567). Antibodies to cytoplasmic proteins may be delivered and reach their targets by conjugation with membrane-penetrating toxin proteins (U.S. Pat. NO. 6,086,900).

When used therapeutically in a patient, the antibodies of the subject invention are typically administered parenterally, when possible at the target site, or intravenously. The therapeutically effective dose and dosage regimen is determined by clinical studies. Typically, the amount of antibody administered is in the range of about 0.1 mg/kg -to about 10 mg/kg of patient weight. For parenteral administration, the antibodies are formulated in a unit dosage injectable form (e.g., solution, suspension, emulsion) in association with a pharmaceutically acceptable vehicle. Such vehicles are inherently nontoxic and non-therapeutic. Examples are water, saline, Ringer's solution, dextrose solution, and 5% human serum albumin. Nonaqueous vehicles such as fixed oils, ethyl oleate, or liposome carriers may also be used. The vehicle may contain minor amounts of additives, such as buffers and preservatives, which enhance isotonicity and chemical stability or otherwise enhance therapeutic potential. The antibodies' concentrations in such vehicles are typically in the range of about 1 mg/ml-to about10 mg/ml. Immunotherapeutic methods are further described in the literature (US Pat. No. 5,859,206; WO0073469).

### Nucleic Acid Modulators

Other preferred ISM-modulating agents comprise nucleic acid molecules, such as antisense oligomers or double stranded RNA (dsRNA), which generally inhibit ISM activity.

Preferred antisense oligomers interfere with the function of ISM nucleic acids, such as DNA replication, transcription, ISM RNA translocation, translation of protein from the ISM RNA, RNA splicing, and any catalytic activity in which the ISM RNA participates. In one embodiment, the antisense oligomer is an oligonucleotide that is sufficiently complementary to an ISM mRNA to bind to and prevent translation from the ISM mRNA, preferably by binding to the 5' untranslated region. ISM-specific antisense oligonucleotides preferably range from at least 6 to about 200 nucleotides. In some embodiments the oligonucleotide is preferably at least 10, 15, or 20 nucleotides in length. In other embodiments, the oligonucleotide is preferably less than 50, 40, or 30 nucleotides in length. The oligonucleotide can be DNA or RNA, a chimeric mixture of DNA and RNA, derivatives or modified versions thereof, single-stranded or double-stranded. The oligonucleotide can be modified at the base moiety, sugar moiety, or phosphate backbone. The oligonucleotide may include other appending groups such as peptides, agents that facilitate transport across the cell membrane, hybridization-triggered cleavage agents, and intercalating agents.

In another embodiment, the antisense oligomer is a phosphorothioate morpholino oligomer (PMO). PMOs are assembled from four different morpholino subunits, each of which containing one of four genetic bases (A, C, G, or T) linked to a six-membered morpholine ring. Polymers of these subunits are joined by non-ionic phosphodiamidate inter-subunit linkages. Methods of producing and using PMOs and other antisense oligonucleotides are well known in the art (e.g. see WO99/18193; Summerton J, and Weller D, Antisense Nucleic Acid Drug Dev 1997, 7:187-95; Probst JC, Methods 2000, 22:271-281; US PAT NO: 5,325,033; US PAT NO: 5,378,841).

Antisense oligomers are commonly used as research reagents, diagnostics, and therapeutics. For example, antisense oligonucleotides, which are able to specifically inhibit gene expression, are often used to elucidate the function of particular genes (see, *e.g.*, US PAT NO 6,165,790). Antisense oligomers are also used, for example, to distinguish between functions of various members of a biological pathway. Antisense oligomers have been employed as therapeutic moieties in the treatment of disease states in animals and humans and have been demonstrated in numerous clinical trials to be safe and effective (Milligan JF et al, 1993, J Med Chem 36:1923-1937; Tonkinson JL et al., 1996, Cancer Invest 14:54-65). Accordingly, in one aspect of the invention, an ISM-specific antisense oligomer is used in an assay to further elucidate the function of ISM in INR signaling. Zebrafish is a particularly useful model for the study of INR signaling using antisense oligomers. For example, PMOs are used to selectively inactive one or more genes *in vivo* in the Zebrafish embryo. By injecting PMOs into Zebrafish at the 1-16 cell stage candidate targets emerging from the *Drosophila* screens are validated in this vertebrate model system. In another aspect of the invention, PMOs are used to screen the Zebrafish genome for identification of other therapeutic modulators of INR signaling. An ISM-specific antisense oligomer may be used as a therapeutic agent for treatment of metabolic pathologies.

Alternative preferred ISM-modulating agents are double-stranded RNA species mediating RNA interference (RNAi). RNAi is the process of sequence-specific, post-transcriptional gene silencing in animals and plants, initiated by double-stranded RNA (dsRNA) that is homologous in sequence to the silenced gene. Methods relating to the use of RNAi to silence genes in *C. elegans, Drosophila*, plants, and mammals are known in the art (Fire A, et al., 1998 Nature 391:806-811; Fire, A. Trends Genet. 15, 358-363 (1999); Sharp, P. A. RNA interference 2001. Genes Dev. 15, 485-490 (2001); Hammond, S. M., et al., Nature Rev Genet 2, 110-1119 (2001); Tuschl, T. Chem Biochem. 2,239-245 (2001); Hamilton, A. et al., Science 286, 950-952 (1999); Hammond, S. M., et al., Nature 404, 293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619, and Elbashir SM, et al., 2001, Nature 411:494-498).

### Assay Systems

The invention provides assay systems for identifying specific modulators of ISM activity. As used herein, an "assay system" encompasses all the components required for performing and analyzing results of an assay that detects and/or measures a particular event or events. In general, primary assays are used to identify or confirm a modulator's specific biochemical or molecular effect with respect to the ISM nucleic acid or protein. In general, secondary assays further assess the activity of an ISM-modulating agent identified by a primary assay and may confirm that the modulating agent affects ISM in a manner relevant to INR signaling. In some cases, ISM-modulators will be directly tested in a "secondary assay," without having been identified or confirmed in a "primary assay."

In a preferred embodiment, the assay system comprises contacting a suitable assay system comprising an ISM polypeptide or nucleic acid with a candidate agent under conditions whereby, but for the presence of the agent, the system provides a reference activity, which is based on the particular molecular event the assay system detects. The method further comprises detecting the same type of activity in the presence of a candidate agent (" the agent-biased activity of the system"). A difference between the agent-biased activity and the reference activity indicates that the candidate agent modulates ISM activity, and hence INR signaling. A difference, as used herein, is statistically significant. The assay systems generally include positive and/or negative controls, as are well known in the art.

### Primary Assays

The type of modulator tested generally determines the type of primary assay.

### Primary assays for small molecule modulators

For small molecule modulators, screening assays are used to identify candidate modulators. Screening assays may be cell-based or may use a cell-free system that recreates or retains the relevant biochemical reaction of the target protein (reviewed in Sittampalam GS et al., Curr Opin Chem Biol (1997) 1:384-91 and accompanying references). As used herein the term "cell-based" refers to assays using live cells, dead cells, or a particular cellular fraction, such as a membrane, endoplasmic reticulum, or mitochondrial fraction. The term "cell free" encompasses assays using substantially purified protein (either endogenous or recombinantly produced), partially purified cellular extracts, or crude cellular extracts. Screening assays may detect a variety of molecular events, including protein-DNA interactions, protein-protein interactions (*e.g.*, receptor-ligand binding), transcriptional activity (*e.g.*, using a reporter gene), enzymatic activity (*e.g.*, via a property of the substrate), activity of second messengers, immunogenicty and changes in cellular morphology or other cellular characteristics. Appropriate screening assays may use a wide range of detection methods including fluorescent, radioactive, colorimetric, spectrophotometric, and amperometric methods, to provide a read-out for the particular molecular event detected.

In a preferred embodiment, screening assays use fluorescence technologies, including fluorescence polarization, time-resolved fluorescence, and fluorescence resonance energy transfer. These systems offer means to monitor protein-protein or DNA-protein interactions in which the intensity of the signal emitted from dye-labeled molecules depends upon their interactions with partner molecules (*e.g.*, Selvin PR, Nat Struct Biol (2000) 7:730-4; Fernandes PB, Curr Opin Chem Biol (1998) 2:597-603; Hertzberg RP and Pope AJ, Curr Opin Chem Biol (2000) 4:445-451).

Suitable assay formats that may be adapted to screen for ISM modulators are known in the art. Examples of assays useful for the various ISM protein classes are provided below.

Protein kinases, key signal transduction proteins that may be either membrane-associated or intracellular, catalyze the transfer of gamma phosphate from adenosine triphosphate (ATP) to a serine, threonine or tyrosine residue in a protein substrate. Radioassays, which monitor the transfer from [gamma-³²P or -³³P]ATP, are frequently used to assay kinase activity. Separation of the phospho-labeled product from the remaining radio-labeled ATP can be accomplished by various methods including SDS-polyacrylamide gel electrophoresis, filtration using glass fiber filters or other matrices which bind peptides or proteins, and adsorption/binding of peptide or protein substrates to solid-phase matrices allowing removal of remaining radiolabeled ATP by washing. In one example, a scintillation assay monitors the transfer of the gamma phosphate from [gamma -³³P] ATP to a biotinylated peptide substrate. The substrate is captured on a streptavidin coated bead that transmits the signal (Beveridge M et al., J Biomol Screen (2000) 5:205-212). This assay uses the scintillation proximity assay (SPA), in which only radio-ligand bound to receptors tethered to the surface of an SPA bead are detected by the scintillant immobilized within it, allowing binding to be measured without separation of bound from free ligand. Other assays for protein kinase activity may use antibodies that specifically recognize phosphorylated substrates. For instance, the kinase receptor activation (KIRA) assay measures receptor tyrosine kinase activity by ligand stimulating the intact receptor in cultured cells, then capturing solubilized receptor with specific antibodies and quantifying phosphorylation via phosphotyrosine ELISA (Sadick MD, Dev Biol Stand (1999) 97:121-133). Another example of antibody based assays for protein kinase activity is TRF (time-resolved fluorometry). This method utilizes europium chelate-labeled anti-phosphotyrosine antibodies to detect phosphate transfer to a polymeric substrate coated onto microtiter plate wells. The amount of phosphorylation is then detected using time-resolved, dissociation-enhanced fluorescence (Braunwalder AF, et al., Anal Biochem 1996 Jul 1;238(2):159-64). Generic assays may be established for protein kinases that rely upon the phosphorylation of substrates such as myelein basic protein, casein, histone, or synthetic peptides such as polyGlutamate/Tyrosine and radiolabeled ATP.

Phosphoinositide kinases catalyze the phosphorylation of phosphatidylinositol substrates. Assays for lipid kinase activity may use labeled, such as radio-labeled substrates to detect transfer of a phosphate to lipid substrates. In one example, assays may use chromatography techniques to detect phosphorylation (Sbrissa D et al. , 1999, J Biol Chem 274:21589-21597). In another example, an assay uses "FlashPlate" technology (U.S. Patent No. 5,972,595), in which the hydrophobic substrate is immobilized on a solid support in each well of a multi-well plate. Phosphorylation of the substrate with a radio-labeled phosphate is measured as an increase in bound radioactivity, which is detected by the close proximity of the scintillant.

Fatty acid CoA ligases catalyze the elongation of fatty acid chains. Assays may detect elongation of fatty acid substrates, for instance using chromatographic (e.g., HPLC) analysis of labeled elongation products. Long chain fatty acids may be labeled with 14-C (Moon YA et al., 2001, J Biol Chem 276:45358-66).

Fatty acid desaturases catalyze the insertion of double bonds into saturated fatty acid molecules. In one application, radioassays for inhibitors of fatty acid desaturase activity use thin layer chromatography to detect conversion of fatty acid substrates (Obukowicz et al., Biochem Pharmacol (1998) 55:1045-1058).

Mitochondrial adenine transporters mediate the passage of adenosine from the mitochondrial compartment to the cytoplasm. The mitochondrial aspartate/glutamate carrier catalyzes an important step in both the urea cycle and the aspartate/malate NADH shuttle. Mitochondrial transport assays may directly measure transported molecules (e.g., Ruck A et al., 1998, FEBS Lett 426:97-101) and may detect transport activity in intact mitochondria or reconstituted vesicles (e.g., Haucke V and Schatz G, 1997, EMBO J 16:4560-7; Genchi G et al., 1996 Plant Physiol 112:845-51). In one example, vesicles are reconstituted in the presence of the labeled substrate (Palmieri F et al., 1995, Methods Enzymol 260:349-69). Assays for mitochondrial transport may alternatively detect electrogenic or fluorogenic properties of the mitochondrial proteins (e.g., Burstovetsky N et al., 1996, Proc Natl Acad Sci USA 93:664-8; Streicher-Scott J, 1994, Arch Biochem Biophys 15:548-54; U.S. Pat. No. 6,183,948).

p-Hydroxybenzoate polyprenyl transferase (COQ2) is involved in the biosynthesis of ubiquinone, an essential component of the electron transfer system (Ashby MN et al., 1992, J Biol Chem 267:4128-4136). Assays for COQ2 activity may use chromatographic methods to detect ubiquinone biosynthesis (e.g., Uchida N et al., 2000 J Bacteriol 182:6933-6939). Alternatively, assays may directly detect the COQ2 polyprenyltransferase activity (condensation of p-hydroxybenzoate and polyprenyl diphosphate).

A variety of assays are available to detect the activity of proteins that have specific binding activity. Exemplary assays use fluorescence polarization, fluorescence polarization, and laser scanning techniques to measure binding of fluorescently labeled proteins, peptides, or other molecules (Lynch BA et al., 1999, Anal Biochem 275:62-73; Li HY, 2001, J Cell Biochem 80:293-303; Zuck P et al., Proc Natl Acad Sci USA 1999, 96: 11122-11127). In another example, binding activity is detected using the scintillation proximity assay (SPA), which uses a biotinylated peptide probe captured on a streptavidin coated SPA bead and a radio-labeled partner molecule. The assay specifically detects the radio-labeled protein bound to the peptide probe via scintillant immobilized within the SPA bead (Sonatore LM et al., 1996, Anal Biochem 240:289-297).

Proteins that are part of a transcriptional complex may be assayed for binding activity, as described above. Other suitable assays may measure transcriptional activity. In one example, transcriptional activity is detected using quantitative RT-PCR (*e.g.*, using the TaqMan®, PE Applied Biosystems). In another example, a transcriptional reporter (e.g., luciferase, GFP, beta-galactosidase, etc.) operably linked to a responsive gene regulatory sequence is used (e.g., Berg M et al, 2000, J Biomol Screen, 5:71-76).

Preferred screening assays are high throughput or ultra high throughput and thus provide automated, cost-effective means of screening compound libraries for lead compounds (Fernandes PB, 1998, *supra*; Sundberg SA, Curr Opin Biotechnol 2000, 11:47-53).

Cell-based screening assays usually require systems for recombinant expression of ISM and any auxiliary proteins demanded by the particular assay. Cell-free assays often use recombinantly produced purified or substantially purified proteins. Appropriate methods for generating recombinant proteins produce sufficient quantities of proteins that retain their relevant biological activities and are of sufficient purity to optimize activity and assure assay reproducibility. Yeast two-hybrid and variant screens, and mass spectrometry provide preferred methods for determining protein-protein interactions and elucidation of protein complexes. In certain applications when ISM-interacting proteins are used in screening assays, the binding specificity of the interacting protein to the ISM protein may be assayed by various known methods, including binding equilibrium constants (usually at least about 10⁷ M⁻¹, preferably at least about 10⁸ M¹, more preferably at least about 10⁹ M⁻¹), and immunogenic properties. For enzymes and receptors, binding may be assayed by, respectively, substrate and ligand processing.

The screening assay may measure a candidate agent's ability to specifically bind to or modulate activity of an ISM polypeptide, a fusion protein thereof, or to cells or membranes bearing the polypeptide or fusion protein. The ISM polypeptide can be full length or a fragment thereof that retains functional ISM activity. The ISM polypeptide may be fused to another polypeptide, such as a peptide tag for detection or anchoring, or to another tag. The ISM polypeptide is preferably human ISM, or is an ortholog or derivative thereof as described above. In a preferred embodiment, the screening assay detects candidate agent-based modulation of ISM interaction with a binding target, such as an endogenous or exogenous protein or other substrate that has ISM -specific binding activity, and can be used to assess normal ISM gene function.

Certain screening assays may also be used to test antibody and nucleic acid modulators; for nucleic acid modulators, appropriate assay systems involve ISM mRNA expression.

### Primary assays for antibody modulators

For antibody modulators, appropriate primary assays are binding assays that test the antibody's affinity to and specificity for the ISM protein. Methods for testing antibody affinity and specificity are well known in the art (Harlow and Lane, 1988, 1999, *supra*). The enzyme-linked immunosorbant assay (ELISA) is a preferred method for detecting ISM-specific antibodies; others include FACS assays, radioimmunoassays, and fluorescent assays.

### Primary assays for nucleic acid modulators

For nucleic acid modulators, primary assays may test the ability of the nucleic acid modulator to inhibit ISM gene expression, preferably mRNA expression. In general, expression analysis comprises comparing ISM expression in like populations of cells (*e.g.*, two pools of cells that endogenously or recombinantly express ISM) in the presence and absence of the nucleic acid modulator. Methods for analyzing mRNA and protein expression are well known in the art. For instance, Northern blotting, slot blotting, ribonuclease protection, quantitative RT-PCR (*e.g.*, using the TaqMan®, PE Applied Biosystems), or microarray analysis may be used to confirm that ISM mRNA expression is reduced in cells treated with the nucleic acid modulator (*e.g.*, Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm DH and Guiseppi-Elie, ACurr Opin Biotechnol 2001, 12:41-47). Protein expression may also be monitored. Proteins are most commonly detected with specific antibodies or antisera directed against either the ISM protein or specific peptides. A variety of means including Western blotting, ELISA, or in situ detection, are available (Harlow E and Lane D, 1988 and 1999, *supra*).

### Secondary Assays

Secondary assays may be used to further assess the activity of an ISM-modulating agent identified by any of the above methods to confirm that the modulating agent affects ISM in a manner relevant to INR signaling. As used herein, ISM-modulating agents encompass candidate clinical compounds or other agents derived from previously identified modulating agent. Secondary assays can also be used to test the activity of a modulator on a particular genetic or biochemical pathway or to test the specificity of the modulator's interaction with ISM.

Secondary assays generally compare like populations of cells or animals (*e.g.*, two pools of cells or animals that endogenously or recombinantly express ISM) in the presence and absence of the candidate modulator. In general, such assays test whether treatment of cells or animals with a candidate ISM-modulating agent results in changes in INR signaling, in comparison to untreated (or mock- or placebo-treated) cells or animals. Changes in INR signaling may be detected as modifications to INR pathway components, or changes in their expression or activity. Assays may also detect an output of normal or defective INR signaling, used herein to encompass immediate outputs, such as glucose uptake, or longer-term effects, such as changes in glycogen and triglycerides metabolism, adipocyte differentiation, or development of diabetes or other INR-related pathologies. Certain assays use sensitized genetic backgrounds, used herein to describe cells or animals engineered for altered expression of genes in the INR or interacting pathways, or pathways associated with INR signaling or an output of INR signaling.

### Cell-based assays

Cell-based assays may use a variety of insulin-sensitive mammalian cells and may detect endogenous INR signaling or may rely on recombinant expression of INR and/or other INR pathway components. Exemplary insulin-sensitive cells include adipocytes, hepatocytes, and pancreatic beta cells. Suitable adipocytes include 3T3L1 cells, which are most commonly used for insulin sensitivity assays, as well as primary cells from mice or human biopsy. Suitable hepatocytes include the rat hepatoma H4-II-E cell line. Suitable beta cells include rat INS-1 cells with optimized glucose-sensitive insulin secretion (such as clone 823-13, Hohmeier et al., 2000, Diabetes 49:424). Other suitable cells include muscle cells, such as L6 myotubes, and CHO cells engineered to over-express INR. For certain assay systems it may be useful to treat cells with factors such as glucosamine, free fatty acids or TNF alpha, which induce an insulin resistant state. Candidate modulators are typically added to the cell media but may also be injected into cells or delivered by any other efficacious means.

Cell based assays generally test whether treatment of insulin responsive cells with the ISM - modulating agent alters INR signaling in response to insulin stimulation ("insulin sensitivity"); such assays are well-known in the art (see, e.g., Sweeney et al., 1999, J Biol Chem 274:10071). In a preferred embodiment, assays are performed to determine whether inhibition of ISM function increases insulin sensitivity.

In one example, INR signaling is assessed by measuring expression of insulin-responsive genes. Hepatocytes are preferred for these assays. Many insulin responsive genes are known (e.g., p85 PI3 kinase, hexokinase II, glycogen synthetase, lipoprotein lipase, etc; PEPCK is specifically down-regulated in response to INR signaling). Any available means for expression analysis, as previously described, may be used. Typically, mRNA expression is detected. In a preferred application, Taqman analysis is used to directly measure mRNA expression. Alternatively, expression is indirectly monitored from a transgenic reporter construct comprising sequences encoding a reporter gene (such as luciferase, GFP or other fluorescent proteins, beta-galactosidase, etc.) under control of regulatory sequences (e.g., enhancer/promoter regions) of an insulin responsive gene. Methods for making and using reporter constructs are well known.

INR signaling may also be detected by measuring the activity of components of the INR-signaling pathway, which are well-known in the art (see, e.g., Kahn and Weir, Eds., Joslin's Diabetes Mellitus, Williams & Wilkins, Baltimore, MD, 1994). Suitable assays may detect phosphorylation of pathway members, including IRS, PI3K, Akt, GSK3 etc., for instance, using an antibody that specifically recognizes a phosphorylated protein. Assays may also detect a change in the specific signaling activity of pathway components (e.g., kinase activity of PI3K, GSK3, Akt, etc.). Kinase assays, as well as methods for detecting phosphorylated protein substrates, are well known in the art (see, e.g., Ueki K et al, 2000, Mol Cell Biol;20:8035-46).

In another example, assays measure glycogen synthesis in response to insulin stimulation, preferably using hepatocytes. Glycogen synthesis may be assayed by various means, including measurement of glycogen content, and determination of glycogen synthase activity using labeled, such as radio-labeled, glucose (see, e.g., Aiston S and Agius L, 1999, Diabetes 48:15-20; Rother KI et al., 1998, J Biol Chem 273:17491-7).

Other suitable assays measure cellular uptake of glucose (typically labeled glucose) in response to insulin stimulation. Adipocytes are preferred for these assays. Assays also measure translocation of glucose transporter (GLUT) 4, which is a primary mediator of insulin-induced glucose uptake, primarily in muscle and adipocytes, and which specifically translocates to the cell surface following insulin stimulation. Such assays may detect endogenous GLUT4 translocation using GLUT4-specific antibodies or may detect exogenously introduced, epitope-tagged GLUT4 using an antibody specific to the particular epitope (see, e.g., Sweeney, 1999, *supra*; Quon MJ et al., 1994, Proc Natl Acad Sci U S A 91:5587-91).

Other preferred assays detect insulin secretion from beta cells in response to glucose. Such assays typically use ELISA (see, e.g., Bergsten and Hellman, 1993, Diabetes 42:670-4) or radioimmunoassay (RIA; see, e.g., Hohmeier et al., 2000, *supra*).

### Animal assays

A variety of non-human animal models of metabolic disorders may be used to test candidate ISM modulators. Such models typically use genetically modified animals that have been engineered to mis-express (e.g., over-express or lack expression in) genes involved in lipid metabolism, adipogenesis, and/or the INR signaling pathway. Additionally, particular feeding conditions, and/or administration or certain biologically active compounds, may contribute to or create animal models of lipid and/or metabolic disorders. Assays generally required systemic delivery of the candidate modulators, such as by oral administration, injection (intravenous, subcutaneous, intraperitoneous), bolus administration, etc.

Assays may use mouse models of diabetes and/or insulin resistance. Mice carrying knockouts of genes in the leptin pathway, such as *ob* (leptin) or *db* (leptin receptor), or the INR signaling pathway, such as INR or the insulin receptor substrate (IRS), develop symptoms of diabetes, and show hepatic lipid accumulation (fatty liver) and, frequently, increased plasma lipid levels (Nishina et al., 1994, Metabolism 43:549-553; Michael et al., 2000, Mol Cell 6:87-97; Bruning JC et al., 1998, Mol Cell 2:559-569). Certain susceptible wild type mice, such as C57BL/6, exhibit similar symptoms when fed a high fat diet (Linton and Fazio, 2001, Current Opinion in Lipidology 12:489-495). Accordingly, appropriate assays using these models test whether administration of a candidate modulator alters, preferably decreases lipid accumulation in the liver. Lipid levels in plasma and adipose tissue may also be tested. Methods for assaying lipid content, typically by FPLC or colorimetric assays (Shimano H et al., 1996, J Clin Invest 98:1575-1584; Hasty et al., 2001, J Biol Chem 276:37402-37408), and lipid synthesis, such as by scintillation measurement of incorporation of radio-labeled substrates (Horton JD et al., 1999, J Clin Invest 103:1067-1076), are well known in the art. Other useful assays test blood glucose levels, insulin levels, and insulin sensitivity (e.g., Michael MD, 2000, Molecular Cell 6: 87). Insulin sensitivity is routinely tested by a glucose tolerance test or an insulin tolerance test.

In another example, assays may use mouse models of lipoprotein biology and cardiovascular disease. For instance, mouse knockouts of apolipoprotein E (apoE) display elevated plasma cholesterol and spontaneous arterial lesions (Zhang SH, 1992, Science 258:468-471). Transgenic mice over-expressing cholesterol ester transfer protein (CETP) also display increased plasma lipid levels (specifically, very-low-density lipoprotein [VLDL] and low-density lipoprotein [LDL] cholesterol levels) and plaque formation in arteries (Marotti KR et al., 1993, Nature 364:73-75). Assays using these models may test whether administration of candidate modulators alters plasma lipid levels, such as by decreasing levels of the pro-atherogenic LDL and VLDL, increasing HDL, or by decreasing overall lipid (including trigyceride) levels. Additionally histological analysis of arterial morphology and lesion formation (i.e., lesion number and size) may indicate whether a candidate modulator can reduce progression and/or severity of atherosclerosis. Numerous other mouse models for atherosclerosis are available, including knockouts of Apo-A1, PPARgamma, and scavenger receptor (SR)-B1 in LDLR- or ApoE-null background (reviewed in, e.g., Glass CK and Witztum JL, 2001, Cell 104:503-516).

The ability of candidate modulators to alter plasma lipid levels and atherosclerotic progression may be tested in mouse models for multiple lipid disorders. For instance, mice with knockouts in both leptin and LDL receptor genes display hypercholesterolemia, hypertriglyceridemia and arterial lesions and provide a model for the relationship between impaired fuel metabolism, increased plasma remnant lipoproteins, diabetes, and atherosclerosis (Hasty AH et al, 2001, *supra.*).

### Diagnostic Methods

The discovery that ISM is implicated in INR signaling provides for a variety of methods that can be employed for the diagnostic and prognostic evaluation of diseases and disorders associated with INR signaling and for the identification of subjects having a predisposition to such diseases and disorders. Any method for assessing ISM expression in a sample, as previously described, may be used. Such methods may, for example, utilize reagents such as the ISM oligonucleotides and antibodies directed against ISM, as described above for: (1) the detection of the presence of ISM gene mutations, or the detection of either over- or under-expression of ISM mRNA relative to the non-disorder state; (2) the detection of either an over- or an under-abundance of ISM gene product relative to the non-disorder state; and (3) the detection of perturbations or abnormalities in a biological pathway mediated by ISM.

## Claims

1. A method of identifying a candidate INR signaling enhancing agent, said method comprising the steps of:
(a) providing a non-human assay system comprising an ISM polypeptide or nucleic acid, wherein said ISM polypeptide is a full length ISM protein consisting of amino acid sequence SEQ ID NO: 22, or a fragment thereof that has kinase activity and comprises at least 50 contiguous amino acids of the ISM protein, wherein said ISM nucleic acid is a DNA or RNA molecule that encodes said ISM polypeptide;
(b) contacting the assay system with a test agent under conditions whereby, but for the presence of the test agent, the system provides a reference activity; and
(c) detecting reduced activity or expression of the ISM polypeptide or nucleic acid, wherein the reduced activity or expression of the ISM polypeptide or nucleic acid between the presence and absence of the test agent identifies the test agent as a candidate INR signaling enhancing agent.

2. The method according to Claim 1, wherein the method is *in vitro.*

3. The method according to Claim 1 or Claim 2, wherein the ISM nucleic acid is an ISM nucleic acid which consists of the nucleic acid sequence shown as SEQ ID NO: 21, or a nucleic acid sequence which hybridizes to the nucleic acid sequence shown as SEQ ID NO: 21 under low stringency conditions that comprise incubation for 8 hours to overnight at 37°C in a solution comprising 20% formamide, 5x SSC, 50mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1x SSC at about 37°C for 1 hour.

4. The method of Claim 1 or Claim 2 wherein the assay system includes a screening assay comprising the ISM polypeptide, and the candidate test agent is a small molecule modulator.

5. The method of Claim 4 wherein the screening assay detects kinase activity.

6. The method of Claim 1 or Claim 2 wherein the assay system includes a binding assay comprising the ISM polypeptide and the candidate test agent is an antibody.

7. The method of Claim 1 or Claim 2 wherein the assay system includes an expression assay comprising the ISM nucleic acid and the candidate test agent is a nucleic acid selected from an antisense oligomer, a phosphorothioate morpholino oligomer (PMO), and dsRNA which mediates RNAi.

8. The method of Claim 7 wherein the nucleic acid modulator is an antisense oligomer.

9. The method of Claim 7 wherein the nucleic acid modulator is a phosphorothioate morpholino oligomer (PMO).

10. The method of Claim 1 or Claim 2 wherein the assay system comprises cultured cells expressing the ISM, and wherein the assay system includes an assay that detects a test agent-biased change in INR signaling or an output of INR signaling.

11. The method of Claim 1 or Claim 2 wherein the assay system comprises cultured cells that have been engineered for altered expression of genes in the INR or interacting pathways, pathways associated with INR signaling or an output of INR signaling.

12. The method of Claim 1 or Claim 2 wherein the assay system is a cell-free system.

13. The method of Claim 11 wherein the assay detects an event selected from the group consisting of expression of insulin-responsive genes, phosphorylation of an INR signaling pathway component, kinase activity of an INR signaling pathway component, glycogen synthesis, glucose uptake, GLUT4 translocation, and insulin secretion.

14. The method of Claim 1 or Claim 2, comprising the additional steps of
(d) providing a second assay system, said second assay system comprising cultured cells expressing the ISM, wherein the second assay system includes a second assay that detects an agent-biased change in INR signaling or an output of INR signaling;
(e) contacting the second assay system with the test agent of (b) or an agent derived therefrom under conditions whereby, but for the presence of the test agent or agent derived therefrom, the system provides a reference activity; and
(f) detecting an agent-biased activity of the second assay system, wherein a difference between the agent-biased activity and the reference activity of the second assay system confirms the test agent or agent derived therefrom as a candidate INR signaling enhancing agent.

15. The method of Claim 14 wherein the second assay detects an event selected from the group consisting of expression of insulin-responsive genes, phosphorylation of an INR signaling pathway component, kinase activity of an INR signaling pathway component, glycogen synthesis, glucose uptake, GLUT4 translocation, and insulin secretion.

16. The method of Claim 14, wherein the second assay system comprises cultured cells that have been engineered for altered expression of genes in the INR or interacting pathways, pathways associated with INR signaling or an output of INR signaling.

17. An in vitro method of enhancing INR signaling in a mammalian cell comprising contacting the cell with an agent that inhibits an ISM polypeptide or nucleic acid; wherein said ISM polypeptide is a full length ISM protein of amino acid sequence SEQ ID NO: 22, or a fragment thereof that has kinase activity and comprises at least 50 contiguous amino acids of the ISM protein; wherein said ISM nucleic acid is a DNA or RNA molecule that encodes said ISM polypeptide; and wherein the agent is an antibody, a T cell antigen receptor, or a nucleic acid selected from an antisense oligomer, a phosphorothioate morpholino oligomer (PMO), and dsRNA which mediates RNAi.

18. The method according to Claim 17, wherein the ISM nucleic acid is an ISM nucleic acid which consists of the nucleic acid sequence shown as SEQ ID NO: 21, or a nucleic acid sequence which hybridizes to the nucleic acid sequence shown as SEQ ID NO: 21 under low stringency conditions that comprise incubation for 8 hours to overnight at 37°C in a solution comprising 20% formamide, 5x SSC, 50mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1x SSC at about 37°C for 1 hour.

## Patentansprüche

1. Ein Verfahren zum Identifizieren eines INR-Signalisierung verbessernden Wirkstoffkandidaten, wobei das Verfahren die folgenden Schritte beinhaltet:
(a) Bereitstellen eines nicht menschlichen Assay-Systems, das ein ISM-Polypeptid oder eine ISM-Nukleinsäure beinhaltet, wobei das ISM-Polypeptid ein ISM-Protein voller Länge ist, das aus der Aminosäuresequenz SEQ ID NO: 22 besteht, oder ein Fragment davon, das Kinaseaktivität aufweist und mindestens 50 zusammenhängende Aminosäuren des ISM-Proteins beinhaltet, wobei die ISM-Nukleinsäure ein DNA- oder RNA-Molekül ist, das das ISM-Polypeptid kodiert;
(b) In-Kontakt-Bringen des Assay-Systems mit einem Testwirkstoff unter Bedingungen, unter denen, außer bei Vorhandensein des Testwirkstoffs, das System eine Referenzaktivität bereitstellt; und
(c) Erkennen einer reduzierten Aktivität oder Expression des ISM-Polypeptids oder der ISM-Nukleinsäure, wobei die reduzierte Aktivität oder Expression des ISM-Polypeptids oder der ISM-Nukleinsäure zwischen dem Vorhandensein und dem Nichtvorhandensein des Testwirkstoffs den Testwirkstoff als einen INR-Signalisierung verbessernden Wirkstoffkandidaten identifiziert.

2. Verfahren gemäß Anspruch 1, wobei das Verfahren *in vitro* ist.

3. Verfahren gemäß Anspruch 1 oder Anspruch 2, wobei die ISM-Nukleinsäure eine ISM-Nukleinsäure ist, die aus der als SEQ ID NO: 21 gezeigten Nukleinsäuresequenz besteht, oder eine Nukleinsäuresequenz, die mit der als SEQ ID NO: 21 gezeigten Nukleinsäuresequenz unter Bedingungen geringer Stringenz hybridisiert, welche Folgendes beinhalten: Inkubation über 8 Stunden bis über Nacht bei 37 °C in einer Lösung, die 20 % Formamid, 5x SSC, 50 mM Natriumphosphat (pH 7,6), 5X Denhardt-Lösung, 10 % Dextransulfat und 20 µg/ml denaturierte gescherte Lachssperma-DNA beinhaltet; Hybridisierung in dem gleichen Puffer über 18 bis 20 Stunden; und Waschen der Filter in 1x SSC bei etwa 37 °C über 1 Stunde.

4. Verfahren gemäß Anspruch 1 oder Anspruch 2, wobei das Assay-System einen Screening-Assay umfasst, der das ISM-Polypeptid beinhaltet, und der Testwirkstoffkandidat ein Kleinmolekülmodulator ist.

5. Verfahren gemäß Anspruch 4, wobei der Screening-Assay Kinaseaktivität erkennt.

6. Verfahren gemäß Anspruch 1 oder Anspruch 2, wobei das Assay-System einen Bindungs-Assay umfasst, der ein ISM-Polypeptid beinhaltet, und der Testwirkstoffkandidat ein Antikörper ist.

7. Verfahren gemäß Anspruch 1 oder Anspruch 2, wobei das Assay-System einen Expressions-Assay umfasst, der die ISM-Nukleinsäure beinhaltet, und der Testwirkstoffkandidat eine Nukleinsäure ist, die aus einem Antisense-Oligomer, einem Phosphorothioat-Morpholino-Oligomer (PMO) und einer RNAi vermittelnden dsRNA ausgewählt ist.

8. Verfahren gemäß Anspruch 7, wobei der Nukleinsäuremodulator ein Antisense-Oligomer ist.

9. Verfahren gemäß Anspruch 7, wobei der Nukleinsäuremodulator ein Phosphorothioat-Morpholino-Oligomer (PMO) ist.

10. Verfahren gemäß Anspruch 1 oder Anspruch 2, wobei das Assay-System kultivierte Zellen beinhaltet, die den ISM exprimieren, und wobei das Assay-System einen Assay umfasst, der eine durch den Testwirkstoff beeinflusste Änderung bei der INR-Signalisierung oder einem Ergebnis der INR-Signalisierung erkennt.

11. Verfahren gemäß Anspruch 1 oder Anspruch 2, wobei das Assay-System kultivierte Zellen beinhaltet, die für eine veränderte Expression von Genen in dem INR-Weg oder wechselwirkenden Wegen, Wegen, die mit der INR-Signalisierung oder einem Ergebnis der INR-Signalisierung assoziiert sind, manipuliert worden sind.

12. Verfahren gemäß Anspruch 1 oder Anspruch 2, wobei das Assay-System ein zellloses System ist.

13. Verfahren gemäß Anspruch 11, wobei der Assay ein Ereignis erkennt, das aus der Gruppe ausgewählt ist, die aus Folgendem besteht: Expression von auf Insulin reagierenden Genen, Phosphorylierung einer Komponente des INR-Signalwegs, Kinaseaktivität einer Komponente des INR-Signalwegs, Glykogensynthese, Glukoseaufnahme, GLUT4-Translokation und Insulinausschüttung.

14. Verfahren gemäß Anspruch 1 oder Anspruch 2, das die folgenden zusätzlichen Schritte beinhaltet:
(d) Bereitstellen eines zweiten Assay-Systems, wobei das zweite Assay-System kultivierte Zellen beinhaltet, die den ISM exprimieren, wobei das zweite Assay-System einen zweiten Assay umfasst, der eine durch einen Wirkstoff beeinflusste Änderung bei der INR-Signalisierung oder einem Ergebnis der INR-Signalisierung erkennt;
(e) In-Kontakt-Bringen des zweiten Assay-Systems mit dem Testwirkstoff aus (b) oder einem davon abgeleiteten Wirkstoff unter Bedingungen, unter denen, außer bei Vorhandensein des Testwirkstoffs oder des davon abgeleiteten Wirkstoffs, das System eine Referenzaktivität bereitstellt; und
(f) Erkennen einer durch den Wirkstoff beeinflussten Aktivität des zweiten Assay-Systems, wobei eine Differenz zwischen der durch den Wirkstoff beeinflussten Aktivität und der Referenzaktivität des zweiten Assay-Systems bestätigt, dass der Testwirkstoff oder der davon abgeleitete Wirkstoff ein INR-Signalisierung verbessernder Wirkstoffkandidat ist.

15. Verfahren gemäß Anspruch 14, wobei der zweite Assay ein Ereignis erkennt, das aus der Gruppe ausgewählt ist, die aus Folgendem besteht: Expression von auf Insulin reagierenden Genen, Phosphorylierung einer Komponente des INR-Signalwegs, Kinaseaktivität einer Komponente des INR-Signalwegs, Glykogensynthese, Glukoseaufnahme, GLUT4-Translokation und Insulinausschüttung.

16. Verfahren gemäß Anspruch 14, wobei das zweite Assay-System kultivierte Zellen beinhaltet, die für eine veränderte Expression von Genen in dem INR-Weg oder wechselwirkenden Wegen, Wegen, die mit der INR-Signalisierung oder einem Ergebnis der INR-Signalisierung assoziiert sind, manipuliert worden sind.

17. Ein In-vitro-Verfahren der Verbesserung der INR-Signalisierung in einer Säugetierzelle, das das In-Kontakt-Bringen der Zelle mit einem Wirkstoff, welcher ein ISM-Polypeptid oder eine ISM-Nukleinsäure inhibiert, beinhaltet; wobei das ISM-Polypeptid ein ISM-Protein voller Länge der Aminosäuresequenz SEQ ID NO: 22 oder ein Fragment davon, das Kinaseaktivität aufweist und mindestens 50 zusammenhängende Aminosäuren des ISM-Proteins beinhaltet, ist; wobei die ISM-Nukleinsäure ein DNA- oder RNA-Molekül ist, das das ISM-Polypeptid kodiert; und wobei der Wirkstoff ein Antikörper, ein T-Zell-Antigenrezeptor oder eine Nukleinsäure, ausgewählt aus einem Antisense-Oligomer, einem Phosphorothioat-Morpholino-Oligomer (PMO) und einer RNAi vermittelnden dsRNA, ist.

18. Verfahren gemäß Anspruch 17, wobei die ISM-Nukleinsäure eine ISM-Nukleinsäure ist, die aus der als SEQ ID NO: 21 gezeigten Nukleinsäuresequenz besteht, oder eine Nukleinsäuresequenz, die mit der als SEQ ID NO: 21 gezeigten Nukleinsäuresequenz unter Bedingungen geringer Stringenz hybridisiert, welche Folgendes beinhalten:
Inkubation über 8 Stunden bis über Nacht bei 37 °C in einer Lösung, die 20 % Formamid, 5x SSC, 50 mM Natriumphosphat (pH 7,6), 5X Denhardt-Lösung, 10 % Dextransulfat und 20 µg/ml denaturierte gescherte Lachssperma-DNA beinhaltet;
Hybridisierung in dem gleichen Puffer über 18 bis 20 Stunden; und Waschen der Filter in 1x SSC bei etwa 37°C über 1 Stunde.

## Revendications

1. Une méthode d'identification d'un agent facilitant la signalisation d'INR candidat, ladite méthode comprenant les étapes suivantes :
(a) fournir un système d'essai non humain comprenant un polypeptide ou acide nucléique ISM, ledit polypeptide ISM étant une protéine ISM de longueur totale consistant en la séquence d'acides aminés SEQ ID N° : 22, ou un fragment de celle-ci qui présente une activité kinase et comprend au moins 50 acides aminés contigus de la protéine ISM, ledit acide nucléique ISM étant une molécule d'ADN ou d'ARN qui code pour ledit polypeptide ISM ;
(b) mettre en contact le système d'essai avec un agent de test dans des conditions selon lesquelles, sans la présence de l'agent de test, le système fournit une activité de référence ; et
(c) détecter une réduction d'activité ou d'expression du polypeptide ou acide nucléique ISM, dans laquelle la réduction d'activité ou d'expression du polypeptide ou acide nucléique ISM entre la présence et l'absence de l'agent de test identifie l'agent de test comme étant un agent facilitant la signalisation d'INR candidat.

2. La méthode selon la revendication 1, dans laquelle la méthode est *in vitro.*

3. La méthode selon la revendication 1 ou la revendication 2, dans laquelle l'acide nucléique ISM est un acide nucléique ISM qui consiste en la séquence d'acides nucléiques montrée comme étant SEQ ID N° : 21, ou une séquence d'acides nucléiques qui s'hybride à la séquence d'acides nucléiques montrée comme étant SEQ ID N° : 21 dans des conditions de faible stringence qui comprennent une incubation pendant 8 heures à une nuit à 37 °C dans une solution comprenant du formamide à 20 %, du SSC 5x, du phosphate de sodium 50 mM (pH 7,6), de la solution de Denhardt 5X, du sulfate de dextran à 10 %, et de l'ADN de sperme de saumon cisaillé et dénaturé à 20 µg/ml ; une hybridation dans le même tampon pendant 18 à 20 heures ; et un lavage des filtres dans du SSC 1x à environ 37 °C pendant 1 heure.

4. La méthode de la revendication 1 ou de la revendication 2 dans laquelle le système d'essai comporte un essai de criblage comprenant le polypeptide ISM, et l'agent de test candidat est un modulateur à petites molécules.

5. La méthode de la revendication 4 dans laquelle l'essai de criblage détecte l'activité kinase.

6. La méthode de la revendication 1 ou de la revendication 2 dans laquelle le système d'essai comporte un essai de liaison comprenant le polypeptide ISM et l'agent de test candidat est un anticorps.

7. La méthode de la revendication 1 ou de la revendication 2 dans laquelle le système d'essai comporte un essai d'expression comprenant l'acide nucléique ISM et l'agent de test candidat est un acide nucléique sélectionné parmi un oligomère anti-sens, un oligomère morpholino phosphorothioate (PMO), et un ARNds qui médie un ARNi.

8. La méthode de la revendication 7 dans laquelle le modulateur d'acides nucléiques est un oligomère anti-sens.

9. La méthode de la revendication 7 dans laquelle le modulateur d'acides nucléiques est un oligomère morpholino phosphorothioate (PMO).

10. La méthode de la revendication 1 ou de la revendication 2 dans laquelle le système d'essai comprend des cellules de culture exprimant l'ISM, et dans laquelle le système d'essai comporte un essai qui détecte un changement de la signalisation d'INR influencé par l'agent de test ou une sortie de signalisation d'INR.

11. La méthode de la revendication 1 ou de la revendication 2 dans laquelle le système d'essai comprend des cellules de culture qui ont été mises au point pour une expression altérée de gènes dans l'INR ou dans les voies d'interaction, voies associées à la signalisation d'INR ou une sortie de signalisation d'INR.

12. La méthode de la revendication 1 ou de la revendication 2 dans laquelle le système d'essai est un système acellulaire.

13. La méthode de la revendication 11 dans laquelle l'essai détecte un événement sélectionné dans le groupe consistant en l'expression de gènes sensibles à l'insuline, la phosphorylation d'un composant de voie de signalisation d'INR, l'activité kinase d'un composant de voie de signalisation d'INR, la synthèse de glycogène, l'absorption de glucose, la translocation de GLUT4, et la sécrétion d'insuline.

14. La méthode de la revendication 1 ou de la revendication 2, comprenant les étapes additionnelles suivantes :
(d) fournir un deuxième système d'essai, ledit deuxième système d'essai comprenant des cellules de culture exprimant l'ISM, dans laquelle le deuxième système d'essai comporte un deuxième essai qui détecte un changement de la signalisation d'INR influencé par l'agent ou une sortie de signalisation d'INR ;
(e) mettre en contact le deuxième système d'essai avec l'agent de test de (b) ou un agent dérivé de celui-ci dans des conditions selon lesquelles, sans la présence de l'agent de test ou de l'agent dérivé de celui-ci, le système fournit une activité de référence ; et
(f) détecter une activité du deuxième système d'essai influencée par l'agent, dans laquelle une différence entre l'activité influencée par l'agent et l'activité de référence du deuxième système d'essai confirme que l'agent de test ou agent dérivé de celui-ci est un agent facilitant la signalisation d'INR candidat.

15. La méthode de la revendication 14 dans laquelle le deuxième essai détecte un événement sélectionné dans le groupe consistant en l'expression de gènes sensibles à l'insuline, la phosphorylation d'un composant de voie de signalisation d'INR, l'activité kinase d'un composant de voie de signalisation d'INR, la synthèse de glycogène, l'absorption de glucose, la translocation de GLUT4, et la sécrétion d'insuline.

16. La méthode de la revendication 14, dans laquelle le deuxième système d'essai comprend des cellules de culture qui ont été mises au point pour une expression altérée de gènes dans l'INR ou les voies d'interaction, voies associées à la signalisation d'INR ou une sortie de signalisation d'INR.

17. Une méthode in vitro pour faciliter la signalisation d'INR dans une cellule de mammifère comprenant la mise en contact de la cellule avec un agent qui inhibe un polypeptide ou acide nucléique ISM ; dans laquelle ledit polypeptide ISM est une protéine ISM de longueur totale de séquence d'acides aminés SEQ ID N° : 22, ou un fragment de celle-ci qui présente une activité kinase et comprend au moins 50 acides aminés contigus de la protéine ISM ; dans laquelle ledit acide nucléique ISM est une molécule d'ADN ou d'ARN qui code pour ledit polypeptide ISM ; et dans laquelle l'agent est un anticorps, un récepteur d'antigène de cellule T, ou un acide nucléique sélectionné parmi un oligomère anti-sens, un oligomère morpholino phosphorothioate (PMO), et un ARNds qui médie un ARNi.

18. La méthode selon la revendication 17, dans laquelle l'acide nucléique ISM est un acide nucléique ISM qui consiste en la séquence d'acides nucléiques montrée comme étant SEQ ID N° : 21, ou une séquence d'acides nucléiques qui s'hybride à la séquence d'acides nucléiques montrée comme étant SEQ ID N° : 21 dans des conditions de faible stringence qui comprennent une incubation pendant 8 heures à une nuit à 37 °C dans une solution comprenant du formamide à 20 %, du SSC 5x, du phosphate de sodium 50 mM (pH 7,6), de la solution de Denhardt 5X, du sulfate de dextran à 10 %, et de l'ADN de sperme de saumon cisaillé et dénaturé à 20 µg/ml ; une hybridation dans le même tampon pendant 18 à 20 heures ; et un lavage des filtres dans du SSC 1x à environ 37 °C pendant 1 heure.
